# EUROPEAN PATENT SPECIFICATION

(11) **EP 2 297 117 B1**
(45) Date of publication and mention of the grant of the patent: **31.10.2012**
(21) Application number: 09751278.4
(22) Date of filing: 18.05.2009
(51) Int. Cl.: C07D 239/88, C07D 239/90, C07D 239/95, C07D 401/06, C07D 401/12, C07D 401/14, C07D 407/06, C07D 407/12, C07D 413/04, C07D 413/12, C07D 417/12, A61K 31/517, A61P 3/04, A61P 3/10, A61P 9/00

(54) **BICYCLIC HETEROCYCLE DERIVATIVES AND USE THEREOF AS GPR119 MODULATORS**
BICYCLISCHE HETEROCYCLUSDERIVATE UND DEREN VERWENDUNG ALS GPR119-MODULATOREN
DÉRIVÉS HÉTÉROCYCLIQUES BICYCLIQUES ET LEUR UTILISATION EN TANT QUE MODULATEURS DE GPR119

(30) Priority: 19.05.2008 US 54319 P
(43) Date of publication of application: 23.03.2011
(73) Proprietor: Merck Sharp & Dohme Corp., Rahway, NJ 07065-0907 (US)
(72) Inventor: HARRIS, Joel, M., Summit, NJ 07901 (US); NEELAMKAVIL, Santhosh, Francis, Scotch Plains New Jersey 07076 (US); NEUSTADT, Bernard, R., West Orange, NJ 07052 (US); BOYLE, Craig, D., Branchburg, NJ 08876 (US); LIU, Hong, Hillsborough New Jersey 08844 (US); HAO, Jinsong, Belle Mead, NJ 08502 (US); STAMFORD, Andrew, Chatham Township, NJ 07928 (US); CHACKALAMANNIL, Samuel, Califon, NJ 07830 (US); GREENLEE, William, J., Teaneck, NJ 07666 (US)
(74) Representative: Buchan, Gavin MacNicol
(86) International application number: PCT/US2009/044323
(87) International publication number: WO 2009/143049

(56) References cited:
- WO-A-94/21259
- WO-A-2006/012577
- WO-A-2007/003486
- WO-A-2008/025798
- WO-A-2008/130584
- US-A- 5 264 439
- US-A- 5 385 894
- US-B1- 6 337 332
- US-B2- 7 132 426

## Description

### FIELD OF THE INVENTION

The present invention relates to Bicyclic Heterocycle Derivatives, compositions comprising a Bicyclic Heterocycle Derivative, and said Bicyclic Heterocycle Derivatives for use in treating or preventing obesity, diabetes, a diabetic complication, a metabolic disorder, a cardiovascular disease or a disorder related to the activity of GPR119 in a patient.

### BACKGROUND OF THE INVENTION

Although a number of receptor classes exist in humans, by far the most abundant and therapeutically relevant is represented by the G protein-coupled receptor (GPCR or GPCRs) class. It is estimated that there are some 100,000 genes within the human genome, and of these, approximately 2% or 2,000 genes, are estimated to code for GPCRs. Receptors, including GPCRs, for which the endogenous ligand has been identified are referred to as "known" receptors, while receptors for which the endogenous ligand has not been identified are referred to as "orphan" receptors. GPCRs represent an important area for the development of pharmaceutical products, as evidenced by the fact that pharmaceutical products have been developed from approximately 20 of the 100 known GPCRs. This distinction is not merely semantic, particularly in the case of GPCRs. Thus, the orphan GPCRs are to the pharmaceutical industry what gold was to California in the late 19th century--an opportunity to drive growth, expansion, enhancement and development.

GPCRs share a common structural motif. All these receptors have seven sequences of between 22 to 24 hydrophobic amino acids that form seven alpha helices, each of which spans the membrane (each span is identified by number, *i.e*., transmembrane-1 (TM-1), transmembrane-2 (TM-2), etc.). The transmembrane helices are joined by strands of amino acids between transmembrane-2 and transmembrane-3, transmembrane-4 and transmembrane-5, and transmembrane-6 and transmembrane-7 on the exterior, or "extracellular" side, of the cell membrane (these are referred to as "extracellular" regions 1, 2 and 3 (EC-1, EC-2 and EC-3), respectively). The transmembrane helices are also joined by strands of amino acids between transmembrane-1 and transmembrane-2, transmembrane-3 and transmembrane-4, and transmembrane-5 and transmembrane-6 on the interior, or "intracellular" side, of the cell membrane (these are referred to as "intracellular" regions 1, 2 and 3 (IC-1, IC-2 and IC-3), respectively). The "carboxy" ("C") terminus of the receptor lies in the intracellular space within the cell, and the "amino" ("N") terminus of the receptor lies in the extracellular space outside of the cell.

Generally, when an endogenous ligand binds with the receptor (often referred to as "activation" of the receptor), there is a change in the conformation of the intracellular region that allows for coupling between the intracellular region and an intracellular "G-protein." It has been reported that GPCRs are "promiscuous" with respect to G proteins, *i.e*., that a GPCR can interact with more than one G protein. See, Kenakin, T., Life Sciences 43, 1095 (1988). Although other G proteins exist, currently, Gq, Gs, Gi, and Go are G proteins that have been identified. Endogenous ligand-activated GPCR coupling with the G-protein begins a signaling cascade process (referred to as "signal transduction"). Under normal conditions, signal transduction ultimately results in cellular activation or cellular inhibition. It is thought that the IC-3 loop as well as the carboxy terminus of the receptor interact with the G protein.

Under physiological conditions, GPCRs exist in the cell membrane in equilibrium between two different conformations: an "inactive" state and an "active" state. A receptor in an inactive state is unable to link to the intracellular signaling transduction pathway to produce a biological response. Changing the receptor conformation to the active state allows linkage to the transduction pathway (via the G-protein) and produces a biological response. A receptor can be stabilized in an active state by an endogenous ligand or a compound such as a drug.

Modulation of G-protein coupled receptors has been well-studied for controlling various metabolic disorders. Small molecule modulators of the receptor GPR119, a G-protein coupled-receptor described in, for example, GenBank (see, e.g., accession numbers XM.sub.-066873 and AY288416), have been shown to be useful for treating or preventing certain metabolic disorders. GPR119 is a G protein-coupled receptor that is selectively expressed on pancreatic beta cells. GPR119 activation leads to elevation of a level of intracellular cAMP, consistent with GPR119 being coupled to Gs. Agonists to GPR119 stimulate glucose-dependent insulin secretion in vitro and lower an elevated blood glucose level *in vivo.* See, e.g., International Publication Nos. WO 04/065380 and WO 04/076413, and European Patent No. EP 1338651.

U.S. Patent No. 7,132,426 discloses pyrazolo[3,4-d]pyrimidine ethers and related compounds as modulators of the GPR119 receptor that are useful for the treatment of various metabolic-related disorders such as type I diabetes, type II diabetes, inadequate glucose tolerance, insulin resistance, hyperglycemia, hyperlipidemia, hypertriglyceridemia, hypercholesterolemia, dyslipidemia or syndrome X. The compounds are also reported as being useful for controlling weight gain, controlling food intake, and inducing satiety in mammals. The promising nature of these GPCR119 modulators indicates a need in the art for additional small molecule GRP119 modulators with improved efficacy and safety profiles. This invention addresses that need.

WO2006/012577 relates to quinazolinone derivatives, compositions and methods of treating diabetes, obesity and related disorders, such as regulation of glucose homeostasis and food intake. There is no indication, however, that the compounds disclosed therein can act as GPR119 modulators.

### SUMMARY OF THE INVENTION

In one aspect, the present invention provides compounds of Formula (I): 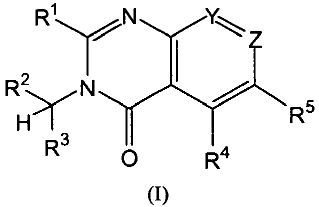 or a pharmaceutically acceptable salt or solvate thereof,
wherein
Y is -N- or -C(R⁷)-;
Z is -N- or -C(R⁶)-, such that at least one of Y and Z is other than -N-;
R¹ is -H, alkyl, -OH, -OR⁹, -SR⁹, or -N(R¹⁰)₂, wherein an alkyl group can be optionally substituted with one or more groups, which are each independently selected from halo, aryl, - OH, -O-haloalkyl, -O-alkyl, -CN, -N(R¹⁰)₂, -C(O)R⁹, -C(O)OR⁹, -C(O)N(R¹⁰)₂ and - NHC(O)R⁹;
R² and R³ are each independently selected from haloalkyl, -(alkylene)ₙ-aryl, -(alkylene)ₙ-heteroaryl, -(alkylene)ₙ-cycloalkyl and -(alkylene)-Oalkyl or
R² and R³ and the carbon atom to which they are both attached, combine to form: 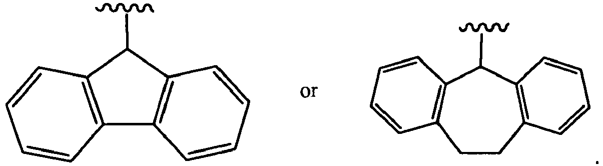
R⁴, R⁵, R⁶ and R⁷ are each independently selected from H, -(alkylene)ₙ-N(R⁸)₂, - S(O)ₚR¹³, -OR¹², -C(O)OR¹¹, -C(O)R¹¹, alkyl, halo, haloalkyl, -CN, cycloalkyl, heteroaryl, heterocycloalkyl, -C(O)N(R⁸)₂, -C(=NOH)-NH₂ and -(alkylene)ₙ-aryl, wherein any aryl, heteroaryl or heterocycloalkyl group can be optionally substituted with one or more groups, which are each independently selected from halo, alkyl, aryl, hydroxyalkyl, -O-alkyl, haloalkyl, -C(O)O-alkyl, -C(O)-alkyl, -NHC(O)O-alkyl, -C(O)NH-alkyl, -CN, -NO₂, -S(O)₂-alkyl and -S-alkyl, such that R⁴, R⁵, R⁶ and R⁷ are not each H, and wherein a heterocycloalkyl or heteroaryl group can be optionally fused to a benzene ring;
each occurrence of R⁸ is independently H, alkyl, -alkylene-C(O)OR¹¹, -(alkylene)ₙ-aryl, -(alkylene)ₙ-cycloalkyl, -(alkylene)ₙ-heterocycloalkyl, -(alkylene)ₙ-heteroaryl, -alkylene-O-alkyl, cyanoalkyl, alkenyl, alkynyl, haloalkyl or haloalkenyl, wherein an aryl, cycloalkyl, heterocycloalkyl or heteroaryl group can be optionally substituted with one or more groups, which are each independently selected from halo, alkyl, hydroxyalkyl, -OR¹⁰, haloalkyl, -CN, - NO₂, -O-haloalkyl, -S-alkyl, -S-haloalkyl, -alkylene-O-alkyl, -CN, -N(R¹²)₂, -C(O)R¹², - C(O)OR¹², -C(O)N(R¹²)₂, -NHC(O)R¹², -NHS(O)_{q}R¹⁴, -S(O)ₚR¹⁴ and -S(O)_{q}N(R¹²)₂;
each occurrence of R⁹ is alkyl, alkenyl, alkynyl, haloalkyl, -alkylene-O-aryl, -alkyleneS-aryl, -alkylene-N(R⁸)C(O)O-alkyl, -(alkylene)ₙ-aryl, -(alkylene)ₙ-cycloalkyl, -(alkylene)ₙ-cycloalkenyl, -(alkylene)ₙ-heterocycloalkyl or -(alkylene)ₙ-heteroaryl, wherein an aryl, cycloalkyl, cycloalkenyl, heterocycloalkyl or heteroaryl group can be optionally substituted with one or more groups, which are each independently selected from halo, alkyl, hydroxyalkyl, -OR¹⁰, haloalkyl, -CN, -NO₂, -O-haloalkyl, -S-haloalkyl, -alkylene-O-alkyl, - CN, -N(R¹²)₂, -C(O)R¹², -C(O)OR¹², -C(O)N(R¹²)₂, -NHC(O)R¹², -NHS(O)_{q}R¹⁴, -S(O)ₚR¹⁴ and -S(O)_{q}N(R¹²)₂;
each occurrence of R¹⁰ is independently H, alkyl, -(alkylene)ₙ-aryl, -(alkylene)ₙ-cycloalkyl, -(alkylene)ₙ-cycloalkenyl, -(alkylene)ₙ-heterocycloalkyl or -(alkylene)ₙ-heteroaryl, wherein any of the above groups, excluding H, can be optionally substituted with one or more groups, which are each independently selected from alkyl, haloalkyl, hydroxyalkyl, halo, -OH, -O-haloalkyl, -O-alkyl, -O-aryl, -alkylene-O-alkyl, -CN, -N(R¹²)₂, -C(O)H, -C(O)R¹², - C(O)OR¹², -C(O)N(R¹²)₂, -NHC(O)R¹², -NHS(O)_{q}R¹⁴, -S(O)ₚR¹⁴ and -S(O)_{q}N(R¹²)₂;
each occurrence of R¹¹ is independently H, alkyl, aryl, heterocycloalkyl, heteroaryl or cycloalkyl, wherein any of the above groups can be optionally substituted with one or more groups, which are each independently selected from alkyl, haloalkyl, hydroxyalkyl, halo, -OH, -O-haloalkyl, -O-alkyl, -O-aryl, -alkylene-O-alkyl, -CN, -N(R¹²)₂, -C(O)H, -C(O)R¹², - C(O)OR¹², -C(O)N(R¹²)₂, -NHC(O)R¹², -NHS(O)_{q}R¹⁴, -S(O)ₚR¹⁴ and -S(O)_{q}N(R¹²)₂;
each occurrence of R¹² is independently H, alkyl, -(alkylene)ₙ-aryl, heterocycloalkyl, heteroaryl or cycloalkyl;
each occurrence of R¹³ is independently alkyl, aryl, heterocycloalkyl, heteroaryl or cycloalkyl, wherein any of the above groups can be optionally substituted with one or more groups, which are each independently selected from alkyl, haloalkyl, hydroxyalkyl, halo, -OH, -O-haloalkyl, -O-alkyl, -O-aryl, -alkylene-O-alkyl, -CN, -N(R¹²)₂, -C(O)H, -C(O)R¹², - C(O)OR¹², -C(O)N(R¹²)₂, -NHC(O)R¹², -NHS(O)_{q}R¹⁴, -S(O)ₚR¹⁴ and -S(O)_{q}N(R¹²)₂;
each occurrence of R¹⁴ is independently alkyl, -(alkylene)ₙ-aryl, heterocycloalkyl, heteroaryl or cycloalkyl;
each occurrence of n is independently 0 or 1;
each occurrence of p is independently 0, 1 or 2; and
each occurrence of q is independently 1 or 2 with the exception of

The compounds of formula (I) and pharmaceutically acceptable salts or solvates thereof (referred to collectively herein as the "Bicyclic Heterocycle Derivatives") can be useful for treating or preventing obesity, diabetes, a diabetic complication, a metabolic disorder, a cardiovascular disease or a disorder related to the activity of GPR119 (each being a "Condition") in a patient.

The present invention further provides compositions comprising an effective amount of one or more Bicyclic Heterocycle Derivatives or a pharmaceutically acceptable salt or solvate, thereof, and a pharmaceutically acceptable carrier. The compositions can be useful for treating or preventing a Condition in a patient.

The details of the invention are set forth in the accompanying detailed description below.

Although any methods and materials similar to those described herein can be used in the practice or testing of the present invention, illustrative methods and materials are now described. Other features, objects, and advantages of the invention will be apparent from the description and the claims.

### DETAILED DESCRIPTION OF THE INVENTION

In an embodiment, the present invention provides Bicyclic Heterocycle Derivatives of Formula (I), compositions comprising one or more Bicyclic Heterocycle Derivatives, and use of said Bicyclic Heterocycle Derivatives for treating or preventing a Condition in a patient.

### Definitions and Abbreviations

As used above, and throughout this disclosure, the following terms, unless otherwise indicated, shall be understood to have the following meanings:
A "patient" is a human or non-human mammal. In one embodiment, a patient is a human. In another embodiment, a patient is a non-human mammal, including, but not limited to, a monkey, dog, baboon, rhesus, mouse, rat, horse, cat or rabbit. In another embodiment, a patient is a companion animal, including but not limited to a dog, cat, rabbit, horse or ferret. In one embodiment, a patient is a dog. In another embodiment, a patient is a cat.
The term "obesity" as used herein, refers to a patient being overweight and having a body mass index (BMI) of 25 or greater. In one embodiment, an obese patient has a BMI of 25 or greater. In another embodiment, an obese patient has a BMI from 25 to 30. In another embodiment, an obese patient has a BMI greater than 30. In still another embodiment, an obese patient has a BMI greater than 40.
The term "obesity-related disorder" as used herein refers to: (i) disorders which result from a patient having a BMI of 25 or greater; and (ii) eating disorders and other disorders associated with excessive food intake. Non-limiting examples of an obesity-related disorder include edema, shortness of breath, sleep apnea, skin disorders and high blood pressure.
The term "metabolic syndrome" as used herein, refers to a set of risk factors that make a patient more succeptible to cardiovascular disease and/or type 2 diabetes. A patient is said to have metabolic syndrome if the patient simultaneously has three or more of the following five risk factors:
   1) central/abdominal obesity as measured by a waist circumference of greater than 40 inches in a male and greater than 35 inches in a female;
   2) a fasting triglyceride level of greater than or equal to 150 mg/dL;
   3) an HDL cholesterol level in a male of less than 40 mg/dL or in a female of less than 50 mg/dL;
   4) blood pressure greater than or equal to 130/85 mm Hg; and
   5) a fasting glucose level of greater than or equal to 110 mg/dL.

The term "effective amount" as used herein, refers to an amount of Bicyclic Heterocycle Derivative and/or an additional therapeutic agent, or a composition thereof that is effective in producing the desired therapeutic, ameliorative, inhibitory or preventative effect when administered to a patient suffering from a Condition. In the combination therapies of the present invention, an effective amount can refer to each individual agent or to the combination as a whole, wherein the amounts of all agents administered are together effective, but wherein the component agent of the combination may not be present individually in an effective amount.

The term "alkyl," as used herein, refers to an aliphatic hydrocarbon group which may be straight or branched and which contains from about 1 to about 20 carbon atoms. In one embodiment, an alkyl group contains from about 1 to about 12 carbon atoms. In another embodiment, an alkyl group contains from about 1 to about 6 carbon atoms. Non-limiting examples of alkyl groups include methyl, ethyl, n-propyl, isopropyl, n-butyl, sec-butyl, isobutyl, tert-butyl, n-pentyl, neopentyl, isopentyl, n-hexyl, isohexyl and neohexyl. In another embodiment, an alkyl group is linear. In another embodiment, an alkyl group is branched.

The term "alkenyl," as used herein, refers to an aliphatic hydrocarbon group containing at least one carbon-carbon double bond and which may be straight or branched and contains from about 2 to about 15 carbon atoms. In one embodiment, an alkenyl group contains from about 2 to about 12 carbon atoms. In another embodiment, an alkenyl group contains from about 2 to about 6 carbon atoms. Non-limiting examples of alkenyl groups include ethenyl, propenyl, n-butenyl, 3-methylbut-2-enyl, n-pentenyl, octenyl and decenyl.

The term "alkynyl," as used herein, refers to an aliphatic hydrocarbon group containing at least one carbon-carbon triple bond and which may be straight or branched and contains from about 2 to about 15 carbon atoms. In one embodiment, an alkynyl group contains from about 2 to about 12 carbon atoms. In another embodiment, an alkynyl group contains from about 2 to about 6 carbon atoms. Non-limiting examples of alkynyl groups include ethynyl, propynyl, 2-butynyl and 3-methylbutynyl.

The term "alkylene," as used herein, refers to an alkyl group, as defined above, wherein one of the alkyl group's hydrogen atoms has been replaced with a bond. Non-limiting examples of alkylene groups include -CH₂-, -CH₂CH₂-, -CH₂CH₂CH₂-, -CH₂CH₂CH₂CH₂-, - CH(CH₃)CH₂CH₂-, -CH(CH₃)- and -CH₂CH(CH₃)CH₂-. In one embodiment, an alkylene group has from 1 to about 6 carbon atoms. In another embodiment, an alkylene group is branched. In another embodiment, an alkylene group is linear.

The term "aryl," as used herein, refers to an aromatic monocyclic or multicyclic ring system comprising from about 6 to about 14 carbon atoms. In one embodiment, an aryl group contains from about 6 to about 10 carbon atoms. Non-limiting examples of aryl groups include phenyl and naphthyl. In one embodiment, an aryl group is phenyl.

The term "arylene," as used herein, refers to an aryl group, as defined above, wherein one of the aryl group's hydrogen atoms has been replaced with a bond. Non-limiting examples of arylene groups include: 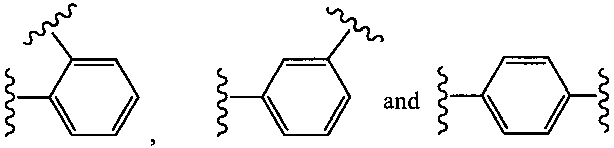 In one embodiment, an arylene group is a phenylene group.

The term "cycloalkyl," as used herein, refers to a non-aromatic mono- or multicyclic ring system comprising from about 3 to about 10 ring carbon atoms. In one embodiment, a cycloalkyl contains from about 5 to about 10 ring carbon atoms. In another embodiment, a cycloalkyl contains from about 5 to about 7 ring atoms. The term "cycloalkyl" also encompasses a cycloalkyl group, as defined above, that is fused to an aryl (*e.g*., benzene) or heteroaryl ring. A ring carbon atom of a cycloalkyl group may be functionalized as a carbonyl group. Non-limiting examples of monocyclic cycloalkyls include cyclopropyl, cyclobutyl, cyclopentyl, cyclohexyl, cycloheptyl and cyclooctyl. Non-limiting examples of multicyclic cycloalkyls include 1-decalinyl, norbornyl and adamantyl.

The term "cycloalkenyl," as used herein, refers to a non-aromatic mono- or multicyclic ring system comprising from about 3 to about 10 ring carbon atoms and containing at least one endocyclic double bond. In one embodiment, a cycloalkenyl contains from about 5 to about 10 ring carbon atoms. In another embodiment, a cycloalkenyl contains 5 or 6 ring atoms. Non-limiting examples of monocyclic cycloalkenyls include cyclopentenyl, cyclohexenyl, cyclohepta-1,3-dienyl, and the like. In another embodiment, a cycloalkenyl group is a 5-membered cycloalkenyl.

The term "heteroaryl," as used herein, refers to an aromatic monocyclic or multicyclic ring system comprising about 5 to about 14 ring atoms, wherein from 1 to 4 of the ring atoms is independently O, N or S and the remaining ring atoms are carbon atoms. In one embodiment, a heteroaryl group has 5 to 10 ring atoms. In another embodiment, a heteroaryl group is monocyclic and has 5 or 6 ring atoms. In another embodiment, a heteroaryl group is bicyclic and has from 8 to 14 ring atoms. A heteroaryl group is joined via a ring carbon atom, and any nitrogen atom of a heteroaryl can be optionally oxidized to the corresponding N-oxide. The term "heteroaryl" also encompasses a heteroaryl group, as defined above, that is fused to a benzene ring. Non-limiting examples of heteroaryls include pyridyl, pyrazinyl, furanyl, thienyl, pyrimidinyl, pyridone (including N-substituted pyridones), isoxazolyl, isothiazolyl, oxazolyl, thiazolyl, pyrazolyl, furazanyl, pyrrolyl, triazolyl, 1,2,4-thiadiazolyl, pyrazinyl, pyridazinyl, quinoxalinyl, phthalazinyl, oxindolyl, imidazo[1,2-a]pyridinyl, imidazo[2,1-b]thiazolyl, benzofurazanyl, indolyl, azaindolyl, benzimidazolyl, benzothienyl, quinolinyl, imidazolyl, thienopyridyl, quinazolinyl, thienopyrimidyl, pyrrolopyridyl, imidazopyridyl, isoquinolinyl, benzoazaindolyl, 1,2,4-triazinyl, benzothiazolyl and the like. The term "heteroaryl" also refers to partially saturated heteroaryl moieties such as, for example, tetrahydroisoquinolyl, tetrahydroquinolyl and the like. In another embodiment, a heteroaryl group is a 5-membered heteroaryl. In another embodiment, a heteroaryl group is a 6-membered heteroaryl.

The term "heteroarylene," as used herein, refers to an heteroaryl group, as defined above, wherein one of the heteroaryl group's hydrogen atoms has been replaced with a bond. Non-limiting examples of heteroarylene groups include those derived from a pyridyl group or from a pyrimidinyl group. In one embodiment, a heteroarylene group is 5-membered. In another embodiment, a heteroarylene group is 6-membered.

The term "heterocycloalkyl," as used herein, refers to a non-aromatic saturated monocyclic or multicyclic ring system comprising 3 to about 10 ring atoms, wherein from 1 to 4 of the ring atoms are independently O, S or N and the remainder of the ring atoms are carbon atoms. In one embodiment, a heterocycloalkyl group has from about 5 to about 10 ring atoms. In another embodiment, a heterocycloalkyl group has 5 or 6 ring atoms. There are no adjacent oxygen and/or sulfur atoms present in the ring system. Any -NH group in a heterocycloalkyl ring may exist protected such as, for example, as an -N(BOC), -N(Cbz), -N(Tos) group and the like; such protected heterocycloalkyl groups are considered part of this invention. The term "heterocycloalkyl" also encompasses a heterocycloalkyl group, as defined above, that is fused to an aryl (*e.g*., benzene) or heteroaryl ring. The nitrogen or sulfur atom of the heterocycloalkyl can be optionally oxidized to the corresponding N-oxide, S-oxide or S,S-dioxide. Non-limiting examples of monocyclic heterocycloalkyl rings include piperidyl, pyrrolidinyl, piperazinyl, morpholinyl, thiomorpholinyl, thiazolidinyl, 1,4-dioxanyl, tetrahydrofuranyl, tetrahydrothiophenyl, lactam, lactone, and the like. A ring carbon atom of a heterocycloalkyl group may be functionalized as a carbonyl group. An illustrative example of such a heterocycloalkyl group is pyrrolidonyl:

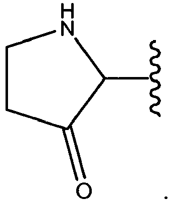

In another embodiment, a heterocycloalkyl group is a 5-membered heterocycloalkyl. In another embodiment, a heterocycloalkyl group is a 6-membered heterocycloalkyl.

The term "heterocycloalkenyl," as used herein, refers to a heterocycloalkyl group, as defined above, wherein the heterocycloalkyl group contains from 3 to 10 ring atoms, and at least one endocyclic carbon-carbon or carbon-nitrogen double bond. In one embodiment, a heterocycloalkenyl group has from 5 to 10 ring atoms. In another embodiment, a heterocycloalkenyl group is monocyclic and has 5 or 6 ring atoms. The nitrogen or sulfur atom of the heterocycloalkenyl can be optionally oxidized to the corresponding N-oxide, S-oxide or S,S-dioxide. Non-limiting examples of heterocycloalkenyl groups include 1,2,3,4-tetrahydropyridinyl, 1,2-dihydropyridinyl, 1,4-dihydropyridinyl, 1,2,3,6-tetrahydropyridinyl, 1,4,5,6-tetrahydropyrimidinyl, 2-pyrrolinyl, 3-pyrrolinyl, 2-imidazolinyl, 2-pyrazolinyl, dihydroimidazolyl, dihydrooxazolyl, dihydrooxadiazolyl, dihydrothiazolyl, 3,4-dihydro-2H-pyranyl, dihydrofuranyl, fluoro-substituted dihydrofuranyl, 7-oxabicyclo[2.2.1]heptenyl, dihydrothiophenyl, dihydrothiopyranyl, and the like. A ring carbon atom of a heterocycloalkenyl group may be functionalized as a carbonyl group. An illustrative example of such a heterocycloalkenyl group is:

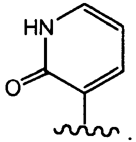

In another embodiment, a heterocycloalkenyl group is a 5-membered heterocycloalkenyl. In another embodiment, a heterocycloalkenyl group is a 6-membered heterocycloalkenyl.

It should also be noted that tautomeric forms such as, for example, the moieties: 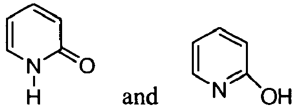 are considered equivalent in certain embodiments of this invention.

"Halo" means -F, -Cl, -Br or -I. In one embodiment, halo refers to -F, -Cl or -Br.

The term "haloalkyl," as used herein, refers to an alkyl group as defined above, wherein one or more of the alkyl group's hydrogen atoms has been replaced with a halogen. In one embodiment, a haloalkyl group has from 1 to 6 carbon atoms. In another embodiment, a haloalkyl group is substituted with from 1 to 3 F atoms. Non-limiting examples of haloalkyl groups include ―CH₂F, -CHF₂, -CF₃, -CH₂Cl and -CCl₃.

The term "haloalkenyl," as used herein, refers to an alkenyl group as defined above, wherein one or more of the alkenyl group's hydrogen atoms has been replaced with a halogen. In one embodiment, a haloalkenyl group has from 2 to 6 carbon atoms. In another embodiment, a haloalkenyl group is substituted with from 1 to 6 F atoms. In another embodiment, a haloalkenyl group is substituted with from 1 to 3 F atoms. Non-limiting examples of haloalkenyl groups include ―CH=CH₂F, -CH=CHF₂, and ―CH=CHCF₃.

The term "cyanoalkyl," as used herein, refers to an alkyl group as defined above, wherein one or more of the alkyl group's hydrogen atoms has been replaced with a -CN group. In one embodiment, a cyanoalkyl group has from 1 to 6 carbon atoms. In another embodiment, a cyanoalkyl group is substituted with 1 -CN group. Non-limiting examples of cyanoalkyl groups include ―CH₂CN, -CH₂CH₂CN and -CH₂CH₂CH₂CN.

The term "hydroxyalkyl," as used herein, refers to an alkyl group as defined above, wherein one or more of the alkyl group's hydrogen atoms has been replaced with an -OH group. In one embodiment, a hydroxyalkyl group has from 1 to 6 carbon atoms. Non-limiting examples of hydroxyalkyl groups include ―CH₂OH, -CH₂CH₂OH, -CH₂CH₂CH₂OH and - CH₂CH(OH)CH₃.

The term "alkoxy" as used herein, refers to an -O-alkyl group, wherein an alkyl group is as defined above. Non-limiting examples of alkoxy groups include methoxy, ethoxy, n-propoxy, isopropoxy, n-butoxy and t-butoxy. An alkoxy group is bonded via its oxygen atom.

The term "substituted" means that one or more hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds. By "stable compound' or "stable structure" is meant a compound that is sufficiently robust to survive isolation to a useful degree of purity from a reaction mixture, and formulation into an efficacious therapeutic agent.

The term "purified", "in purified form" or "in isolated and purified form" for a compound refers to the physical state of the compound after being isolated from a synthetic process (e.g. from a reaction mixture), or natural source or combination thereof. Thus, the term "purified", "in purified form" or "in isolated and purified form" for a compound refers to the physical state of the compound after being obtained from a purification process or processes described herein or well known to the skilled artisan (*e.g*., chromatography, recrystallization and the like) , in sufficient purity to be characterizable by standard analytical techniques described herein or well known to the skilled artisan.

It should also be noted that any carbon as well as heteroatom with unsatisfied valences in the text, schemes, examples and tables herein is assumed to have the sufficient number of hydrogen atom(s) to satisfy the valences.

When a functional group in a compound is termed "protected", this means that the group is in modified form to preclude undesired side reactions at the protected site when the compound is subjected to a reaction. Suitable protecting groups will be recognized by those with ordinary skill in the art as well as by reference to standard textbooks such as, for example, T. W. Greene *et al, Protective Groups in Organic Synthesis* (1991), Wiley, New York.

When any variable (e.g., R¹, R², n, etc...) occurs more than one time in any constituent or in Formula (I), its definition on each occurrence is independent of its definition at every other occurrence.

As used herein, the term "composition" is intended to encompass a product comprising the specified ingredients in the specified amounts, as well as any product which results, directly or indirectly, from combination of the specified ingredients in the specified amounts.

One or more compounds of the invention may exist in unsolvated as well as solvated forms with pharmaceutically acceptable solvents such as water, ethanol, and the like, and it is intended that the invention embrace both solvated and unsolvated forms. "Solvate" means a physical association of a compound of this invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolatable solvates. Non-limiting examples of solvates include ethanolates, methanolates, and the like. "Hydrate" is a solvate wherein the solvent molecule is H₂O.

One or more compounds of the invention may optionally be converted to a solvate. Preparation of solvates is generally known. Thus, for example, M. Caira et al, J. Pharmaceutical Sci., 93(3), 601-611 (2004) describes the preparation of the solvates of the antifungal fluconazole in ethyl acetate as well as from water. Similar preparations of solvates, hemisolvate, hydrates and the like are described by E. C. van Tonder et al, AAPS PharmSciTechours., 5(1), article 12 (2004); and A. L. Bingham et al, Chem. Commun., 603-604 (2001). A typical, non-limiting, process involves dissolving the inventive compound in desired amounts of the desired solvent (organic or water or mixtures thereof) at a higher than ambient temperature, and cooling the solution at a rate sufficient to form crystals which are then isolated by standard methods. Analytical techniques such as, for example IR spectroscopy, show the presence of the solvent (or water) in the crystals as a solvate (or hydrate).

The Bicyclic Heterocycle Derivatives can form salts which are also within the scope of this invention. Reference to a Bicyclic Heterocycle Derivative herein is understood to include reference to salts thereof, unless otherwise indicated. The term "salt(s)", as employed herein, denotes acidic salts formed with inorganic and/or organic acids, as well as basic salts formed with inorganic and/or organic bases. In addition, when a Bicyclic Heterocycle Derivative contains both a basic moiety, such as, but not limited to a pyridine or imidazole, and an acidic moiety, such as, but not limited to a carboxylic acid, zwitterions ("inner salts") may be formed and are included within the term "salt(s)" as used herein. In one embodiment, the salt is a pharmaceutically acceptable (*i.e*., non-toxic, physiologically acceptable) salt. In another embodiment, the salt is other than a pharmaceutically acceptable salt. Salts of the compounds of the Formula (I) may be formed, for example, by reacting a Bicyclic Heterocycle Derivative with an amount of acid or base, such as an equivalent amount, in a medium such as one in which the salt precipitates or in an aqueous medium followed by lyophilization.

Exemplary acid addition salts include acetates, ascorbates, benzoates, benzenesulfonates, bisulfates, borates, butyrates, citrates, camphorates, camphorsulfonates, fumarates, hydrochlorides, hydrobromides, hydroiodides, lactates, maleates, methanesulfonates, naphthalenesulfonates, nitrates, oxalates, phosphates, propionates, salicylates, succinates, sulfates, tartarates, thiocyanates, toluenesulfonates (also known as tosylates) and the like. Additionally, acids which are generally considered suitable for the formation of pharmaceutically useful salts from basic pharmaceutical compounds are discussed, for example, by P. Stahl et al, Camille G. (eds.) Handbook of Pharmaceutical Salts. Properties, Selection and Use. (2002) Zurich: Wiley-VCH; S. Berge et al, Journal of Pharmaceutical Sciences (1977) 66(1) 1-19; P. Gould, International J. of pharmaceutics (1986) 33 201-217; Anderson et al, The Practice of Medicinal Chemistry (1996), Academic Press, New York; and in The Orange Book (Food & Drug Administration, Washington, D.C. on their website). These disclosures are incorporated herein by reference thereto.

Exemplary basic salts include ammonium salts, alkali metal salts such as sodium, lithium, and potassium salts, alkaline earth metal salts such as calcium and magnesium salts, salts with organic bases (for example, organic amines) such as dicyclohexylamine, choline, t-butyl amine, and salts with amino acids such as arginine, lysine and the like. Basic nitrogen-containing groups may be quarternized with agents such as lower alkyl halides (*e.g*., methyl, ethyl, and butyl chlorides, bromides and iodides), dialkyl sulfates (*e.g*., dimethyl, diethyl, and dibutyl sulfates), long chain halides (*e.g*., decyl, lauryl, and stearyl chlorides, bromides and iodides), aralkyl halides (*e.g*., benzyl and phenethyl bromides), and others.

All such acid salts and base salts are intended to be pharmaceutically acceptable salts within the scope of the invention and all acid and base salts are considered equivalent to the free forms of the corresponding compounds for purposes of the invention.

Pharmaceutically acceptable esters of the present compounds include the following groups: (1) carboxylic acid esters obtained by esterification of the hydroxy group of a hydroxyl compound, in which the non-carbonyl moiety of the carboxylic acid portion of the ester grouping is selected from straight or branched chain alkyl (for example, methyl, ethyl, n-propyl, isopropyl, t-butyl, sec-butyl or n-butyl), alkoxyalkyl (for example, methoxymethyl), aralkyl (for example, benzyl), aryloxyalkyl (for example, phenoxymethyl), aryl (for example, phenyl optionally substituted with, for example, halogen, C₁₋₄alkyl, or C₁₋₄alkoxy or amino); (2) sulfonate esters, such as alkyl- or aralkylsulfonyl (for example, methanesulfonyl); (3) amino acid esters (for example, L-valyl or L-isoleucyl); (4) phosphonate esters and (5) mono-, di- or triphosphate esters. The phosphate esters may be further esterified by, for example, a C₁₋₂₀ alcohol or reactive derivative thereof, or by a 2,3-di (C₆₋₂₄)acyl glycerol.

Diastereomeric mixtures can be separated into their individual diastereomers on the basis of their physical chemical differences by methods well known to those skilled in the art, such as, for example, by chromatography and/or fractional crystallization. Enantiomers can be separated by converting the enantiomeric mixture into a diastereomeric mixture by reaction with an appropriate optically active compound (*e.g*., chiral auxiliary such as a chiral alcohol or Mosher's acid chloride), separating the diastereomers and converting (*e.g.,* hydrolyzing) the individual diastereomers to the corresponding pure enantiomers. Sterochemically pure compounds may also be prepared by using chiral starting materials or by employing salt resolution techniques. Also, some of the Bicyclic Heterocycle Derivatives may be atropisomers (*e.g*., substituted biaryls) and are considered as part of this invention. Enantiomers can also be separated by use of chiral HPLC methods.

It is also possible that the Bicyclic Heterocycle Derivatives may exist in different tautomeric forms, and all such forms are embraced within the scope of the invention. Also, for example, all keto-enol and imine-enamine forms of the compounds are included in the invention.

All stereoisomers (for example, geometric isomers, optical isomers and the like) of the present compounds (including those of the salts, solvates, hydrates, esters and prodrugs of the compounds as well as the salts, solvates and esters of the prodrugs), such as those which may exist due to asymmetric carbons on various substituents, including enantiomeric forms (which may exist even in the absence of asymmetric carbons), rotameric forms, atropisomers, and diastereomeric forms, are contemplated within the scope of this invention, as are positional isomers (such as, for example, 4-pyridyl and 3-pyridyl). For example, if a Bicyclic Heterocycle Derivative incorporates a double bond or a fused ring, both the cis- and transforms, as well as mixtures, are embraced within the scope of the invention. Also, for example, all keto-enol and imine-enamine forms of the compounds are included in the invention.

Individual stereoisomers of the compounds of the invention may, for example, be substantially free of other isomers, or may be admixed, for example, as racemates or with all other, or other selected, stereoisomers. The chiral centers of the present invention can have the S or R configuration as defined by the *IUPAC* 1974 Recommendations. The use of the terms "salt", "solvate", "ester", "prodrug" and the like, is intended to apply equally to the salt, solvate, ester and prodrug of enantiomers, stereoisomers, rotamers, tautomers, positional isomers, racemates or prodrugs of the inventive compounds.

The present invention also embraces isotopically-labelled compounds of the present invention which are identical to those recited herein, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorus, fluorine and chlorine, such as ²H, ³H, ¹³C, ¹⁴C, ¹⁵N, ¹⁸O, ¹⁷O, ³¹P, ³²P, ³⁵S, ¹⁸F and ³⁶Cl, respectively.

Certain isotopically-labelled Bicyclic Heterocycle Derivatives (*e.g*., those labeled with ³H and ¹⁴C) are useful in compound and/or substrate tissue distribution assays. In one embodiment, ritiated (*i.e*., ³H) and carbon-14 (*i.e*., ¹⁴C) isotopes are employed for their ease of preparation and detectability. In another embodiment, substitution with heavier isotopes such as deuterium (*i.e*., ²H) may afford certain therapeutic advantages resulting from greater metabolic stability (*e.g*., increased in vivo half-life or reduced dosage requirements). Isotopically labelled compounds of Formula (I) can generally be prepared by following procedures analogous to those disclosed in the Schemes and/or in the Examples herein below, by substituting an appropriate isotopically labelled reagent for a non-isotopically labelled reagent.

Synthetic chemical procedures analogous to those disclosed herein for making the Bicyclic Heterocycle Derivatives, by substituting an appropriate isotopically labelled starting material or reagent for a non-isotopically labelled starting material or reagent.

Polymorphic forms of the Bicyclic Heterocycle Derivatives, and of the salts, solvates, hydrates, esters and prodrugs of the Bicyclic Heterocycle Derivatives, are intended to be included in the present invention.

The following abbreviations are used below and have the following meanings: Ac ie acetyl, AcOH is acetic acid, BINAP is rac-2,2'-bis(diphenylphosphino)-1,1'-binaphthyl, Boc or BOC is *tert*-butoxycarbonyl, BSA is *N,O*-(bistrimethylsilyl)acetamide, Bu is butyl, n-BuLi is n-butyllithium, t-butyl or tert-butyl is tertiary butyl, catacXium C is *trans*-bis(acetate)bis[o-(di-o-tolylphosphino)benzyl]dipalladium(II), DAST is diethylaminosulfur trichloride, dba is dibenzylidene acetone, DCE is dichloroethane, DCM is dichloromethane is dichloromethane, DIAD is diisopropylazodicarboxylate, DIEA is diisopropylethylamine, DIPEA is diisoproylethylamine, DMAP is 4-dimethylaminopyridine, DMEM is Dulbecco's modified eagle medium, DMF is *N,N*-dimethylformamide, DMSO is dimethylsulfoxide, dppf is 1,1'-bis(diphenylphosphino)ferrocene, EDCI is 1-(3-dimethylaminopropyl)-3-ethylcarbodiimide, EDTA is ethylenediaminetetraacetic acid, Et is ethyl, EtOAc is ethyl acetate, EtOH is ethanol, Et₃N is triethylamine, EtNH₂ is ethylamine, HOBt is 1-hydroxybenzotriazole, LAH is lithium aluminum hydride, LCMS is liquid chromatography mass spectrometry, LDA is lithium diisopropylamide, mCPBA is meta-chloroperoxybenzoic acid, Me is methyl, MeOH is methanol, MS is mass spectroscopy, NaOEt is sodium ethoxide, NaOtBu is sodium t-butoxide, NMM is n-methylmorpholine, NMR is nuclear magnetic resonance, Ph is phenyl, PhMe is toluene, TBAF is tetra-*n*-butyl-ammonium fluoride, TBS is *tert*-butyldimethylsilyl, TFA is trifluoroacetic acid, THF is tetrahydrofuran, TLC is thin-layer chromatography, TMS is trimethylsilyl, TMSOTf is trimethylsilyl trifluoromethanesulfonate and X-Phos is 2-dicyclohexylphosphino-2',4',6'-triisopropylbiphenyl.

### The Bicyclic Heterocycle Derivatives of Formula (I)

The present invention provides Bicyclic Heterocycle Derivatives of Formula (I): 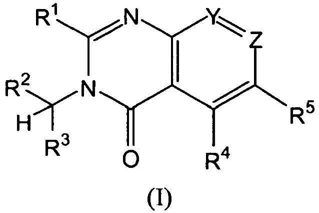 and pharmaceutically acceptable salts, solvates, esters, prodrugs and stereoisomers thereof, wherein R¹, R², R³, R⁴, R⁵, Y and Z are defined above for the compounds of formula (I).

In one embodiment, Y is C(R⁷).

In another embodiment, Y is CH.

In another embodiment, Y is N.

In one embodiment, Z is C(R⁶).

In another embodiment, Z is CH.

In another embodiment, Z is N.

In one embodiment, Y is C(R⁷) and Z is C(R⁶).

In another embodiment, Y and Z are each CH.

In another embodiment, one of Y and Z is N and the other is not N. In still another embodiment, Y is C(R⁷) and Z is N.

In another embodiment, Y is N and Z is C(R⁶).

In one embodiment, Y is CH and Z is N.

In another embodiment, Y is N and Z is CH.

In one embodiment, R¹ is -H.

In one embodiment, R¹ is other than -H.

In another embodiment, R¹ is alkyl.

In another embodiment, R¹ is ―N(R¹⁰)₂.

In still another embodiment, R¹ is ―OR⁹.

In yet another embodiment, R¹ is ―SR⁹.

In one embodiment, R¹ is -NH₂.

In another embodiment, R¹ is -NH-alkyl.

In another embodiment, R¹ is -N(alkyl)₂.

In still another embodiment, R¹ is -O-alkyl.

In a further embodiment, R¹ is -S-alkyl.

In another embodiment, R¹ is -(alkylene)-aryl.

In one embodiment, R¹ is haloalkyl.

In another embodiment, R¹ is fluoromethyl.

In another embodiment, R¹ is difluoromethyl.

In still another embodiment, R¹ is trifluoromethyl.

In one embodiment, R¹ is methyl.

In another embodiment, R¹ is ethyl.

In another embodiment, R¹ is n-propyl.

In still another embodiment, R¹ isopropyl.

In a further embodiment, R¹ is benzyl.

In another embodiment, R² is aryl.

In another embodiment, R² is heteroaryl.

In another embodiment, R² is benzyl.

In yet another embodiment, R² is cycloalkyl.

In another embodiment, R² is cyclopropyl, cyclobutyl, cyclopentyl or cyclohexyl.

In one embodiment, R² is biaryl.

In a further embodiment, R² is -alkylene-aryl.

In another embodiment, R² is -alkylene-O-alkyl.

In another embodiment, R² is phenyl.

In another embodiment, R² is pyridyl.

In still another embodiment, R² is 2-pyridyl.

In another embodiment, R² is trifluoromethyl.

In yet another embodiment, R² is cyclopropyl.

In still another embodiment, R² is cyclobutyl.

In another embodiment, R² is cyclopentyl.

In one embodiment, R² is cyclohexyl.

In another embodiment, R² is -CH₂-O-CH₃.

In yet another embodiment, R² is ―CH₂-O-CH₂CH₃.

In one embodiment, R³ is aryl.

In another embodiment, R³ is heteroaryl.

In another embodiment, R³ is benzyl.

In yet another embodiment, R³ is cycloalkyl.

In another embodiment, R³ is cyclopropyl, cyclobutyl, cyclopentyl or cyclohexyl.

In one embodiment, R³ is biaryl.

In a further embodiment, R³ is -alkylene-aryl.

In another embodiment, R³ is ―alkylene-O-alkyl.

In another embodiment, R³ is phenyl.

In another embodiment, R³ is pyridyl.

In still another embodiment, R³ is 2-pyridyl.

In another embodiment, R³ is trifluoromethyl.

In yet another embodiment, R³ is cyclopropyl.

In still another embodiment, R³ is cyclobutyl.

In another embodiment, R³ is cyclopentyl.

In one embodiment, R³ is cyclohexyl.

In another embodiment, R³ is ―CH₂-O-CH₃.

In yet another embodiment, R³ is -CH₂-O-CH₂CH₃.

In one embodiment, R² and R³ and the carbon atom to which they are both attached, combine to form:

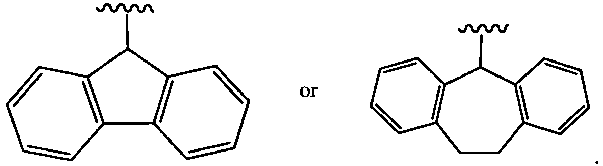

In another embodiment, R² and R³ are each independently selected from haloalkyl, - (alkylene)ₙ-aryl, -(alkylene)ₙ-heteroaryl, -(alkylene)ₙ-cycloalkyl and -alkylene-O-alkyl.

In another embodiment, R² and R³ are each aryl.

In yet another embodiment, R² and R³ are each heteroaryl.

In another embodiment, R² and R³ are each phenyl.

In another embodiment, R² and R³ are each cycloalkyl.

In another embodiment, R² is aryl and R³ is heteroaryl.

In still another embodiment, R² is phenyl and R³ is heteroaryl.

In yet another embodiment, R² is phenyl and R³ is pyridyl.

In a further embodiment, R² is phenyl and R³ is 2-pyridyl.

In still another embodiment, R² and R³ are each benzyl.

In another embodiment, R² is aryl and R³ is cycloalkyl.

In still another embodiment, R² is phenyl and R³ is cycloalkyl.

In a further embodiment, R² is phenyl and R³ is cyclopentyl.

In another embodiment, R² is phenyl and R³ is cyclobutyl.

In yet another embodiment, R² is phenyl and R³ is pyrimidinyl.

In still another embodiment, R² is phenyl and R³ is thienyl.

In one embodiment, R² is phenyl and R³ is benzyl.

In another embodiment, R² is phenyl and R³ is cyclohexyl.

In another embodiment, R² is phenyl and R³ is heterocycloalkyl.

In still another embodiment, R² is phenyl and R³ is -alkylene-O-alkyl.

In another embodiment, R² is phenyl and R³ is alkylene-cyclolalkyl.

In a further embodiment, R² is phenyl and R³ is -CH₂-O-CH₃.

In another embodiment, R² is phenyl and R³ is -CH₂-O-CH₂CH₃.

In another embodiment, R² is benzyl and R³ is -CH₂-O-CH₃.

In another embodiment, R² is benzyl and R³ is -CH₂-O-CH₂CH₃.

In one embodiment, the group -CH(R²)(R³) is:

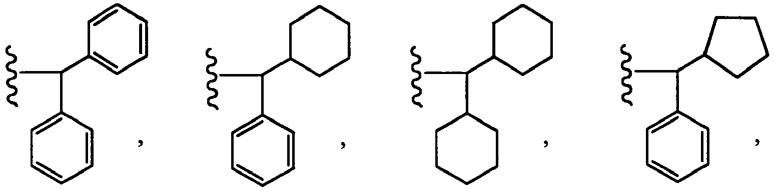

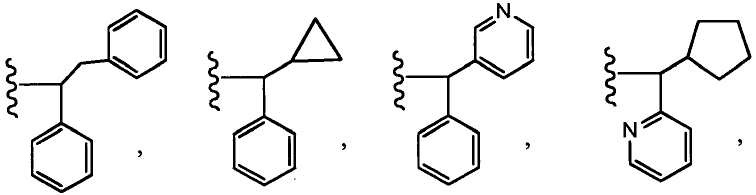

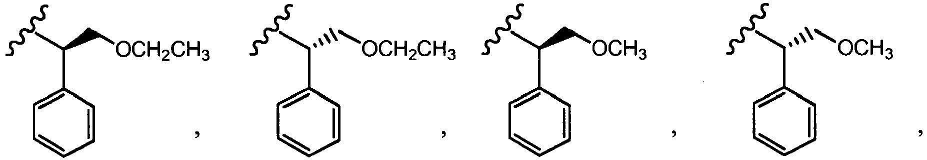

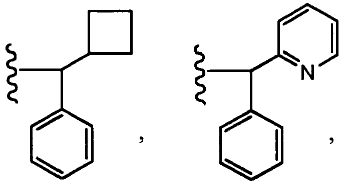

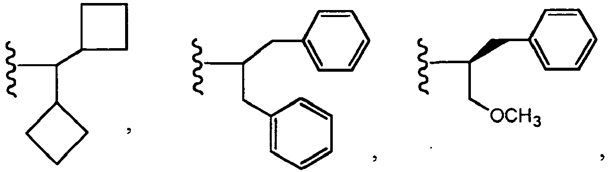

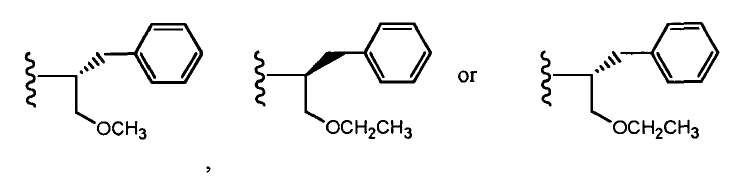

In one embodiment, R⁴ is H.

In one embodiment, R⁴ is -N(R⁸)_{2.}

In another embodiment, R⁴ is -NH₂.

In another embodiment, R⁴ is -NH-alkyl.

In yet another embodiment, R⁴ is ―N(akyl)₂.

In a further embodiment, R⁴ is ―N(CH₃)₂.

In another embodiment, R⁴ is ―N(CH₂CH₃)₂.

In one embodiment, R⁴ is ―NHCH₃.

In still another embodiment, R⁴ is -NH-aryl.

In another embodiment, R⁴ is ―NH-C(O)OR¹¹.

In another embodiment, R⁴ is ―NH-C(O)R¹¹.

In another embodiment, R⁴ is ―N(alkyl)-C(O)OR¹¹.

In another embodiment, R⁴ is ―N(alkyl)-C(O)R¹¹.

In another embodiment, R⁴ is -NH-C(O)alkyl.

In still another embodiment, R⁴ is -NH-C(O)-aryl.

In another embodiment, R⁴ is -NH-C(O)-phenyl.

In yet another embodiment, R⁴ is -NHC(O)O-alkyl.

In another embodiment, R⁴ is -NHC(O)O-heteroaryl.

In another embodiment, R⁴ is -NHC(O)O-aryl.

In one embodiment, R⁴ is -NHC(O)O-phenyl.

In another embodiment, R⁴ is -NHC(O)O-t-butyl.

In another embodiment, R⁴ is -NH-phenyl.

In another embodiment, R⁴ is -N(alkyl)(aryl).

In one embodiment, R⁴ is -N(heteroaryl)(alkyl).

In another embodiment, R⁴ is -N(pyridyl)(CH₃).

In another embodiment, R⁴ is -N(phenyl)(alkyl).

In a further embodiment, R⁴ is -N(phenyl)(CH₃).

In another embodiment, R⁴ is -N(alkylene-aryl)(alkyl).

In another embodiment, R⁴ is -N(benzyl)(-SO₂-alkyl).

In one embodiment, R⁴ is -NH-alkylene-aryl.

In another embodiment, R⁴ is -NH-benzyl.

In one embodiment, R⁴ is ―OR¹⁴.

In another embodiment, R⁴ is -O-alkyl.

In another embodiment, R⁴ is -OCH₃.

In still another embodiment, R⁴ is -O-ethyl, -O-isopropyl or -O-t-butyl.

In another embodiment, R⁴ is -O-aryl.

In yet another embodiment, R⁴ is -O-phenyl.

In one embodiment, R⁴ is -O-heteroaryl.

In another embodiment, R⁴ is -O-pyridyl.

In another embodiment, R⁴ is -O-alkylene-C(O)OR¹¹.

In still another embodiment, R⁴ is -O-alkylene-C(O)O-alkyl.

In another embodiment, R⁴ is -O-alkylene-C(O)O-t-butyl.

In yet another embodiment, R⁴ is -O-alkylene-heterocycloalkyl.

In another embodiment, R⁴ is -O-haloalkyl.

In a further embodiment, R⁴ is -OCF₃.

In one embodiment, R⁴ is -C(NH₂)(=N-OH).

In another embodiment, R⁴ is heterocycloalkyl.

In another embodiment, R⁴ is morpholinyl.

In still another embodiment, R⁴ is piperidinyl.

In another embodiment, R⁴ is indolyl.

In yet another embodiment, R⁴ is alkyl.

In another embodiment, R⁴ is aryl.

In a further embodiment, R⁴ is phenyl.

In another embodiment, R⁴ is -CN.

In another embodiment, R⁴ is -NO₂.

In still another embodiment, R⁴ is -halo.

In another embodiment, R⁴ is -Br.

In one embodiment, R⁴ is -C(O)R¹¹.

In another embodiment, R⁴ is -C(O)alkyl.

In another embodiment, R⁴ is -C(O)aryl.

In one embodiment, R⁴ is -C(O)OR¹¹.

In another embodiment, R⁴ is -C(O)Oalkyl.

In another embodiment, R⁴ is -C(O)Oaryl.

In yet another embodiment, R⁴ is -C(O)N(R⁸)₂.

In another embodiment, R⁴ is -C(O)NH-alkyl.

In still another embodiment, R⁴ is -C(O)NH-aryl.

In another embodiment, R⁴ is -C(O)NH-phenyl.

In one embodiment, R⁵ is H.

In one embodiment, R⁵ is -N(R⁸)₂.

In another embodiment, R⁵ is -NH₂.

In another embodiment, R⁵ is -NH-alkyl.

In yet another embodiment, R⁵ is ―N(alkyl)₂.

In a further embodiment, R⁵ is ―N(CH₃)₂.

In another embodiment, R⁵ is ―N(CH₂CH₃)₂.

In one embodiment, R⁵ is -NHCH₃.

In still another embodiment, R⁵ is -NH-aryl.

In another embodiment, R⁵ is ―NH-C(O)OR¹¹.

In another embodiment, R⁵ is ―NH-C(O)R¹¹.

In another embodiment, R⁵ is ―N(alkyl)-C(O)OR¹¹.

In another embodiment, R⁵ is ―N(alkyl)-C(O)R¹¹.

In another embodiment, R⁵ is -NH-C(O)alkyl.

In still another embodiment, R⁵ is -NH-C(O)-aryl.

In another embodiment, R⁵ is -NH-C(O)-phenyl.

In yet another embodiment, R⁵ is -NHC(O)O-alkyl.

In another embodiment, R⁵ is ―NHC(O)O-heteroaryl.

In another embodiment, R⁵ is ―NHC(O)O-aryl.

In one embodiment, R⁵ is -NHC(O)O-phenyl.

In another embodiment, R⁵ is -NHC(O)O-t-butyl.

In another embodiment, R⁵ is -NH-phenyl.

In another embodiment, R⁵ is -N(alkyl)(aryl).

In one embodiment, R⁵ is -N(heteroaryl)(alkyl).

In another embodiment, R⁵ is -N(pyridyl)(CH₃).

In another embodiment, R⁵ is -N(phenyl)(alkyl).

In a further embodiment, R⁵ is -N(phenyl)(CH₃).

In another embodiment, R⁵ is -N(alkylene-aryl)(alkyl).

In another embodiment, R⁵ is -N(benzyl)(-SO₂-alkyl).

In one embodiment, R⁵ is -NH-alkylene-aryl.

In another embodiment, R⁵ is -NH-benzyl.

In another embodiment, R⁵ is ―NHC(O)OR¹¹, -NH-phenyl, or -N(alkyl)(phenyl), wherein the phenyl moiety of an -NH-phenyl or N(alkyl)(phenyl) group can be unsubstituted or substituted as set forth above for the compounds of formula (I).

In one embodiment, R⁵ is ―OR¹⁴.

In another embodiment, R⁵ is -O-alkyl.

In another embodiment, R⁵ is -OCH₃.

In still another embodiment, R⁵ is ―O-ethyl, -O-isopropyl or -O-t-butyl.

In another embodiment, R⁵ is -O-aryl.

In yet another embodiment, R⁵ is -O-phenyl.

In one embodiment, R⁵ is -O-heteroaryl.

In another embodiment, R⁵ is -O-pyridyl.

In another embodiment, R⁵ is -O-alkylene-C(O)OR¹¹.

In still another embodiment, R⁵ is -O-alkylene-C(O)O-alkyl.

In another embodiment, R⁵ is -O-akylene-C(O)O-t-butyl.

In yet another embodiment, R⁵ is -O-akylene-heterocycloalkyl.

In another embodiment, R⁵ is -O-haloalkyl.

In a further embodiment, R⁵ is -OCF₃.

In one embodiment, R⁵ is -C(NH₂)(=N-OH).

In another embodiment, R⁵ is heterocycloalkyl.

In another embodiment, R⁵ is morpholinyl.

In still another embodiment, R⁵ is piperidinyl.

In another embodiment, R⁵ is indolyl.

In yet another embodiment, R⁵ is alkyl.

In another embodiment, R⁵ is aryl.

In a further embodiment, R⁵ is phenyl.

In another embodiment, R⁵ is -CN.

In another embodiment, R⁵ is -NO₂.

In still another embodiment, R⁵ is -halo.

In another embodiment, R⁵ is -Br.

In one embodiment, R⁵ is -C(O)R¹¹.

In another embodiment, R⁵ is -C(O)alkyl.

In another embodiment, R⁵ is -C(O)aryl.

In one embodiment, R⁵ is -C(O)OR¹¹.

In another embodiment, R⁵ is -C(O)Oalkyl.

In another embodiment, R⁵ is -C(O)Oaryl.

In yet another embodiment, R⁵ is -C(O)N(R⁸)₂.

In another embodiment, R⁵ is -C(O)NH-alkyl.

In still another embodiment, R⁵ is -C(O)NH-aryl.

In another embodiment, R⁵ is -C(O)NH-phenyl.

In one embodiment, R⁶ is H.

In one embodiment, R⁶ is -N(R⁸)₂.

In another embodiment, R⁶ is -NH₂.

In another embodiment, R⁶ is -NH-alkyl.

In yet another embodiment, R⁶ is ―N(alkyl)₂.

In a further embodiment, R⁶ is ―N(CH₃)₂.

In another embodiment, R⁶ is ―N(CH₂CH₃)₂.

In one embodiment, R⁶ is -NHCH₃.

In still another embodiment, R⁶ is -NH-aryl.

In another embodiment, R⁶ is ―NH-C(O)OR¹¹.

In another embodiment, R⁶ is ―NH-C(O)R¹¹.

In another embodiment, R⁶ is ―N(alkyl)-C(O)OR¹¹.

In another embodiment, R⁶ is ―N(alkyl)-C(O)R¹¹.

In another embodiment, R⁶ is -NH-C(O)alkyl.

In still another embodiment, R⁶ is -NH-C(O)-aryl.

In another embodiment, R⁶ is -NH-C(O)-phenyl.

In yet another embodiment, R⁶ is -NHC(O)O-alkyl.

In another embodiment, R⁶ is -NHC(O)O-heteroaryl.

In another embodiment, R⁶ is NHC(O)O-aryl.

In one embodiment, R⁶ is -NHC(O)O-phenyl.

In another embodiment, R⁶ is -NHC(O)O-t-butyl.

In another embodiment, R⁶ is -NH-phenyl.

In another embodiment, R⁶ is -N(alkyl)(aryl).

In one embodiment, R⁶ is -N(heteroaryl)(alkyl).

In another embodiment, R⁶ is -N(pyridyl)(CH₃).

In another embodiment, R⁶ is -N(phenyl)(alkyl).

In a further embodiment, R⁶ is -N(phenyl)(CH₃).

In another embodiment, R⁶ is -N(alkylene-aryl)(alkyl).

In another embodiment, R⁶ is -N(benzyl)(-SO₂-akyl).

In one embodiment, R⁶ is -NH-alkylene-aryl.

In another embodiment, R⁶ is -NH-benzyl.

In another embodiment, R⁶ is -NHC(O)OR¹¹, -NH-phenyl, or -N(alkyl)(phenyl), wherein the phenyl moiety of an -NH-phenyl or -N(alkyl)(phenyl) group can be unsubstituted or substituted as set forth above for the compounds of formula (I).

In one embodiment, R⁶ is -OR¹⁴.

In another embodiment, R⁶ is -O-alkyl.

In another embodiment, R⁶ is -OCH₃.

In still another embodiment, R⁶ is -O-ethyl, -O-isopropyl or -O-t-butyl.

In another embodiment, R⁶ is -O-aryl.

In yet another embodiment, R⁶ is -O-phenyl.

In one embodiment, R⁶ is -O-heteroaryl.

In another embodiment, R⁶ is -O-pyridyl.

In another embodiment, R⁶ is -O-alkylene-C(O)OR¹¹.

In still another embodiment, R⁶ is -O-alkylene-C(O)O-alkyl.

In another embodiment, R⁶ is -O-alkylene-C(O)O-t-butyl.

In yet another embodiment, R⁶ is -O-alkylene-heterocycloalkyl.

In another embodiment, R⁶ is -O-haloalkyl.

In a further embodiment, R⁶ is -OCF₃.

In one embodiment, R⁶ is -C(NH₂)(=N-OH).

In another embodiment, R⁶ is heterocycloalkyl.

In another embodiment, R⁶ is morpholinyl.

In still another embodiment, R⁶ is piperidinyl.

In another embodiment, R⁶ is indolyl.

In yet another embodiment, R⁶ is alkyl.

In another embodiment, R⁶ is aryl.

In a further embodiment, R⁶ is phenyl.

In another embodiment, is -CN.

In another embodiment, R⁶ is -NO₂.

In still another embodiment, R⁶ is -halo.

In another embodiment, R⁶ is -Br.

In one embodiment, R⁶ is -C(O)R¹¹.

In another embodiment, R⁶ is -C(O)alkyl.

In another embodiment, R⁶ is -C(O)aryl.

In one embodiment, R⁶ is -C(O)OR¹¹.

In another embodiment, R⁶ is -C(O)Oalkyl.

In another embodiment, R⁶ is -C(O)Oaryl.

In yet another embodiment, R⁶ is -C(O)N(R⁸)_{2.}

In another embodiment, R⁶ is -C(O)NH-alkyl.

In still another embodiment, R⁶ is -C(O)NH-aryl.

In another embodiment, R⁶ is -C(O)NH-phenyl.

In another embodiment, R⁶ is other than H.

In one embodiment, R⁷ is H.

In another embodiment, R⁷ is other than H.

In one embodiment, Y is C(R⁷); Z is C(R⁶); and R⁴ is H.

In another embodiment, Y and Z are each CH and R⁴ is H.

In still another embodiment, Y is C(R⁷); Z is N; and R⁴ is H.

In another embodiment, Y is N; Z is C(R⁶); and R⁴ is H.

In one embodiment, Y is CH; Z is N; and R⁴ is H.

In another embodiment, Y is N; Z is CH; and R⁴ is H.

In one embodiment, Y is C(R⁷); Z is C(R⁶); and R⁵ is H.

In another embodiment, Y and Z are each CH and R⁵ is H.

In still another embodiment, Y is C(R⁷); Z is N; and R⁵ is H.

In another embodiment, Y is N; Z is C(R⁶); and R⁵ is H.

In one embodiment, Y is CH; Z is N; and R⁵ is H.

In another embodiment, Y is N; Z is CH; and R⁵ is H.

In one embodiment, Y is CH; Z is C(R⁶); R⁴ is H; and R⁵ is H.

In one embodiment, R¹ is alkyl, -OH or -NH₂; R² and R³ are each independently selected from aryl, cycloalkyl, heteroaryl or -alkylene-O-alkyl.

In another embodiment, R¹ is alkyl; R² and R³ are each independently selected from aryl, cycloalkyl, heteroaryl or -alkylene-O-alkyl.

In another embodiment, R¹ is methyl; R² and R³ are each independently selected from aryl, cycloalkyl, heteroaryl or -alkylene-O-alkyl.

In one embodiment, R¹ is alkyl, -OH or -NH₂ and the group -CH(R²)(R³) is:

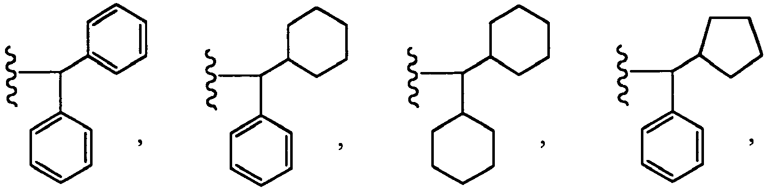

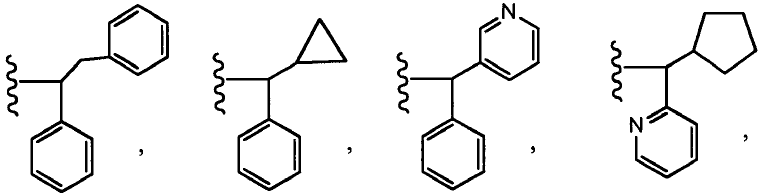

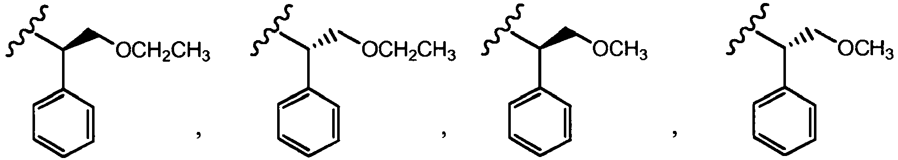

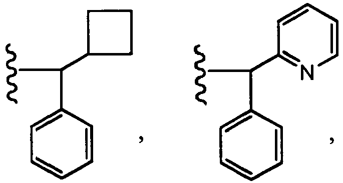

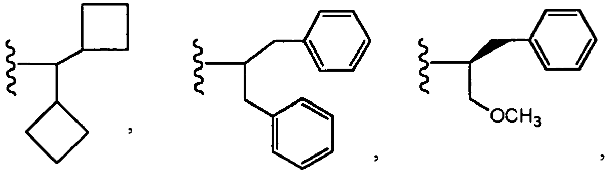

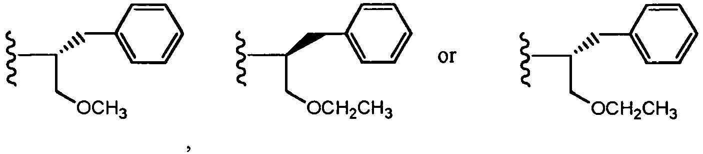

In one embodiment, R¹ is alkyl and the group -CH(R²)(R³) is:

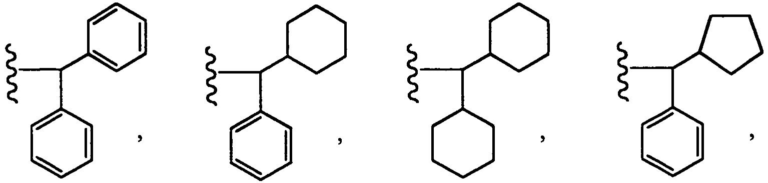

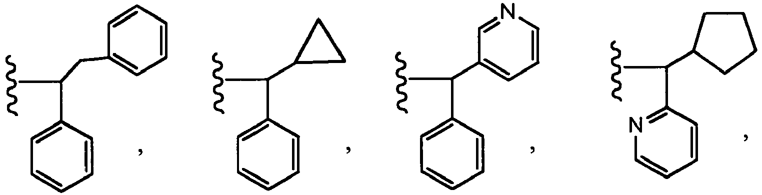

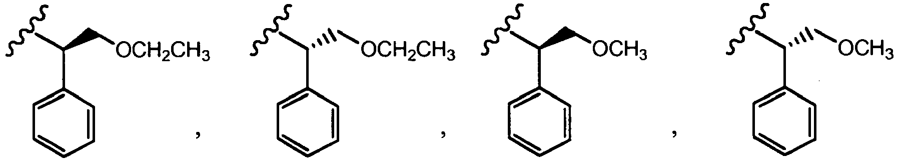

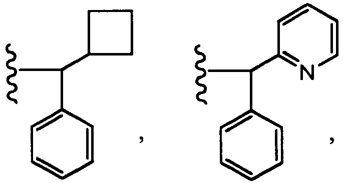

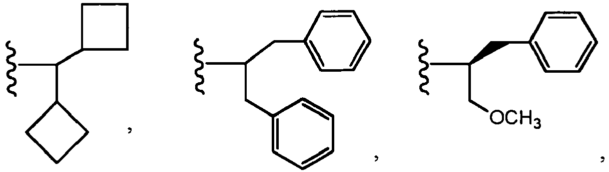

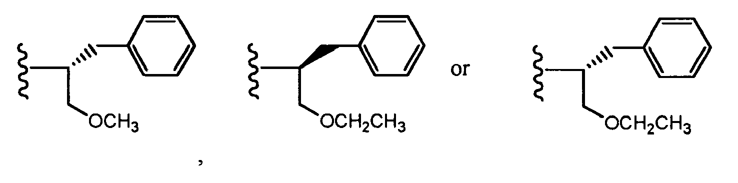

In another embodiment, R¹ is alkyl, R² is aryl and R³ is heteroaryl.

In still another embodiment, R¹ is alkyl, R² is phenyl and R³ is heteroaryl.

In another embodiment, R¹ is alkyl, R² is phenyl and R³ is pyridyl.

In another embodiment, R¹ is alkyl, R² is phenyl and R³ is cycloalkyl.

In yet another embodiment, R¹ is alkyl, R² is phenyl and R³ is -alkylene-O-alkyl.

In another embodiment, R¹ is alkyl, R² is benzyl and R³ is -alkylene-O-alkyl.

In another embodiment, R¹ is alkyl, R² is phenyl and R³ is benzyl.

In another embodiment, R¹ is alkyl, R² is phenyl and R³ is 2-pyridyl.

In a further embodiment, R¹ is alkyl, and R² and R³ are each aryl.

In another embodiment, R¹ is alkyl, and R² and R³ are each heteroaryl.

In yet another embodiment, R¹ is alkyl, and R² and R³ are each phenyl.

In another embodiment, R¹ is benzyl, R² is aryl and R³ is heteroaryl.

In still another embodiment, R¹ is benzyl, R² is phenyl and R³ is heteroaryl.

In yet another embodiment, R¹ is benzyl, R² is phenyl and R³ is pyridyl.

In another embodiment, R¹ is benzyl, R² is phenyl and R³ is 2-pyridyl.

In another embodiment, R¹ is benzyl, R² is phenyl and R³ is cycloalkyl.

In yet another embodiment, R¹ is benzyl, R² is phenyl and R³ is -alkylene-O-alkyl.

In another embodiment, R¹ is benzyl, R² is benzyl and R³ is -alkylene-O-alkyl.

In another embodiment, R¹ is benzyl, R² is phenyl and R³ is benzyl.

In a further embodiment, R¹ is benzyl, and R² and R³ are each aryl.

In another embodiment, R¹ is benzyl, and R² and R³ are each heteroaryl.

In yet another embodiment, R¹ is benzyl, and R² and R³ are each phenyl.

In one embodiment, R¹ is -N(R¹⁰)₂, R² is aryl and R³ is heteroaryl.

In another embodiment, R¹ is -N(R¹⁰)₂, R² is phenyl and R³ is heteroaryl.

In yet another embodiment, R¹ is -N(R¹⁰)₂, R² is phenyl and R³ is pyridyl.

In another embodiment, R¹ is -N(R¹⁰)₂, R² is phenyl and R³ is 2-pyridyl.

In yet another embodiment, R¹ is -N(R¹⁰)₂, R² is phenyl and R³ is 4-fluorophenyl.

In a further embodiment, R¹ is -N(R¹⁰)₂, and R² and R³ are each aryl.

In another embodiment, R¹ is -N(R¹⁰)₂, and R² and R³ are each heteroaryl.

In yet another embodiment, R¹ is -N(R¹⁰)₂, and R² and R³ are each phenyl.

In one embodiment, R¹ is -NH₂, R² is aryl and R³ is heteroaryl.

In another embodiment, R¹ is -NH₂, R² is phenyl and R³ is heteroaryl.

In yet another embodiment, R¹ is -NH₂, R² is phenyl and R³ is pyridyl.

In another embodiment, R¹ is NH₂, R² is phenyl and R³ is 2-pyridyl.

In another embodiment, R¹ is -NH₂, R² is phenyl and R³ is cycloalkyl.

In yet another embodiment, R¹ is -NH₂, R² is phenyl and R³ is -alkylene-O-alkyl.

In another embodiment, R¹ is -NH₂, R² is benzyl and R³ is -alkylene-O-alkyl.

In another embodiment, R¹ is -NH₂, R² is phenyl and R³ is benzyl.

In a further embodiment, R¹ is -NH₂, and R² and R³ are each aryl.

In another embodiment, R¹ is -NH₂, and R² and R³ are each heteroaryl.

In yet another embodiment, R¹ is -NH₂, and R² and R³ are each phenyl.

In one embodiment, R¹ is methyl, R² is aryl and R³ is heteroaryl.

In still another embodiment, R¹ is methyl, R² is phenyl and R³ is heteroaryl.

In yet another embodiment, R¹ is methyl, R² is phenyl and R³ is pyridyl.

In another embodiment, R¹ is methyl, R² is phenyl and R³ is 2-pyridyl.

In another embodiment, R¹ is methyl, R² is phenyl and R³ is cycloalkyl.

In yet another embodiment, R¹ is methyl, R² is phenyl and R³ is -alkylene-O-alkyl.

In another embodiment, R' is methyl, R² is benzyl and R³ is -alkylene-O-alkyl.

In another embodiment, R¹ is methyl, R² is phenyl and R³ is benzyl.

In a further embodiment, R¹ is methyl and R² and R³ are each aryl.

In another embodiment, R¹ is methyl and R² and R³ are each heteroaryl.

In yet another embodiment, R¹ is alkyl and R² and R³ are each phenyl.

In another embodiment, R¹ is methyl and R² and R³ are each phenyl.

In one embodiment, R¹ is -OH, R² is aryl and R³ is heteroaryl.

In still another embodiment, R¹ is -OH, R² is phenyl and R³ is heteroaryl.

In yet another embodiment, R¹ is -OH, R² is phenyl and R³ is pyridyl.

In another embodiment, R¹ is -OH, R² is phenyl and R³ is 2-pyridyl.

In another embodiment, R¹ is -OH, R² is phenyl and R³ is cycloalkyl.

In yet another embodiment, R¹ is -OH, R² is phenyl and R³ is -alkylene-O-alkyl.

In another embodiment, R¹ is -OH, R² is benzyl and R³ is -alkylene-O-alkyl.

In another embodiment, R¹ is -OH, R² is phenyl and R³ is benzyl.

In a further embodiment, R¹ is -OH and R² and R³ are each aryl.

In another embodiment, R¹ is -OH and R² and R³ are each heteroaryl.

In another embodiment, R¹ is -OH and R² and R³ are each phenyl.

In one embodiment, R¹ is alkyl, -OH or NH₂; R² and R³ are each independently selected from aryl, cycloalkyl, heteroaryl or -alkylene-O-alkyl; Y is CH; and Z is N or CH.

In another embodiment, R¹ is alkyl, -OH or -NH₂; R² and R³ are each independently selected from aryl, cycloalkyl, heteroaryl or -alkylene-O-alkyl; R⁴ is H; Y is CH; and Z is N or CH.

In another embodiment, R¹ is alkyl, -OH or NH₂; R² and R³ are each independently selected from aryl, cycloalkyl, heteroaryl or -alkylene-O-alkyl; R⁴ is H; R⁵ is H; Y is CH; and Z is C(R⁶).

In one embodiment, for the compounds of formula (I), R¹, R², R³, R⁴, R⁵, Y and Z are selected independently of each other.

In another embodiment, a compound of formula (I) is in purified form.

In one embodiment, the compounds of formula (I) have the formula (Ia): 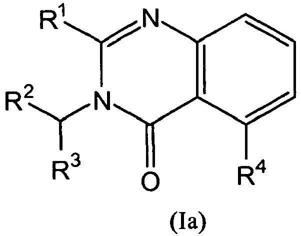 wherein R¹, R², R³ and R⁴ are defined above for the compounds of formula (I).

In one embodiment, R¹ is alkyl, -alkylene-aryl, -OH or -NH₂.

In another embodiment, R¹ is alkyl.

In another embodiment, R¹ is -alkylene-aryl.

In still another embodiment, R¹ is -OH.

In another embodiment, R¹ is -NH₂.

In yet another embodiment, R¹ is methyl.

In a further embodiment, R¹ is ethyl.

In one embodiment, R² and R³ are each independently selected from haloalkyl, - (alkylene)ₙ-aryl, -(alkylene)ₙ-heteroaryl, -(alkylene)ₙ-cycloalkyl and -alkylene-O-alkyl.

In another embodiment, R² and R³ are each aryl.

In yet another embodiment, R² and R³ are each heteroaryl.

In another embodiment, R² and R³ are each cycloalkyl.

In another embodiment, R² is aryl and R³ is heteroaryl.

In one embodiment, the group -CH(R²)(R³) is:

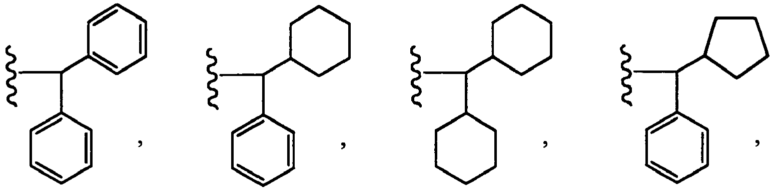

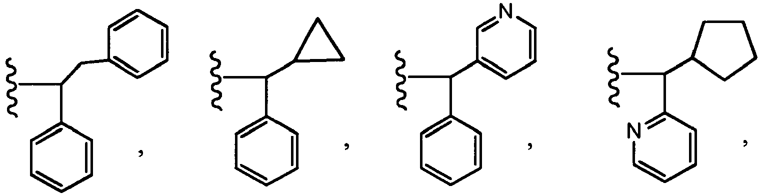

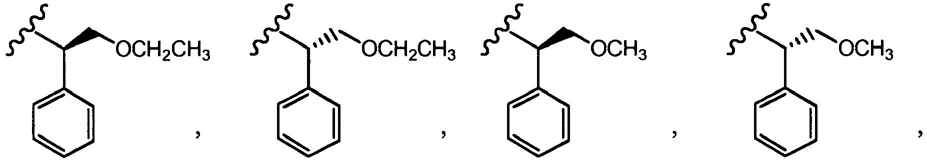

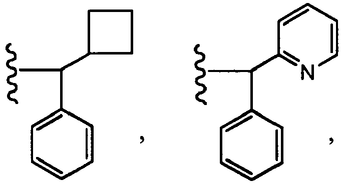

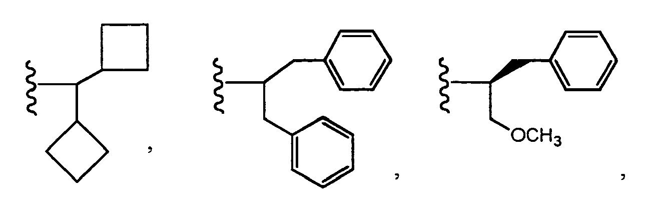

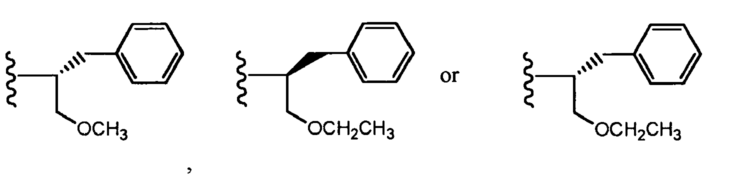

In another embodiment, R² and R³ are each phenyl.

In another embodiment, R² and R³ and the carbon atom to which they are both attached, combine to form:

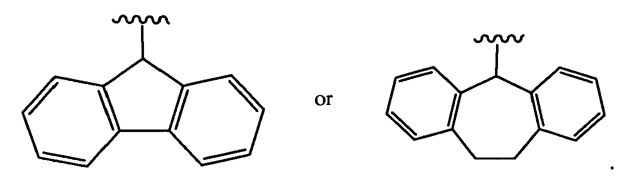

In one embodiment, R¹ is alkyl, -OH or -NH₂; R² and R³ are each independently selected from aryl, cycloalkyl, heteroaryl or -alkylene-O-alkyl.

In another embodiment, R¹ is alkyl; R² and R³ are each independently selected from aryl, cycloalkyl, heteroaryl or -alkylene-O-alkyl.

In another embodiment, R¹ is methyl; R² and R³ are each independently selected from aryl, cycloalkyl, heteroaryl or -alkylene-O-alkyl.

In one embodiment, R¹ is alkyl, -OH or -NH₂ and the group -CH(R²)(R³) is:

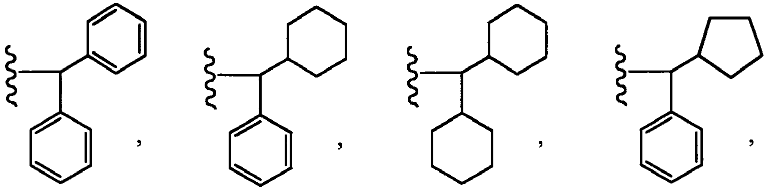

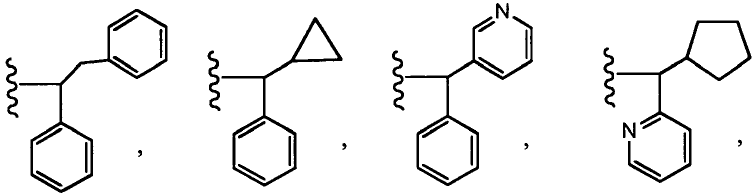

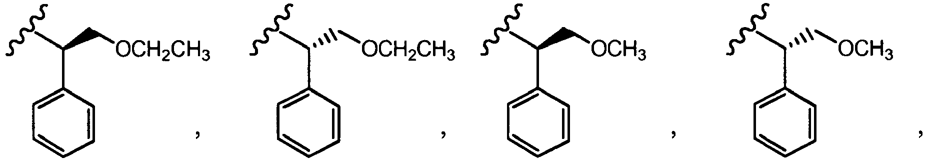

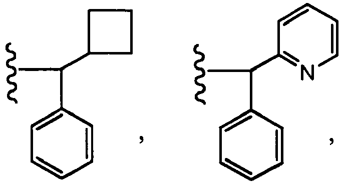

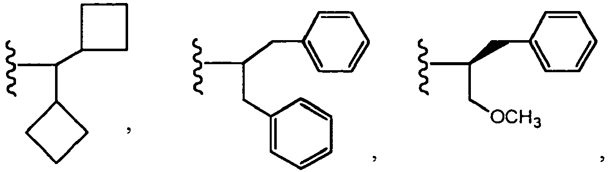

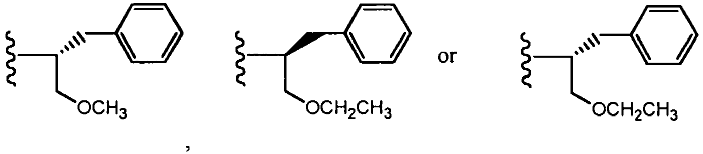

In one embodiment, R¹ is alkyl and the group -CH(R²)(R³) is:

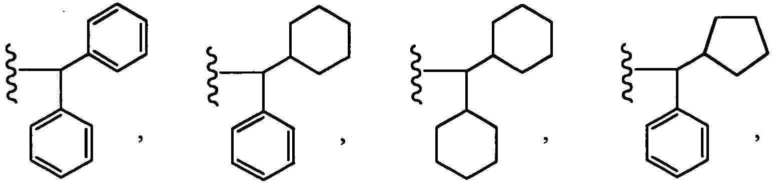

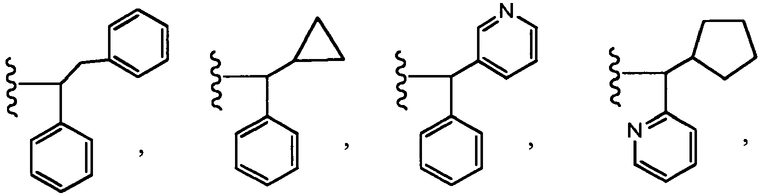

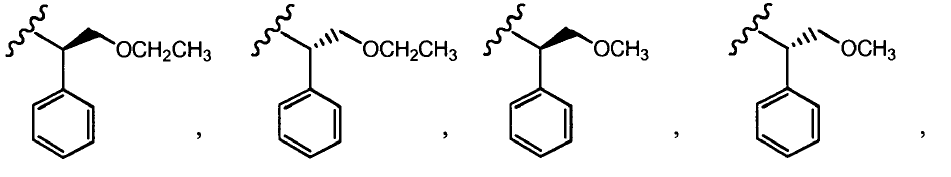

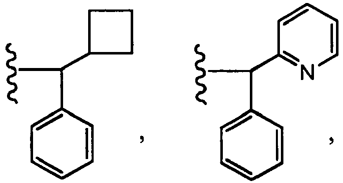

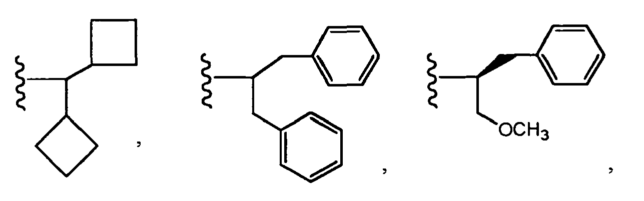

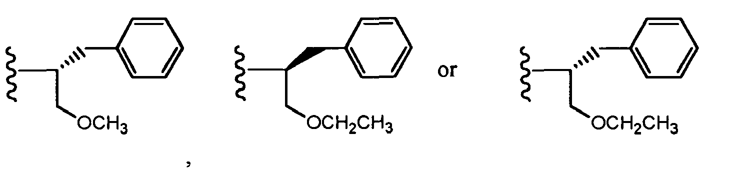

In one embodiment, R⁴ is H.

In one embodiment, R⁴ is -N(R⁸)₂.

In another embodiment, R⁴ is -NH₂.

In another embodiment, R⁴ is -NH-alkyl.

In yet another embodiment, R⁴ is -N(alkyl)₂.

In a further embodiment, R⁴ is -N(CH₃)₂.

In another embodiment, R⁴ is -N(CH₂CH₃)₂.

In one embodiment, R⁴ is -NHCH₃.

In still another embodiment, R⁴ is -NH-aryl.

In another embodiment, R⁴ is -NH-C(O)OR¹¹.

In another embodiment, R⁴ is -NH-C(O)R¹¹.

In another embodiment, R⁴ is -N(alkyl)-C(O)OR¹¹.

In another embodiment, R⁴ is -N(alkyl)-C(O)R¹¹.

In another embodiment, R⁴ is -NH-C(O)alkyl.

In still another embodiment, R⁴ is -NH-C(O)-aryl.

In another embodiment, R⁴ is -NH-C(O)-phenyl.

In yet another embodiment, R⁴ is -NHC(O)O-alkyl.

In another embodiment, R⁴ is NHC(O)O-heteroaryl.

In another embodiment, R⁴ is -NHC(O)O-aryl.

In one embodiment, R⁴ is -NHC(O)O-phenyl.

In another embodiment, R⁴ is -NHC(O)O-t-butyl.

In another embodiment, R⁴ is -NH-phenyl.

In another embodiment, R⁴ is -N(alkyl)(aryl).

In one embodiment, R⁴ is -N(heteroaryl)(alkyl).

In another embodiment, R⁴ is -N(pyridyl)(CH₃).

In another embodiment, R⁴ is -N(phenyl)(alkyl).

In a further embodiment, R⁴ is -N(phenyl)(CH₃).

In another embodiment, R⁴ is -N(alkylene-aryl)(alkyl).

In another embodiment, R⁴ is -N(benzyl)(-SO₂-alkyl).

In one embodiment, R⁴ is -NH-alkylene-aryl.

In another embodiment, R⁴ is -NH-benzyl.

In one embodiment, R⁴ is -OR¹⁴.

In another embodiment, R⁴ is -O-alkyl.

In another embodiment, R⁴ is -OCH₃.

In still another embodiment, R⁴ is -O-ethyl, -O-isopropyl or -O-t-butyl.

In another embodiment, R⁴ is -O-aryl.

In yet another embodiment, R⁴ is -O-phenyl.

In one embodiment, R⁴ is -O-heteroaryl.

In another embodiment, R⁴ is -O-pyridyl.

In another embodiment, R⁴ is -O-alkylene-C(O)OR¹¹.

In still another embodiment, R⁴ is -O-alkylene-C(O)O-alkyl.

In another embodiment, R⁴ is -O-alkylene-C(O)O-t-butyl.

In yet another embodiment, R⁴ is -O-alkylene-heterocycloalkyl.

In another embodiment, R⁴ is -O-haloalkyl.

In a further embodiment, R⁴ is -OCF₃.

In one embodiment, R⁴ is -C(NH₂)(=N-OH).

In another embodiment, R⁴ is heterocycloalkyl.

In another embodiment, R⁴ is morpholinyl.

In still another embodiment, R⁴ is piperidinyl.

In another embodiment, R⁴ is indolyl.

In yet another embodiment, R⁴ is alkyl.

In another embodiment, R⁴ is aryl.

In a further embodiment, R⁴ is phenyl.

In another embodiment, R⁴ is -CN.

In another embodiment, R⁴ is -NO₂.

In still another embodiment, R⁴ is halo.

In another embodiment, R⁴ is -Br.

In one embodiment, R⁴ is -C(O)R¹¹.

In another embodiment, R⁴ is -C(O)alkyl.

In another embodiment, R⁴ is -C(O)aryl.

In one embodiment, R⁴ is -C(O)OR¹¹.

In another embodiment, R⁴ is -C(O)Oalkyl.

In another embodiment, R⁴ is -C(O)Oaryl.

In yet another embodiment, R⁴ is -C(O)N(R⁸)₂.

In another embodiment, R⁴ is -C(O)NH-alkyl.

In still another embodiment, R⁴ is -C(O)NH-aryl.

In another embodiment, R⁴ is -C(O)NH-phenyl.

In one embodiment, for the compounds of formula (Ia), R¹, R², R³ and R⁴ are selected independently of each other.

In another embodiment, a compound of formula (Ia) is in purified form.

In one embodiment, the compounds of formula (I) have the formula (Ib): 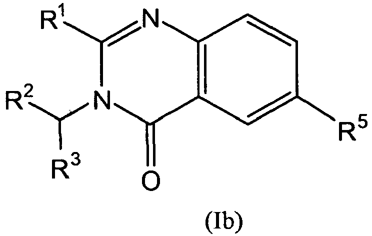 wherein R¹, R², R³ and R⁵ are defined above for the compounds of formula (I).

In one embodiment, R¹ is alkyl, -alkylene-aryl, -OH or -NH₂.

In another embodiment, R¹ is alkyl.

In another embodiment, R¹ is -alkylene-aryl.

In still another embodiment, R¹ is -OH.

In another embodiment, R' is -NH₂.

In yet another embodiment, R¹ is methyl.

In a further embodiment, R¹ is ethyl.

In one embodiment, R² and R³ are each independently selected from haloalkyl, - (alkylene)ₙ-aryl, -(alkylene)ₙ-heteroaryl, -(alkylene)ₙ-cycloalkyl and -alkylene-O-alkyl.

In another embodiment, R² and R³ are each aryl.

In yet another embodiment, R² and R³ are each heteroaryl.

In another embodiment, R² and R³ are each cycloalkyl.

In another embodiment, R² is aryl and R³ is heteroaryl.

In one embodiment, the group -CH(R²)(R³) is:

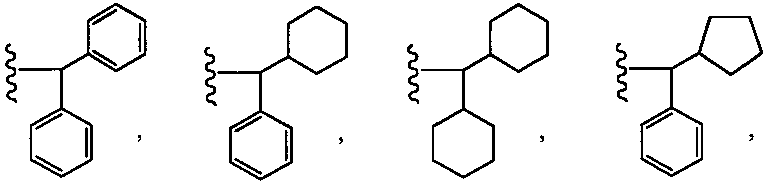

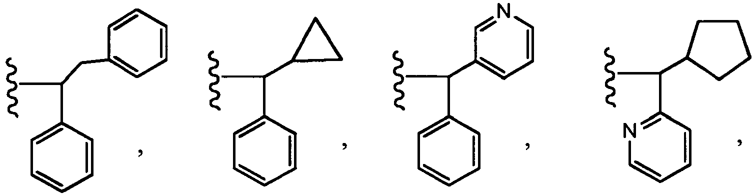

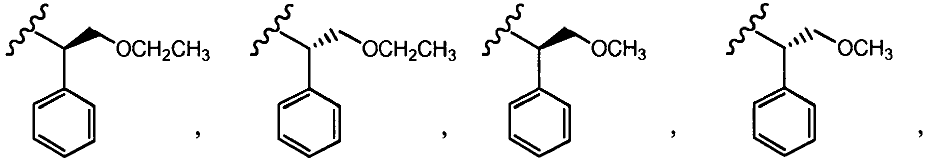

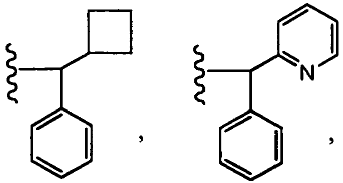

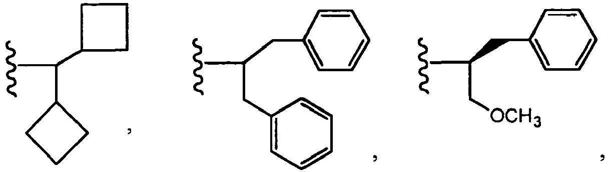

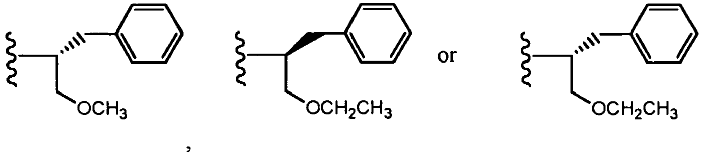

In another embodiment, R² and R³ are each phenyl.

In another embodiment, R² and R³ and the carbon atom to which they are both attached, combine to form:

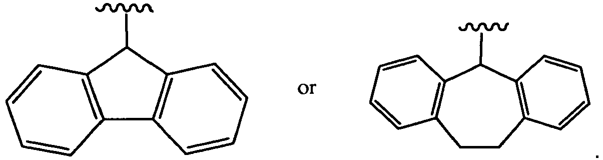

In one embodiment, R¹ is alkyl, -OH or -NH₂; R² and R³ are each independently selected from aryl, cycloalkyl, heteroaryl or -alkylene-O-alkyl.

In another embodiment, R¹ is alkyl; R² and R³ are each independently selected from aryl, cycloalkyl, heteroaryl or -alkylene-O-alkyl.

In another embodiment, R¹ is methyl; R² and R³ are each independently selected from aryl, cycloalkyl, heteroaryl or -alkylene-O-alkyl.

In one embodiment, R¹ is alkyl, -OH or -NH₂ and the group -CH(R²)(R³) is:

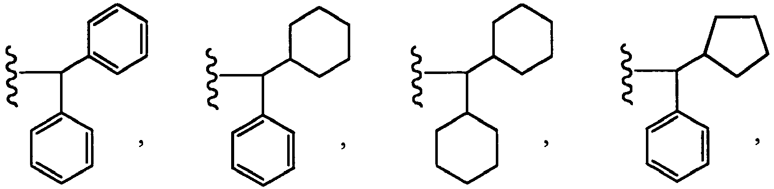

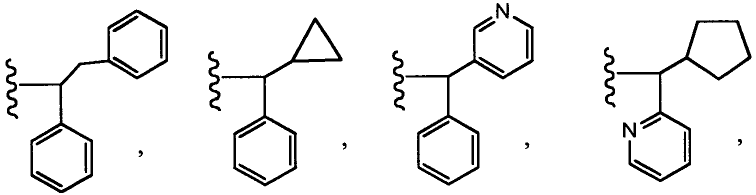

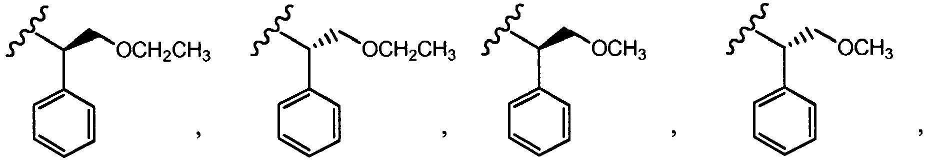

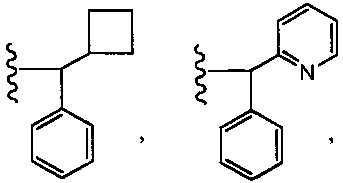

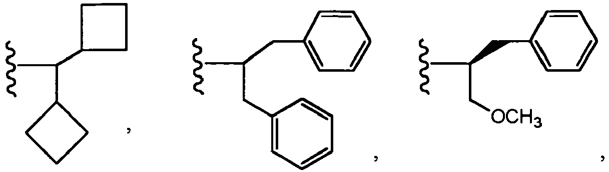

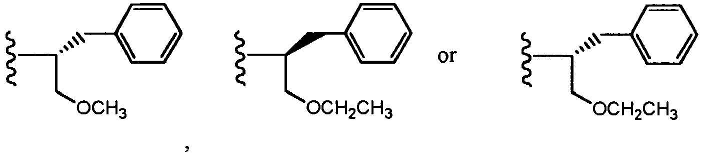

In one embodiment, R¹ is alkyl and the group -CH(R²)(R³) is:

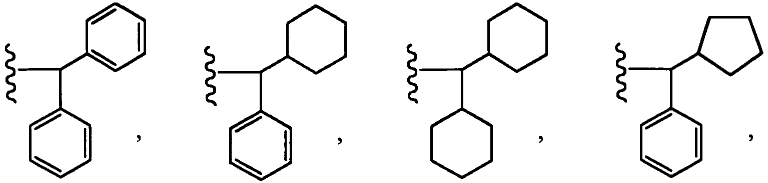

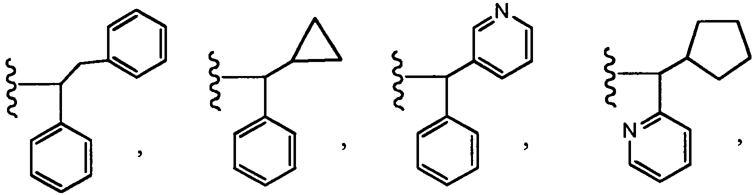

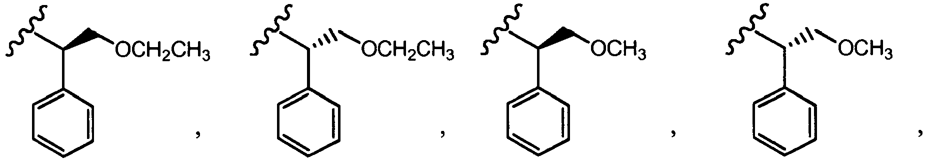

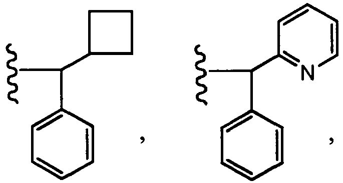

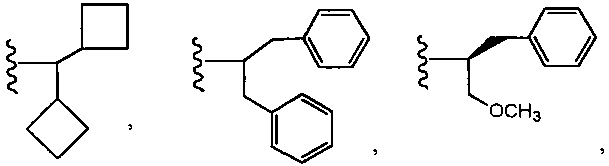

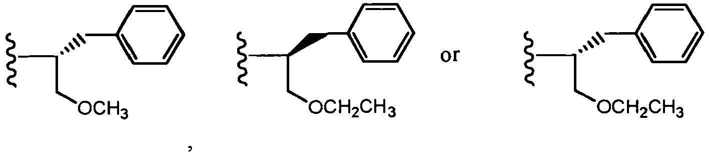

In one embodiment, R⁵ is H.

In one embodiment, R⁵ is -N(R⁸)₂.

In another embodiment, R⁵ is -NH₂.

In another embodiment, R⁵ is -NH-alkyl.

In yet another embodiment, R⁵ is -N(alkyl)₂.

In a further embodiment, R⁵ is -N(CH₃)₂.

In another embodiment, R⁵ is -N(CH₂CH₃)₂.

In one embodiment, R⁵ is -NHCH₃.

In still another embodiment, R⁵ is -NH-aryl.

In another embodiment, R⁵ is -NH-C(O)OR¹¹.

In another embodiment, R⁵ is -NH-C(O)R¹¹.

In another embodiment, R⁵ is -N(alkyl)-C(O)OR¹¹.

In another embodiment, R⁵ is -N(alkyl)-C(O)R¹¹.

In another embodiment, R⁵ is -NH-C(O)alkyl.

In still another embodiment, R⁵ is -NH-C(O)-aryl.

In another embodiment, R⁵ is -NH-C(O)-phenyl.

In yet another embodiment, R⁵ is NHC(O)O-alkyl.

In another embodiment, R⁵ is -NHC(O)O-heteroaryl.

In another embodiment, R⁵ is -NHC(O)O-aryl.

In one embodiment, R⁵ is -NHC(O)O-phenyl.

In another embodiment, R⁵ is -NHC(O)O-t-butyl.

In another embodiment, R⁵ is -NH-phenyl.

In another embodiment, R⁵ is -N(alkyl)(aryl).

In one embodiment, R⁵ is -N(heteroaryl)(alkyl).

In another embodiment, R⁵ is -N(pyridyl)(CH₃).

In another embodiment, R⁵ is -N(phenyl)(alkyl).

In a further embodiment, R⁵ is -N(phenyl)(CH₃).

In another embodiment, R⁵ is -N(alkylene-aryl)(alkyl).

In another embodiment, R⁵ is -N(benzyl)(-SO₂-alkyl).

In one embodiment, R⁵ is -NH-alkylene-aryl.

In another embodiment, R⁵ is -NH-benzyl.

In another embodiment, R⁶ is NHC(O)OR¹¹, -NH-phenyl, or -N(alkyl)(phenyl), wherein the phenyl moiety of an -NH-phenyl or -N(alkyl)(phenyl) group can be unsubstituted or substituted as set forth above for the compounds of formula (I).

In one embodiment, R⁵ is -OR¹⁴.

In another embodiment, R⁵ is -O-alkyl.

In another embodiment, R⁵ is -OCH₃.

In still another embodiment, R⁵ is -O-ethyl, -O-isopropyl or -O-t-butyl.

In another embodiment, R⁵ is -O-aryl.

In yet another embodiment, R⁵ is -O-phenyl.

In one embodiment, R⁵ is -O-heteroaryl.

In another embodiment, R⁵ is -O-pyridyl.

In another embodiment, R⁵ is -O-alkylene-C(O)OR¹¹.

In still another embodiment, R⁵ is -O-alkylene-C(O)O-alkyl.

In another embodiment, R⁵ is -O-alkylene-C(O)O-t-butyl.

In yet another embodiment, R⁵ is -O-alkylene-heterocycloalkyl.

In another embodiment, R⁵ is -O-haloalkyl.

In a further embodiment, R⁵ is -OCF₃.

In one embodiment, R⁵ is -C(NH₂)(=N-OH).

In another embodiment, R⁵ is heterocycloalkyl.

In another embodiment, R⁵ is morpholinyl.

In still another embodiment, R⁵ is piperidinyl.

In another embodiment, R⁵ is indolyl.

In yet another embodiment, R⁵ is alkyl.

In another embodiment, R⁵ is aryl.

In a further embodiment, R⁵ is phenyl.

In another embodiment, R⁵ is -CN.

In another embodiment, R⁵ is -NO₂.

In still another embodiment, R⁵ is -halo.

In another embodiment, R⁵ is -Br.

In one embodiment, R⁵ is -C(O)R¹¹.

In another embodiment, R⁵ is -C(O)alkyl.

In another embodiment, R⁵ is -C(O)aryl.

In one embodiment, R⁵ is -C(O)OR¹¹.

In another embodiment, R⁵ is -C(O)Oalkyl.

In another embodiment, R⁵ is -C(O)Oaryl.

In yet another embodiment, R⁵ is -C(O)N(R⁸)₂.

In another embodiment, R⁵ is -C(O)NH-alkyl.

In still another embodiment, R⁵ is -C(O)NH-aryl.

In another embodiment, R⁵ is -C(O)NH-phenyl.

In one embodiment, for the compounds of formula (Ib), R', R², R³ and R⁵ are selected independently of each other.

In another embodiment, a compound of formula (Ib) is in purified form.

In one embodiment, the compounds of formula (I) have the formula (Ic): 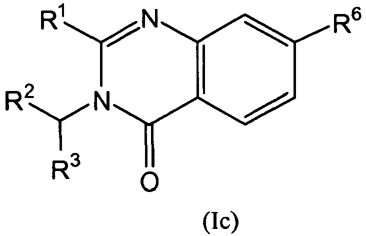 wherein R', R², R³ and R⁶ are defined above for the compounds of formula (I).

In one embodiment, R¹ is alkyl, -alkylene-aryl, -OH or -NH₂.

In another embodiment, R¹ is alkyl.

In another embodiment, R' is -alkylene-aryl.

In still another embodiment, R¹ is -OH.

In another embodiment, R¹ is -NH₂.

In yet another embodiment, R¹ is methyl.

In a further embodiment, R¹ is ethyl.

In one embodiment, R² and R³ are each independently selected from haloalkyl, - (alkylene)ₙ-aryl, -(alkylene)ₙ-heteroaryl, -(alkylene)ₙ-cycloalky and -alkylene-O-alkyl.

In another embodiment, R² and R³ are each aryl.

In yet another embodiment, R² and R³ are each heteroaryl.

In another embodiment, R² and R³ are each cycloalkyl.

In another embodiment, R² is aryl and R³ is heteroaryl.

In one embodiment, the group -CH(R²)(R³) is:

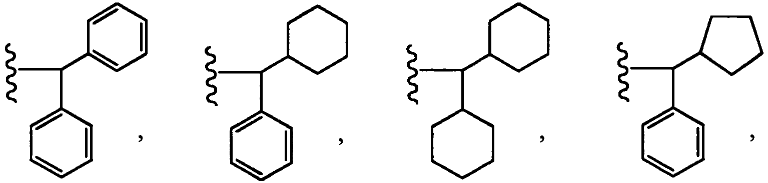

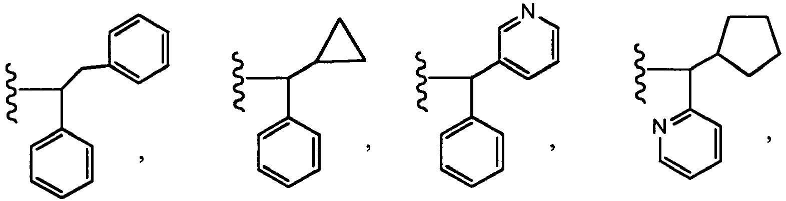

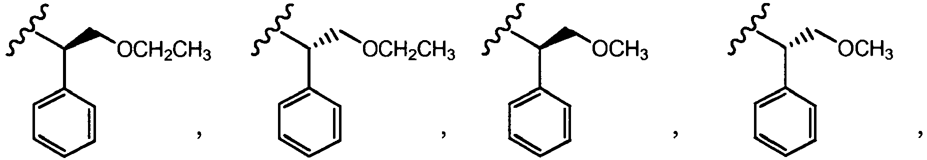

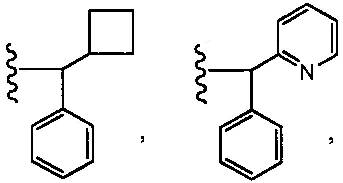

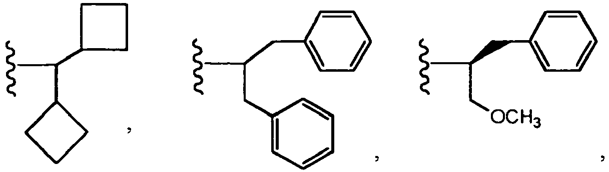

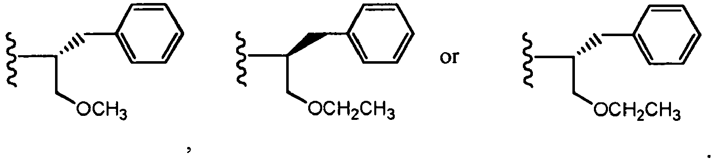

In another embodiment, R² and R³ are each phenyl.

In another embodiment, R² and R³ and the carbon atom to which they are both attached, combine to form:

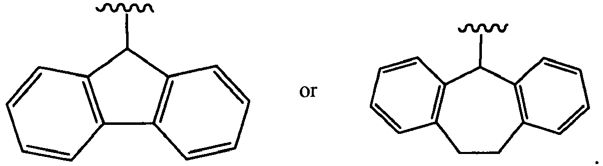

In one embodiment, R¹ is alkyl, -OH or -NH₂; R² and R³ are each independently selected from aryl, alkyl, cycloalkyl, heteroaryl or -alkylene-O-alkyl.

In another embodiment, R¹ is alkyl; R² and R³ are each independently selected from aryl, alkyl, cycloalkyl, heteroaryl or -alkylene-O-alkyl.

In another embodiment, R¹ is methyl; R² and R³ are each independently selected from aryl, alkyl, cycloalkyl, heteroaryl or -alkylene-O-alkyl.

In one embodiment, R¹ is alkyl, -OH or -NH₂ and the group -CH(R²)(R³) is:

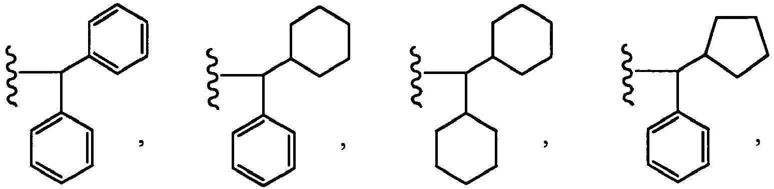

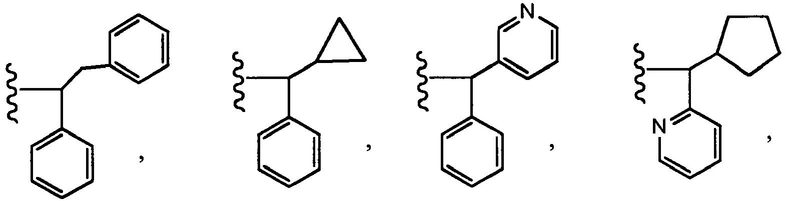

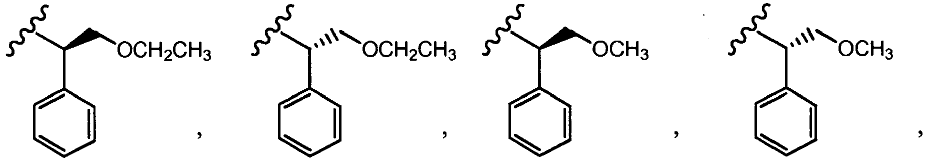

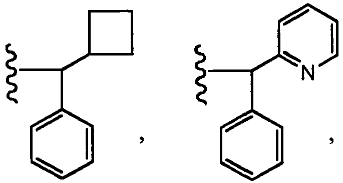

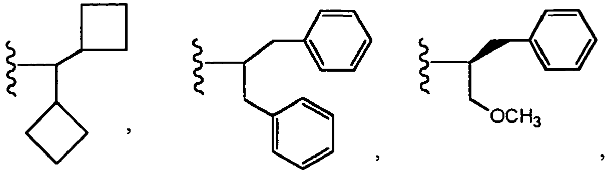

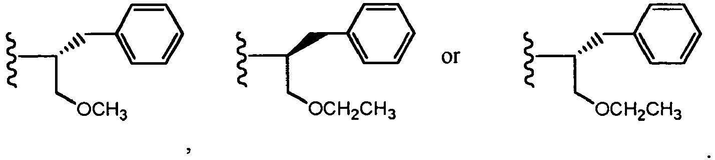

In one embodiment, R¹ is alkyl, and the group -CH(R²)(R³) is:

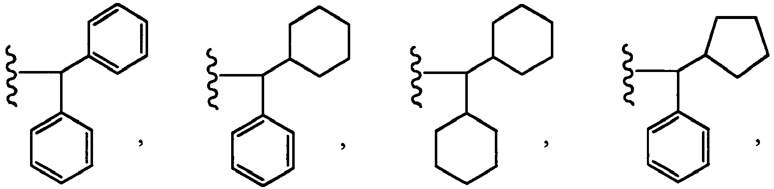

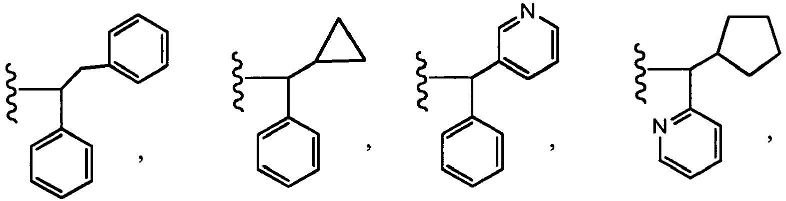

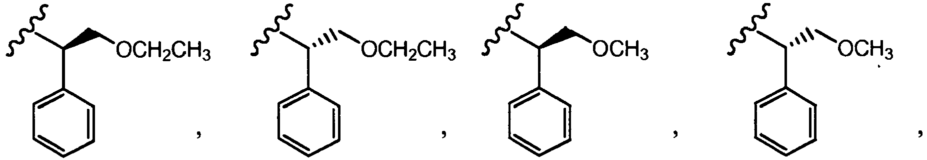

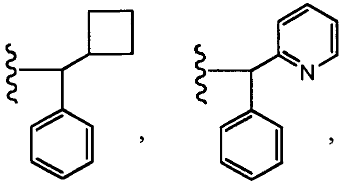

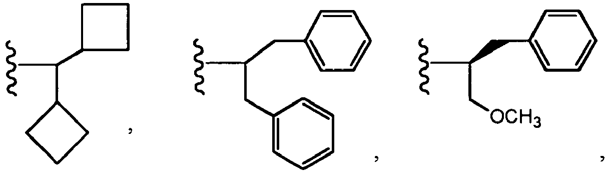

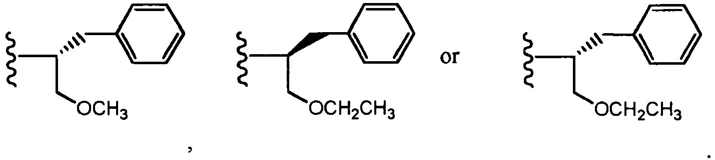

In one embodiment, R⁶ is H.

In one embodiment, R⁶ is -N(R⁸)₂.

In another embodiment, R⁶ is -NH₂.

In another embodiment, R⁶ is -NH-alkyl.

In yet another embodiment, R⁶ is -N(alkyl)₂.

In a further embodiment, R⁶ is -N(CH₃)₂.

In another embodiment, R⁶ is -N(CH₂CH₃)₂.

In one embodiment, R⁶ is -NHCH₃.

In still another embodiment, R⁶ is -NH-aryl.

In another embodiment, R⁶ is -NH-C(O)OR¹¹.

In another embodiment, R⁶ is -NH-C(O)R¹¹.

In another embodiment, R⁶ is -N(alkyl)-C(O)OR¹¹.

In another embodiment, R⁶ is -N(alkyl)-C(O)R¹¹.

In another embodiment, R⁶ is -NH-C(O)alkyl.

In still another embodiment, R⁶ is -NH-C(O)-aryl.

In another embodiment, R⁶ is -NH-C(O)-phenyl.

In yet another embodiment, R⁶ is -NHC(O)O-alkyl.

In another embodiment, R⁶ is -NHC(O)O-heteroaryl.

In another embodiment, R⁶ is -NHC(O)O-aryl.

In one embodiment, R⁶ is -NHC(O)O-phenyl.

In another embodiment, R⁶ is -NHC(O)O-t-butyl.

In another embodiment, R⁶ is -NH-phenyl.

In another embodiment, R⁶ is -N(alkyl)(aryl).

In one embodiment, R⁶ is -N(heteroaryl)(alkyl).

In another embodiment, R⁶ is -N(pyridyl)(CH₃).

In another embodiment, R⁶ is -N(phenyl)(alkyl).

In a further embodiment, R⁶ is -N(phenyl)(CH₃).

In another embodiment, R⁶ is -N(alkylene-aryl)(alkyl).

In another embodiment, R⁶ is -N(benzyl)(-SO₂-alkyl).

In one embodiment, R⁶ is -NH-alkylene-aryl.

In another embodiment, R⁶ is -NH-benzyl.

In another embodiment, R⁶ is ―NHC(O)OR¹¹, -NH-phenyl, or -N(alkyl)(phenyl), wherein the phenyl moiety of an -NH-phenyl or -N(alkyl)(phenyl) group can be unsubstituted or substituted as set forth above for the compounds of formula (I).

In one embodiment, R⁶ is ―OR¹⁴.

In another embodiment, R⁶ is -O-alkyl.

In another embodiment, R⁶ is -OCH₃.

In still another embodiment, R⁶ is ―O-ethyl, -O-isopropyl or -O-t-butyl.

In another embodiment, R⁶ is -O-aryl.

In yet another embodiment, R⁶ is -O-phenyl

In one embodiment, R⁶ is -O-heteroaryl.

In another embodiment, R⁶ is -O-pyridyl.

In another embodiment, R⁶ is -O-alkylene-C(O)OR¹¹.

In still another embodiment, R⁶ is -O-alkylene-C(O)O-alkyl.

In another embodiment, R⁶ is -O-alkylene-C(O)O-t-butyl.

In yet another embodiment, R⁶ is -O-alkylene-heterocycloalkyl.

In another embodiment, R⁶ is -O-haloalkyl.

In a further embodiment, R⁶ is -OCF₃.

In one embodiment, R⁶ is -C(NH₂)(=N-OH).

In another embodiment, R⁶ is heterocycloalkyl.

In another embodiment, R⁶ is morpholinyl.

In still another embodiment, R⁶ is piperidinyl.

In another embodiment, R⁶ is indolyl.

In yet another embodiment, R⁶ is alkyl.

In another embodiment, R⁶ is aryl.

In a further embodiment, R⁶ is phenyl.

In another embodiment, R⁶ is -CN.

In another embodiment, R⁶ is -NO₂.

In still another embodiment, R⁶ is -halo.

In another embodiment, R⁶ is -Br.

In one embodiment, R⁶ is -C(O)R¹¹.

In another embodiment, R⁶ is -C(O)alkyl.

In another embodiment, R⁶ is -C(O)aryl.

In one embodiment, R⁶ is -C(O)OR¹¹.

In another embodiment, R⁶ is -C(O)Oalkyl.

In another embodiment, R⁶ is -C(O)Oaryl.

In yet another embodiment, R⁶ is -C(O)N(R⁸)₂.

In another embodiment, R⁶ is -C(O)NH-alkyl.

In still another embodiment, R⁶ is -C(O)NH-aryl.

In another embodiment, R⁶ is -C(O)NH-phenyl.

In another embodiment, R⁶ is other than H.

In one embodiment, for the compounds of formula (Ic), R¹, R², R³ and R⁶ are selected independently of each other.

In another embodiment, a compound of formula (Ic) is in purified form.

In one embodiment, the compounds of formula (I) have the formula (Id): 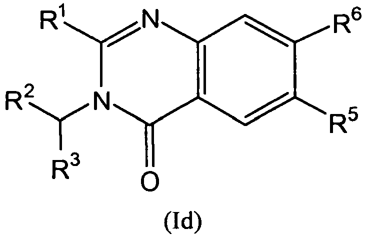 wherein R¹, R², R³, R⁵ and R⁶ are defined above for the compounds of formula (I).

In one embodiment, R¹ is alkyl, -akylene-aryl, -OH or -NH₂.

In another embodiment, R¹ is alkyl.

In another embodiment, R¹ is -alkylene-aryl.

In still another embodiment, R¹ is -OH.

In another embodiment, R¹ is -NH₂.

In yet another embodiment, R¹ is methyl.

In a further embodiment, R¹ is ethyl.

In one embodiment, R² and R³ are each independently selected from haloalkyl, - (alkylene)ₙ-aryl, -(alkylene)ₙ-heteroaryl, -(alkylene)ₙ-cycloalkyl and -alkylene-O-alkyl.

In another embodiment, R² and R³ are each aryl.

In yet another embodiment, R² and R³ are each heteroaryl.

In another embodiment, R² and R³ are each cycloalkyl.

In another embodiment, R² is aryl and R³ is heteroaryl.

In one embodiment, the group ―CH(R²)(R³) is:

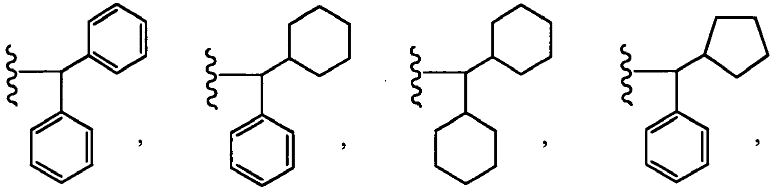

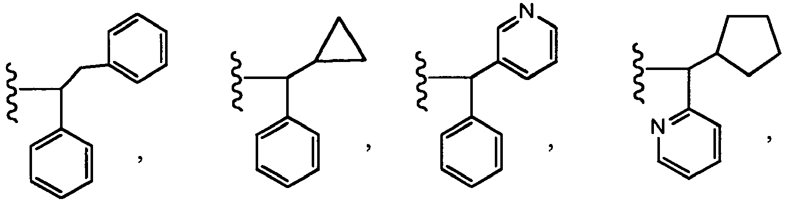

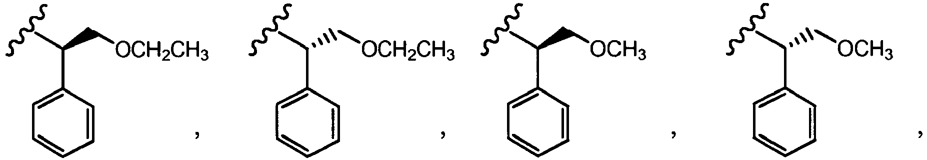

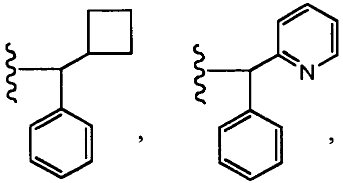

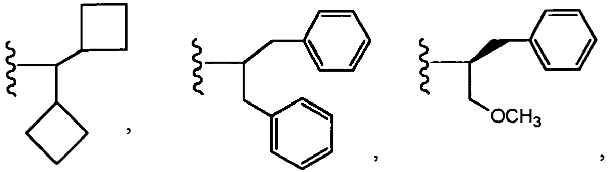

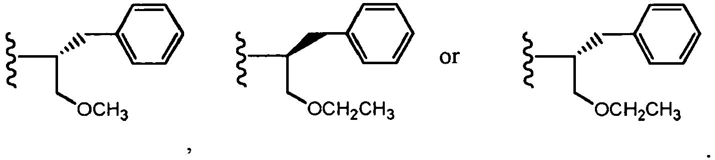

In another embodiment, R² and R³ are each phenyl.

In one embodiment, R² and R³ and the carbon atom to which they are both attached, combine to form a cycloalkyl or heterocycloalkyl group, wherein the cycloalkyl or heterocycloalkyl group can be optionally fused to one or two benzene rings.

In another embodiment, R² and R³ and the carbon atom to which they are both attached, combine to form:

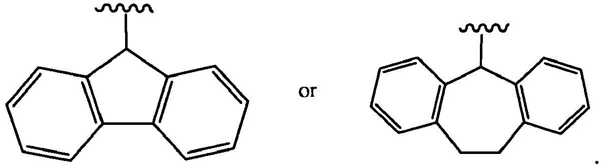

In one embodiment, R¹ is alkyl, -OH or -NH₂; R² and R³ are each independently selected from aryl, alkyl, cycloalkyl, heteroaryl or -alkylene-O-alkyl.

In another embodiment, R¹ is alkyl; R² and R³ are each independently selected from aryl, alkyl, cycloalkyl, heteroaryl or -alkylene-O-alkyl.

In another embodiment, R¹ is methyl; R² and R³ are each independently selected from aryl, alkyl, cycloalkyl, heteroaryl or -alkylene-O-alkyl.

In one embodiment, R¹ is alkyl, -OH or -NH₂ and the group ―CH(R²)(R³) is:

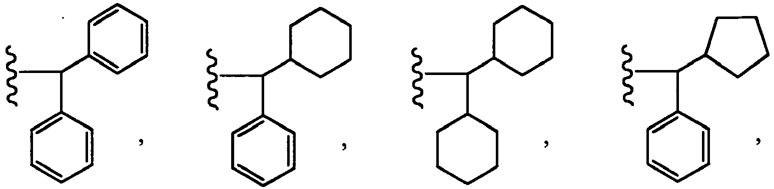

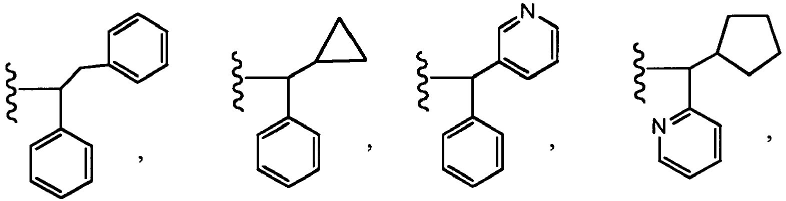

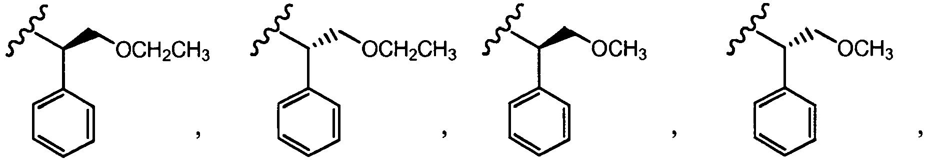

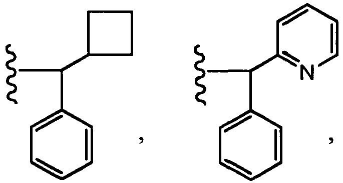

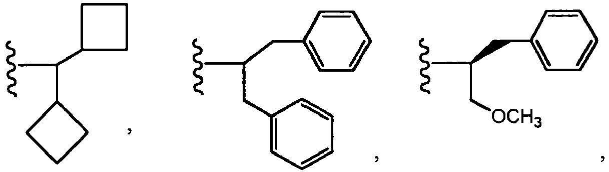

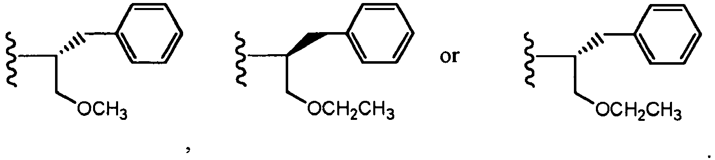

In one embodiment, R¹ is alkyl, and the group ―CH(R²)(R³) is:

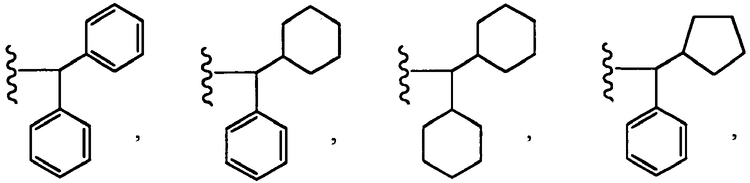

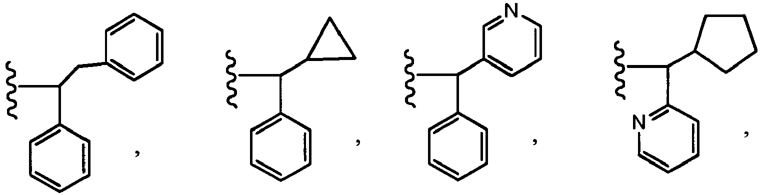

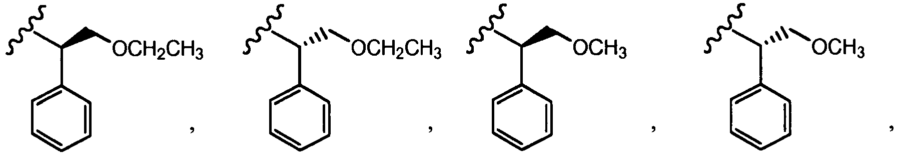

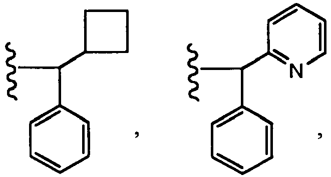

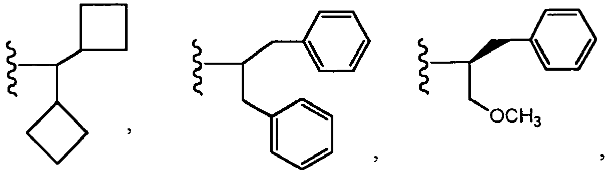

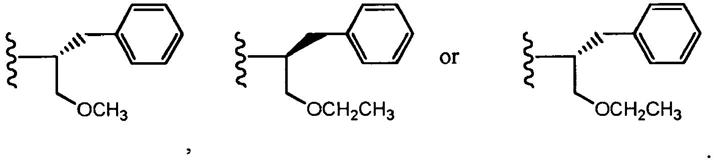

In one embodiment, R⁵ is H.

In one embodiment, R⁵ is -N(R⁸)₂.

In another embodiment, R⁵ is -NH₂.

In another embodiment, R⁵ is -NH-alkyl.

In yet another embodiment, R⁵ is ―N(alkyl)₂.

In a further embodiment, R⁵ is ―N(CH₃)₂.

In another embodiment, R⁵ is ―N(CH₂CH₃)₂.

In one embodiment, R⁵ is -NHCH₃.

In still another embodiment, R⁵ is -NH-aryl.

In another embodiment, R⁵ is ―NH-C(O)OR¹¹.

In another embodiment, R⁵ is ―NH-C(O)R¹¹.

In another embodiment, R⁵ is ―N(alkyl)-C(O)OR¹¹.

In another embodiment, R⁵ is ―N(alkyl)-C(O)R¹¹.

In another embodiment, R⁵ is -NH-C(O)alkyl.

In still another embodiment, R⁵ is -NH-C(O)-aryl.

In another embodiment, R⁵ is -NH-C(O)-phenyl.

In yet another embodiment, R⁵ is -NHC(O)O-alkyl.

In another embodiment, R⁵ is -NHC(O)O-heteroaryl.

In another embodiment, R⁵ is -NHC(O)O-aryl.

In one embodiment, R⁵ is -NHC(O)O-phenyl.

In another embodiment, R⁵ is ―NHC(O)O-t-butyl.

In another embodiment, R⁵ is -NH-phenyl.

In another embodiment, R⁵ is -N(alkyl)(aryl).

In one embodiment, R⁵ is -N(heteroaryl)(alkyl).

In another embodiment, R⁵ is -N(pyridyl)(CH₃).

In another embodiment, R⁵ is -N(phenyl)(alkyl).

In a further embodiment, R⁵ is -N(phenyl)(CH₃).

In another embodiment, R⁵ is -N(alkylene-aryl)(alkyl).

In another embodiment, R⁵ is -N(benzyl)(-SO₂-alkyl).

In one embodiment, R⁵ is -NH-alkylene-aryl.

In another embodiment, R⁵ is -NH-benzyl.

In another embodiment, R⁵ is ―NHC(O)OR¹¹, -NH-phenyl, or -N(alkyl)(phenyl), wherein the phenyl moiety of an -NH-phenyl or -N(alkyl)(phenyl) group can be unsubstituted or substituted as set forth above for the compounds of formula (I).

In one embodiment, R⁵ is ―OR¹⁴.

In another embodiment, R⁵ is -O-alkyl.

In another embodiment, R⁵ is -OCH₃.

In still another embodiment, R⁵ is -O-ethyl, -O-isopropyl or -O-t-butyl.

In another embodiment, R⁵ is -O-aryl.

In yet another embodiment, R⁵ is -O-phenyl.

In one embodiment, R⁵ is -O-heteroaryl.

In another embodiment, R⁵ is -O-pyridyl.

In another embodiment, R⁵ is -O-akylene-C(O)OR¹¹.

In still another embodiment, R⁵ is -O-alkylene-C(O)O-alkyl.

In another embodiment, R⁵ is -O-alkylene-C(O)O-t-butyl.

In yet another embodiment, R⁵ is -O-alkylene-heterocycloalkyl.

In another embodiment, R⁵ is -O-haloalkyl.

In a further embodiment, R⁵ is -OCF₃.

In one embodiment, R⁵ is -C(NH₂)(=N-OH).

In another embodiment, R⁵ is heterocycloalkyl.

In another embodiment, R⁵ is morpholinyl.

In still another embodiment, R⁵ is piperidinyl.

In another embodiment, R⁵ is indolyl.

In yet another embodiment, R⁵ is alkyl.

In another embodiment, R⁵ is aryl.

In a further embodiment, R⁵ is phenyl.

In another embodiment, R⁵ is -CN.

In another embodiment, R⁵ is -NO₂.

In still another embodiment, R⁵ is -halo.

In another embodiment, R⁵ is -Br.

In one embodiment, R⁵ is -C(O)R¹¹.

In another embodiment, R⁵ is -C(O)alkyl.

In another embodiment, R⁵ is -C(O)aryl.

In one embodiment, R⁵ is -C(O)OR¹¹.

In another embodiment, R⁵ is -C(O)Oalkyl.

In another embodiment, R⁵ is -C(O)Oaryl.

In yet another embodiment, R⁵ is -C(O)N(R⁸)₂.

In another embodiment, R⁵ is -C(O)NH-alkyl.

In still another embodiment, R⁵ is -C(O)NH-aryl.

In another embodiment, R⁵ is -C(O)NH-phenyl.

In one embodiment, R⁶ is H.

In one embodiment, R⁶ is -N(R⁸)₂.

In another embodiment, R⁶ is -NH₂.

In another embodiment, R⁶ is -NH-alkyl.

In yet another embodiment, R⁶ is ―N(alkyl)₂.

In a further embodiment, R⁶ is ―N(CH₃)₂.

In another embodiment, R⁶ is ―N(CH₂CH₃)₂.

In one embodiment, R⁶ is -NHCH₃.

In still another embodiment, R⁶ is -NH-aryl.

In another embodiment, R⁶ is ―NH-C(O)OR¹¹.

In another embodiment, R⁶ is ―NH-C(O)R¹¹.

In another embodiment, R⁶ is ―N(alkyl)-C(O)OR¹¹.

In another embodiment, R⁶ is ―N(alkyl)-C(O)R¹¹.

In another embodiment, R⁶ is -NH-C(O)alkyl.

In still another embodiment, R⁶ is -NH-C(O)-aryl.

In another embodiment, R⁶ is -NH-C(O)-phenyl.

In yet another embodiment, R⁶ is ―NHC(O)O-alkyl.

In another embodiment, R⁶ is -NHC(O)O-heteroaryl.

In another embodiment, R⁶ is -NHC(O)O-aryl.

In one embodiment, R⁶ is -NHC(O)O-phenyl.

In another embodiment, R⁶ is -NHC(O)O-t-butyl.

In another embodiment, R⁶ is -NH-phenyl.

In another embodiment, R⁶ is -N(alkyl)(aryl).

In one embodiment, R⁶ is -N(heteroaryl)(alkyl).

In another embodiment, R⁶ is -N(pyridyl)(CH₃).

In another embodiment, R⁶ is -N(phenyl)(alkyl).

In a further embodiment, R⁶ is -N(phenyl)(CH₃).

In another embodiment, R⁶ is -N(alkylene-aryl)(alkyl).

In another embodiment, R⁶ is -N(benzyl)(-SO₂-alkyl).

In one embodiment, R⁶ is -NH-alkylene-aryl.

In another embodiment, R⁶ is -NH-benzyl.

In another embodiment, R⁶ is ―NHC(O)OR¹¹, -NH-phenyl, or -N(alkyl)(phenyl), wherein the phenyl moiety of an -NH-phenyl or -N(alkyl)(phenyl) group can be unsubstituted or substituted as set forth above for the compounds of formula (I).

In one embodiment, R⁶ is ―OR¹⁴.

In another embodiment, R⁶ is -O-alkyl.

In another embodiment, R⁶ is -OCH₃.

In still another embodiment, R⁶ is -O-ethyl, -O-isopropyl or -O-t-butyl.

In another embodiment, R⁶ is -O-aryl.

In yet another embodiment, R⁶ is -O-phenyl.

In one embodiment, R⁶ is -O-heteroaryl.

In another embodiment, R⁶ is -O-pyridyl.

In another embodiment, R⁶ is -O-alkylene-C(O)OR¹¹.

In still another embodiment, R⁶ is -O-alkylene-C(O)O-alkyl.

In another embodiment, R⁶ is -O-alkylene-C(O)O-t-butyl.

In yet another embodiment, R⁶ is -O-alkylene-heterocycloalkyl.

In another embodiment, R⁶ is -O-haloalkyl.

In a further embodiment, R⁶ is -OCF₃.

In one embodiment, R⁶ is -C(NH₂)(=N-OH).

In another embodiment, R⁶ is heterocycloalkyl.

In another embodiment, R⁶ is morpholinyl.

In still another embodiment, R⁶ is piperidinyl.

In another embodiment, R⁶ is indolyl.

In yet another embodiment, R⁶ is alkyl.

In another embodiment, R⁶ is aryl.

In a further embodiment, R⁶ is phenyl.

In another embodiment, R⁶ is -CN.

In another embodiment, R⁶ is -NO₂.

In still another embodiment, R⁶ is -halo.

In another embodiment, R⁶ is -Br.

In one embodiment, R⁶ is -C(O)R¹¹.

In another embodiment, R⁶ is -C(O)alkyl.

In another embodiment, R⁶ is -C(O)aryl.

In one embodiment, R⁶ is -C(O)OR¹¹.

In another embodiment, R⁶ is -C(O)Oalkyl.

In another embodiment, R⁶ is -C(O)Oaryl.

In yet another embodiment, R⁶ is -C(O)N(R⁸)₂.

In another embodiment, R⁶ is -C(O)NH-alkyl.

In still another embodiment, R⁶ is -C(O)NH-aryl.

In another embodiment, R⁶ is -C(O)NH-phenyl.

In another embodiment, R⁶ is other than H.

In one embodiment, R⁵ is H and R⁶ is other than H.

In another embodiment, R⁶ is H and R⁵ is other than H.

In another embodiment, R⁵ and R⁶ are each other than H.

In still another embodiment, R⁵ and R⁶ are each -O-alkyl.

In one embodiment, R⁵ and R⁶ are each methoxy.

In one embodiment, for the compounds of formula (Id), R¹, R², R³, R⁵ and R⁶ are selected independently of each other.

In another embodiment, a compound of formula (Id) is in purified form.

Non-limiting examples of the Bicyclic Heterocycle Derivatives of Formula (I) include, but are not limited to, the following compounds:

| **Compound No.** | **Structure** | **Observed Mass [M+1]** |
|---|---|---|
| **1** | 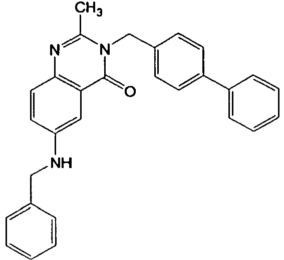 | 432.2 |
| **2** | 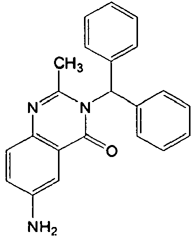 | 342.2 |
| **3** | 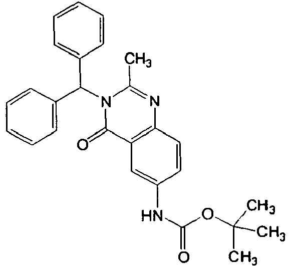 | 442.2 |
| **5** | 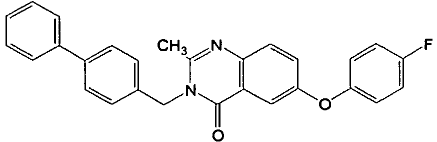 | 437.2 |
| **6** | 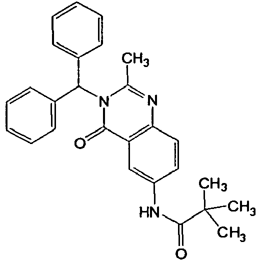 | 426.2 |
| **7** | 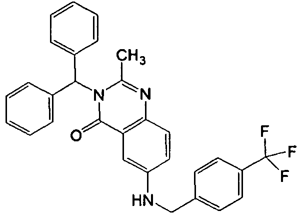 | 500.3 |
| **8** | 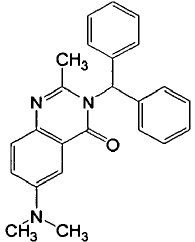 | 370.2 |
| **9** | 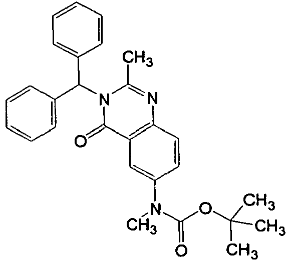 | 456.3 |
| **10** | 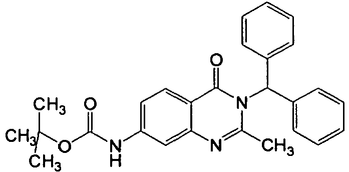 | 442.1 |
| **11** | 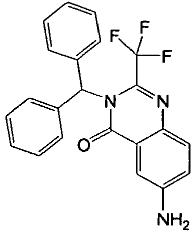 | 396.2 |
| **12** | 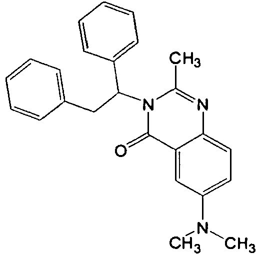 | 384.2 |
| **13** | 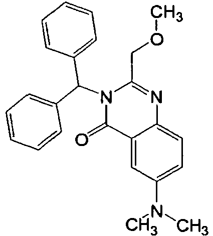 | 400.2 |
| **14** | 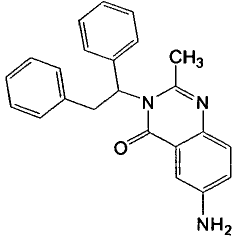 | 356.2 |
| **15** | 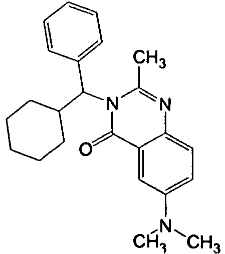 | 376.2 |
| **16** | 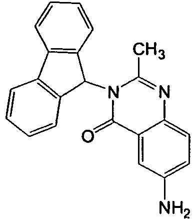 | 340.2 |
| **17** | 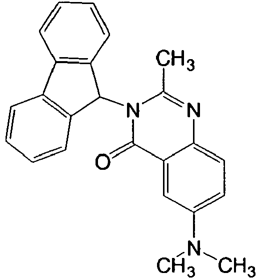 | 368.2 |
| **18** | 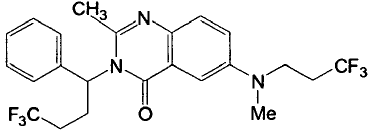 | 472.3 |
| **19** | 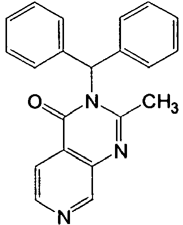 | 328 |
| **20** | 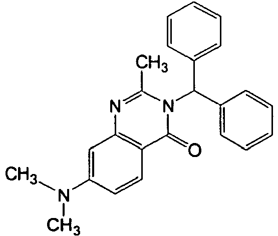 | 370.2 |
| **21** | 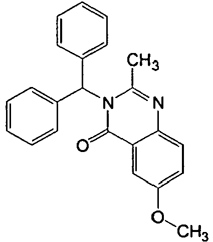 | 357 |
| **22** | 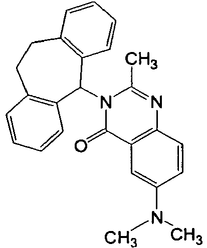 | 396.2 |
| **23** | 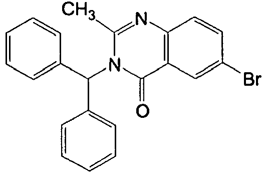 | 405,407 |
| **24** | 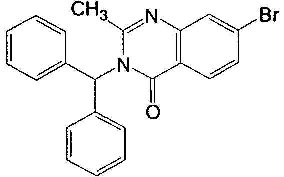 | 405,407 |
| **25** | 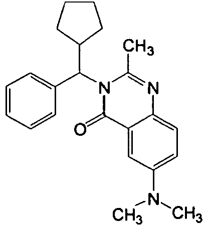 | 362.2 |
| **26** | 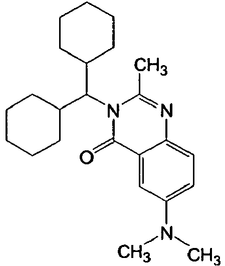 | 382.2 |
| **27** | 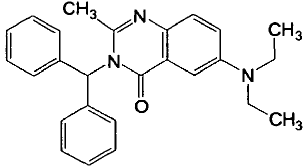 | 398.2 |
| **28** | 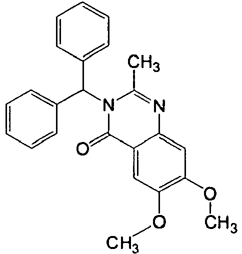 | 387 |
| **29** | 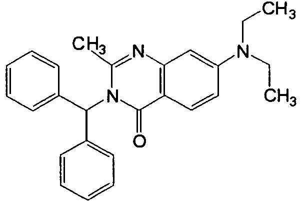 | 398.2 |
| **30** | 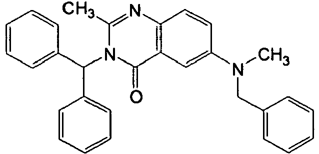 | 446.2 |
| **31** | | 357 |
| | 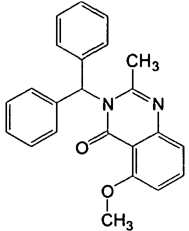 | |
| **32** | 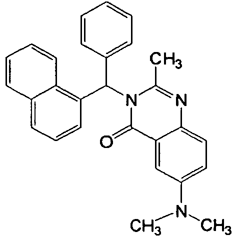 | 420.2 |
| **33** | 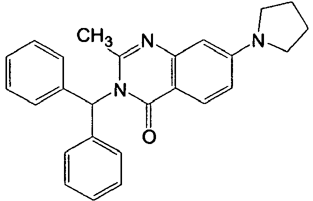 | 396.2 |
| **34** | 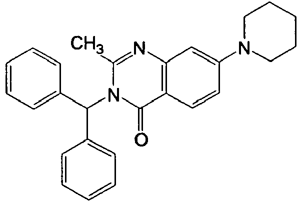 | 410.2 |
| **35** | 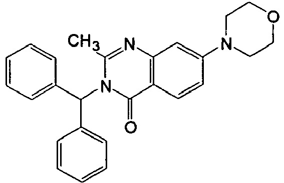 | 412.2 |
| **36** | 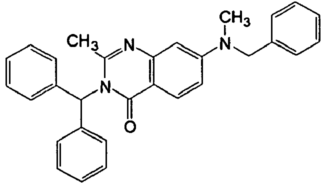 | 446.2 |
| **38** | 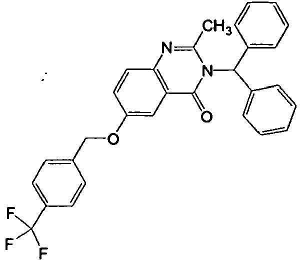 | 501.3 |
| **39** | 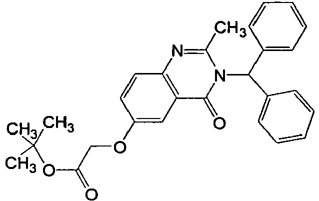 | 457.3 |
| **40** | 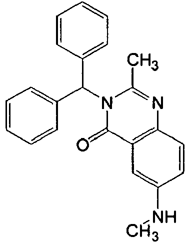 | 356 |
| **41** | 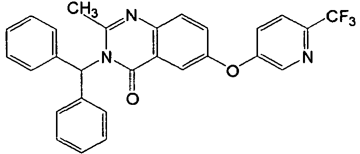 | 488.3 |
| **42** | 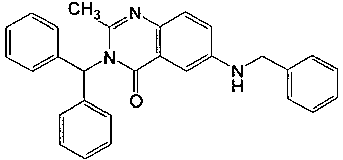 | 432.2 |
| **43** | 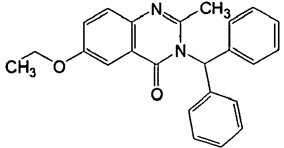 | 371.2 |
| **44** | 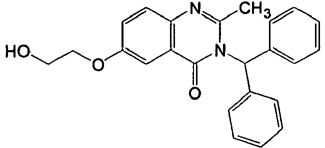 | 387.2 |
| **45** | 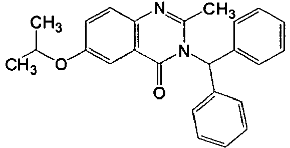 | 385.2 |
| **46** | 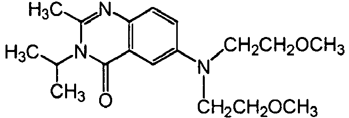 | NA |
| **48** | 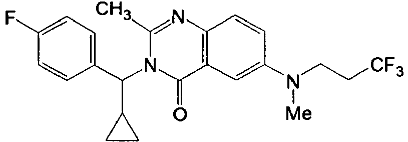 | 434.2 |
| **49** | 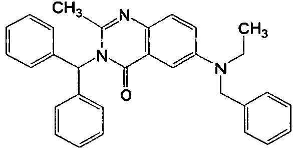 | 460.3 |
| **50** | 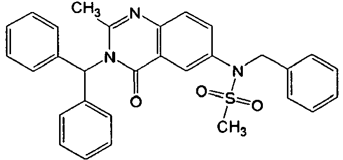 | 510.3 |
| **51** | 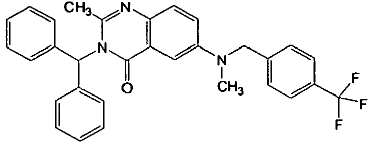 | 514.3 |
| **52** | 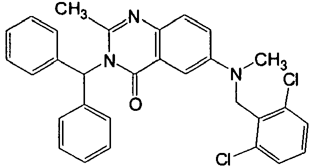 | 514.3 |
| **53** | 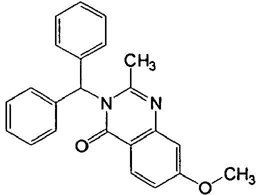 | 357 |
| **54** | 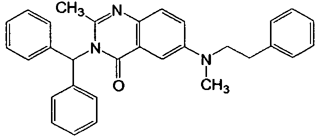 | 460.3 |
| **56** | 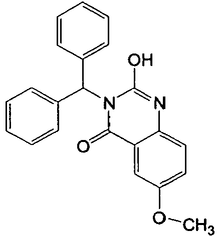 | 359 |
| **57** | 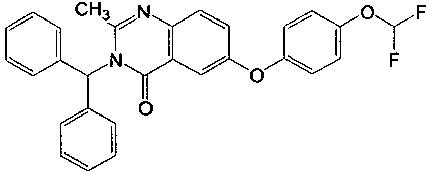 | 485.3 |
| **58** | 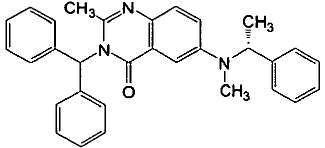 | 460.3 |
| **59** | 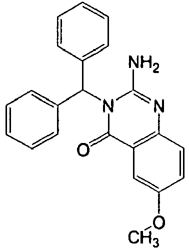 | 358 |
| **60** | 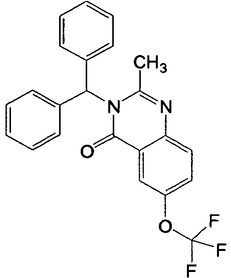 | 411 |
| **61** | 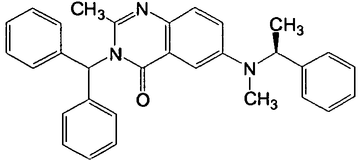 | 460.3 |
| **63** | 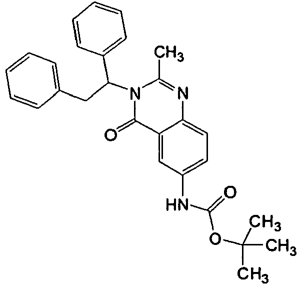 | 456.3 |
| **64** | 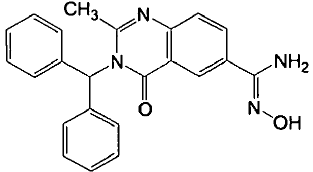 | 385.2 |
| **65** | 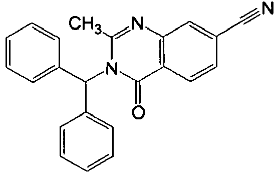 | 352.2 |
| **66** | 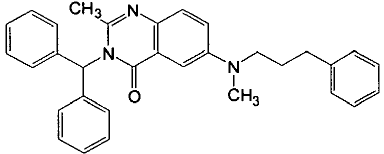 | 474.3 |
| **67** | 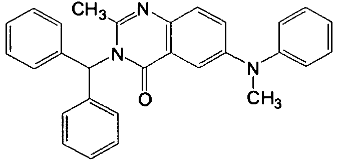 | 432.2 |
| **68** | 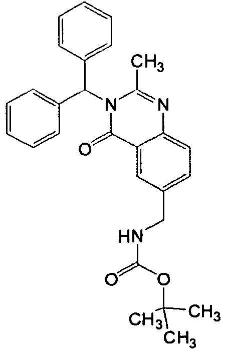 | 456.3 |
| **69** | 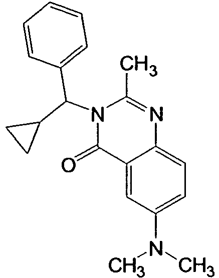 | 334.2 |
| **70** | 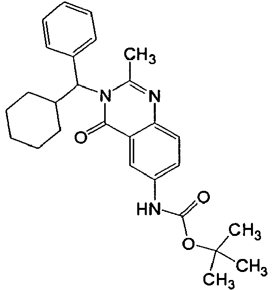 | 448.2 |
| **71** | 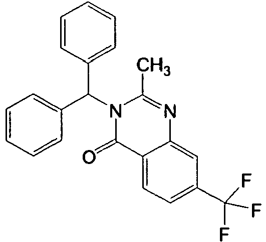 | 395 |
| **72** | 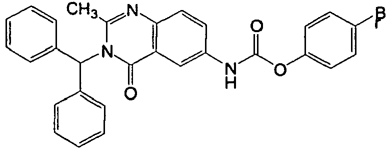 | 540.3 |
| **73** | 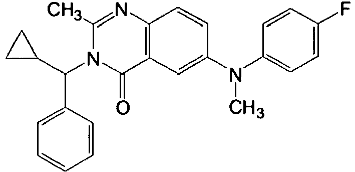 | 413.2 |
| **74** | 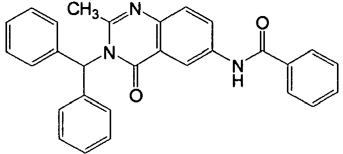 | 446.2 |
| **76** | 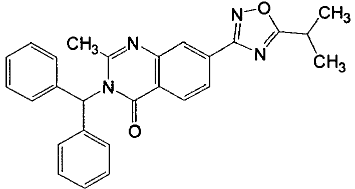 | 437.2 |
| **77** | 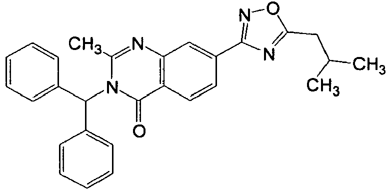 | 451.2 |
| **78** | 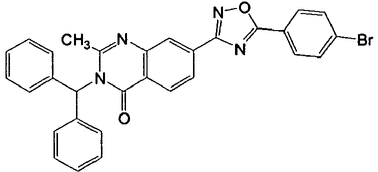 | 549.3 |
| **79** | 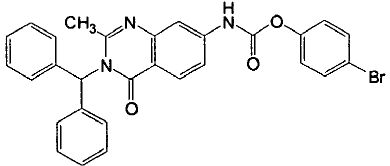 | 540,542 |
| **80** | 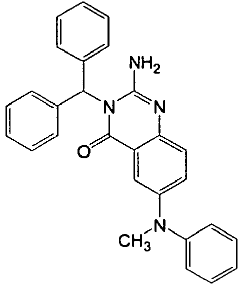 | 433 |
| **81** | 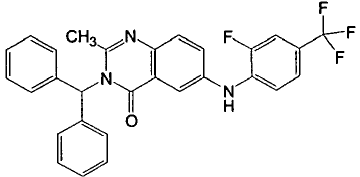 | 504.3 |
| **82** | 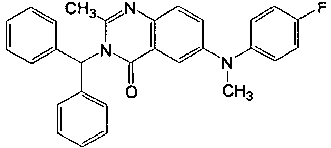 | 450.2 |
| **83** | 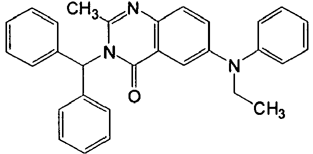 | 446.2 |
| **84** | 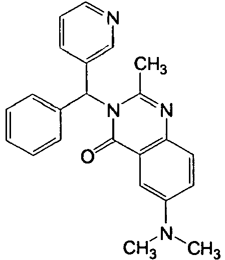 | 371.2 |
| **85** | 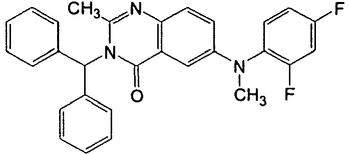 | 468.3 |
| **86** | 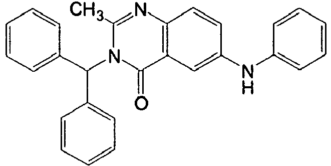 | 418.2 |
| **87** | 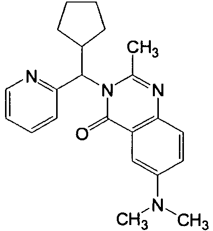 | 363.2 |
| **88** | 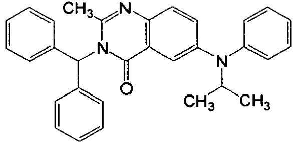 | 460.3 |
| **89** | 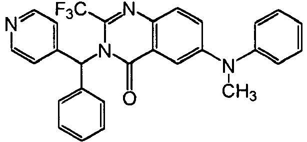 | NA |
| **91** | 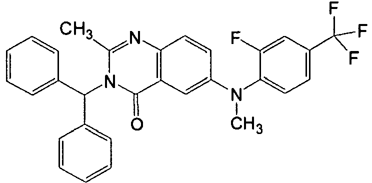 | 518.3 |
| **92** | 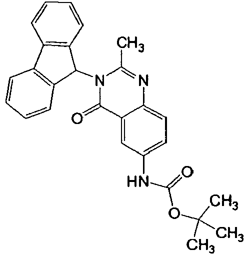 | 440.2 |
| **93** | 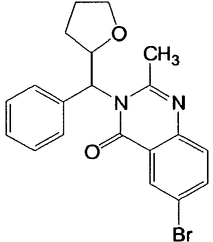 | 400 |
| **94** | 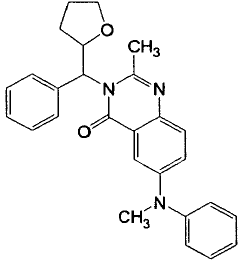 | 426 |
| **95** | 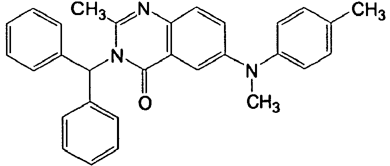 | 446.2 |
| **96** | 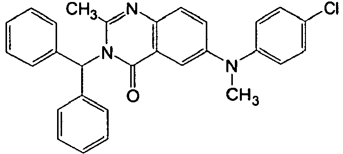 | 466.3 |
| **97** | 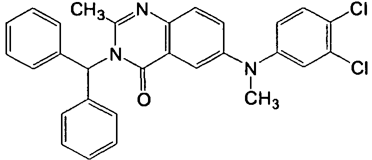 | 500.3 |
| **98** | 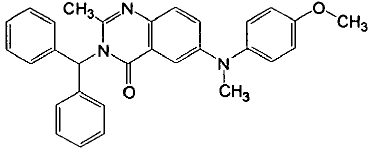 | 462.3 |
| **99** | 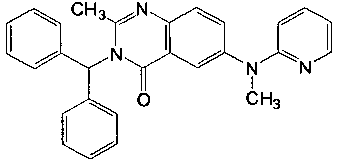 | 433.2 |
| **100** | 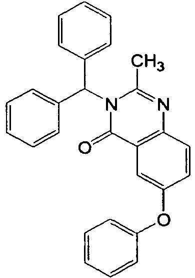 | 419.2 |
| **101** | 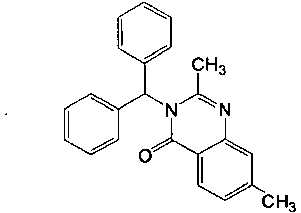 | 341 |
| **102** | 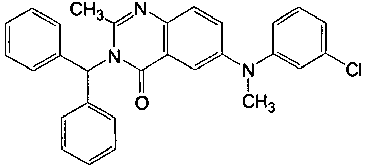 | 466.3 |
| **103** | 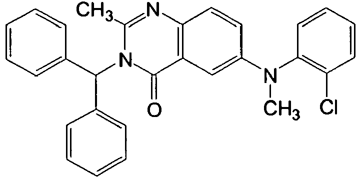 | 466.3 |
| **104** | 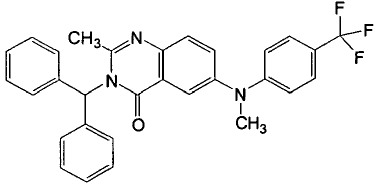 | 500.3 |
| **105** | 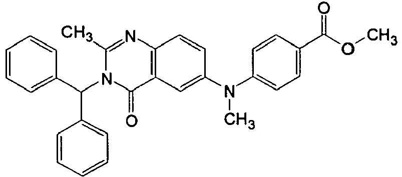 | 490.3 |
| **106** | 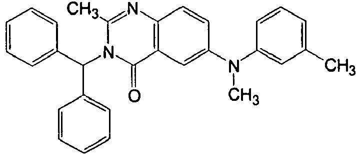 | 446.2 |
| **107** | 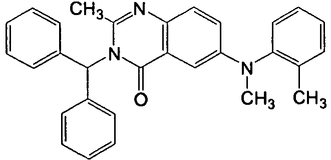 | 446.2 |
| **108** | 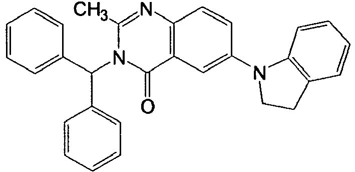 | 444.2 |
| **109** | 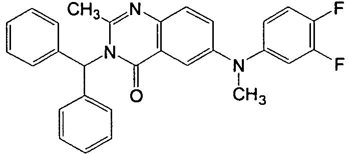 | 468.2 |
| **111** | 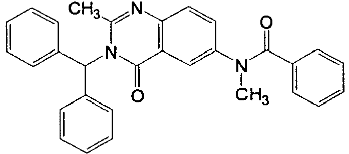 | 460.3 |
| **112** | 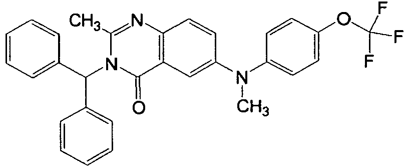 | 516.3 |
| **113** | 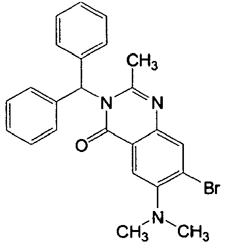 | 449.2 |
| **114** | 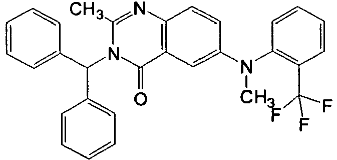 | 500.3 |
| **115** | 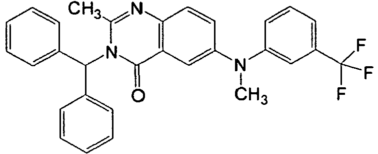 | 500.3 |
| **116** | 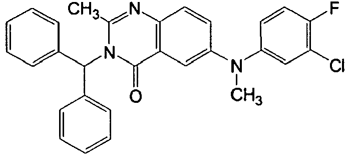 | 484.3 |
| **117** | 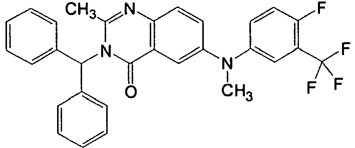 | 518.3 |
| **118** | 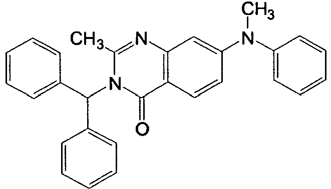 | 432.2 |
| **119** | 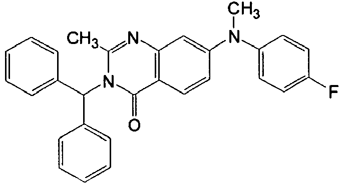 | 450.2 |
| **120** | 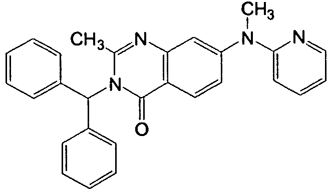 | 433.2 |
| **121** | 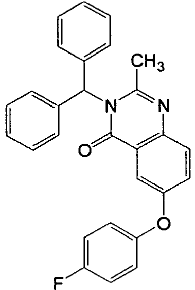 | 437.2 |
| **122** | 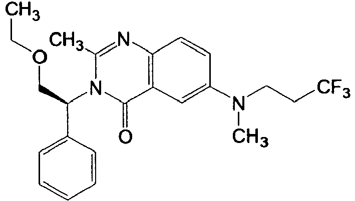 | 434.2 |
| **123** | 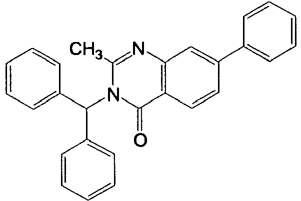 | 403.2 |
| **124** | 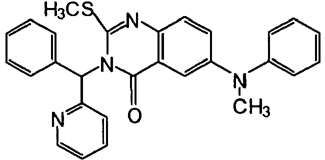 | NA |
| **125** | 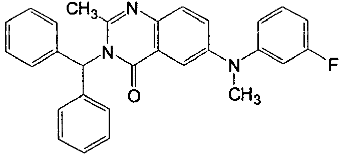 | 450.2 |
| **127** | 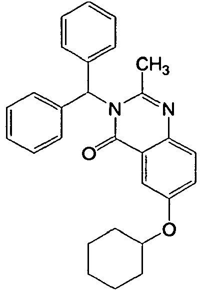 | 425.2 |
| **128** | 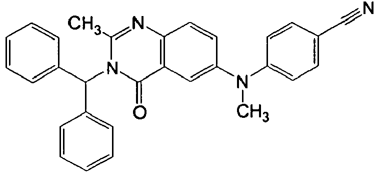 | 457.3 |
| **129** | 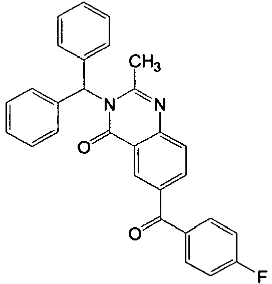 | 449.2 |
| **130** | 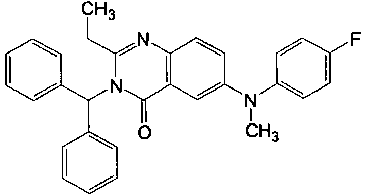 | 464.3 |
| **131** | 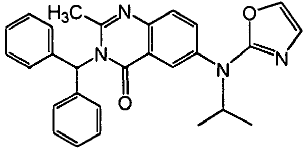 | NA |
| **132** | 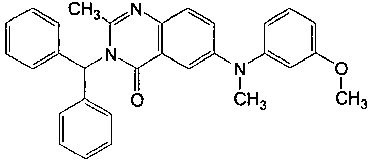 | 462.3 |
| **133** | 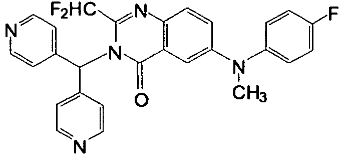 | NA |
| **136** | 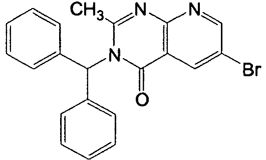 | 406,408 |
| **137** | 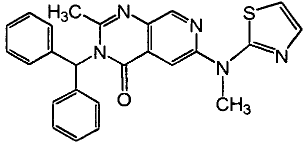 | NA |
| **138** | 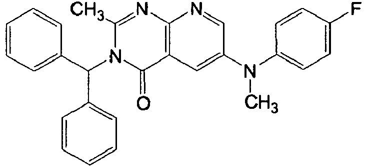 | 451.2 |
| **139** | 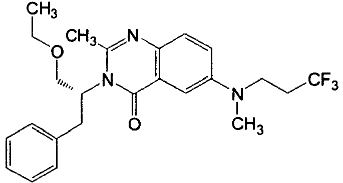 | 434.2 |
| **140** | 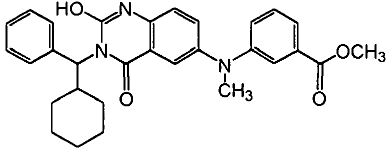 | NA |
| **141** | 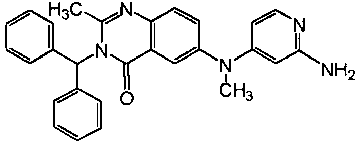 | NA |
| **142** | 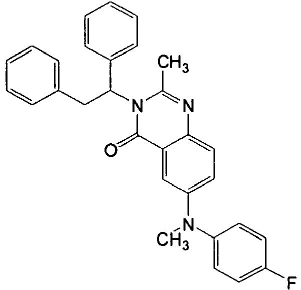 | 464.3 |
| **144** | 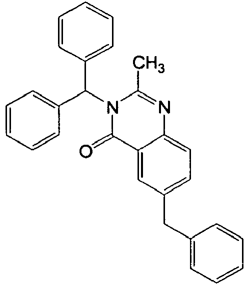 | 417.2 |
| **145** | 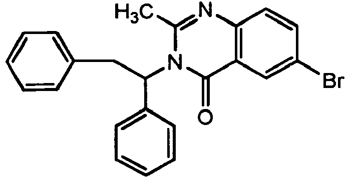 | 419,421 |
| **147** | 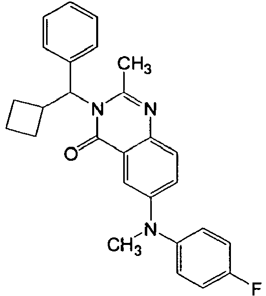 | 428.2 |
| **149** | 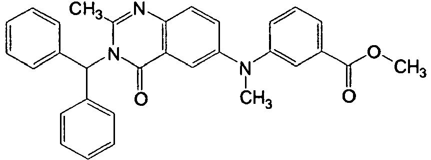 | 490.3 |
| **150** | 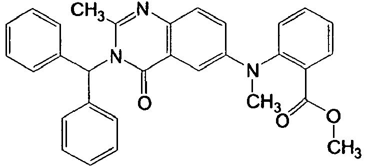 | 490.3 |
| **151** | 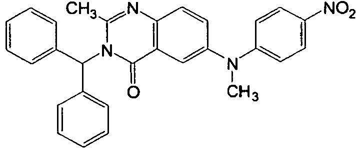 | 477.3 |
| **152** | 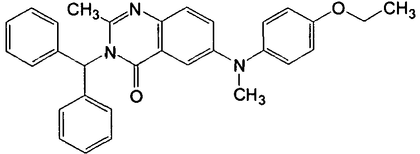 | 476.3 |
| **153** | 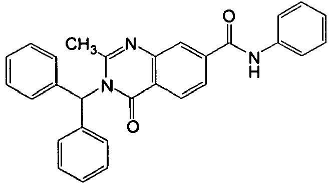 | 446.2 |
| **154** | 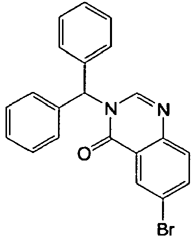 | 391,393 |
| **155** | 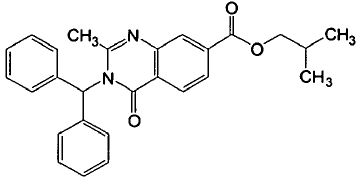 | 427.2 |
| **156** | 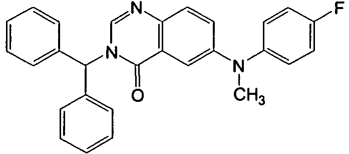 | 436.2 |
| **157** | 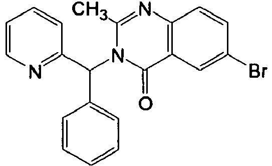 | 406,408 |
| **158** | 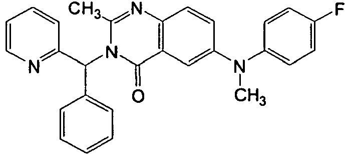 | 451.2 |
| **159** | 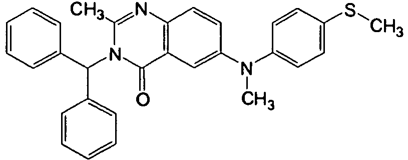 | 478.3 |
| **160** | 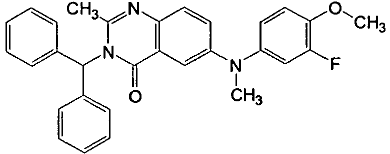 | 480.3 |
| **161** | 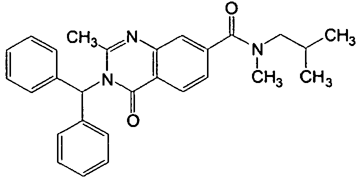 | 440.2 |
| **162** | 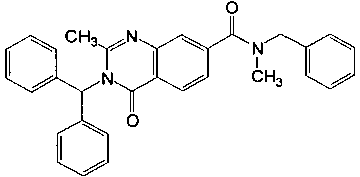 | 474.3 |
| **163** | 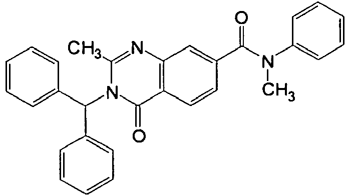 | 460.2 |
| **164** | 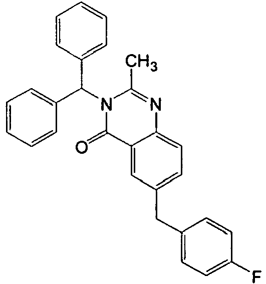 | 435.2 |
| **165** | 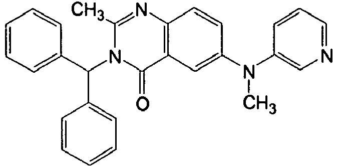 | 433.2 |
| **166** | 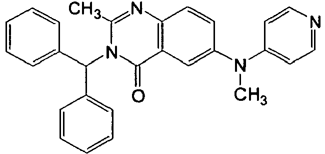 | 433.2 |
| **167** | 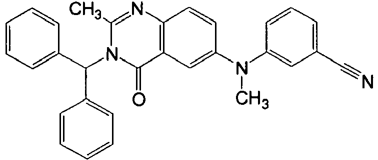 | 457.3 |
| **168** | 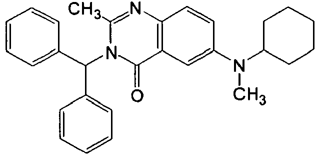 | 438.2 |
| **169** | 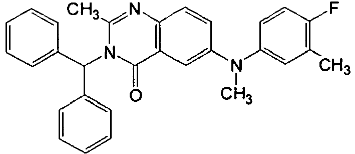 | 464.3 |
| **170** | 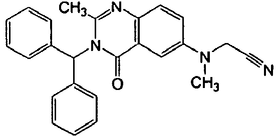 | 395.2 |
| **171** | 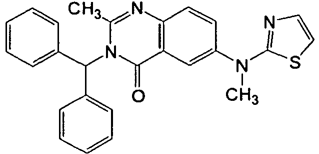 | 439.2 |
| **173** | 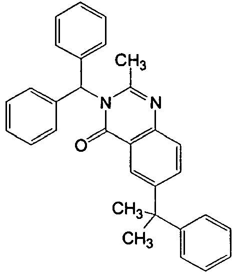 | 445.2 |
| **174** | 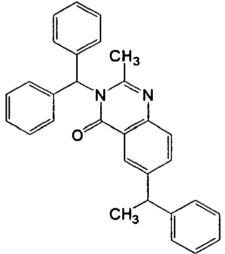 | 431.2 |
| **175** | 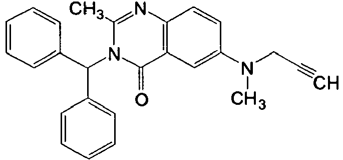 | 394.2 |
| **176** | 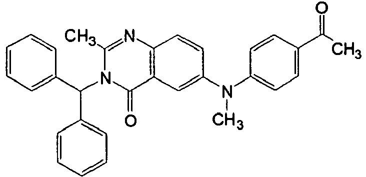 | 474.3 |
| **177** | 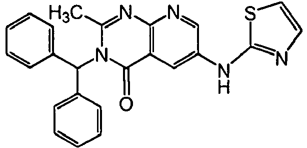 | NA |
| **178** | 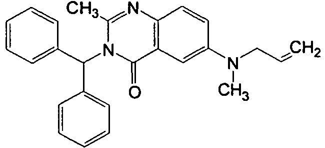 | 396.2 |
| **179** | 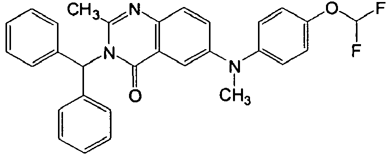 | 498.3 |
| **180** | 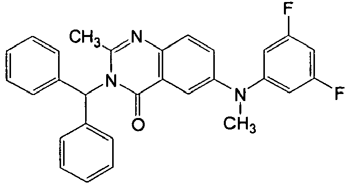 | 468.3 |
| **182** | 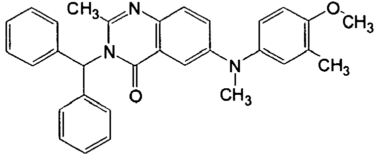 | 476.3 |
| **183** | 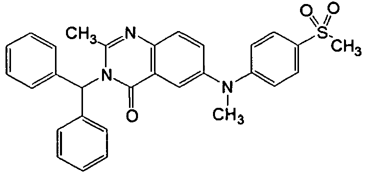 | 510.3 |
| **185** | 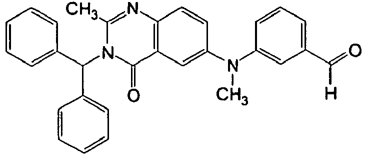 | 460.3 |
| **186** | 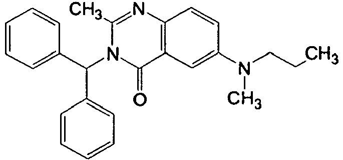 | 398.2 |
| **187** | 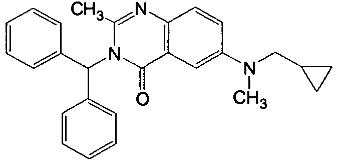 | 410.2 |
| **188** | 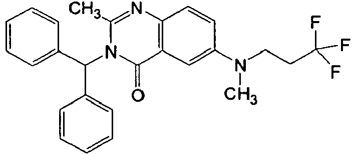 | 452.2 |
| **189** | 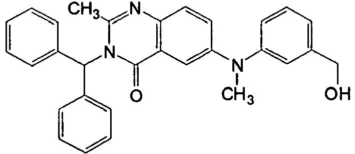 | 462.3 |
| **190** | 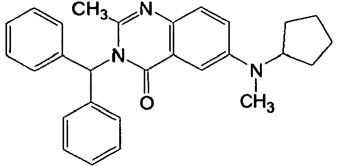 | 424.2 |
| **191** | 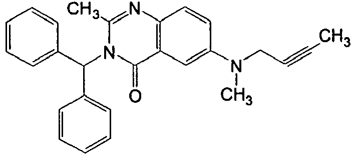 | 408.2 |
| **192** | 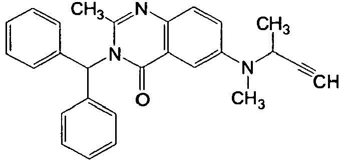 | 408.2 |
| **193** | 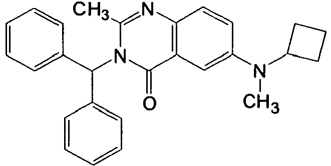 | 410.2 |
| **194** | 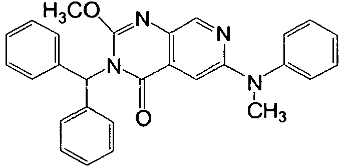 | NA |
| **195** | 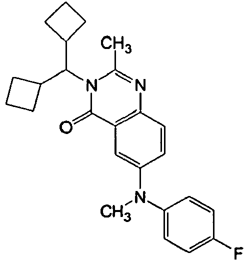 | 406.2 |
| **196** | 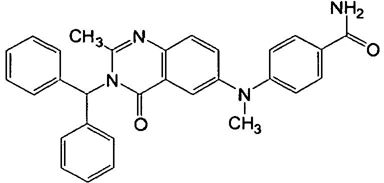 | 475.3 |
| **199** | 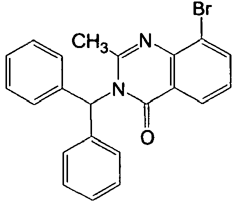 | 405,407 |
| **200** | 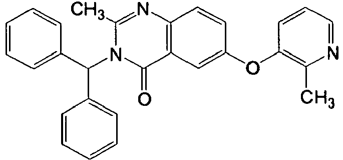 | 432.2 |
| **202** | 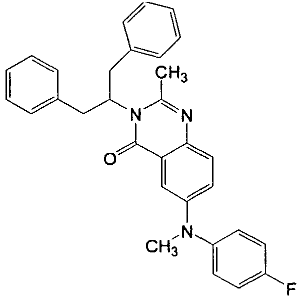 | 510.2 |
| **203** | 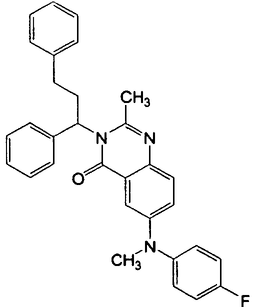 | 478.3 |
| **204** | 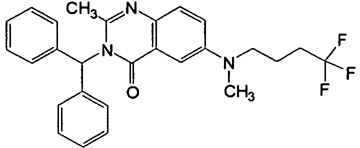 | 478.3 |
| **205** | 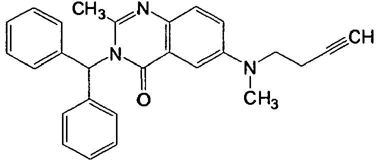 | 466.3 |
| **206** | 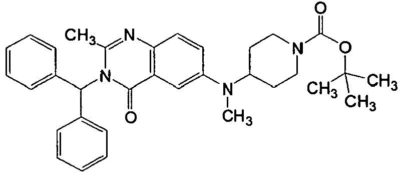 | 408.2 |
| **207** | 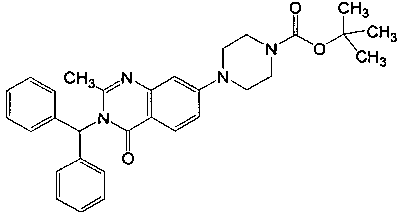 | 539.3 |
| **208** | 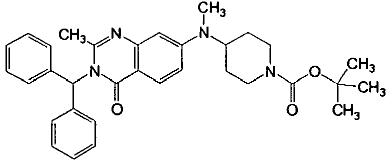 | 511.3 |
| **209** | 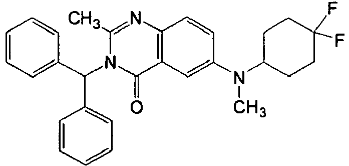 | 539.3 |
| **210** | 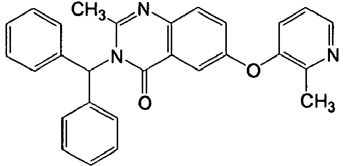 | 474.3 |
| **211** | 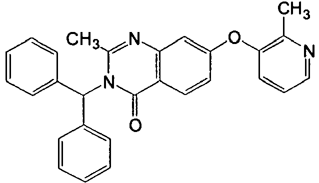 | 434.2 |
| **214** | 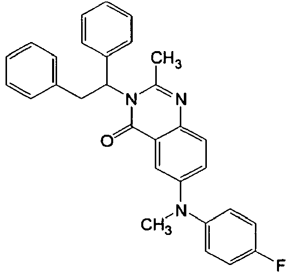 | 464.3 |
| | **Enantiomer A** | |
| **215** | 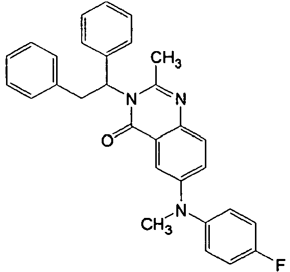 | 464.3 |
| | **Enantiomer B** | |
| **216** | 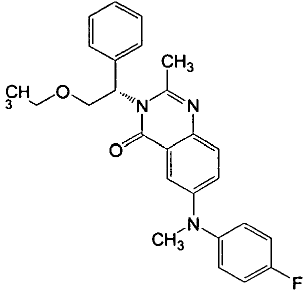 | 432.2 |
| **217** | 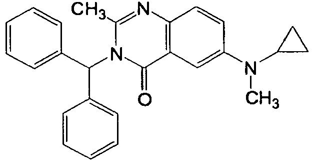 | 396.2 |
| **218** | 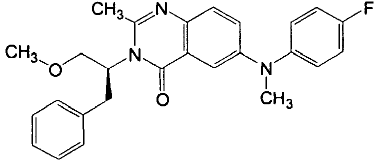 | 432.2 |
| **219** | 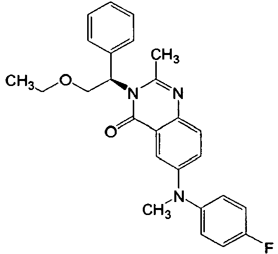 | 432.2 |
| **220** | 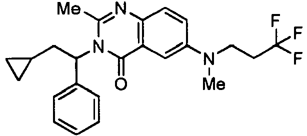 | 430.2 |
| **221** | 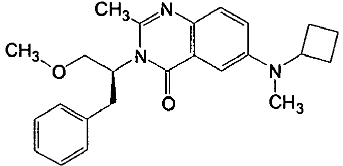 | 392.2 |
| **222** | 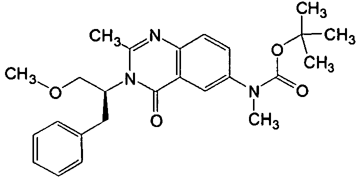 | 438.2 |
| **223** | 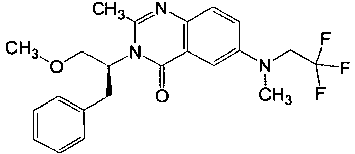 | 420.2 |
| **224** | 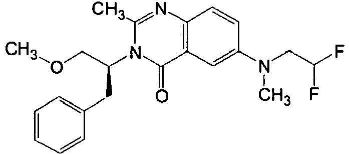 | 402.2 |
| **226** | 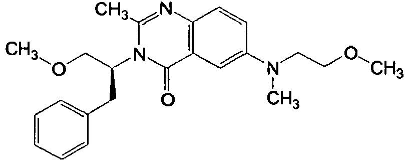 | 396.2 |
| **227** | 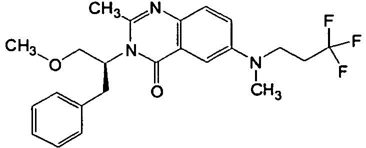 | 434.2 |
| **228** | 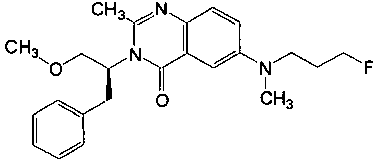 | 398.2 |
| **229** | 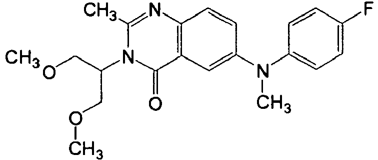 | 386.2 |
| **231** | 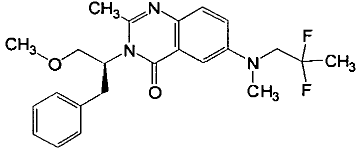 | 416.2 |
| **232** | 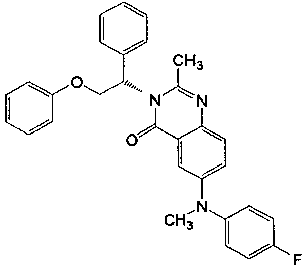 | 480.3 |
| **233** | 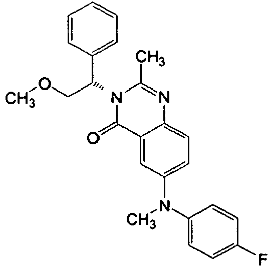 | 418.2 |
| **234** | 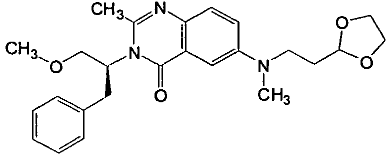 | 438.2 |
| **235** | 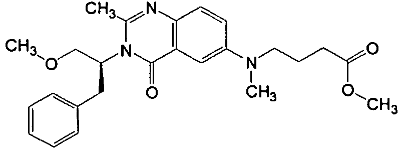 | 438.2 |
| **236** | 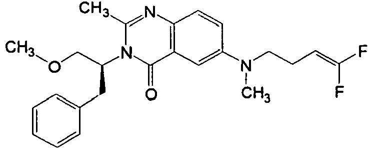 | 428.2 |
| **237** | 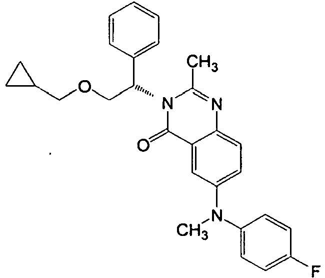 | 458.3 |
| **238** | 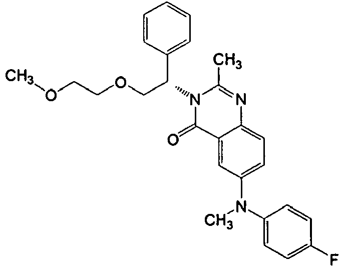 | 462.3 |
| **242** | 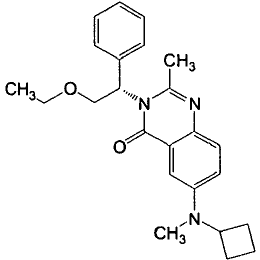 | 392.2 |
| **243** | 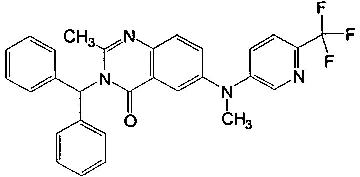 | 501.3 |
| **244** | 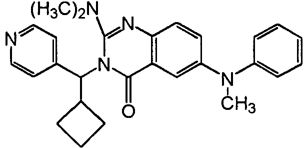 | NA |
| **245** | 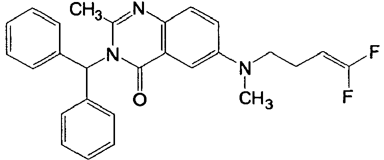 | 446.2 |
| **246** | 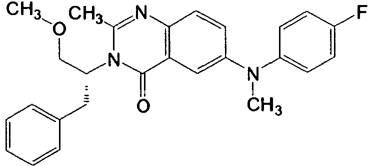 | 432.2 |
| **247** | 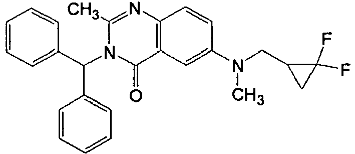 | 446.2 |
| **248** | 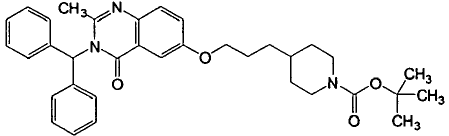 | 568.3 |
| **249** | 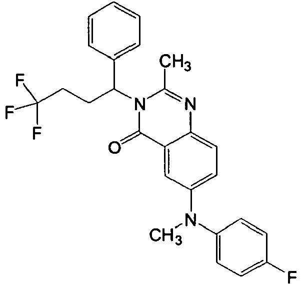 | 470.3 |
| **250** | 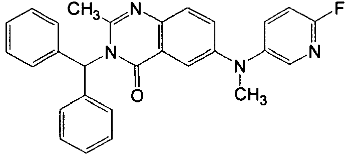 | 451.2 |
| **251** | 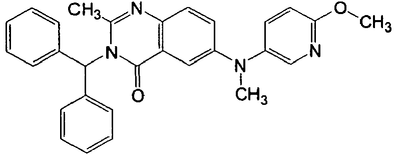 | 463.3 |
| **253** | 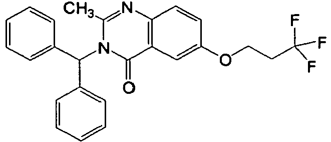 | 439.2 |
| **254** | 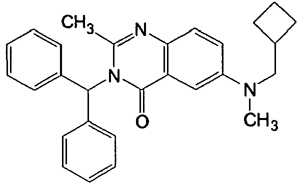 | 424.2 |
| **255** | 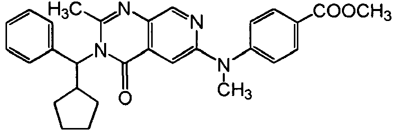 | NA |
| **256** | 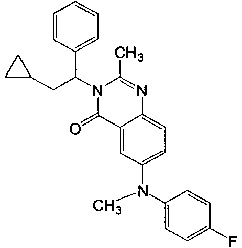 | 428.2 |
| **257** | 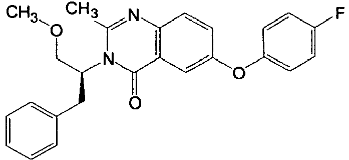 | 419.2 |
| **258** | 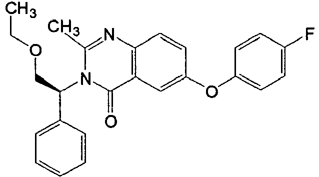 | 419.2 |
| **259** | 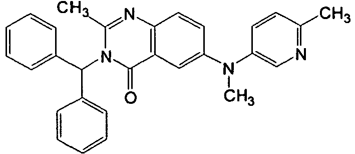 | 447.2 |
| **260** | 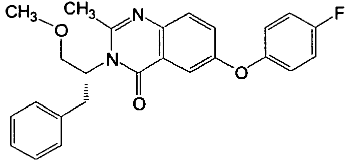 | 419.2 |
| **261** | 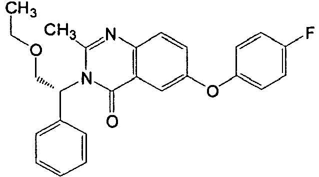 | 419.2 |
| **263** | 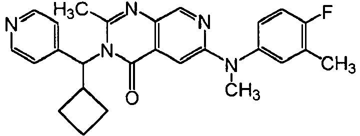 | NA |
| **264** | 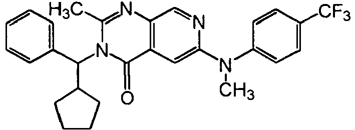 | NA |
| **265** | 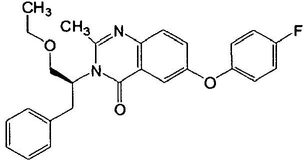 | 433.2 |
| **266** | 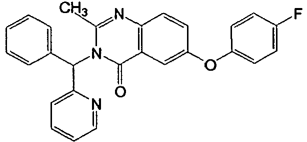 | 438.2 |
| **267** | 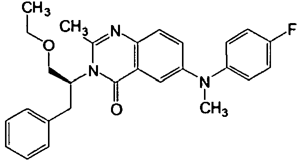 | 446.2 |
| **268** | 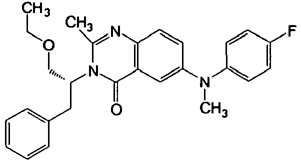 | 446.2 |
| **269** | 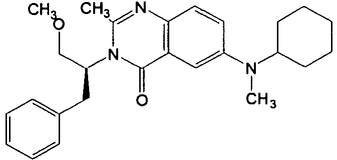 | 420.2 |
| **271** | 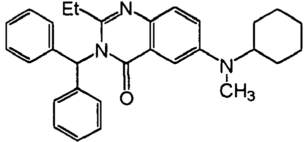 | NA |
| **272** | 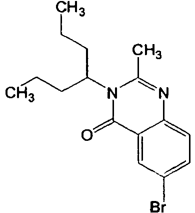 | 337,339 |
| **273** | 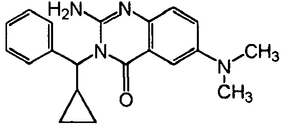 | NA |
| **274** | 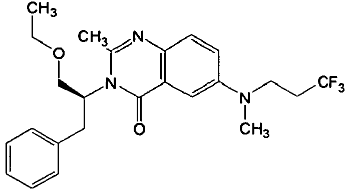 | 448.2 |

and pharmaceutically acceptable salts, solvates, esters, prodrugs and stereoisomers thereof.

### Methods For Making the Bicyclic Heterocycle Derivatives

The Compounds of Formula (I) may be prepared from known or readily prepared starting materials, following methods known to one skilled in the art of organic synthesis. Methods useful for making the Bicyclic Heterocycle Derivatives are set forth in the Examples below and generalized in Schemes **1-8**. Alternative synthetic pathways and analogous structures will be apparent to those skilled in the art or organic synthesis. All stereoisomers and tautomeric forms of the compounds are contemplated.

Scheme 1 shows a method useful for making compound **D** which is a useful intermediate for making Bicyclic Heterocycle Derivatives wherein Y and Z are each CH and R⁵ is Br, I, OH, or OMe.

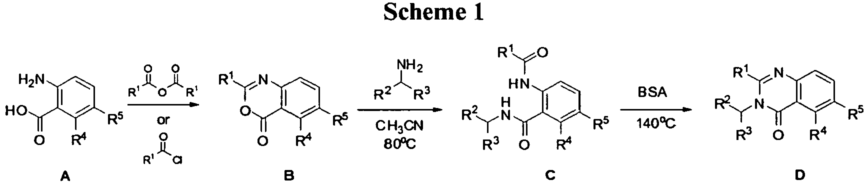

An anthranilic acid of formula **A** can be treated with an anhydride or acid chloride to provide benzoxazinone compounds of formula **B.** The compounds of formula **B** can then be treated with various amines in acetonitrile at 80 °C or toluene at 110 °C to provide the bis-amido compounds of formula **C.** The compounds of formula **C** are then cyclized using N,O-bis(trimethylsilyl)acetamide at 140 °C, or other well-known cyclization methods, to yield compound **D.**

Scheme 2 shows a method for converting intermediate compounds of formula **D** (where R⁵ is halo or a sulfonate, such as trifluormethanesulfonyloxy) to the Bicyclic Heterocycle Derivatives of formula **E.**

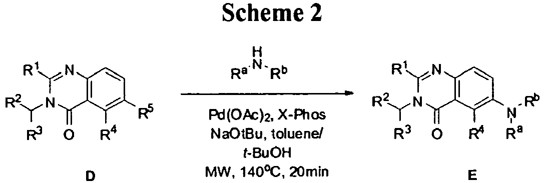

Amination of compound **D** with an amine of formula R^{a}R^{b}NH using Buchwald-Hartwig reaction conditions in the microwave at 140 °C for 20 minutes yields quinazolinone derivatives of formula **E**.

Scheme 3 illustrates an alternative method for making compounds of formula **E.**

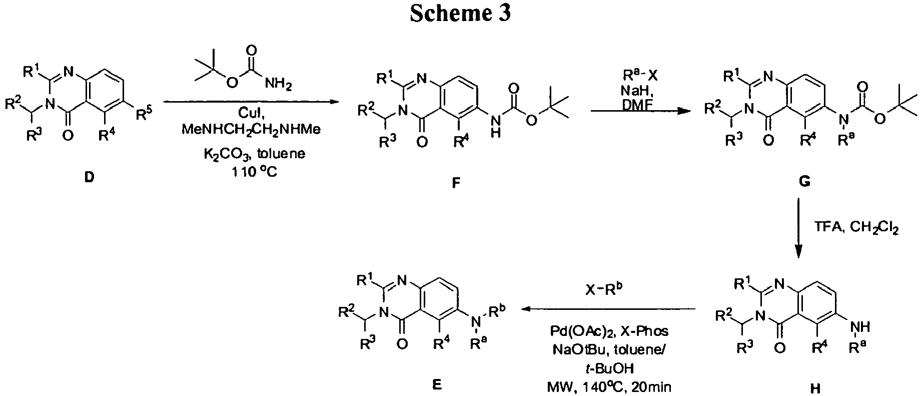

Amidation of compound **D** (where R⁵ is halo or a sulfonate, such as trifluormethanesulfonyloxy) using copper iodide catalyzed coupling of tert-butyl carbamate yields compound **F.** Alkylation with alkyl halide R^{a}-X and sodium hydride in DMF yields compound **G.** Deprotection of the tert-butoxycarbonyl group with TFA yields compound **H.** Reaction of compound **H** with an aryl/heteroaryl halide of formula R^{b}-X using Buchwald-Hartwig reaction conditions, yields the compounds of formula **E.**

Scheme 4 illustrates an alternative method for making compounds of formula **E.**

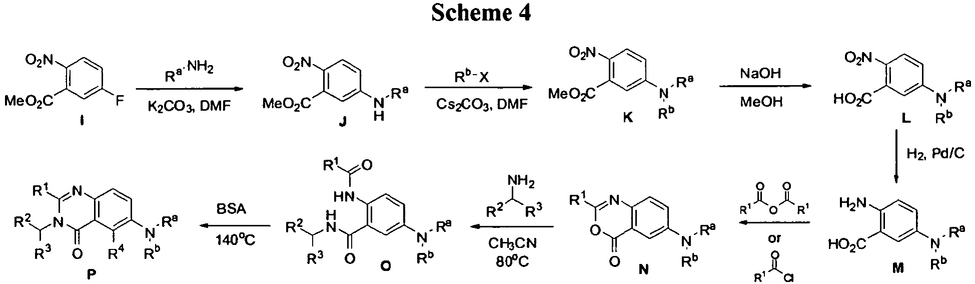

Treatment of methyl 5-fluoro-2-nitrobenzoate with an amine of formula R^{a}-NH₂ in the presence of a base, such as potassium carbonate, yields aniline **J.** Reaction of aniline **J** with an alkyl halide of formula R^{b}-X in the presence of a base, such as potassium carbonate, provides aniline **K.** Hydrolysis of compound **K** followed by reduction of the nitro group yields aniline **M,** which can be further elaborated using methods described in Scheme 1 to provide the Bicyclic Heterocycle Derivatives of formula **E.**

Scheme 5 shows a method for converting intermediate compounds of formula P to Bicyclic Heterocycle Derivatives of formula Q.

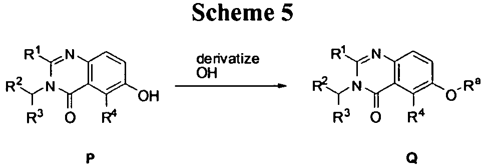

The compounds of formula **P** may be further derivatized using well-known methods to provide the compounds of formula **Q,** which correspond to the Bicyclic Heterocycle Derivatives where in ―OR^{a} is representative of all R⁵ substituents, as defined for the compounds of formula (I), that are connected via an oxygen atom.

Scheme 6 shows a method useful for making Bicyclic Heterocycle Derivatives wherein R¹ is ―NR^{c}R^{d}.

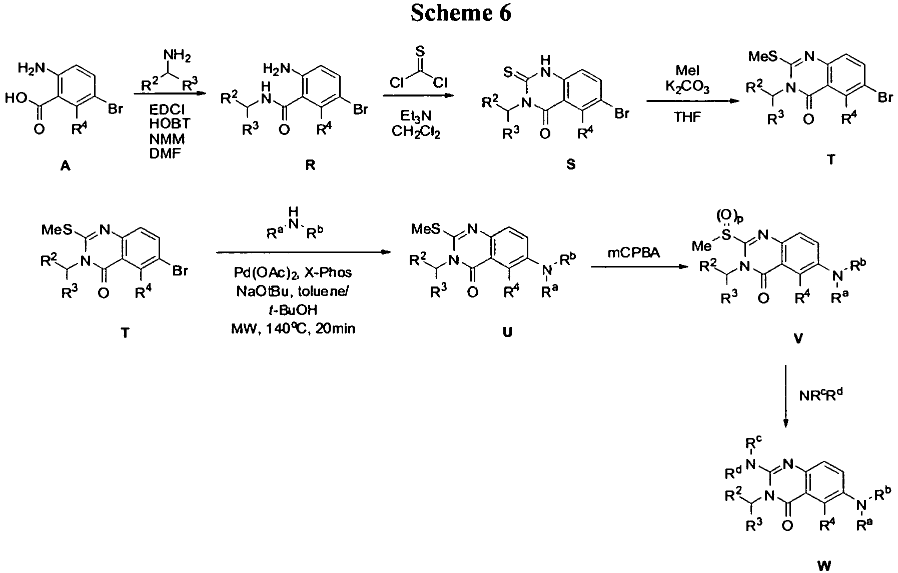

Anthranilic acids of formula **A** can be coupled with amine of formula R²CH(NH₂)R³ using standard amide coupling conditions to yield amide **R.** Cyclization with thiophosgene yields thiourea compound **S.** Compounds of formula **S** can then be alkylated using, for example, an alkyl halide and a base such as K₂CO₃ to provide compounds of formula **T,** which are then coupled with amines R^{a}R^{b}NH using palladium catalysis to provide compounds **U,** which are then oxidized to the corresponding sulfoxide or sulfone compounds of formula V, depending on the choice of oxidizing conditions. Reaction of a sulfoxide or sulfone of formula V with ammonia, an alkyl amine, or dialkylamine provides amines of formula **W.**

Scheme 7 shows a method for making Bicyclic Heterocycle Derivatives of formula **BB** wherein Y is -N- using the methods described in Schemes 1-2, wherein R⁵ is halo or a sulfonate, such as trifluormethanesulfonyloxy.

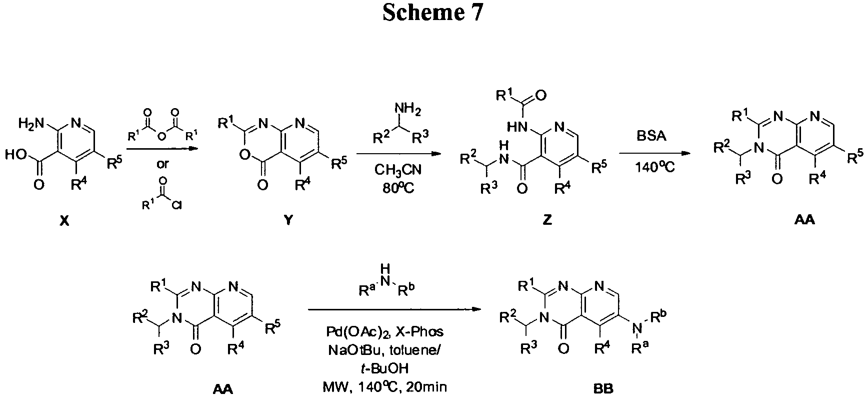

Scheme 8 shows a method useful for making Bicyclic Heterocycle Derivatives **FF** wherein Z is -N-.

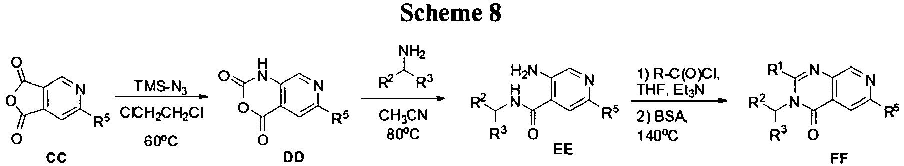

Furo[3,4-c]pyridine-1,3-dione is reacted with TMS-N₃ to provide 1H-pyrido[3,4-d][1,3]oxazine-2,4-dione **DD.** Using methods from Schemes 1-2, compounds of formula **FF** are prepared.

The starting materials and reagents depicted in Schemes 1-8 are either available from commercial suppliers such as Sigma-Aldrich (St. Louis, MO) and Acros Organics Co. (Fair Lawn, NJ), or can be prepared using methods well-known to those of skill in the art of organic synthesis.

One skilled in the art will recognize that the synthesis of compounds of Formula (I) may require the need for the protection of certain functional groups (i.e., derivatization for the purpose of chemical compatibility with a particular reaction condition). Suitable protecting groups for the various functional groups of the compounds of formula (I) and methods for their installation and removal may be found in Greene et. al., Protective Groups in Organic Synthesis, Wiley-Interscience, New York, (1999).

### EXAMPLES

The following examples exemplify illustrative preparations of compounds of the present invention and are not to be construed as limiting the scope of the disclosure. Alternative mechanistic pathways and analogous structures within the scope of the invention may be apparent to those skilled in the art.

### General Methods

Solvents, reagents, and intermediates that are commercially available were used as received. Reagents and intermediates that are not commercially available were prepared in the manner described below. ¹H NMR spectra were obtained on a Gemini AS-400 (400 MHz) and are reported as ppm down field from Me₄Si with number of protons, multiplicities, and coupling constants in Hertz indicated parenthetically. Where LC/MS data are presented, analyses was performed using an Applied Biosystems API-100 mass spectrometer and Shimadzu SCL-10A LC column: Altech platinum C18, 3 micron, 33 mm x 7mm ID; gradient flow: 0 minutes- 10% CH₃CN, 5 minutes- 95% CH₃CN, 7 minutes- 95% CH₃CN, 7.5 minutes- 10% CH₃CN, 9 minutes- stop. The retention time and observed parent ion are given.

### Example 1

### Preparation of Compound 23

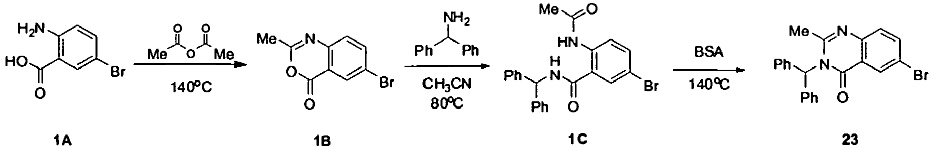

### Step A - Synthesis of Compound 1B

A solution of 2-amino-5-bromobenzoic acid (1A, 10g, 46mmol) in acetic anhydride (65mL) was heated to 140°C for 1 hour. Allowed to cool and removed excess acetic anhydride under vacuum to obtain the benzoxazinone 1B as a tan solid.

### Step B - Synthesis of Compound 1C

To the product of Step A, acetonitrile (75mL) was added, aminodiphenylmethane (7.9mL, 46.3mmol) was added, and the solution was heated to 80°C for 16 hours. Toluene can be substituted for acetonitrile. Allowed reaction to cool, solid precipitated from solution, collected solid by vacuum filtration, and dried solid under vacuum to give bis-amide 1C as a tan solid.

### Step C - Synthesis of Compound 23

To the product of Step B, BSA (40mL) was added and the solution was heated to 140°C for 16 hours. Allowed to cool, removed excess BSA under vacuum, slowly added methanol (50mL) and stirred for 1 hour. Removed methanol under vacuum and purified using flash column chromatography on silica gel (30% EtOAc-hexanes) to provide compound **23** as a white solid (8.6g, overall yield 46%).

### Example 2

### Preparation of Compound 24

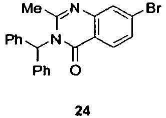

Compound 24 was synthesized using the method described in Example 1 and starting from 2-amino-4-bromobenzoic acid.

### Example 3

### Preparation of Compound 21

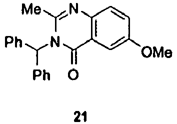

Compound 21 was synthesized using the method described in Example 1 and starting from 2-amino-5-methoxybenzoic acid.

### Example 4

### Preparation of Compound 28

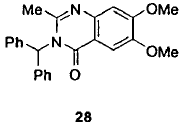

Compound 28 was synthesized using the method described in Example 1 and starting from 2-amino-4,5-dimethoxybenzoic acid.

### Example 5

### Preparation of Compound 31

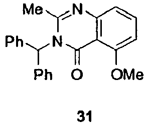

Compound 31 was synthesized using the method described in Example 1 and starting from 2-amino-6-methoxybenzoic acid.

### Example 6

### Preparation of Compound 53

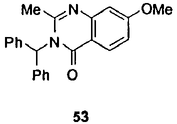

Compound 53 was synthesized using the method described in Example 1 and starting from 2-amino-4-methoxybenzoic acid.

### Example 7

### Preparation of Compound 71

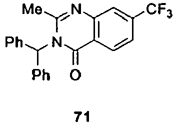

Compound 71 was synthesized using the method described in Example 1 and starting from 2-amino-4-(trifluoromethyl)benzoic acid.

### Example 8

### Preparation of Compound 101

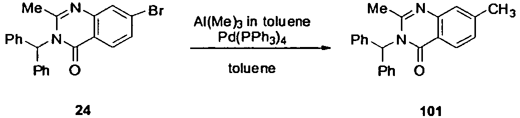

To a solution of compound 24 (0.08g, 0.2 mmol) in toluene (2 mL) was added Pd(PPh₃)₄ (0.01 g, 0.01 mmol) and Al(Me)₃ (2.0M in toluene, 0.1 mL, 0.2 mmol) and the resulting solution was heated to 110°C and stirred for 2 hours. The reaction was allowed to cool and was filtered to remove precipitated palladium. The filtrate was dried (MgSO₄), filtered and concentrated. The resulting residue was purified using preparative TLC (%MeOH/CH₂Cl₂) to provide compound 101 as a light yellow solid (0.06g, 9%).

### Example 9

### Preparation of Compound 145

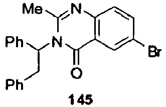

Using the method described in Example 1, and substituting 1,2-diphenylethanamine for aminodiphenylmethane, compound 145 was prepared.

### Example 11

### Preparation of Compound 56

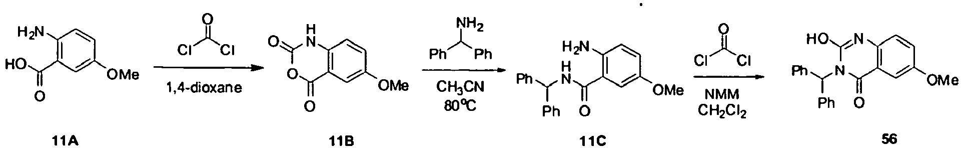

### Step A ― Synthesis of Compound 11B

To a solution of 2-amino-5-methoxybenzoic acid (11A, 1.34g, 8.00mmol) in 1,4-dioxane (12mL) was added phosgene (20% in toluene, 1.04g, 10.5mmol). The resulting solution was allowed to stir for 1 hour. A solid precipitate formed and was filtered, rinsed with Et₂O, and dried to yield compound 11B as a purple solid (0.63g, 40%).

### Step B - Synthesis of Compound 11C

To a solution of 11B (0.63g, 3.3mmol) in acetonitrile (10 mL) was added aminodiphenylmethane (0.71g, 3.9mmol), and the solution was heated to 80 °C for 2.5 hours. The reaction was allowed to cool to room temperature, then partitioned between EtOAc (40mL) and 5% aqueous citric acid (40 mL). The EtOAc layer was separated, washed with H₂O, washed with saturated aqueous NaHCO₃, washed with brine, dried (MgSO₄), filtered, and concentrated *in vacuo* to yield compound 11C as a tan solid (0.86g, 78%).

### Step C ― Synthesis of Compound 56

To the product of Step B was added CH₂Cl₂ (3mL), NMM (0.07g, 0.70mmol), phosgene (20% in toluene, 0.15g, 0.30mmol) was added, and the solution was stirred for 16 hours. The solution was partitioned with EtOAc (10mL) and H₂O (10mL), the EtOAc layer was washed with 5%aqueous citric acid, washed with H₂O, washed with saturated aqueous NaHCO₃, washed with brine, dried (MgSO₄), filtered, and concentrated. The resulting residue was purified using preparative TLC (2%MeOH/CH₂Cl₂) to provide compound 56 as a white solid (0.005g, 7%).

### Example 12

### Preparation of Compound 60

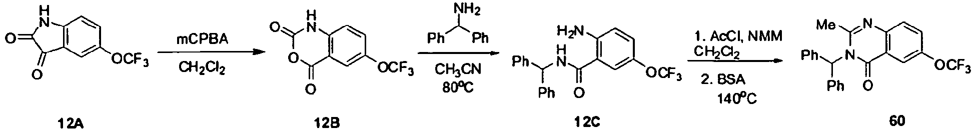

### Step A - Synthesis of Compound 12B

To a solution of 5-trifluoromethoxyisatin (12A, 0.46g, 2.00mmol) in CH₂Cl₂ (15mL) was added mCPBA (70% pure, 0.54g, 2.2mmol). The resulting solution was allowed to stir for 30 minutes. A solid precipitate formed and was filtered, rinsed with CH₂Cl₂, and dried to yield compound 12B as a solid (0.39g, 79%).

### Step B - Synthesis of Compound 12C

To the product of Step A (0.37g, 1.5mmol) was added acetonitrile (8mL), aminodiphenylmethane (0.29g, 1.58mmol), and the solution was heated to 80°C for 1 hour. Allowed reaction to cool, partitioned between EtOAc (40mL) and 5%aqueous citric acid (40mL), the EtOAc layer was separated, washed with H₂O, washed with saturated aqueous NaHCO₃, washed with brine, dried (MgSO₄), filtered and concentrated to yield compound 12C as solid (0.39g, 67%).

### Step C - Synthesis of Compound 60

To the product of Step B (0.39 g, 1.0 mmol) was added CH₂Cl₂ (4 mL), NMM (0.13 g, 1.3 mmol), and acetyl chloride (0.115 g, 1.3 mmol) was added and the solution was stirred for 30 minutes. The solution was partitioned with CH₂Cl₂ (10mL) and H₂O (10mL), the organic layer was washed with H₂O, washed with saturated aqueous NaHCO₃, washed with brine, dried (MgSO₄), filtered and concentrated. The resulting residue was purified by recrystallization from Et₂O to provide the bis-amide product as a white solid (0.32g, 75%). The resulting product (0.16g, 0.37mmol), was subjected to the method of Step C from Example 1 to provide compound 60 as a white solid (41mg, 27%).

### Example 13

### Preparation of Compound 220

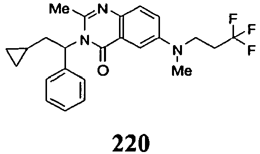

Using Steps B and C from Example 1, substituting compound 248B for compound 1B and substituting compound 99B for aminodiphenylmethane, compound 220 was prepared.

### Example 14

### Preparation of Compound 154

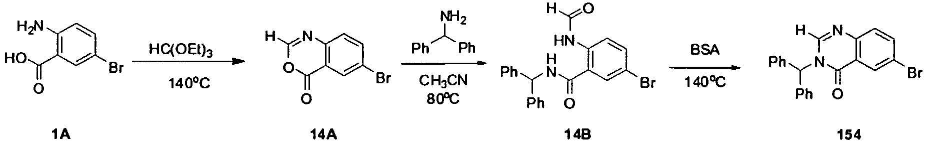

### Step A - Synthesis of Compound 14A

A solution of 2-amino-5-bromobenzoic acid (1A, 1.0g, 4.6mmol) in triethylorthoformate (8mL) was heated to 140°C for 4 hours. Allowed solution to cool and concentrated under vacuum to yield compound 14A as a yellow foam (1.0g, 96%)

### Step B - Synthesis of Compound 14B

To the product of Step A, using Step B described in Example 1, compound 14B was prepared.

### Step C - Synthesis of Compound 154

Using Step C described in Example 1, compound 154 was prepared from compound 14B as a white solid (0.7g, 39% overall yield).

### Example 15

### Preparation of Compound 19

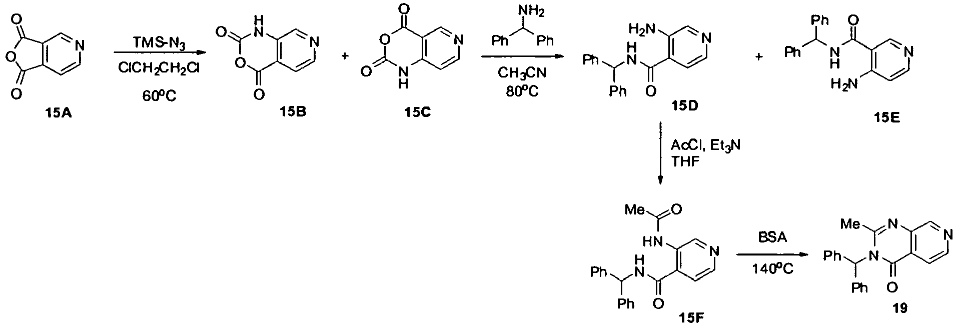

### Step A - Synthesis of Compounds 15B and 15C

To a solution of 3,4-pyridinedicarboxylic anhydride (5.00 g, 33.5 mmol) in 1,2-dichloroethane (25 mL) was added TMS-N₃ (5.02 mL, 38.2 mmol) and the resulting mixture was gently heated to 60°C and allowed to stir at this temperature for 1 hour. Allowed solution to cool and added 2 mL of EtOH and stirred for 15 minutes. Solid precipitate formed and was filtered, washed with CH₂Cl₂ and dried. To the crude product was added CH₃CN (80 mL) and the resulting reaction was heated to 80 °C and allowed to stir at this temperature for 2 hours, then the warm reaction mixture was filtered and the mother liquor was concentrated *in vacuo* to yield a mixture of compounds 15B and 15C (1.78 g, 32%).

### Step B - Synthesis of Compounds 15D and 15E

To a mixture of compounds 15B and 15C from step A (1.78g, 10.8mmol) in THF (40mL) was added aminodiphenylmethane (1.98g, 10.8mmol) and the solution was heated to 60°C for 1 hour. Allowed the reaction to cool and concentrated to give a mixture of 15D and 15E. The crude mixture was purified by using preparative TLC (3%MeOH/CH₂Cl₂) to provide compound 15D (TLC Rf= 0.3, 0.70g, 21 %) and compound 15E (TLC Rf = 0.2, 0.93g, 28%).

### Step C - Synthesis of Compound 15F

To compound 15D (0.70g, 2.31mmol) was added THF (10mL), Et₃N (0.64mL, 4.62mmol) and acetyl chloride (0.25mL, 3.46mmol), and the solution was stirred and heated to 60°C for 12 hours. Allowed the reaction to cool, partitioned between Et₂O and saturated aqueous NaHCO₃, ether layer was dried (MgSO₄), filtered, and concentrated to yield compound 15F as a yellow solid (0.80g, 100%).

### Step D - Synthesis of Compound 19

Using Step C described in Example 1 and using 15F as a starting material, compound 19 was prepared as a white solid (0.2g, 26% yield).

### Example 16

### Preparation of Compound 40

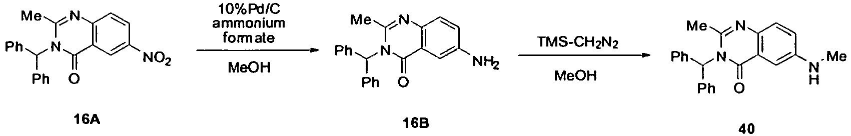

### Step A - Synthesis of Compound 16A

Compound 16A was synthesized using steps A, B, and C described in Example 1 starting from 2-amino-5-nitrobenzoic acid.

### Step B - Synthesis of Compound 16B

To a solution of compound 16A (0.12g, 0.32mmol) in MeOH was added ammonium formate (0.12g, 1.94mmol), and 10% Pd/C (70 mg), and the solution was stirred and heated to 60°C for 1 hour. Allowed reaction to cool, removed Pd/C by filtration through celite, and concentrated the filtrate to yield compound 16B as a yellow solid.

### Step C - Synthesis of Compound 40

To a solution of compound 16B in MeOH (5mL) was added trimethylsilyldiazomethane (1.3mL) of a 2.0M solution in hexanes) and the solution was stirred for 12 hours. Reaction was concentrated under vacuum and the crude mixture was purified by using preparative TLC (2%MeOH/CH₂Cl₂) to provide compound 40 as a yellow solid (0.025g, 22%).

### Example 17

### Preparation of Compound 59

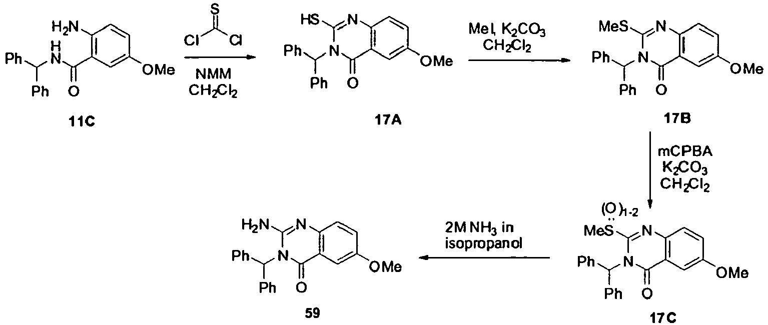

### Step A - Synthesis of Compound 17A

To compound 11C (0.32g, 0.95mmol from Example 11) was added CH₂Cl₂ (10mL), NMM (0.16mL, 1.5mmol), and the solution was cooled to 0 °C, and thiophosgene (0.095mL, 1.2mmol) was added and the solution was stirred for 2 hours at 0°C and 1h at room temperature. The solution was partitioned with CH₂Cl₂ (20mL) and saturated aqueous NaHCO₃ (20mL), the CH₂Cl₂ layer was dried (MgSO₄), filtered and concentrated. The resulting residue was triturated with MeOH to provide compound 17A as a yellow solid (0.20g, 56%).

### Step B - Synthesis of Compound 17B

To compound 17A (0.19g, 0.51mmol) was added CH₂Cl₂ (3mL), K₂CO₃ (0.085g, 0.62mmol), iodomethane (0.04mL, 0.62mmol), and the solution was stirred for 12 hours. Removed the excess K₂CO₃ by filtration and concentrated the filtrate to yield compound 17B as a yellow solid (0.20g, 100%).

### Step C - Synthesis of Compound 17C

To a solution of compound 17B (0.06g, 0.15mmol) in CH₂Cl₂ was added K₂CO₃ (0.04g, 0.31mmol) followed by mCPBA (0.053g, 0.31mmol) and the resulting solution was stirred for 3 hours. The excess solid was removed by filtration and the filtrate was concentrated to yield compound 17C.

### Step D - Synthesis of Compound 59

To the product from Step C was added 2M NH₃ in isopropanol (4mL) in a sealed tube, sealed tube, stirred and heated to 100°C for 12 hours. Reaction was concentrated and the crude mixture was purified by using preparative TLC (30%EtOAc/hexanes) to provide compound 59 as a solid (0.012g, 22%).

### Example 18

### Preparation of Compound 80

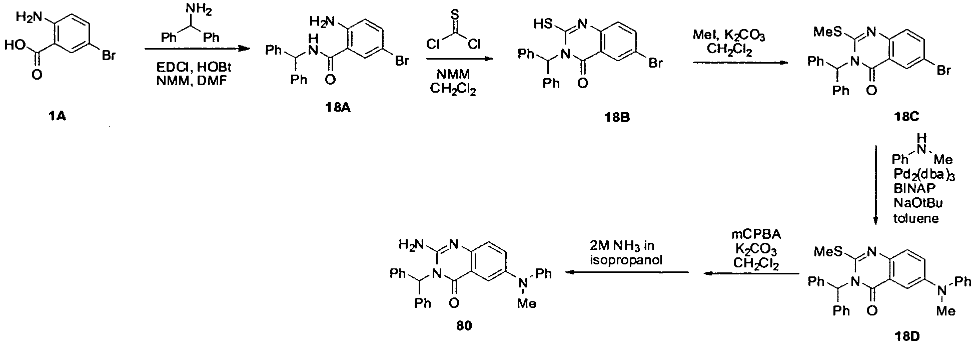

### Step A - Synthesis of Compound 18A

To compound 1A (1.50g, 6.94mmol) was added EDCI (1.60g, 8.33mmol) followed by HOBt (1.12g, 8.33mmol) followed by DMF (15mL) followed by NMM (0.91ml, 8.33mmol) and aminodiphenylmethane (1.4mL, 8.33mmol) and the solution was stirred for 12 hours. Removed DMF under vacuum, residue partitioned between EtOAc (50mL) and H₂O (50mL), washed EtOAc layer with saturated aqueous NaHCO₃, dried organic layer (MgSO₄), filtered and concentrated to yield compound 18A as a yellow solid (1.5g, 55%).

### Step B - Synthesis of Compound 18C

Using Steps A and B from Example 17, and starting with compound 18A, compound 18C was prepared.

### Step C - Synthesis of Compound 18D

To a solution of compound 18C (0.12g, 0.27mmol) in toluene (2mL) was added *N-*methylaniline (0.12mL, 1.08mmol), Pd₂(dba)₃ (0.013g, 0.014mmol), BINAP (0.017g, 0.027mmol), NaOtBu (0.039g, 0.41mmol), and the solution was heated in a microwave at 120°C for 2 hours. The crude mixture was purified by using preparative TLC (10%EtOAc/hexanes) to provide compound 18D as a yellow solid (0.093g, 74%).

### Step D - Synthesis of Compound 80

Using Steps C and D from Example 17, and starting with compound 18D, compound 80 was prepared.

### Example 22

### Preparation of Compound 3

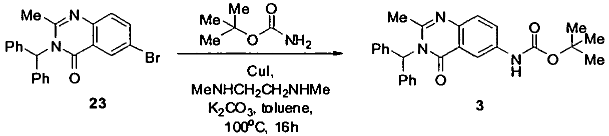

To a solution of compound 23 (250 mg, 0.62mmol) was added tert-butylcarbamate (87 mg, 0.74mmol), CuI (12 mg, 0.062mmol), K₂CO₃ (171mg, 1.24mmol), toluene (1mL), MeNHCH₂CH₂NHMe (0.007mL, 0.062mmol) in a sealed tube. Tube was sealed and heated to 100°C for 16 hours. Allowed to cool, filtered solution through a pad of celite and concentrated filtrate. Purified residue using preparative TLC (30%acetone/hexanes) to yield compound 3 as a solid (240 mg, 88%).

### Example 23

### Preparation of Compound 27

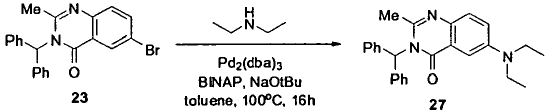

To a solution of compound 23 (150 mg, 0.37mmol) was added Pd₂(dba)₃ (17 mg, 0.019mmol), BINAP (23 mg, 0.037mmol), NaOtBu (54 mg, 0.56mmol), toluene (5mL), and diethylamine (0.058mL, 0.56mmol) in a sealed tube. Solution was stirred and heated at 100°C for 16 hours. Allowed to cool and purified solution using preparative TLC (30%acetone/hexanes) to yield compound 27 (7 mg, 5%).

### Example 24

### Preparation of Compound 29

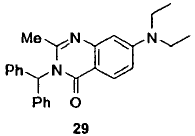

Using Step A from Example 23, and substituting compound 24 for compound 23, compound 29 was prepared.

### Example 25

### Preparation of Compound 10

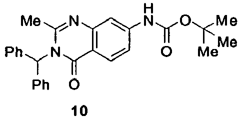

Using Step A from Example 22, and substituting compound 24 for compound 23, compound 10 was prepared.

### Example 26

### Preparation of Compound 30

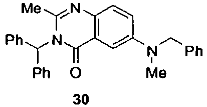

Using Step A from Example 23, and substituting N-methylbenzylamine for diethylamine, compound **30** was prepared.

### Example 27

### Preparation of Compound 33

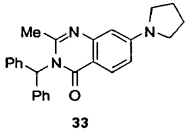

Using Step A from Example 23, and substituting compound 24 for compound 23 and substituting pyrrolidine for diethylamine, compound 33 was prepared.

### Example 28

### Preparation of Compound 34

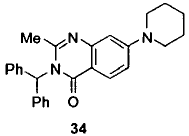

Using Step A from Example 23, and substituting compound 24 for compound 23 and substituting piperidine for diethylamine, compound 34 was prepared.

### Example 29

### Preparation of Compound 35

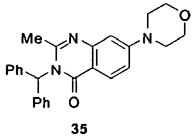

Using Step A from Example 23, and substituting compound 24 for compound 23 and substituting morpholine for diethylamine, compound 35 was prepared.

### Example 30

### Preparation of Compound 36

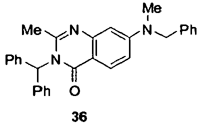

Using Step A from Example 23, and substituting compound 24 for compound 23 and substituting N-methylbenzylamine for diethylamine, compound 36 was prepared.

### Example 31

### Preparation of Compound 42

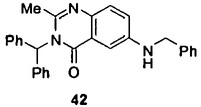

Using Step A from Example 23, and substituting benzylamine for diethylamine, compound 42 was prepared.

### Example 32

### Preparation of Compound 49

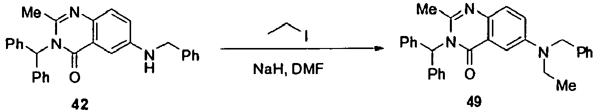

To a solution of compound 42 (50 mg, 0.12 mmol) in DMF (1 mL) was added NaH (60% dispersion in oil, 6.0 mg, 0.14 mmol) and the solution was stirred for 10 minutes. To the solution was added ethyl iodide (0.013 mL, 0.16 mmol) and the solution was stirred for 2 hours. Concentrated under vacuum. Purified residue by preparative TLC (30% acetone/hexanes) to yield compound 49 (21mg, 38%).

### Example 33

### Preparation of Compound 52

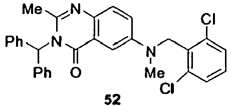

Using Step A from Example 23, and substituting N-methyl-2,6-dichlorobenzylamine hydrochloride for diethylamine, compound 52 was prepared.

### Example 34

### Preparation of Compound 54

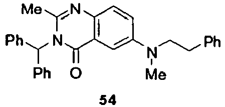

Using Step A from Example 23, and substituting N-methylphenethylamine for diethylamine, compound 54 was prepared.

### Example 35

### Preparation of Compound 50

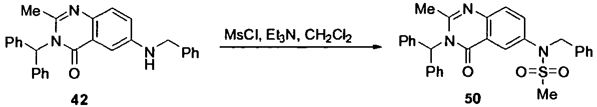

To a solution of compound 42 (20 mg, 0.046mmol) and Et₃N (0.01mL, 0.069mmol) in CH₂Cl₂ (0.5mL) was added methanesulfonyl chloride (0.005mL), 0.06mmol) and the solution was stirred for 1 hour. Purified solution by preparative TLC (30%acetone/hexanes) to yield compound 50 (4 mg, 17%).

### Example 36

### Preparation of Compound 51

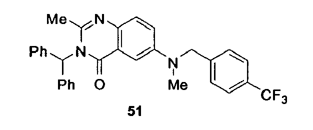

Using Step A from Example 23, and substituting *N*-methyl-(4-trifluoromethyl)benzylamine for diethylamine, compound 51 was prepared.

### Example 37

### Preparation of Compound 58

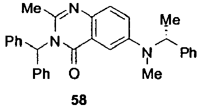

Using Step A from Example 23, and substituting (R)-*N*-methyl-α-methylbenzylamine for diethylamine, compound 58 was prepared.

### Example 38

### Preparation of Compound 61

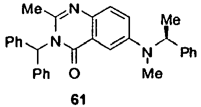

Using Step A from Example 23, and substituting (S)-*N*-methyl-α-methylbenzylamine for diethylamine, compound 61 was prepared.

### Example 39

### Preparation of Compound 65

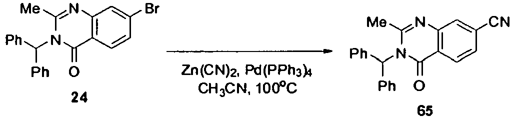

To a solution of compound 24 (300 mg, 0.74mmol) and Pd(PPh₃)₄ (43 mg, 0.04 mmol) in CH₃CN (1 mL) was added Zn(CN)₂ (174 mg, 1.48 mmol) in a sealed tube. Stirred and heated to 100°C for 16 hours. Allowed to cool and purified solution using preparative TLC (30% acetone/hexanes) to yield compound 65 (15 mg, 6%).

### Example 40

### Preparation of Compound 64

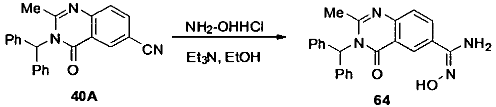

### Step A - Synthesis of Compound 40A

Using Step A from example 39, substituting compound 23 for compound 24, compound 40A was prepared.

### Step B - Synthesis of Compound 64

To a solution of compound 40A (350 mg, 1.0mmol), Et₃N (0.49mL, 3.5mmol) in EtOH (5mL) was added hydroxylamine hydrochloride (208 mg, 3.0mmol), and the solution was stirred and heated to 80°C for 16 hours. Allowed to cool, removed EtOH under vacuum, added H₂O (50mL), added EtOAc (50mL), transferred to separatory funnel, separated layers, extracted aqueous layer with EtOAc, combined EtOAc layers, dried (MgSO₄), filtered, and concentrated under vacuum to yield compound 64 (307 mg, 80%).

### Example 41

### Preparation of Compound 66

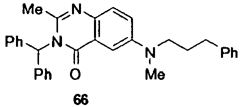

Using Step A from Example 23, and substituting *N*-methyl-3-phenylpropan-1-amine for diethylamine, compound 66 was prepared.

### Example 42

### Preparation of Compound 67

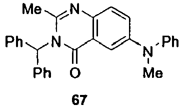

Using Step A from Example 23, and substituting *N*-methylaniline for diethylamine, compound 67 was prepared.

### Example 43

### Preparation of Compound 2

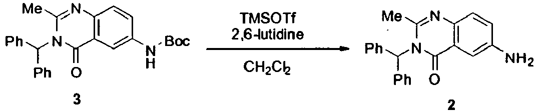

To a solution of compound 3 (800 mg, 1.81mmol) and 2,6-lutidine (0.53mL, 3.62mmol) in CH₂Cl₂ (6mL) was added TMSOTf (0.98mL, 5.43mmol) and the solution was stirred for 3 hours. Transferred to separatory funnel, added CH₂Cl₂ (30mL), added H₂O (30mL), mixed, separated layers, extracted aqueous layer with CH₂Cl₂, combined organic layers, dried (MgSO₄), filtered, and concentrated under vacuum to yield compound 2 as a yellow foam (600 mg, 97%).

### Example 44

### Preparation of Compound 72

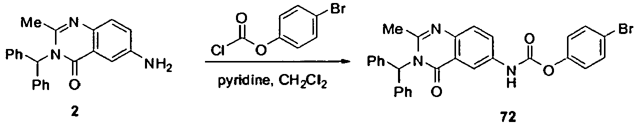

To a solution of compound 2 (60 mg, 0.18mmol) and pyridine (0.016mL, 0.19mmol) in CH₂Cl₂ (2mL) was added 4-bromophenyl chloroformate (0.025mL, 0.18 mmol) and the solution was stirred for 2 hours. Purified solution by preparative TLC (30%EtOAc/hexanes) to yield compound 72 (18 mg, 19%).

### Example 45

### Preparation of Compound 74

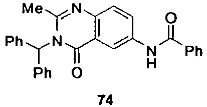

Using Step A from Example 44, and substituting benzoyl chloride for 4-bromophenylchloroformate, compound 74 was prepared.

### Example 46

### Preparation of Compound 6

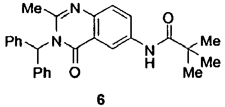

Using Step A from Example 44, and substituting pivaloyl chloride for 4-bromophenylchloroformate, compound 6 was prepared.

### Example 47

### Preparation of Compound 9

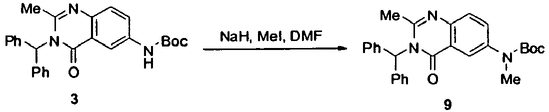

Using Step A from Example 32, and substituting compound 3 for compound 42 and substituting iodomethane for iodoethane, compound 9 was prepared.

### Example 48

### Preparation of Compound 14

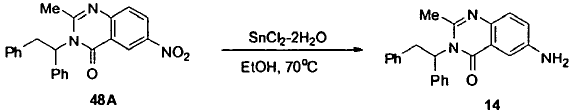

### Step A - Synthesis of Compound 48A

Using Steps A, B, and C from Example 1, substituting 2-amino-5-nitrobenzoic acid for compound 1A and substituting 1,2-diphenylethanamine for aminodiphenylmethane, compound 48A was prepared.

### Step B - Synthesis of Compound 14

To a solution of compound 48A (500 mg, 1.3mmol) in EtOH (10mL) was added SnCl₂•2H₂O (587 mg, 2.6 mmol) and the solution was heated to 70°C 30 minutes. Allowed to cool, poured onto ice water, precipitate formed, basified to pH 8 using saturated aqueous NaHCO₃, extracted with EtOAc, dried organic layer (MgSO₄), filtered, and concentrated under vacuum. Purified residue using preparative TLC (5%MeOH/CH₂Cl₂) to provide compound 14 as a yellow foam (434 mg, 94%).

### Example 49

### Preparation of Compound 12

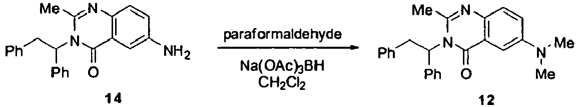

To a solution of compound 14 (60 mg, 0.17mmol) in CH₂Cl₂ (8mL) was added paraformaldehyde (30 mg, 1mmol) and Na(OAc)₃BH (180 mg, 0.85mmol) and the solution was stirred for 24 hours. To the solution was added paraformaldehyde (15eq.) and Na(OAc)₃BH (5eq.) and the solution was stirred for 24 hours. The reaction was concentrated under vacuum and purified by preparative TLC (30%EtOAc/hexanes) to provide compound 12 (62 mg, 96%).

### Example 50

### Preparation of Compound 8

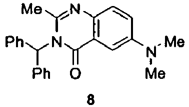

Using Step A from Example 49, substituting compound 2 from Example 43, compound 8 was prepared.

### Example 51

### Preparation of Compound 11

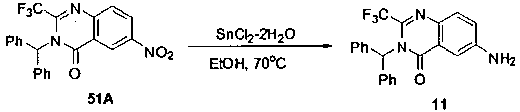

Using Steps A, B, and C from Example 1, substituting 2-amino-5-nitrobenzoic acid for compound 1A and substituting trifluoroacetic anhydride for acetic anhydride, compound 51 A was prepared. Using Step B from Example 48, compound 11 was prepared.

### Example 52

### Preparation of Compound 7

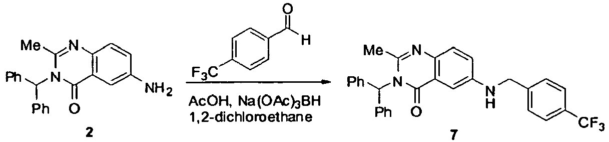

To a solution of compound 2 (0.015g, 0.04mmol) in 1,2-dichloroethane (1mL) was added acetic acid (0.012mL, 0.20mmol), 4-(trifluoromethyl)benzaldehyde (0.0075mL, 0.05mmol) and the solution was allowed to stir for 30 minutes. To the solution NaBH(OAc)₃ (0.03g, 0.14mmol) was added and stirred for 12 hours. The reaction mixture was diluted with CH₂Cl₂, washed with NaHCO₃, dried (MgSO₄), filtered, concentrated, and purified by preparative TLC (5%MeOH/CH₂Cl₂) to provide compound 7 (0.014g, 70%).

### Example 53

### Preparation of Compound 13

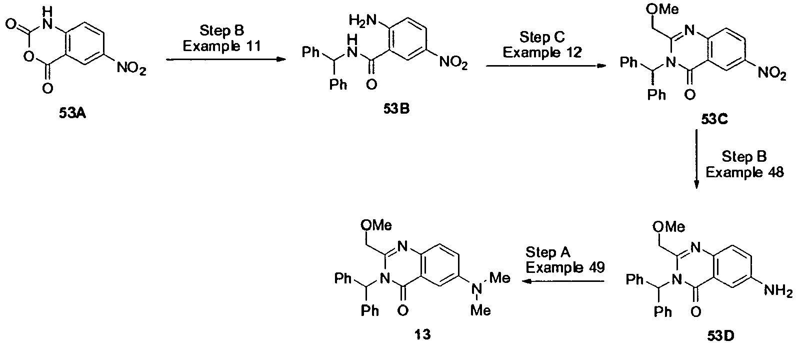

Using Step B from Example 11, substituting 5-nitroisatoic anhydride for compound 11B, compound 53B was prepared. Using Step C from Example 12, substituting compound 53B for compound 12C and substituting 2-methoxyacetyl chloride for acetyl chloride, compound 53C was prepared. Using Step B from Example 48, substituting compound 53C for 48A, compound 53D was prepared. Using Step A from Example 49, substituting compound 53D for compound 14, compound 13 was prepared.

### Example 54

### Preparation of Compound 16

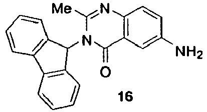

Using Step B from Example 11, substituting 5-nitroisatoic anhydride for compound 11B and substituting 9*H*-fluoren-9-amine for aminodiphenylmethane, followed by Step C from Example 12, and followed by Step B from Example 48, compound 16 was prepared.

### Example 55

### Preparation of Compound 17

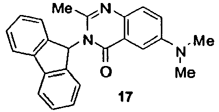

Using Step A from example 49, substituting compound 16 for compound 14, compound 17 was prepared.

### Example 56

### Preparation of Compound 15

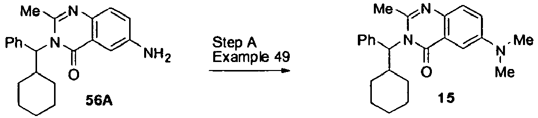

Using Step B from Example 11, substituting 5-nitroisatoic anhydride for compound 11B and substituting cyclohexyl(phenyl)methanamine for aminodiphenylmethane, followed by Step C from Example 12, and followed by Step B from Example 48, compound 56A was prepared. Using Step A from example 49, compound 15 was prepared from compound 56A.

### Example 57

### Preparation of Compound 20

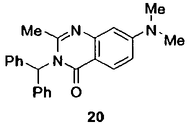

Using Step A from Example 43, substituting compound 10 for compound 3, followed by Step A from Example 49, compound 20 was prepared.

### Example 58

### Preparation of Compound 22

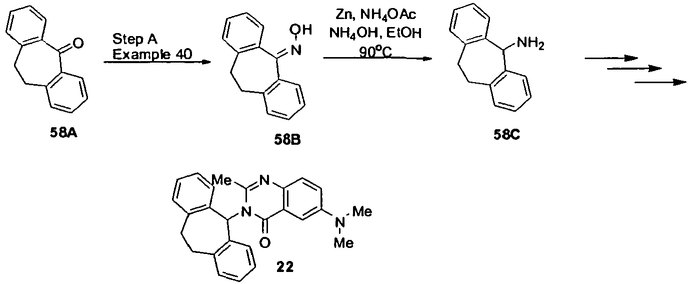

### Step A - Synthesis of Compound 58B

Using Step A from Example 40, substituting compound 58A for compound 40A, compound 58B was prepared.

### Step B - Synthesis of Compound 58C

To a solution of compound 58B (2.0g, 9.0mmol) in EtOH (9mL) was added NH₄OH (45mL), NH₄OAc (0.35g, 4.48mmol),and slowly added Zn powder (2.9g, 45mmol) in a sealed tube, stirred and heated to 90°C for 16 hours. Allowed to cool, diluted with 30mL EtOAc and stirred for 30 minutes. Removed solid by vacuum filtration, filtrate was washed with brine, organic layer dried (MgSO₄), filtered, and concentrated under vacuum to yield compound 58C as a yellow solid (1.9g, 95%).

### Step C - Synthesis of Compound 22

Using Step B from Example 11, substituting 5-nitroisatoic anhydride for compound 11B and substituting compound 58C for aminodiphenylmethane, followed by Step C from Example 12, and followed by Step B from Example 48, followed by Step A from example 49, compound 22 was prepared.

### Example 59

### Preparation of Compound 25

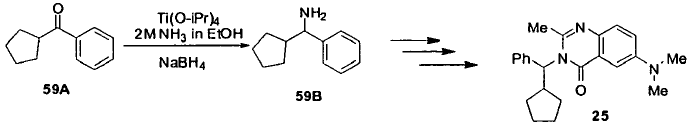

### Step A - Synthesis of Compound 59B

To a solution of compound 59A (3.0g, 17.2mmol) in 2M NH₃ in EtOH (43 mL) was added Ti(O-iPr)₄ (10.8mL, 34.4mmol) and the resulting solution was places in a sealed tube and allowed to stir for 7 hours. To the solution was added NaBH₄ (1.0g, 25.8mmol) and the solution was stirred for 12 hours. The solution was poured into 2M NH₄OH (50mL), solid precipitate was filtered off, rinsed with EtOAc to give a colorless filtrate, the organic layer was separated, the aqueous layer was extracted with EtOAc, combined organic layers, extracted organic layers with 1N HCl, combined aqueous layers, basified aqueous layer with 2N NaOH to pH 10, extracted with EtOAc, dried (MgSO₄), filtered and concentrated to yield compound 59B (0.76g, 25%)

### Step B - Synthesis of Compound 25

Using Step B from Example 11, substituting 5-nitroisatoic anhydride for compound 55B and substituting compound 59B for aminodiphenylmethane, followed by Step C from Example 12, and followed by Step B from Example 48, followed by Step A from example 49, compound 25 was prepared.

### Example 60

### Preparation of Compound 26

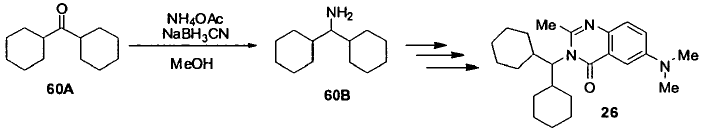

### Step A - Synthesis of Compound 60B

To a solution of compound 60A (3.0g, 15.4mmol) in MeOH (35mL) was added NH₄OAc (11.9g, 154mmol) followed by NaBH₃CN (0.71g, 10.8mmol) and the solution was stirred for 16 hours. Solution was acidified with conc. HCl to pH 2, precipitate formed, removed MeOH under vacuum to give a white solid. Solid was dissolved in H₂O (150mL), extracted with Et₂O and discarded, aqueous layer was basified with solid KOH to pH 10, extracted aqueous layer with Et₂O, dried (MgSO₄), filtered, and concentrated to yield compound 60B (1.6g, 52%).

### Step B - Synthesis of Compound 26

Using Step B from Example 11, substituting 5-nitroisatoic anhydride for compound 11B and substituting compound 60B for aminodiphenylmethane, followed by Step C from Example 12, and followed by Step B from Example 48, followed by Step A from example 49, compound 26 was prepared.

### Example 61

### Preparation of Compound 32

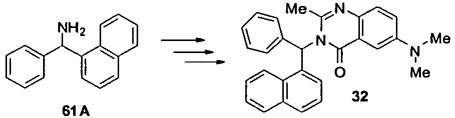

### Step A - Synthesis of Compound 61A

Using Steps A and B from Example 58, substituting 1-naphthylphenyl ketone for compound 58A, compound 61A was prepared.

### Step B - Synthesis of Compound 32

Using Step B from Example 11, substituting 5-nitroisatoic anhydride for compound 11B and substituting compound 61A for aminodiphenylmethane, followed by Step C from Example 12, and followed by Step B from Example 48, followed by Step A from example 49, compound 32 was prepared.

### Example 62

### Preparation of Compound 37

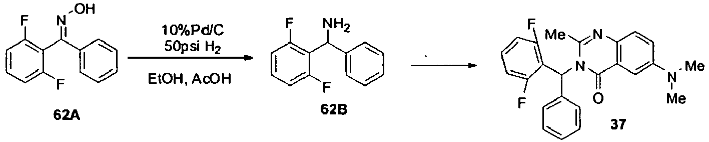

### Step A - Synthesis of Compound 62A

Using Step A from Example 40, substituting (2,6-difluoro-phenyl)-phenyl-methanone for compound 40A, compound 62A was prepared.

### Step B - Synthesis of Compound 62B

To a solution of compound 62A (0.60g, 2.6mmol) in EtOH (10mL) and AcOH (2mL) was added 10%Pd/C (0.60g) and the solution was hydrogenated using 50psi H₂ for 2 hours. Filtered solution through a plug of celite and concentrated to yield compound 62B as a white solid (0.6g, 100%).

### Step C - Synthesis of Compound 37

Using Step B from Example 11, substituting 5-nitroisatoic anhydride for compound 11B and substituting compound 62B for aminodiphenylmethane, followed by Step C from Example 12, and followed by Step B from Example 48, followed by Step A from example 49, compound 37 was prepared.

### Example 66

### Preparation of Compound 63

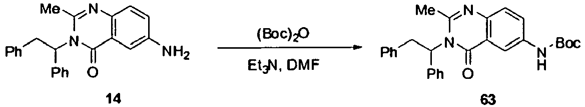

To a solution of compound 14 (20 mg, 0.06 mmol) in DMF (4mL) was added Et₃N (0.009mL, 0.07mmol) and was added (Boc)₂O (18 mg, 0.09 mmol) and the solution was stirred for 16 hours. Reaction was concentrated under vacuum and the residue was purified by preparative TLC (30%EtOAc/hexanes) to yield compound 63 (4 mg, 15%).

### Example 67

### Preparation of Compound 68

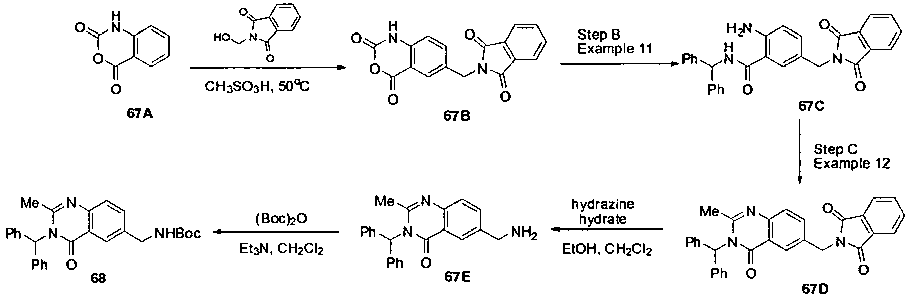

### Step A - Synthesis of Compound 67B

To isatoic anhydride (67A, 4.0g, 24.5 mmol) was added *N*-(hydroxymethyl)phthalimide (4.39g, 24.5 mmol) and methanesulfonic acid (30mL) and the solution was stirred and heated to 50°C for 4 hours. Allowed to cool, diluted with Et₂O (150 mL), stirred for 1h, filtered solid and dried solid to yield compound 67B (2.9g, 37%).

### Step B - Synthesis of Compound 67C

Using Step B from example 11, substituting compound 67B for compound 11B, compound 67C was prepared.

### Step C - Synthesis of Compound 67D

Using Step C from Example 12 compound 67D was prepared.

### Step D - Synthesis of Compound 67E

To a solution of compound 67D (1.43g, 2.95mmol) in EtOH (15mL) and CH₂Cl₂ (15mL) was added hydrazine hydrate (64%, 0.47mL, 9.7mmol) and the solution was stirred for 12 hours. Solution was concentrated under vacuum, 1N HCl (20mL) was added and the solution was stirred for 1 hour. Solid precipitate was removed by filtration, filtrate was basified with KOH to pH=14, extracted with CH₂Cl₂, dried (MgSO₄), filtered, and concentrated to yield compound 67E (0.68g, 64%).

### Step E - Synthesis of Compound 68

To a solution of compound 67E (35 mg, 0.10mmol) in CH₂Cl₂ (5mL) was added Et₃N (0.018mL, 0.13mmol) and was added (Boc)₂O (24 mg, 0.11mmol) and the solution was stirred for 1 hour. Solution was concentrated and purified by preparative TLC (3%MeOH/ CH₂Cl₂) to yield compound 68 (30 mg, 67%).

### Example 68

### Preparation of Compound 69

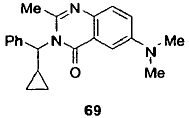

Using Step B from Example 11, substituting 5-nitroisatoic anhydride for compound 11B and substituting 1-cyclopropyl-1-phenylmethanamime for aminodiphenylmethane, followed by Step C from Example 12, and followed by Step B from Example 48, followed by Step A from example 49 compound 69 was prepared.

### Example 69

### Preparation of Compound 70

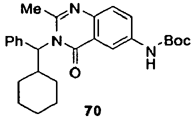

Using Step A from example 66, substituting compound 56A from Example 56 for compound 14, compound 70 was prepared.

### Example 71

### Preparation of Compound 84

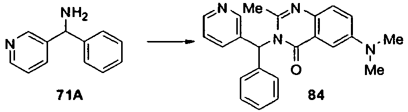

### Step A - Synthesis of Compound 71A

Using Step A from Example 40, substituting 3-benzoylpyridine for compound 40A, followed by Step B from Example 62, compound 71A was prepared.

### Step B - Synthesis of Compound 84

Using Step B from Example 11, substituting 5-nitroisatoic anhydride for compound 11B and substituting compound 71A for aminodiphenylmethane, followed by Step C from Example 12, and followed by Step B from Example 48, followed by Step A from example 49, compound 84 was prepared.

### Example 72

### Preparation of Compound 87

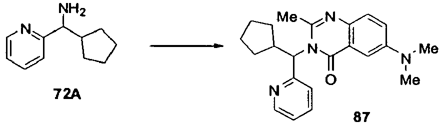

### Step A - Synthesis of Compound 72A

Using Step A from Example 70, substituting 2-cyanopyridine for compound 70A, compound 72A was prepared.

### Step A - Synthesis of Compound 87

Using Step B from Example 11, substituting 5-nitroisatoic anhydride for compound 11B and substituting compound 72A for aminodiphenylmethane, followed by Step C from Example 12, and followed by Step B from Example 48, followed by Step A from example 49, compound 87 was prepared.

### Example 73

### Preparation of Compound 92

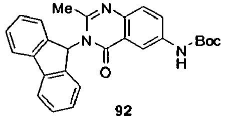

Using Step E from Example 67, substituting compound 16 from Example 54 for compound 67E, compound 92 was prepared.

### Example 74

### Preparation of Compound 113

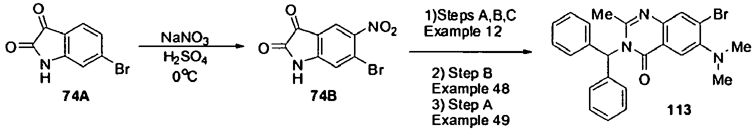

### Step A - Synthesis of Compound 74B

To compound 74A (2.0g, 8.8mmol) in conc. HCl (9mL) was added a solution of NaNO₃ (0.75g, 8.8mmol) in conc. H₂SO₄ (10mL) at 0°C and the solution was stirred for 30minutes. Poured solution into ice water (200mL) and a yellow solid precipitated. Filtered off the solid and dried to yield compound 74B (2.0g, 80%).

### Step B - Synthesis of Compound 113

Using Steps A, B, and C from Example 12, substituting compound 74B for compound 12A, followed by Step B from Example 48, followed by Step A from example 49 compound 113 was prepared.

### Example 75

### Preparation of Compound 142

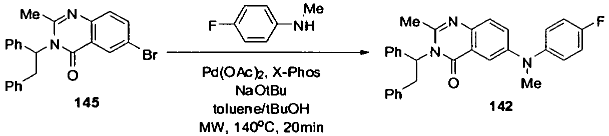

To compound 145 (50 mg, 0.12mmol) was added Pd(OAc)₂ (1.3 mg, 0.006mmol), X-Phos (5.7 mg, 0.012mmol), NaOtBu (16 mg, 0.17mmol), toluene (2mL), tBuOH (1mL), and 4-fluoro-N-methylaniline (20 mg, 0.16mmol) in a microwave vial, sealed vial, stirred, and heated in microwave at 140°C for 20 minutes. The reaction was cooled to room temperature and the reaction mixture was purified using preparative TLC (30%acetone/hexanes) to yield compound 142 (22 mg, 40%).

### Example 78

### Preparation of Compound 147

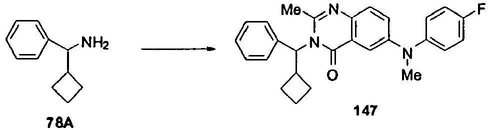

Using methods described in Example 1 and starting with compound 78A (prepared via methods described in Example 62) and Step A from Example 75, compound 147 was prepared.

### Example 82

### Preparation of Compound 195

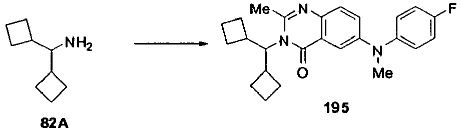

Using methods described in Example 1 (substituting with compound 82A prepared via methods described in Example 60) and Step A from Example 75, compound 195 was prepared.

### Example 83

### Preparation of Compound 200

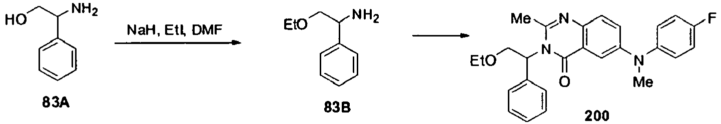

### Step A - Synthesis of Compound 83B

To a solution of D,L-phenylglycinol (83A, 1.0g, 7.3mmol) in DMF (10mL) was added NaH (60% dispersion in oil, 0.36g, 9.0mmol) and the solution was stirred for 30 minutes. To the solution was added EtI (1.35g, 8.3mmol) and the solution was stirred for 1 hour. The reaction was partitioned between EtOAc and H₂O, the organic phase was dried (MgSO₄), filtered, and concentrated. The crude residue was purified by preparative TLC (10%MeOH/CH₂Cl₂) to yield compound 83B (0.14g, 12%).

### Step B - Synthesis of Compound 200

Using methods described in Example 1 (substituting with compound 83B for aminodiphenylmethane) and Step A from Example 75, compound 200 was prepared.

### Example 84

### Preparation of Compound 203

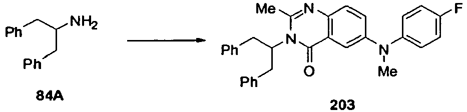

Using methods described in Example 1 (substituting with compound 84A prepared via methods described in Example 62) and Step A from Example 75, compound 203 was prepared.

### Example 85

### Preparation of Compound 204

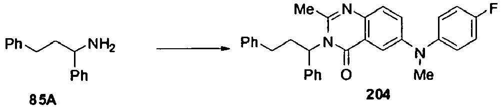

Using methods described in Example 1 (substituting with compound 85A prepared via methods described in Example 62) and Step A from Example 75, compound 204 was prepared.

### Example 86

### Preparation of Compound 216

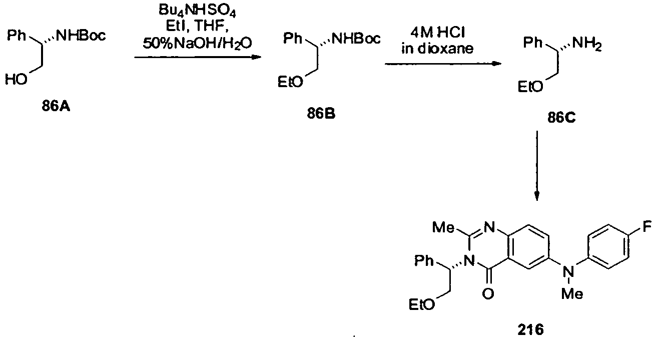

### Step A - Synthesis of Compound 86B

To compound 86A (2.1g, 8.8mmol) was added THF (3mL), 50%NaOH/H₂O (2mL), Bu₄NHSO₄ (0.3g, 0.88mmol), and EtI (0.35mL, 4.4mmol) and the solution was stirred for 12 hours. The solution was partitioned between H₂O and Et₂O, organic phase dried (MgSO₄), filtered, and concentrated. The crude residue was purified by preparative TLC (20%EtOAc/hexanes) to yield compound 86B (0.86g, 37%).

### Step B - Synthesis of Compound 86C

To compound 86B (0.86g, 3.2mmol) was added CH₂Cl₂ (6mL) and 4N HCl in dioxane (2mL) and the solution was stirred for 1 hour. To the solution was added 7M NH₃ in MeOH (8mL) and the solution was concentrated to yield compound 86C (0.43g, 80%).

### Step C - Synthesis of Compound 216

Using methods described in Example 1 (substituting with compound 86C for aminodiphenylmethane) and Step A from Example 75, compound 216 was prepared.

### Example 87

### Preparation of Compound 219

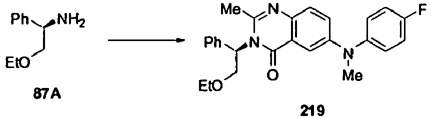

Using methods described in Example 1 (substituting with compound 87A prepared using Steps A and B from Example 86) and Step A from Example 75, compound 219 was prepared.

### Example 89

### Preparation of Compound 230

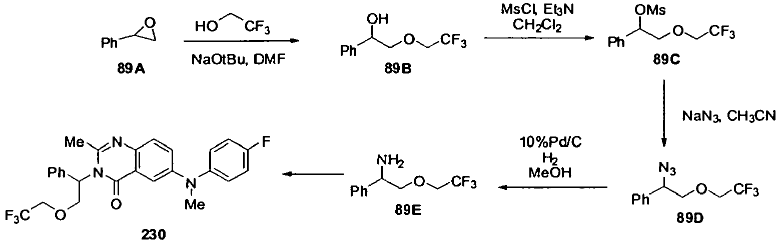

### Step A - Synthesis of Compound 89B

To a solution of 2-phenyloxirane (89A, 3.74g, 31.2mmol) in DMF (40mL) was added trifluoroethanol (15mL), 218mmol) and NaOtBu (0.6g, 6.2mmol) and the solution was stirred and heated to 100°C for 12 hours. Allowed to cool, added H₂O (100mL), extracted with Et₂O, dried (MgSO₄), filtered, and concentrated to yield compound 89B (0.7g, 10%).

### Step B - Synthesis of Compound 89C

Using Step A from Example 35, substituting compound 89B for compound 42, compound 89C was prepared.

### Step C - Synthesis of Compound 89D

To a solution of compound 89C (1.15g, 3.9mmol) in CH₃CN (10mL) was added NaN₃ (0.30g, 4.6mmol) and the solution was stirred and heated to 70°C for 12 hours. Allowed to cool, concentrated under vacuum, partitioned between H₂O and CH₂Cl₂, dried (MgSO₄), filtered, concentrated, and purified by preparative TLC (30%EtOAc/hexanes) to yield compound 89D (450 mg, 40%).

### Step D - Synthesis of Compound 89E

To compound 89D (450 mg, 1.84mmol) was added MeOH (6mL), 10% Pd/C (60 mg), and the solution was stirred under an atmosphere of H₂ for 5 hours. Removed catalyst by filtration and concentrated to yield compound 89E (400 mg, 99%).

### Step E - Synthesis of Compound 230

Using methods described in Example 1 (substituting with compound 89E for aminodiphenylmethane) and Step A from Example 75, compound 230 was prepared.

### Example 90

### Preparation of Compound 232

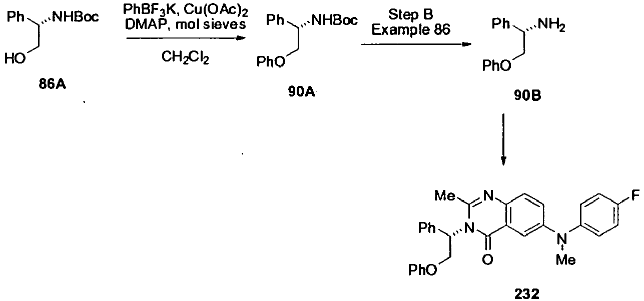

### Step A - Synthesis of Compound 90A

To Cu(OAc)₂ (0.9g, 4.6mmol) was added potassium trifluoroborate (1.55g, 8.4mmol), DMAP (0.10g, 0.8mmol), molecular sieves (4g), and CH₂Cl₂ (50mL) and the solution was stirred for 5 minutes. To the solution was added compound 86A (1.0g, 4.4mmol) and the solution was stirred for 24 hours. The reaction was partitioned between H₂O and CH₂Cl₂, dried (MgSO₄), filtered, and concentrated. The product was purified by preparative TLC (30%EtOAc/hexanes) to yield compound 90A (0.99g, 75%).

### Step B - Synthesis of Compound 90B

Using Step B from Example 86 and substituting compound 90A for compound 86B, compound 90B was prepared.

### Step C - Synthesis of Compound 232

Using methods described in Example 1 (substituting with compound 90B for aminodiphenylmethane) and Step A from Example 75, compound 232 was prepared.

### Example 91

### Preparation of Compound 233

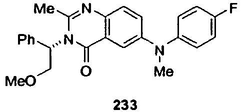

Using methods described in Example 1 (substituting with (S)-(+)-1-amino-1-phenyl-2-methoxyethane for aminodiphenylmethane) and Step A from Example 75, compound 233 was prepared.

### Example 92

### Preparation of Compound 237

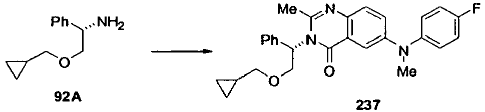

### Step A - Synthesis of Compound 92A

Using Step A from Example 83, substituting (S)-(+)-2-phenylglycinol for compound 83A and substituting cyclopropylmethylbromide for EtI, compound 92A was prepared.

### Step B - Synthesis of Compound 237

Using methods described in Example 1 (substituting compound 92A for aminodiphenylmethane) and methods described in Example 75, compound 237 was prepared.

### Example 97

### Preparation of Compound 242

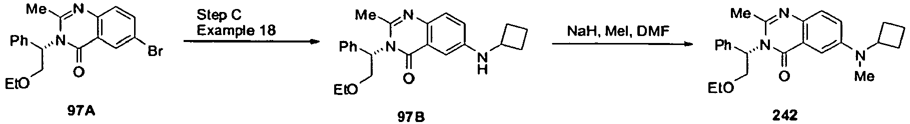

### Step A - Synthesis of Compound 97A

Using the methods of Example 1, substituting compound 86C for aminodiphenylmethane, compound 97A was prepared.

### Step B - Synthesis of Compound 97B

Using Step C from Example 18, substituting 97A for compound 18C and substituting cyclobutylamine for N-methylaniline, compound 97B was prepared.

### Step C - Synthesis of Compound 242

To compound 97B (52 mg, 0.14mmol) was added DMF (3mL), NaH (60% dispersion in oil, 66 mg, 0.17mmol) and the solution was stirred for 10 minutes. To the solution was added iodomethane (0.05mL, 0.84mmol) and the solution was stirred for 12 hours. The solution was concentrated under vacuum and purified by preparative TLC (30%EtOAc/hexanes) to yield compound 242 (39 mg, 72%).

### Example 98

### Preparation of Compound 249

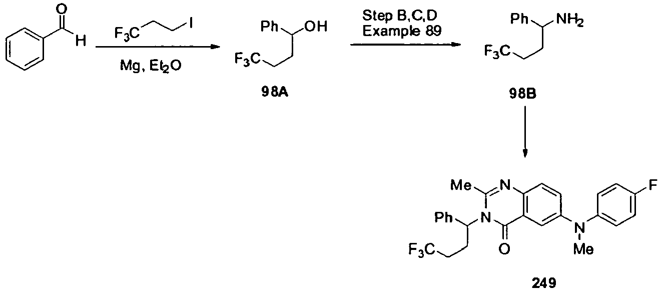

### Step A - Synthesis of Compound 98A

To a mixture of Mg (0.75g, 30mmol) in Et₂O (30mL) was added 1,1,1-trifluoro-3-iodopropane (2.7mL, 22.6mmol) and the solution was heated to reflux for 30 minutes and stirred at room temperature for 1 hour. The solution was transferred to an ether solution of benzaldehyde (2.0g, 18.9mmol) and the solution was stirred for 4h and the reaction was quenched with H₂O. The reaction was extracted with Et₂O, dried (MgSO₄), filtered, concentrated, and purified by flash column chromatography (30%EtOAc/hexanes) to yield compound 98A (2.6g, 67%).

### Step B - Synthesis of Compound 98B

Using Steps B,C,D from Example 89, substituting compound 98A for compound 89B, compound 98B was prepared.

### Step C - Synthesis of Compound 249

Using methods described in Example 1 (substituting compound 98B for aminodiphenylmethane) and Step A from Example 75, compound 249 was prepared.

### Example 99

### Preparation of Compound 256

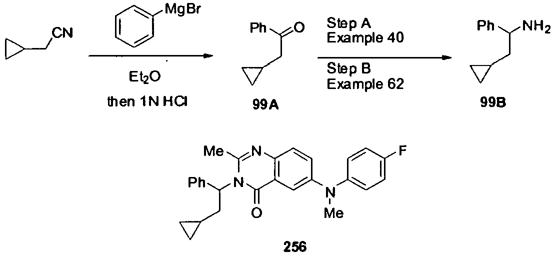

### Step A - Synthesis of Compound 99A

To a solution of phenylmagnesium bromide (3.0M in ether, 10.7mL, 32.1mmol) was added slowly at 0°C a solution of cyclopropylacetonitrile (2.0g, 24.7mmol) in ether and the solution was stirred for 2h, allowed to warm to room temperature, THF (30mL) was added and 1N HCl (30mL) was added and the solution was stirred for 12 hours. The solution was extracted with EtOAc, dried (MgSO₄), filtered, concentrated, and purified by flash column chromatography using silica gel (25%EtOAc/hexanes) to yield compound 99A (3.5g, 89%).

### Step B - Synthesis of Compound 99B

Using Step A from Example 40, substituting compound 99A for compound 40A, followed by Step B from Example 62, compound 99B was prepared.

### Step C - Synthesis of Compound 256

Using methods described in Example 1 (substituting compound 99B for aminodiphenylmethane) and Step A from Example 75, compound 256 was prepared.

### Example 100

### Preparation of Compound 173

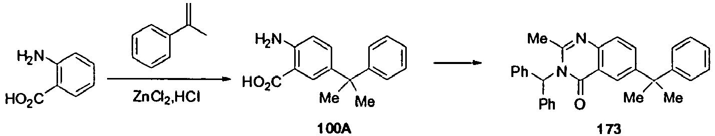

### Step A - Synthesis of Compound 100A

To anthranilic acid (5.0g, 36.5mmol) was added conc. HCl (10mL), α-methylstyrene (9.2g, 76.6mmol), and ZnCl₂ (5.0g, 36.5mmol) and the solution was stirred and heated to 120°C for 5 hours. Diluted reaction with H₂O (100mL), adjusted pH to 4 with 50%NaOH, collected white solid. White solid was partitioned between H₂O and CH₂Cl₂, dried (MgSO₄), filtered, and concentrated. Compound was dissolved in toluene and precipitated with hexanes, filtered solid, and dried to yield compound 100A.

### Step B - Synthesis of Compound 173

Using methods described in Example 1, substituting compound 100A for 2-amino-5-bromobenzoic acid, compound 173 was prepared.

### Example 101

### Preparation of Compound 174

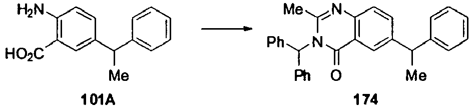

### Step A - Synthesis of Compound 101A

Using Step A in Example 100, substituting styrene for α-methylstyrene, compound 101A was prepared.

### Step B - Synthesis of Compound 174

Using methods described in Example 1, substituting compound 101A for 2-amino-5-bromobenzoic acid, compound 174 was prepared.

### Example 102

### Preparation of Compound 164

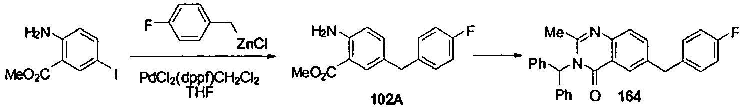

### Step A - Synthesis of Compound 102A

To a solution of 4-fluorobenzyl zinc chloride (0.5M solution in THF, 27.1mL, 13.55mmol) was added PdCl₂(dppf)CH₂Cl₂ (221mg, 0.27mmoL) and the solution was stirred for 5 minutes. To the solution was added 5-iodoanthranilic acid methyl ester (1.5g, 5.4mmol) and the solution was heated to 70 °C for 1 hour. Allowed to cool, quenched with saturated NH₄Cl, followed by saturated aqueous Na₂EDTA, the solution was extracted with CH₂Cl₂, dried (MgSO₄), filtered, and concentrated to yield compound 102A (1.4g, 100%)

### Step B - Synthesis of Compound 164

Using methods described in Example 1, substituting compound 102A for 2-amino-5-bromobenzoic acid, compound 164 was prepared.

### Example 103

### Preparation of Compound 144

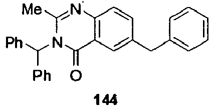

Using the methods described in Example 102, substituting benzylzinc chloride for 4-fluorobenzyl zinc chloride, and methods described in Example 1, compound 144 was prepared.

### Example 104

### Preparation of Compound 129

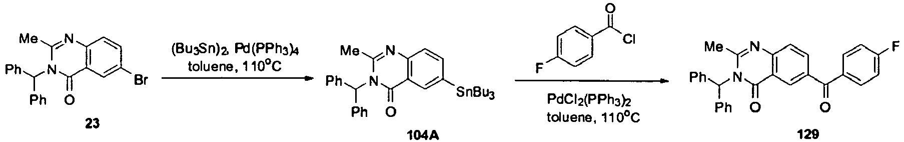

### Step A - Synthesis of Compound 104A

To compound 23 (50 mg, 0.12mmol) was added toluene (5mL), Pd(PPh₃)₄ (14 mg, 0.012mmol), and (Bu₃Sn)₂ (150 mg, 0.24mmol) and the solution was heated to 110°C for 24 hours. Allowed to cool, concentrated under vacuum, and purified by preparative TLC (20%EtOAc/hexanes) to yield compound 104A (20 mg, 26%).

### Step B - Synthesis of Compound 129

To compound 104A (20 mg, 0.033mmol) was added toluene (5mL), PdCl₂(PPh₃)₂ (2 mg, 0.0033mmol), and 4-fluorobenzoyl chloride (0.006mL, 0.05mmol) and the solution was heated to 110 °C for 1 hour. Concentrated under vacuum, purified by preparative TLC (25%EtOAc/hexanes) to yield compound 129 (2 mg, 14%).

### Example 105

### Preparation of Compound 38

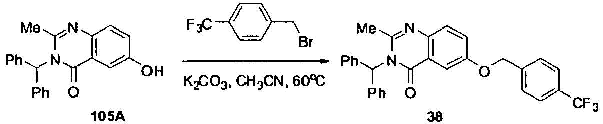

### Step A - Synthesis of Compound 105A

Using the methods of Example 1, substituting 2-amino-5-hydroxybenzoic acid for 2-amino-5-bromobenzoic acid, compound 105A was prepared.

### Step B - Synthesis of Compound 38

To compound 105A (40 mg, 0.09mmol) was added CH₃CN (6mL), K₂CO₃ (17 mg, 0.12mmol), and 2'-bromo-2,2,2-trifluoro-p-xylene (25 mg, 0.10mmol) and the solution was stirred and heated to 60°C for 16 hours. Concentrated solution under vacuum and purified by preparative TLC (3%MeOH/CH₂Cl₂) to yield compound 38 (37 mg, 84%).

### Example 106

### Preparation of Compound 39

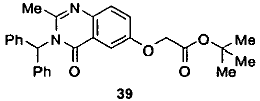

Using the methods described in Example 105, substituting *tert*-butyl bromoacetate for 2'-bromo-2,2,2-trifluoro-*p*-xylene, compound 39 was prepared.

### Example 107

### Preparation of Compound 45

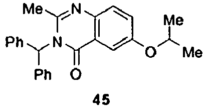

Using the methods described in Example 105, substituting isopropylbromide for 2'-bromo-2,2,2-trifluoro-p-xylene, compound 45 was prepared.

### Example 108

### Preparation of Compound 44

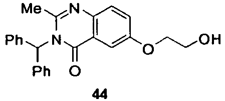

Using the methods described in Example 105, substituting 2-bromoethanol for 2'-bromo-2,2,2-trifluoro-p-xylene, compound 44 was prepared.

### Example 109

### Preparation of Compound 43

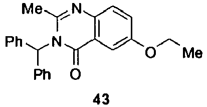

Using the methods described in Example 105, substituting iodoethane for 2'-bromo-2,2,2-trifluoro-*p*-xylene, compound 43 was prepared.

### Example 110

### Preparation of Compound 127

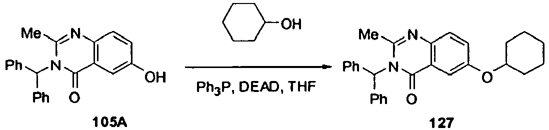

To a solution of compound 105A (50 mg, 0.15 mmol) in THF (5 mL) at -20 °C was added cyclohexanol (0.016 mL, 0.15 mmol), PPh₃ (38 mg, 0.15 mmol) and DEAD (0.023 mL, 0.15 mmol) and the resulting reaction was allowed to stir at -20 °C for 30 minutes, then allowed to warm to room temperature and stirred for an additional 48 hours. The reaction mixture was then concentrated *in vacuo* and the residue obtained was purified using preparative TLC (30% EtOAc/hexanes) to provide compound 127 (13 mg, 21%).

### Example 111

### Preparation of Compound 121

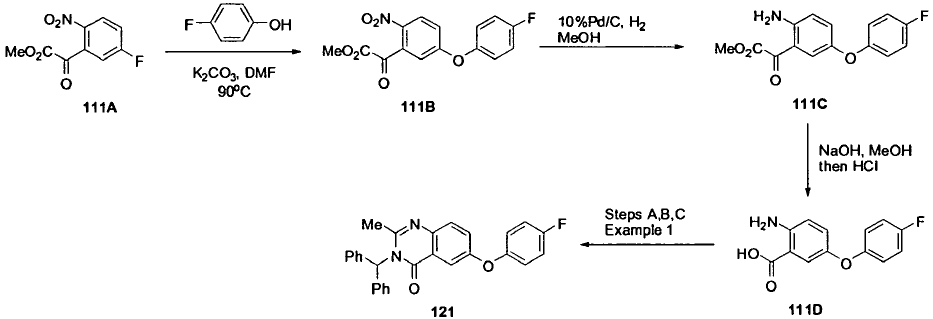

### Step A - Synthesis of Compound 111B

To compound 111A (400 mg, 2.0mmol) was added DMF (6mL), K₂CO₃ (472 mg, 3.4mmol), and 4-fluorophenol (225 mg, 2.0mmol) and the solution was stirred at 90°C for 24 hours. Allowed to cool, reaction was concentrated, partitioned between H₂O and Et₂O, washed with 1N NaOH, washed with 1N HCl, dried (MgSO₄), filtered, and concentrated to give compound 111B (452 mg, 77%)

### Step B - Synthesis of Compound 111C

To compound 111B (450 mg, 1.5mmol) was added MeOH (6mL), 10% Pd/C (90 mg), and the solution was stirred under an atmosphere of H₂ at 50psi for 1 hour. Removed catalyst by filtration through a pad of celite and concentrated to yield compound 111C (354 mg, 88%).

### Step C - Synthesis of Compound 111D

To compound 111C (354 mg, 1.4mmol) was added MeOH (6mL) and 1N NaOH (2.7mL) and the solution was stirred at 60°C for 9 hours. Allowed to cool, added 1N HCl (5.4mL) and concentrated, added EtOH (20mL) and solid was collected by vacuum filtration to yield compound 111D (363 mg, 100%).

### Step D - Synthesis of Compound 121

Using the methods described in Example 1, substituting compound 111D for 2-amino-5-bromobenzoic acid, compound 121 was prepared.

### Example 112

### Preparation of Compound 100

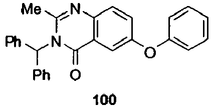

Using the methods described in Example 111, substituting phenol for 4-fluorophenol, compound 100 was prepared.

### Example 113

### Preparation of Compound 77

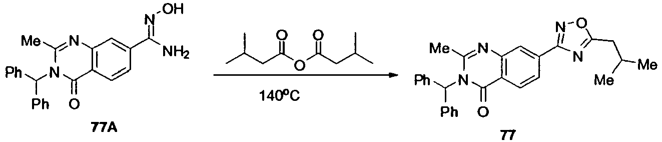

### Step A - Synthesis of Compound 77A

Using Step B from Example 40, substituting compound 65 for compound 40A, compound 77A was prepared.

### Step B - Synthesis of Compound 77

To compound 77A (30 mg, 0.08mmol) was added isovaleric anhydride (1.5mL) and the solution was stirred and heated to 140°C for 4 hours. Allowed to cool and purified by preparative TLC (30%EtOAc/hexanes) to yield compound 77 (20 mg, 55%).

### Example 114

### Preparation of Compound 76

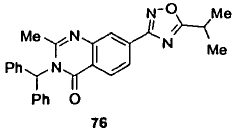

Using Step B from Example 113, substituting isobutyric anhydride for isovaleric anhydride, compound 76 was prepared.

### Example 115

### Preparation of Compound 78

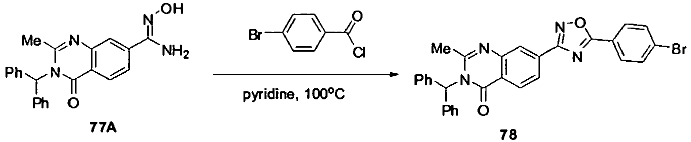

To compound 77A (50 mg, 0.13mmol) was added pyridine (1.5mL) and 4-bromobenzoyl chloride (30 mg, 0.14mmol) and the solution was heated to 100°C for 16 hours. Allowed to cool, concentrated under vacuum, and purified using preparative TLC (30%EtOAc/hexanes) to yield compound 78 (5 mg, 7%).

### Example 116

### Preparation of Compound 79

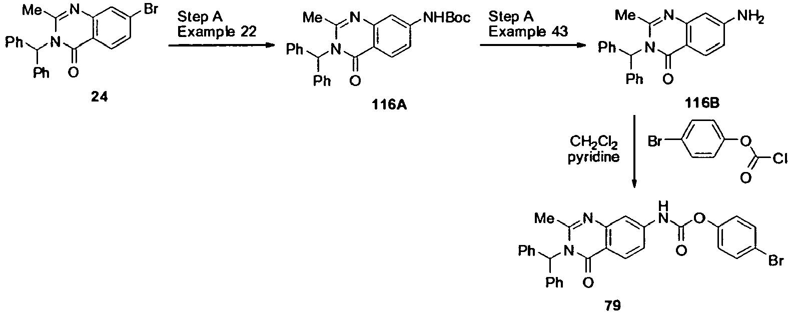

### Step A - Synthesis of Compound 116A

Using Step A from Example 22, substituting compound 24 for compound 23, compound 116A was prepared.

### Step B - Synthesis of Compound 116B

Using Step A from Example 43, substituting compound 116A for compound 3, compound 116B was prepared.

### Step C - Synthesis of Compound 79

To compound 116B (64 mg, 0.19mmol) was added CH₂Cl₂ (1mL), pyridine (0.02mL, 0.24mmol), and 4-bromophenyl chloroformate (0.029mL, 0.21mmol) and the solution was stirred for 1 hour. The solution was purified by preparative TLC (30%EtOAc/hexanes) to yield compound 79 (15 mg, 15%).

### Example 117

### Preparation of Compound 81

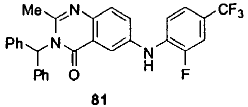

Using Step A from Example 75, substituting compound 23 for compound 145 and substituting 2-fluoro-4-trifluoromethylaniline for 4-fluoro-N-methylaniline, compound 81 was prepared.

### Example 118

### Preparation of Compound 82

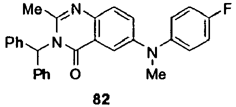

Using Step A from Example 75, substituting compound 23 for compound 145, compound 82 was prepared.

### Example 119

### Preparation of Compound 83

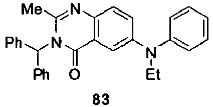

Using Step A from Example 75, substituting compound 23 for compound 145 and substituting *N*-ethylaniline for 4-fluoro-N-methylaniline, compound 83 was prepared.

### Example 120

### Preparation of Compound 85

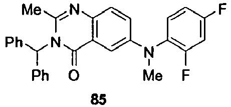

Using Step A from Example 75, substituting compound 23 for compound 145 and substituting 2,4-difluoro-*N*-methylaniline for 4-fluoro-*N*-methylaniline, compound 85 was prepared.

### Example 121

### Preparation of Compound 86

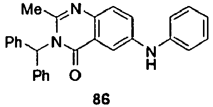

Using Step A from Example 75, substituting compound 23 for compound 145 and substituting aniline for 4-fluoro-*N*-methylaniline, compound 86 was prepared.

### Example 122

### Preparation of Compound 88

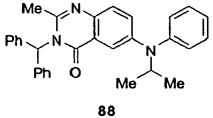

Using Step A from Example 75, substituting compound 23 for compound 145 and substituting *N*-isopropylaniline for 4-fluoro-*N*-methylaniline, compound 88 was prepared.

### Example 123

### Preparation of Compound 91

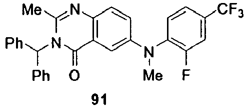

Using Step A from Example 47, substituting compound 81 for compound 3, compound 91 was prepared.

### Example 124

### Preparation of Compound 95

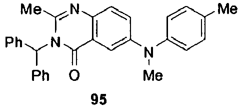

Using Step A from Example 75, substituting compound 23 for compound 145 and substituting *N*-methyl-*p*-toluidine for 4-fluoro-*N*-methylaniline, compound 95 was prepared.

### Example 125

### Preparation of Compound 96

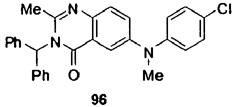

Using Step A from Example 75, substituting compound 23 for compound 145 and substituting 4-chloro-*N*-methylaniline for 4-fluoro-*N*-methylaniline, compound 96 was prepared.

### Example 126

### Preparation of Compound 97

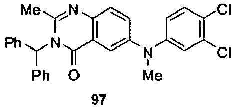

Using Step A from Example 75, substituting compound 23 for compound 145 and substituting 3,4-dichloro-*N*-methylaniline for 4-fluoro-*N*-methylaniline, compound 97 was prepared.

### Example 127

### Preparation of Compound 98

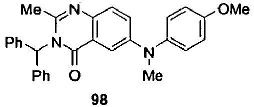

Using Step A from Example 75, substituting compound 23 for compound 145 and substituting 4-methoxy-*N*-methylaniline for 4-fluoro-N-methylaniline, compound 98 was prepared.

### Example 128

### Preparation of Compound 99

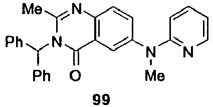

Using Step A from Example 75, substituting compound 23 for compound 145 and substituting 2-(methylamino)pyridine for 4-fluoro-*N*-methylaniline, compound 99 was prepared.

### Example 129

### Preparation of Compound 102

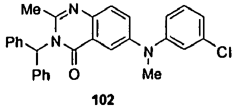

Using Step A from Example 75, substituting compound 23 for compound 145 and substituting 3-chloro-*N*-methylaniline for 4-fluoro-*N*-methylaniline, compound 102 was prepared.

### Example 130

### Preparation of Compound 103

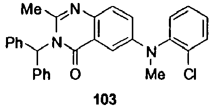

Using Step A from Example 75, substituting compound 23 for compound 145 and substituting 2-chloro-*N*-methylaniline for 4-fluoro-*N*-methylaniline, compound 103 was prepared.

### Example 131

### Preparation of Compound 105

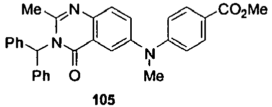

Using Step A from Example 75, substituting compound 23 for compound 145 and substituting methyl 4-methylaminobenzoate for 4-fluoro-*N*-methylaniline, compound 105 was prepared.

### Example 132

### Preparation of Compound 104

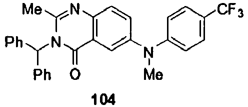

Using Step A from Example 75, substituting compound 23 for compound 145 and substituting 4-trifluoromethyl-*N*-methylaniline for 4-fluoro-N-methylaniline, compound 104 was prepared.

### Example 133

### Preparation of Compound 106

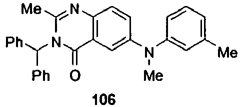

Using Step A from Example 75, substituting compound 23 for compound 145 and substituting *N*-methyl-*m*-toluidine for 4-fluoro-*N*-methylaniline, compound 106 was prepared.

### Example 134

### Preparation of Compound 107

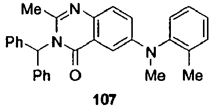

Using Step A from Example 75, substituting compound 23 for compound 145 and substituting N-methyl-o-toluidine for 4-fluoro-*N*-methylaniline, compound 107 was prepared.

### Example 135

### Preparation of Compound 108

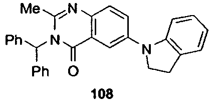

Using Step A from Example 75, substituting compound 23 for compound 145 and substituting indoline for 4-fluoro-*N*-methylaniline, compound 108 was prepared.

### Example 136

### Preparation of Compound 111

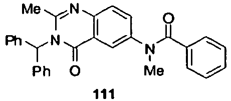

Using Step C from Example 97, substituting compound 74 for compound 97B, compound 111 was prepared.

### Example 137

### Preparation of Compound 109

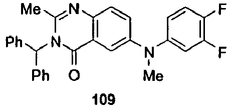

Using Step A from Example 75, substituting compound 23 for compound 145 and substituting 3,4-difluoro-*N*-methylaniline for 4-fluoro-*N*-methylaniline, compound 109 was prepared.

### Example 138

### Preparation of Compound 112

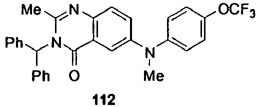

Using Step A from Example 75, substituting compound 23 for compound 145 and substituting 4-trifluoromethoxy-*N*-methylaniline for 4-fluoro-*N*-methylaniline, compound 112 was prepared.

### Example 139

### Preparation of Compound 114

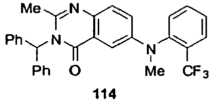

Using Step A from Example 75, substituting compound 23 for compound 145 and substituting 2-trifluoromethyl-*N*-methylaniline for 4-fluoro-*N*-methylaniline, compound 114 was prepared.

### Example 140

### Preparation of Compound 115

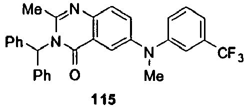

Using Step A from Example 75, substituting compound 23 for compound 145 and substituting 3-trifluoromethyl-*N*-methylaniline for 4-fluoro-*N*-methylaniline, compound 115 was prepared.

### Example 141

### Preparation of Compound 116

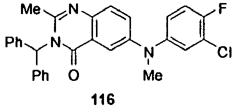

Using Step A from Example 75, substituting compound 23 for compound 145 and substituting 3-chloro-4-fluoro-*N*-methylaniline for 4-fluoro-*N*-methylaniline, compound 116 was prepared.

### Example 142

### Preparation of Compound 120

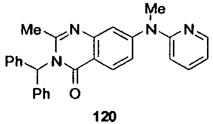

Using Step A from Example 75, substituting compound 24 for compound 145 and substituting 2-(methylamino)pyridine for 4-fluoro-*N*-methylaniline, compound 120 was prepared.

### Example 143

### Preparation of Compound 118

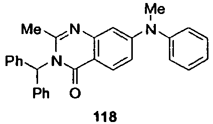

Using Step A from Example 75, substituting compound 24 for compound 145 and substituting N-methylaniline for 4-fluoro-*N*-methylaniline, compound 118 was prepared.

### Example 144

### Preparation of Compound 119

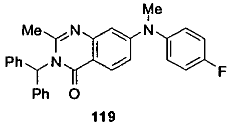

Using Step A from Example 75, substituting compound 24 for compound 145, compound 119 was prepared.

### Example 145

### Preparation of Compound 117

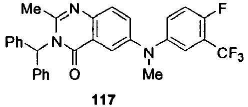

Using Step A from Example 75, substituting compound 23 for compound 145 and substituting 4-fluoro-3-trifluoromethyl-*N*-methylaniline for 4-fluoro-*N*-methylaniline, compound 117 was prepared.

### Example 146

### Preparation of Compound 123

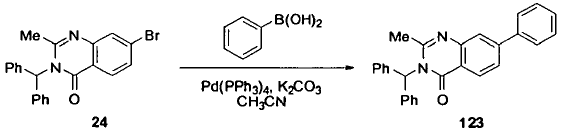

To compound 24 (100 mg, 0.25mmol) was added CH₃CN (2mL), phenylboronic acid (46 mg, 0.38), K₂CO₃ (0.25mL of a 2M solution in H₂O, 0.5mmol), and Pd(PPh₃)₄ (14 mg, 0.013mmol) and the solution was heated in a microwave at 140°C for 20 minutes. Purified solution using preparative TLC (30%acetone/hexanes) to yield compound 123 (96 mg, 96%).

### Example 147

### Preparation of Compound 125

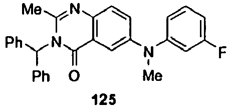

Using Step A from Example 75, substituting compound 23 for compound 145 and substituting 3-fluoro-*N*-methylaniline for 4-fluoro-*N*-methylaniline, compound 125 was prepared.

### Example 148

### Preparation of Compound 128

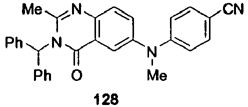

Using Step A from Example 75, substituting compound 23 for compound 145 and substituting 4-cyano-*N*-methylaniline for 4-fluoro-N-methylaniline, compound 128 was prepared.

### Example 149

### Preparation of Compound 130

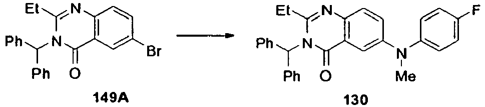

Using methods from Example 1, susbstituting propionic anhydride for acetic anhydride, compound 149A was prepared. Using Step A from Example 75, substituting compound 149A for compound 145, compound 130 was prepared.

### Example 150

### Preparation of Compound 132

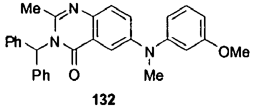

Using Step A from Example 75, substituting compound 23 for compound 145 and substituting 3-methoxy-*N*-methylaniline for 4-fluoro-*N*-methylaniline, compound 132 was prepared.

### Example 153

### Preparation of Compound 149

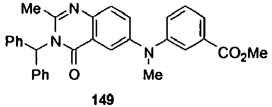

Using Step A from Example 75, substituting compound 23 for compound 145 and substituting methyl 3-aminobenzoate for 4-fluoro-*N*-methylaniline, followed by Step C from Example 97, compound 149 was prepared.

### Example 154

### Preparation of Compound 150

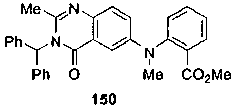

Using Step A from Example 75, substituting compound 23 for compound 145 and substituting methyl-2-methylaminobenzoate for 4-fluoro-*N*-methylaniline, compound 150 was prepared.

### Example 155

### Preparation of Compound 151

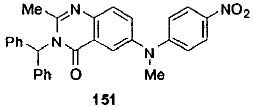

Using Step A from Example 75, substituting compound 23 for compound 145 and substituting 4-nitro-*N*-methylaniline for 4-fluoro-*N*-methylaniline, compound 151 was prepared.

### Example 156

### Preparation of Compound 136

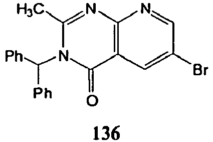

Using methods from Example 1, susbstituting 2-amino-5-bromonicotinic acid for 2-amino-5-bromobenzoic acid, compound 136 was prepared.

### Example 157

### Preparation of Compound 138

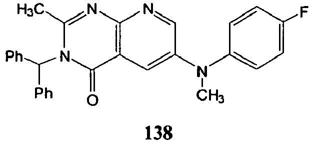

Using Step A from Example 75, substituting compound 136 for compound 145, compound 138 was prepared.

### Example 158

### Preparation of Compound 153

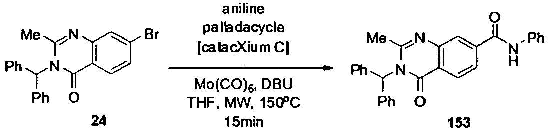

To compound 24 (100 mg, 0.25mmol) was added THF (1mL), DBU (0.045mL, 0.30mmol), aniline (0.035mL, 0.38mmol), palladacycle [catacXiumC] (23 mg, 0.025mmol), and Mo(CO)₆ (66 mg, 0.25mmol), sealed vial, stirred and heated in a microwave at 150°C for 15 minutes. Allowed to cool and purified by preparative TLC (30%acetone/hexanes) to yield compound 153 (72 mg, 67%).

### Example 159

### Preparation of Compound 155

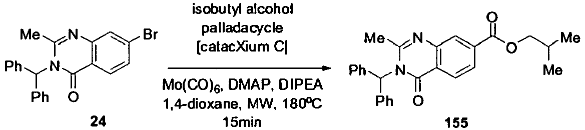

To compound 24 (100 mg, 0.25mmol) was added 1,4-dioxane (1mL), DMAP(61mg, 0.50mmol), DIPEA (0.087mL, 0.50mmol), isobutyl alcohol (1mL), palladacycle [catacXiumC] (23 mg, 0.025mmol), and Mo(CO)₆ (66 mg, 0.25mmol), sealed vial, stirred and heated in a microwave at 180°C for 15 minutes. Allowed to cool and purified by preparative TLC (30%acetone/hexanes) to yield compound 155 (59 mg, 55%).

### Example 160

### Preparation of Compound 156

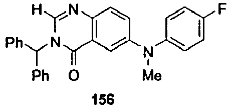

Using Step A from Example 75, substituting compound 154 for compound 145, compound 156 was prepared.

### Example 161

### Preparation of Compound 157

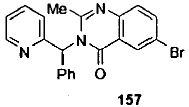

Using methods from Example 1, substituting 1-phenyl-1-pyridin-2-ylmethanamine hydrochloride for aminodiphenylmethane, compound 157 was prepared.

### Example 162

### Preparation of Compound 158

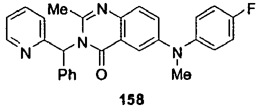

Using Step A from Example 75, substituting compound 157 for compound 145, compound 158 was prepared.

### Example 163

### Preparation of Compound 161

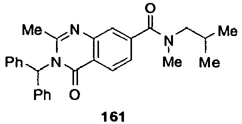

Using Step A from Example 158, substituting N-methylisobutylamine for aniline, compound 161 was prepared.

### Example 164

### Preparation of Compound 162

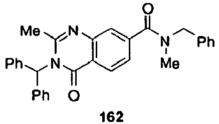

Using Step A from Example 158, substituting N-methylbenzylamine for aniline, compound 162 was prepared.

### Example 165

### Preparation of Compound 163

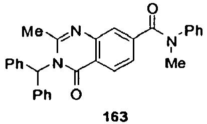

Using Step A from Example 158, substituting *N*-methylaniline for aniline, compound 163 was prepared.

### Example 166

### Preparation of Compounds 40 and 165

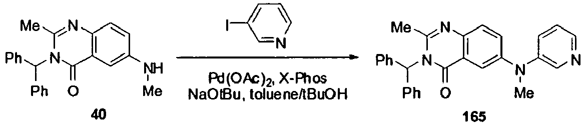

### Step A - Synthesis of Compound 40

An alternative to Example 16 for the synthesis of compound 40, is using Example 47 to prepare compound 9, followed by Example 43, to prepare compound 40.

### Step B - Synthesis of Compound 165

To compound 40 (50 mg, 0.14mmol) was added Pd(OAc)₂ (3 mg, 0.014mmol), X-Phos (13 mg, 0.028mmol), NaOtBu (20 mg, 0.21mmol), 3-iodopyridine (43 mg, 0.21mmol), toluene (0.6mL), and t-BuOH (0.2mL) and the solution was sealed in a vial and heated to 140°C in a microwave for 20 minutes. The solution was purified by preparative TLC (50%acetone/hexanes) to yield compound 165 (44 mg, 73%).

### Example 167

### Preparation of Compounds 167A and 168

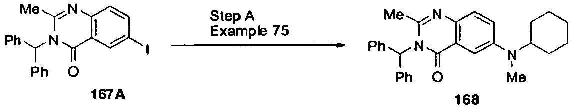

### Step A - Synthesis of Compound 167A

Using the methods of Example 1, substituting 2-amino-5-iodobenzoic acid for 2-amino-5-bromobenzoic acid, compound 167A was prepared.

### Step B - Synthesis of Compound 168

Using Step A from Example 75, substituting compound 167A for compound 145 and substituting N-methylcyclohexylamine for 4-fluoro-*N*-methylaniline, compound 168 was prepared.

### Example 168

### Preparation of Compound 170

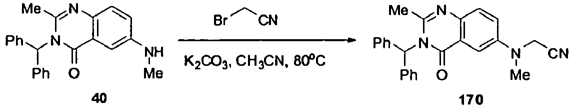

To compound 40 (17 mg, 0.05mmol) was added CH₃CN (0.3mL), K₂CO₃ (13 mg, 0.096mmol) and bromoacetonitrile (0.005mL, 0.062mmol) and the solution was heated to 80°C for 24 hours. Purified solution by preparative TLC (100%CH₂Cl₂) to yield compound 170 (16 mg, 85%).

### Example 169

### Preparation of Compounds 171

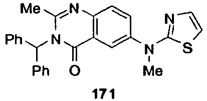

Using Step B from Example 166, substituting 2-bromothiazole for 3-iodopyridine, compound 171 was prepared.

### Example 170

### Preparation of Compound 175

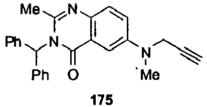

Using Step A from Example 168, substituting propargyl bromide for bromoacetonitrile, compound 175 was prepared.

### Example 171

### Preparation of Compound 178

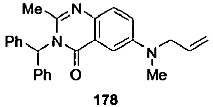

Using Step A from Example 168, substituting allylbromide for bromoacetonitrile, compound 178 was prepared.

### Example 172

### Preparation of Compound 188

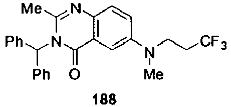

Using the method of Example 47, substituting compound 40 for compound 3 and substituting 1-iodo-3,3,3-trifluoropropane for iodomethane, compound 188 was prepared.

### Example 173

### Preparation of Compound 186

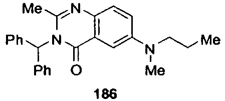

Using the method of Example 47, substituting compound 40 for compound 3 and substituting 1-iodo-propane for iodomethane, compound 186 was prepared.

### Example 174

### Preparation of Compound 187

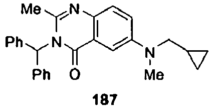

Using the method of Example 47, substituting compound 40 for compound 3 and substituting cyclopropylmethylbromide for iodomethane, compound 187 was prepared.

### Example 175

### Preparation of Compound 199

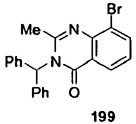

Using the methods of Example 1, substituting 2-amino-3-bromobenzoic acid for 2-amino-5-bromobenzoic acid, compound 199 was prepared.

### Example 177

### Preparation of Compound 208

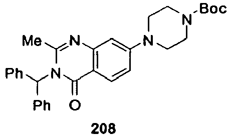

Using Step A from Example 75, substituting compound 24 for compound 145 and substituting Boc-piperazine for 4-fluoro-*N*-methylaniline, compound 208 was prepared.

### Example 178

### Preparation of Compounds 209

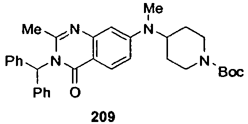

Using Step A from Example 75, substituting compound 24 for compound 145 and substituting 1-Boc-4-methylaminopiperidine for 4-fluoro-*N*-methylaniline, compound 209 was prepared.

### Example 179

### Preparation of Compound 229

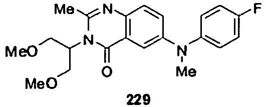

Using Steps B and C from Example 1, substituting compound 176D for compound 1B and substituting 2-amino1,3-dimethoxypropane for aminodiphenylmethane, compound 229 was prepared.

### Example 180

### Preparation of Compound 253

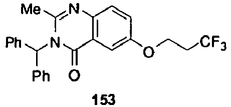

Using Step A from Example 110, substituting trifluoropropanol for cyclohexanol, compound 253 was provided.

### Example 181

### Preparation of Compound 248

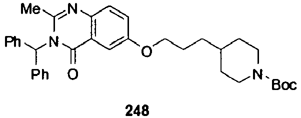

Using Step A from Example 110, substituting 1-N-Boc-4-(3'-hydroxypropyl)-piperidine for cyclohexanol, compound 248 was provided.

### Example 183

### Preparation of Compound 18

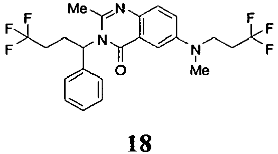

Using Steps B and C from Example 1, substituting compound 248B for compound 1B and substituting compound 98B for aminodiphenylmethane, compound 18 was prepared.

### Example 184

### Preparation of Compound 152

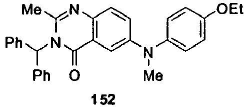

Using Step A from Example 75, substituting compound 23 for compound 145 and substituting 4-ethoxyaniline for 4-fluoro-*N*-methylaniline, followed by Step C from Example 97, compound 152 was prepared.

### Example 185

### Preparation of Compound 159

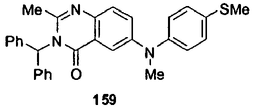

Using Step A from Example 75, substituting compound 23 for compound 145 and substituting 4-methylthioaniline for 4-fluoro-*N*-methylaniline, followed by Step C from Example 97, compound 159 was prepared.

### Example 186

### Preparation of Compound 160

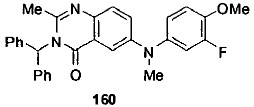

Using Step A from Example 75, substituting compound 23 for compound 145 and substituting 3-fluoro-4-methoxyaniline for 4-fluoro-*N*-methylaniline, followed by Step C from Example 97, compound 160 was prepared.

### Example 187

### Preparation of Compound 166

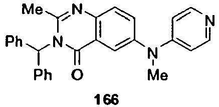

Using Step A from Example 75, substituting compound 23 for compound 145 and substituting 4-(methylamino)-pyridine for 4-fluoro-*N*-methylaniline, followed by Step C from Example 97, compound 166 was prepared.

### Example 188

### Preparation of Compound 167

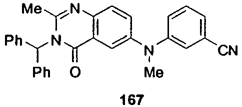

Using Step A from Example 75, substituting compound 23 for compound 145 and substituting 3-aminobenzonitrile for 4-fluoro-*N*-methylaniline, followed by Step C from Example 97, compound 167 was prepared.

### Example 189

### Preparation of Compound 169

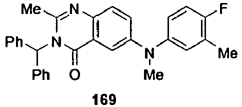

Using Step A from Example 75, substituting compound 23 for compound 145 and substituting 4-fluoro-3-methylaniline for 4-fluoro-*N*-methylaniline, followed by Step C from Example 97, compound 169 was prepared.

### Example 190

### Preparation of Compound 176

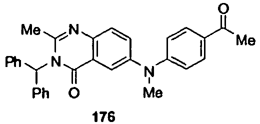

Using Step A from Example 75, substituting compound 23 for compound 145 and substituting 4-aminoacetophenone for 4-fluoro-*N*-methylaniline, followed by Step C from Example 97, compound 176 was prepared.

### Example 191

### Preparation of Compound 179

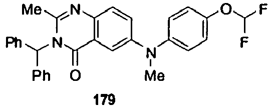

Using Step A from Example 75, substituting compound 23 for compound 145 and substituting 4-difluoromethoxyaniline for 4-fluoro-*N*-methylaniline, followed by Step C from Example 97, compound 179 was prepared.

### Example 192

### Preparation of Compound 180

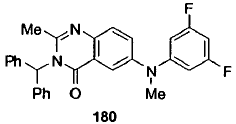

Using Step A from Example 75, substituting compound 23 for compound 145 and substituting 3,5-difluoroaniline for 4-fluoro-*N*-methylaniline, followed by Step C from Example 97, compound 180 was prepared.

### Example 193

### Preparation of Compound 182

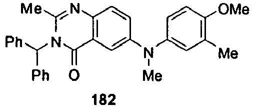

Using Step B from Example 166, substituting 4-bromo-2-methylanisole for 3-iodopyridine, compound 182 was prepared.

### Example 194

### Preparation of Compound 183

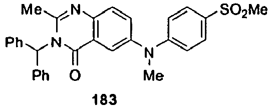

Using Step B from Example 166, substituting 4-bromophenylmethylsulfone for 3-iodopyridine, compound 183 was prepared.

### Example 196

### Preparation of Compound 185

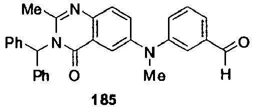

Using Step B from Example 166, substituting 3-bromobenzylalcohol for 3-iodopyridine, compound 185 was prepared.

### Example 197

### Preparation of Compound 189

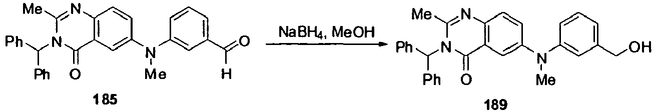

To compound 185 (34 mg, 0.07mmol) was added MeOH (0.3mL) and NaBH₄ (6 mg, 0.14mmol) and the solution was stirred for 12 hours. The solution was purified by preparative TLC using (30%EtOAc/hexanes) to yield compound 189 (15 mg, 46%).

### Example 198

### Preparation of Compound 190

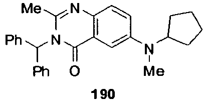

Using Step A from Example 168, substituting cyclopentylbromide for bromoacetonitrile and substituting DMF for CH₃CN, compound 190 was prepared.

### Example 199

### Preparation of Compounds 191

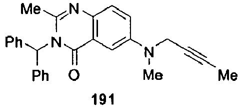

Using Step A from Example 168, substituting 1-bromo-2-butyne for bromoacetonitrile and substituting DMF for CH₃CN, compound 191 was prepared.

### Example 200

### Preparation of Compound 192

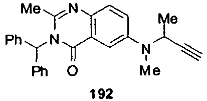

Using Step A from Example 168, substituting 3-chloro-1-butyne for bromoacetonitrile and substituting DMF for CH₃CN, compound 192 was prepared.

### Example 201

### Preparation of Compound 193

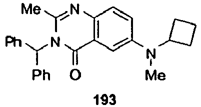

Using Step A from Example 75, substituting compound 23 for compound 145 and substituting cyclobutylamine for 4-fluoro-*N*-methylaniline, followed by Step C from Example 97, compound 193 was prepared.

### Example 202

### Preparation of Compound 196

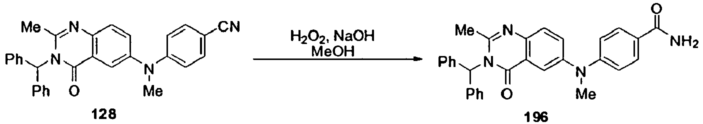

To compound 128 (125 mg, 0.27mmol) was added MeOH (1.5mL), NaOH (1N solution in H₂O, 0.14mL), 0.14mmol), and H₂O₂ (50% in H₂O, 0.082mL, 1.21mmol) and the solution was stirred for 2 hours. Diluted with CH₂Cl₂ and H₂O, separated layers, extracted aqueous layer with CH₂Cl₂, washed organic fraction with 1N HCl, dried (MgSO₄), filtered and concentrated. The residue was purified by preparative TLC using (30%EtOAc/hexanes) to yield compound 196 (59 mg, 46%).

### Example 204

### Preparation of Compound 201

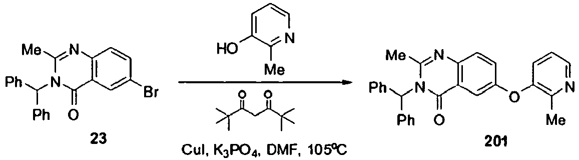

To compound 23 (200 mg, 0.5mmol) was added DMF (5mL), 3-hydroxy-2-methylpyridine (65 mg, 0.6mmol), CuI (5 mg, 0.02mmol), K₃PO₄ (210 mg, 1.0mmol), 2,2,6,6-tetramethylheptan-3,5-dione (18 mg, 0.1mmol) and the solution was stirred and heated to 105 °C for 12 hours. Purified solution using preparative TLC using (50%EtOAc/hexanes) to yield compound 201 (24 mg, 11%).

### Example 205

### Preparation of Compound 205

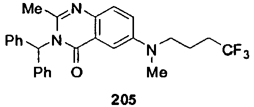

Using Step A from Example 168, substituting 1-iodo-4,4,4-trifluorobutane for bromoacetonitrile and substituting DMF for CH₃CN, compound 205 was prepared.

### Example 206

### Preparation of Compounds 206

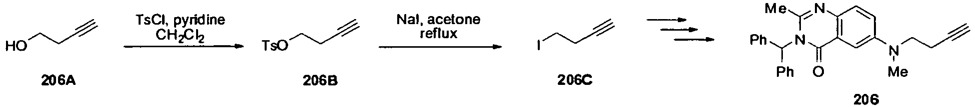

### Step A - Synthesis of Compound 206B

To compound 206A (2.16mL, 28.5mmol) was added CH₂Cl₂ (95mL), pyridine (3.5mL, 42.8mmol) and TsCl (8.2g, 42.8mmol) and the solution was stirred for 12 hours. Purified solution by flash column chromatography using (30%EtOAc/hexanes) to yield compound 206B (3.9g, 62%).

### Step B - Synthesis of Compound 206C

To compound 206B (3.9g, 17.4mmol) was added acetone (58mL) and NaI (5.2g, 34.8mmol) and the solution was heated to reflux for 12 hours. Allowed to cool, removed solid by filtration, and concentrated. Dissolved residue in CH₂Cl₂, washed with H₂O, dried (MgSO₄), filtered, and concentrated to yield compound 206C (1.5g, 50%).

### Step C - Synthesis of Compound 206

Using Step A from Example 168, substituting compound 206C for bromoacetonitrile and substituting DMF for CH₃CN, compound 206 was prepared.

### Example 207

### Preparation of Compound 207

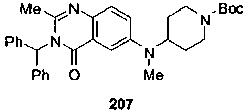

Using Step A from Example 75, substituting compound 23 for compound 145 and substituting 1-Boc-4-(methylamino)piperidine for 4-fluoro-*N*-methylaniline, compound 207 was prepared.

### Example 208

### Preparation of Compound 210

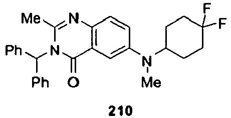

Using Step A from Example 75, substituting compound 23 for compound 145 and substituting 4,4-difluorocyclohexylamine for 4-fluoro-*N*-methylaniline, followed by Step C from Example 97, compound 210 was prepared.

### Example 209

### Preparation of Compound 211

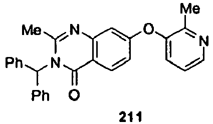

Using Step A from Example 204, substituting compound 24 for compound 23, compound 211 was prepared.

### Example 211

### Preparation of Compounds 214 and 215

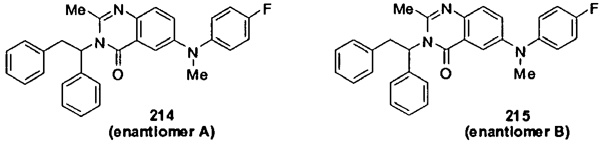

Compound 142 was separated into enantiomers 214 (enantiomer A) and 215 (enantiomer B) using a ChiralAD prep column using (20% IPA/hexanes, 15mL/min).

### Example 212

### Preparation of Compound 217

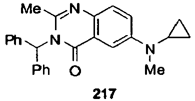

Using Step A from Example 75, substituting compound 23 for compound 145 and substituting cyclopropylamine for 4-fluoro-*N*-methylaniline, followed by Step C from Example 97, compound 217 was prepared.

### Example 213

### Preparation of Compound 218

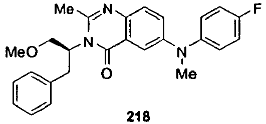

Using Steps B and C from Example 1, substituting compound 176D for compound 1B and substituting (S)-(+)-α-(methoxymethyl)phenethylamine hydrochloride for aminodiphenylmethane, compound 218 was prepared.

### Example 214

### Preparation of Compound 221

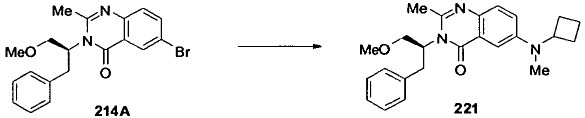

### Step A - Synthesis of Compound 214A

Using Steps A, B, and C from Example 1, substituting (S)-(+)-α-(methoxymethyl)phenethylamine hydrochloride for aminodiphenylmethane, compound 214A was prepared.

### Step B - Synthesis of Compound 222

Using Step A from Example 75, substituting compound 214A for compound 145 and substituting cyclobutylamine for 4-fluoro-*N*-methylaniline, followed by Step C from Example 97, compound 221 was prepared.

### Example 215

### Preparation of Compound 222

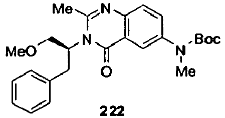

Using Step A from Example 22, substituting compound 214A for compound 23, followed by Step C from Example 97, compound 222 was prepared.

### Example 216

### Preparation of Compound 223

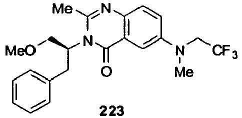

Using Step A from Example 75, substituting compound 214A for compound 145 and substituting 2,2,2-trifluoroethylamine for 4-fluoro-*N*-methylaniline, followed by Step C from Example 97, compound 223 was prepared.

### Example 217

### Preparation of Compound 224

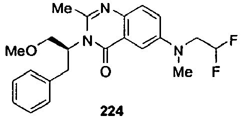

Using Step A from Example 75, substituting compound 214A for compound 145 and substituting 2,2-difluoroethylamine for 4-fluoro-*N*-methylaniline, followed by Step C from Example 97, compound 224 was prepared.

### Example 218

### Preparation of Compound 226

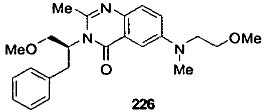

Using Step A from Example 75, substituting compound 214A for compound 145 and substituting 2-methoxyethylamine for 4-fluoro-*N*-methylaniline, followed by Step C from Example 97, compound 226 was prepared.

### Example 219

### Preparation of Compound 227

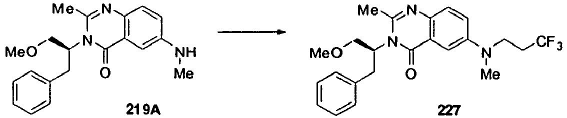

### Step A - Synthesis of Compound 219A

Using Step A from Example 43, substituting compound 222 for compound 3, compound 219A was prepared.

### Step B - Synthesis of Compound 227

Using Step A from Example 168, substituting compound 219A for compound 40 and substituting 3-iodo-1,1,1-trifluoropropane for bromoacetonitrile and substituting DMF for CH₃CN, compound 227 was prepared.

### Example 220

### Preparation of Compound 228

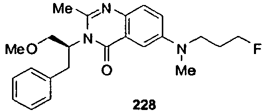

Using Step A from Example 168, substituting compound 219A for compound 40 and substituting 3-iodo-1,1,1-trifluoropropane for bromoacetonitrile and substituting DMF for CH₃CN, compound 228 was prepared.

### Example 221

### Preparation of Compound 231

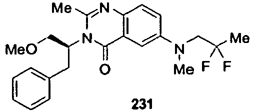

Using Step A from Example 75, substituting compound 214A for compound 145 and substituting 2,2-difluoropropylamine for 4-fluoro-*N*-methylaniline, followed by Step C from Example 97, compound 231 was prepared.

### Example 222

### Preparation of Compound 234

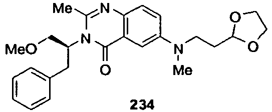

Using Step A from Example 168, substituting compound 219A for compound 40 and substituting 2-(2-bromoethyl)-1,3-dioxolane for bromoacetonitrile and substituting DMF for CH₃CN, compound 234 was prepared.

### Example 223

### Preparation of Compound 235

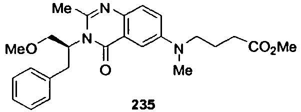

Using Step A from Example 168, substituting compound 219A for compound 40 and substituting methyl-4-iodobutyrate for bromoacetonitrile and substituting DMF for CH₃CN, compound 235 was prepared.

### Example 224

### Preparation of Compound 236

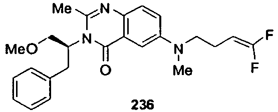

Using Step A from Example 168, substituting compound 219A for compound 40 and substituting 4-bromo-1,1-dilluorobut-1-ene for bromoacetonitrile and substituting DMF for CH₃CN, compound 236 was prepared.

### Example 225

### Preparation of Compound 243

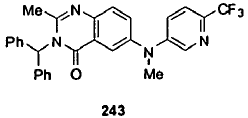

Using Step A from Example 75, substituting 3-amino-5-(trifluoromethyl)pyridine for 4-fluoro-N-methylaniline, followed by Step C from Example 97, compound 243 was prepared.

### Example 226

### Preparation of Compound 245

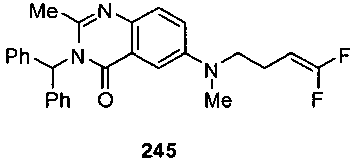

Using Step A from Example 168, substituting 4-bromo-1,1-difluorobut-1-ene for bromoacetonitrile and substituting DMF for CH₃CN, compound 245 was prepared.

### Example 227

### Preparation of Compound 246

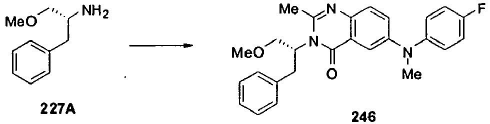

Using Steps B and C from Example 1, substituting compound 176D for compound 1B and substituting compound 227A (prepared using methods from Example 83 substituting (R)-(+)-2-amino-3-phenyl-1-propanol for compound 83A) for aminodiphenylmethane, compound 246 was prepared.

### Example 228

### Preparation of Compound 247

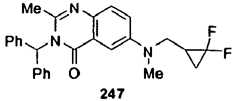

Using Step A from Example 168, substituting 1-bromomethyl-2,2-difluorocyclopropane for bromoacetonitrile and substituting DMF for CH₃CN, compound 247 was prepared.

### Example 229

### Preparation of Compound 250

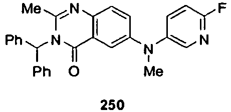

Using Step B from Example 166, substituting 5-bromo-2-fluoropyridine for 3-iodopyridine, compound 250 was prepared.

### Example 230

### Preparation of Compound 251

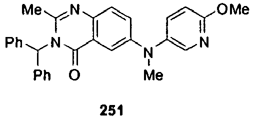

Using Step B from Example 166, substituting 5-bromo-2-methoxypyridine for 3-iodopyridine, compound 251 was prepared.

### Example 231

### Preparation of Compound 254

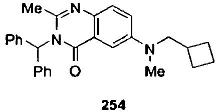

Using Step A from Example 168, substituting bromomethylcyclobutane for bromoacetonitrile and substituting DMF for CH₃CN, compound 254 was prepared.

### Example 232

### Preparation of Compound 257

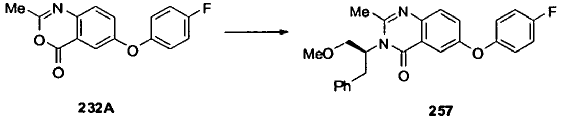

### Step A - Synthesis of Compound 232A

Using Step A from Example 1, substituting compound 111D for compound 1A, compound 232A was prepared.

### Step B - Synthesis of Compound 257

Using Steps B and C from Example 1, substituting compound 232A for compound 1B and substituting (S)-(+)-α-(methoxymethyl)phenethylamine for aminodiphenylmethane, compound 257 was prepared.

### Example 233

### Preparation of Compound 258

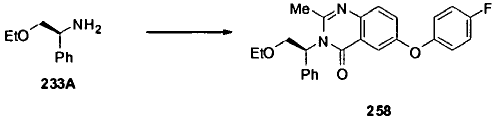

Using Steps B and C from Example 1, substituting compound 232A for compound 1B and substituting compound 233A (prepared using methods from Example 83 substituting 2-ethoxy-1(S)-phenyl-ethylamine for compound 83A) for aminodiphenylmethane, compound 258 was prepared.

### Example 234

### Preparation of Compound 259

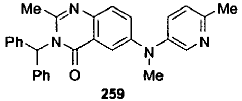

Using Step B from Example 166, substituting 5-bromo-2-methylpyridine for 3-iodopyridine, compound 259 was prepared.

### Example 235

### Preparation of Compound 260

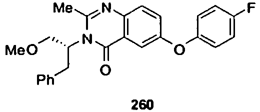

Using Steps B and C from Example 1, substituting compound 232A for compound 1B and substituting compound 227A for aminodiphenylmethane, compound 260 was prepared.

### Example 236

### Preparation of Compound 261

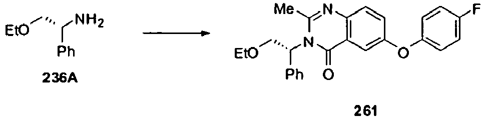

Using Steps B and C from Example 1, substituting compound 232A for compound 1B and substituting compound 236A (prepared using methods from Example 83 substituting 2-ethoxy-1(R)-phenyl-ethylamine for compound 83A) for aminodiphenylmethane, compound 261 was prepared.

### Example 238

### Preparation of Compound 265

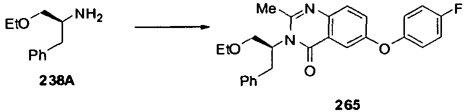

Using Steps B and C from Example 1, substituting compound 232A for compound 1B and substituting compound 238A (prepared using methods from Example 83 substituting (S)-(-)-2-amino-3-phenyl-1-propanol for compound 83A) for aminodiphenylmethane, compound 265 was prepared.

### Example 239

### Preparation of Compound 266

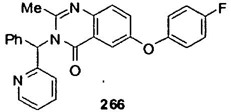

Using Steps B and C from Example 1, substituting compound 232A for compound 1B and substituting phenyl-2-pyridinmethane hydrochloride for aminodiphenylmethane, compound 266 was prepared.

### Example 240

### Preparation of Compound 267

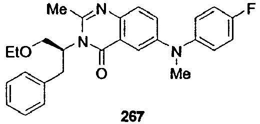

Using Steps B and C from Example 1, substituting compound 176D for compound 1B and substituting compound 238A for aminodiphenylmethane, compound 267 was prepared.

### Example 241

### Preparation of Compound 268

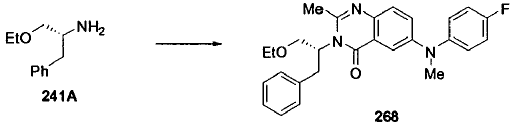

Using Steps B and C from Example 1, substituting compound 176D for compound 1B and substituting compound 241A (prepared analogously to compound 238A) for aminodiphenylmethane, compound 268 was prepared.

### Example 242

### Preparation of Compound 269

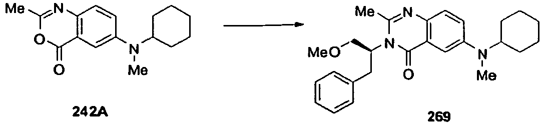

### Step A - Synthesis of Compound 242A

Using methods described for compound 176D in Example 176, compound 242A was prepared substituting cyclohexylamine for 4-fluoro-N-methylaniline.

### Step B - Synthesis of Compound 269

Using Steps B and C from Example 1, substituting compound 242A for compound 1B and substituting (S)-(+)-α-(methoxymethyl)phenethylamine for aminodiphenylmethane, compound 269 was prepared.

### Example 244

### Preparation of Compound 5

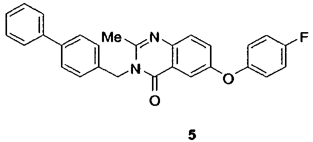

Using Steps B and C from Example 1, substituting compound 232A for compound 1B and substituting 4-phenylbenzylamine for aminodiphenylmethane, compound 5 was prepared.

### Example 245

### Preparation of Compound 41

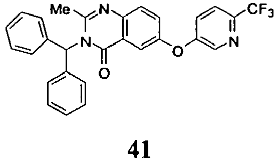

Using the method from Example 75, substituting compound 105A for compound 145 and substituting compound 3-bromo-5-(trifluoromethyl)pyridine for 4-fluoro-N-methylaniline, compound 41 was prepared.

### Example 246

### Preparation of Compound 57

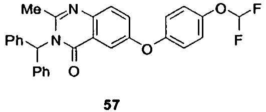

Using Step A from Example 204, substituting 4-(difluoromethoxy)phenol for 3-hydroxy-2-methylpyridine, compound 57 was prepared.

### Example 248

### Preparation of Compounds 122

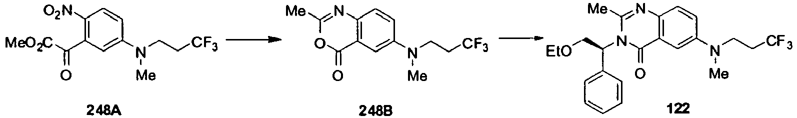

### Step A - Synthesis of Compound 248A

Using Step A from Example 111, substituting 3,3,3-trifluoropropylamine for 4-fluorophenol, followed by Step C from Example 97, compound 248A was prepared.

### Step B - Synthesis of Compound 248B

Using Steps B, C, and D from Example 111, substituting compound 248A for compound 118B, compound 248B was prepared.

### Step C - Synthesis of Compound 122

Using Steps B and C from Example 1, substituting compound 248B for compound 1B and substituting compound 233A for aminodiphenylmethane, compound 122 was prepared.

### Example 249

### Preparation of Compound 139

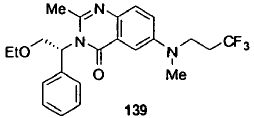

Using Steps B and C from Example 1, substituting compound 248B for compound 1B and substituting compound 236A for aminodiphenylmethane, compound 139 was prepared.

### Example 250

### Preparation of Compound 274

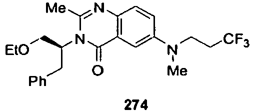

Using Steps B and C from Example 1, substituting compound 248B for compound 1B and substituting compound 238A for aminodiphenylmethane, compound 274 was prepared.

### Example 251

### Preparation of Compound 1

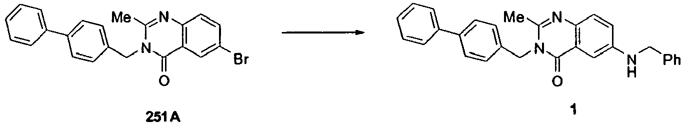

Using Steps A, B, and C from Example 1, substituting 4-phenylbenzylamine for aminodiphenylmethane, compound 251A was prepared. Using Step A from Example 75, substituting compound 251A for 145 and substituting benzylamine for 4-fluoro-*N-*methylaniline, compound 1 was prepared.

### Example 252

### cAMP assay

The ability of illustrative compounds of the invention to activate GPR119 and stimulate increases in cAMP levels was determined using the LANCE™ cAMP kit (Perkin Elmer). HEK293 cells expressing human GPR119 were maintained in culture flasks at 37 °C/5% CO₂ in DMEM containing 10% fetal bovine serum, 100 U/ml Pen/Strep, and 0.5 mg/ml geneticin. The media was changed to Optimem and cells were incubated overnight at 37 °C /5% CO₂. The Optimem was then aspirated and the cells were removed from the flasks using room temperature Hank's balanced saline solution (HBSS). The cells were pelleted using centrifugation (1300 rpm, 7 minutes, room temperature), then resuspended in stimulation buffer (HBSS, 0.1% BSA, 5 mM HEPES, 15 µM RO-20) at 2.5 x 10⁶ cells/mL. Alexa Fluor 647-anti cAMP antibody (1:100) was then added to the cell suspension and incubated for 30 minutes. A representative Bicyclic Heterocycle Derivative (6 µl at 2X concentration) in stimulation buffer containing 2% DMSO were then added to white 384 well Matrix plates. Cell suspension mix (6 µl) was added to each well and incubated with the Bicyclic Heterocycle Derivative for 30 minutes. A cAMP standard curve was also created in each assay according to the kit protocol. Standard concentrations of cAMP in stimulation buffer (6 µl) were added to white 384 well plates. Subsequently, 6 µl of 1:100 anti-cAMP antibody was added to each well. Following the 30 minute incubation period, 12 µl of detection mix (included in kit) was added to all wells and incubated for 2-3 hours at room temperature. Fluorescence was detected on the plates using an Envision instrument. The level of cAMP in each well is determined by extrapolation from the cAMP standard curve.

Using this assay, EC₅₀ values for various illustrative Bicyclic Heterocycle Derivatives pf the present invention were calculated and range from about 10 nM to about 20 µM.

### Example 253

### Effect of the Compounds of the Invention in Oral Glucose Tolerance Test

Male C57Bl/6NCrl mice (6-8 week old) were fasted overnight and randomly dosed with either vehicle (20% hydroxypropyl-β-cyclodextrin) or a representative compound of the invention (at 3, 10 or 30 mg/kg) via oral gavage (n=8 mice/group). Glucose was administered to the animals 30 minutes post-dosing (3 g/kg p.o.). Blood glucose was measured prior to administration of test compound and glucose, and at 20 minutes after glucose administration using a hand-held glucometer (Ascensia Elite, Bayer).

Using this protocol, the effects of various Bicyclic Heterocycle Derivatives of the present invention were measured and indicate that the Bicyclic Heterocycle Derivatives of the present invention are effective in lowering blood glucose levels after glucose challenge.

### Example 254

### Effect of the Compounds of the Invention in an Animal Model of Diabetes

Male C57Bl/6NCrl mice can be used to generate a nongenetic model of type 2 diabetes mellitus as previously described *(*Metabolism 47(6): 663-668, 1998). Briefly, mice (4 weeks of age) are made insulin resistant by high fat feeding (60% of kcal as fat) and hyperglycemia is then induced using a low dose of streptozotocin (100 mg/kg i.p.). Eight weeks after streptozotocin administration, the diabetic mice are placed into one of 3 groups (n = 13/gp), wherein group I receives vehicle (20% hydroxypropyl-β-cyclodextrin p.o.); group II receives test compound (30 mg/kg p.o.) and group III receives either glipizide (20 mg/kg p.o.) or exendin-4 (10 ug/kg i.p.). Mice are dosed once daily for 13 consecutive days, and blood glucose levels are measured daily using, for example, a hand held glucometer, to determine the effects of the test compound(s) on glucose levels of the diabetic animals.

### Uses of the Bicyclic Heterocycle Derivatives

The Bicyclic Heterocycle Derivatives are useful in human and veterinary medicine for treating or preventing a Condition in a patient. In accordance with the invention, the Bicyclic Heterocycle Derivatives can be administered to a patient in need of treatment or prevention of a Condition.

In one embodiment, the present invention provides a method for treating diabetes, a diabetic complication, obesity, metabolic syndrome or a cardiovascular disease in a patient, the method comprising administering to the patient an effective amount of one or more compounds of formula (I) or a pharmaceutically acceptable salt, solvate, ester or prodrug thereof.

### Treatment of Obesity and Obesity-Related Disorders

The Bicyclic Heterocycle Derivatives can also be useful for treating obesity or an obesity-related disorder.

Accordingly, in one embodiment, the invention provides methods for treating obesity or an obesity-related disorder in a patient, wherein the method comprises administering to the patient an effective amount of one or more Bicyclic Heterocycle Derivatives, or a pharmaceutically acceptable salt, solvate, ester, prodrug or stereoisomer thereof.

### Treatment of Diabetes

The Bicyclic Heterocycle Derivatives are useful for treating diabetes in a patient. Accordingly, in one embodiment, the present invention provides treating diabetes in a patient, comprising administering to the patient an effective amount of one or more Bicyclic Heterocycle Derivatives.

Examples of diabetes treatable or preventable using the Bicyclic Heterocycle Derivatives include, but are not limted to, type I diabetes (insulin-dependent diabetes mellitus), type II diabetes (non-insulin dependent diabetes mellitus), gestational diabetes, autoimmune diabetes, insulinopathies, idiopathic type I diabetes (Type 1b), latent autoimmumne diabetes in adults, early-onset type 2 diabetes (EOD), youth-onset atypical diabetes (YOAD), maturity onset diabetes of the young (MODY), malnutrition-related diabetes, diabetes due to pancreatic disease, diabetes associated with other endocrine diseases (such as Cushing's Syndrome, acromegaly, pheochromocytoma, glucagonoma, primary aldosteronism or somatostatinoma), type A insulin resistance syndrome, type B insulin resistance syndrome, lipatrophic diabetes, diabetes induced by β-cell toxins, and diabetes induced by drug therapy (such as diabetes induced by antipsychotic agents).

In one embodiment, the diabetes is type I diabetes.

In another embodiment, the diabetes is type II diabetes.

### Treatment of a Diabetic Complication

The Bicyclic Heterocycle Derivatives are also useful for treating a diabetic complication in a patient. Accordingly, in one embodiment, the present invention provides treating a diabetic complication in a patient, comprising administering to the patient an effective amount of one or more Bicyclic Heterocycle Derivatives.

Examples of diabetic complications treatable or preventable using the Bicyclic Heterocycle Derivatives include, but are not limted to, diabetic cataract, glaucoma, retinopathy, aneuropathy (such as diabetic neuropathy, polyneuropathy, mononeuropathy, autonomic neuropathy, microaluminuria and progressive diabetic neuropathyl), nephropathy, gangrene of the feet, immune-complex vasculitis, systemic lupsus erythematosus (SLE), atherosclerotic coronary arterial disease, peripheral arterial disease, nonketotic hyperglycemic-hyperosmolar coma, foot ulcers, joint problems, a skin or mucous membrane complication (such as an infection, a shin spot, a candidal infection or necrobiosis lipoidica diabeticorumobesity), hyperlipidemia, cataract, hypertension, syndrome of insulin resistance, coronary artery disease, a fungal infection, a bacterial infection, and cardiomyopathy.

### Treatment of a Metabolic Disorder

The Bicyclic Heterocycle Derivatives can also be useful for treating a metabolic disorder. Examples of metabolic disorders treatable include, but are not limited to, metabolic syndrome (also known as "Syndrome X"), impaired glucose tolerance, impaired fasting glucose, hypercholesterolemia, hyperlipidemia, hypertriglyceridemia, low HDL levels, hypertension, phenylketonuria, post-prandial lipidemia, a glycogen-storage disease, Gaucher's Disease, Tay-Sachs Disease, Niemann-Pick Disease, ketosis and acidosis.

Accordingly, in one embodiment, the invention provides methods for treating a metabolic disorder in a patient, wherein the method comprises administering to the patient an effective amount of one or more Bicyclic Heterocycle Derivatives, or a pharmaceutically acceptable salt, solvate, ester, prodrug or stereoisomer thereof

In one embodiment, the metabolic disorder is hypercholesterolemia.

In another embodiment, the metabolic disorder is hyperlipidemia.

In another embodiment, the metabolic disorder is hypertriglyceridemia.

In still another embodiment, the metabolic disorder is metabolic syndrome.

In a further embodiment, the metabolic disorder is low HDL levels.

### Treating a Cardiovascular Disease

The Bicyclic Heterocycle Derivatives are useful for treating or preventing a cardiovascular disease in a patient.

Accordingly, in one embodiment, the present invention provides treatment of a cardiovascular disease in a patient, comprising administering to the patient an effective amount of one or more Bicyclic Heterocycle Derivatives.

Illustrative examples of cardiovascular diseases treatable or preventable, include, but are not limited to atherosclerosis, congestive heart failure, cardiac arrhythmia, myocardial infarction, atrial fibrillation, atrial flutter, circulatory shock, left ventricular hypertrophy, ventricular tachycardia, supraventricular tachycardia, coronary artery disease, angina, infective endocarditis, non-infective endocarditis, cardiomyopathy, peripheral artery disease, Reynaud's phenomenon, deep venous thrombosis, aortic stenosis, mitral stenosis, pulmonic stenosis and tricuspid stenosis.

In one embodiment, the cardiovascular disease is atherosclerosis.

In another embodiment, the cardiovascular disease is congestive heart failure.

In another embodiment, the cardiovascular disease is coronary artery disease.

### Combination Therapy

In one embodiment, the present invention provides methods for treating a Condition in a patient, the method comprising administering to the patient one or more Bicyclic Heterocycle Derivatives, or a pharmaceutically acceptable salt, solvate, ester, prodrug or stereoisomer thereof and at least one additional therapeutic agent that is not a Bicyclic Heterocycle Derivative, wherein the amounts administered are together effective to treat or prevent a Condition.

Non-limiting examples of additional therapeutic agents useful in the present methods for treating or preventing a Condition include, anti-obesity agents, antidiabetic agents, any agent useful for treating metabolic syndrome, any agent useful for treating a cardiovascular disease, cholesterol biosynthesis inhibitors, cholesterol absorption inhibitors, bile acid sequestrants, probucol derivatives, IBAT inhibitors, nicotinic acid receptor (NAR) agonists, ACAT inhibitors, cholesteryl ester transfer proten (CETP) inhibitors, low-denisity lipoprotein (LDL) activators, fish oil, water-soluble fibers, plant sterols, plant stanols, fatty acid esters of plant stanols, or any combination of two or more of these additional therapeutic agents.

Non-limiting examples of anti-obesity agents useful in the present methods for treating a Condition include CB1 antagonists or inverse agonists such as rimonabant, neuropeptide Y antagonists, MCR4 agonists, MCH receptor antagonists, histamine H₃ receptor antagonists or inverse agonists, metabolic rate enhancers, nutrient absorption inhibitors, leptin, appetite suppressants and lipase inhibitors.

Non-limiting examples of appetite suppressant agents useful in the present methods for treating or preventing a Condition include cannabinoid receptor 1 (CB₁) antagonists or inverse agonists (*e.g.,* rimonabant); Neuropeptide Y (NPY1, NPY2, NPY4 and NPY5) antagonists; metabotropic glutamate subtype 5 receptor (mGluR5) antagonists (*e.g.,* 2-methyl-6-(phenylethynyl)-pyridine and 3[(2-methyl-1,4-thiazol-4-yl)ethynyl]pyridine); melanin-concentrating hormone receptor (MCH1R and MCH2R) antagonists; melanocortin receptor agonists (*e.g.,* Melanotan-II and Mc4r agonists); serotonin uptake inhibitors (e.g., dexfenfluramine and fluoxetine); serotonin (5HT) transport inhibitors (*e.g*., paroxetine, fluoxetine, fenfluramine, fluvoxamine, sertaline and imipramine); norepinephrine (NE) transporter inhibitors (*e.g.,* desipramine, talsupram and nomifensine); ghrelin antagonists; leptin or derivatives thereof; opioid antagonists (*e.g.,* nalmefene, 3-methoxynaltrexone, naloxone and nalterxone); orexin antagonists; bombesin receptor subtype 3 (BRS3) agonists; Cholecystokinin-A (CCK-A) agonists; ciliary neurotrophic factor (CNTF) or derivatives thereof (*e.g.,* butabindide and axokine); monoamine reuptake inhibitors (*e.g.,* sibutramine); glucagon-like peptide 1 (GLP-1) agonists; topiramate; and phytopharm compound 57.

Non-limiting examples of metabolic rate enhancers useful in the present methods for treating or preventing a Condition include acetyl-CoA carboxylase-2 (ACC2) inhibitors; beta adrenergic receptor 3 (β3) agonists; diacylglycerol acyltransferase inhibitors (DGAT1 and DGAT2); fatty acid synthase (FAS) inhibitors (*e.g.,* Cerulenin); phosphodiesterase (PDE) inhibitors (*e.g.,* theophylline, pentoxifylline, zaprinast, sildenafil, amrinone, milrinone, cilostamide, rolipram and cilomilast); thyroid hormone β agonists; uncoupling protein activators (UCP-1,2 or 3) (*e.g.,* phytanic acid, 4-[(E)-2-(5,6,7,8-tetramethyl-2-naphthalenyl)-1-propenyl]benzoic acid and retinoic acid); acyl-estrogens (*e.g.,* oleoyl-estrone); glucocorticoid antagonists; 11-beta hydroxy steroid dehydrogenase type 1 (11β HSD-1) inhibitors; melanocortin-3 receptor (Mc3r) agonists; and stearoyl-CoA desaturase-1 (SCD-1) compounds.

Non-limiting examples of nutrient absorption inhibitors useful in the present methods for treating or preventing a Condition include lipase inhibitors (e.g., orlistat, lipstatin, tetrahydrolipstatin, teasaponin and diethylumbelliferyl phosphate); fatty acid transporter inhibitors; dicarboxylate transporter inhibitors; glucose transporter inhibitors; and phosphate transporter inhibitors.

Non-limiting examples of cholesterol biosynthesis inhibitors useful in the present methods for treating or preventing a Condition include HMG-CoA reductase inhibitors, squalene synthase inhibitors, squalene epoxidase inhibitors, and mixtures thereof.

Non-limiting examples of cholesterol absorption inhibitors useful in the present methods for treating or preventing a Condition include ezetimibe. In one embodiment, the cholesterol absorption inhibitor is ezetimibe.

HMG-CoA reductase inhibitors useful in the present methods for treating or preventing a Condition include, but are not limited to, statins such as lovastatin, pravastatin, fluvastatin, simvastatin, atorvastatin, cerivastatin, CI-981, resuvastatin, rivastatin, pitavastatin, rosuvastatin or L-659,699 ((E,E)-11-[3'R-(hydroxy-methyl)-4'-oxo-2'R-oxetanyl]-3,5,7R-trimethyl-2,4-undecadienoic acid).

Squalene synthesis inhibitors useful in the present methods for treating or preventing a Condition include, but are not limited to, squalene synthetase inhibitors; squalestatin 1; and squalene epoxidase inhibitors, such as NB-598 ((E)-N-ethyl-N-(6,6-dimethyl-2-hepten-4-ynyl)-3-[(3,3'-bithiophen-5-yl)methoxy]benzene-methanamine hydrochloride).

Bile acid sequestrants useful in the present methods for treating or preventing a Condition include, but are not limited to, cholestyramine (a styrene-divinylbenzene copolymer containing quaternary ammonium cationic groups capable of binding bile acids, such as QUESTRAN® or QUESTRAN LIGHT® cholestyramine which are available from Bristol-Myers Squibb), colestipol (a copolymer of diethylenetriamine and 1-chloro-2,3-epoxypropane, such as COLESTID® tablets which are available from Pharmacia), colesevelam hydrochloride (such as WelChol® Tablets (poly(allylamine hydrochloride) cross-linked with epichlorohydrin and alkylated with 1-bromodecane and (6-bromohexyl)-trimethylammonium bromide) which are available from Sankyo), water soluble derivatives such as 3,3-ioene, N-(cycloalkyl) alkylamines and poliglusam, insoluble quatemized polystyrenes, saponins and mixtures thereof. Suitable inorganic cholesterol sequestrants include bismuth salicylate plus montmorillonite clay, aluminum hydroxide and calcium carbonate antacids.

Probucol derivatives useful in the present methods for treating or preventing a Condition include, but are not limited to, AGI-1067 and others disclosed in U.S. Patents Nos. 6,121,319 and 6,147,250.

IBAT inhibitors useful in the present methods for treating or preventing a Condition include, but are not limited to, benzothiepines such as therapeutic compounds comprising a 2,3,4,5-tetrahydro-1-benzothiepine 1,1-dioxide structure such as are disclosed in International Publication No. WO 00/38727.

Nicotinic acid receptor agonists useful in the present methods for treating or preventing a Condition include, but are not limited to, those having a pyridine-3-carboxylate structure or a pyrazine-2-carboxylate structure, including acid forms, salts, esters, zwitterions and tautomers, where available. Other examples of nicotinic acid receptor agonists useful in the present methods include nicotinic acid, niceritrol, nicofuranose and acipimox. An example of a suitable nicotinic acid product is NIASPAN® (niacin extended-release tablets) which are available from Kos Pharmaceuticals, Inc. (Cranbury, NJ). Further nicotinic acid receptor agonists useful in the present methods for treating or preventing a Condition include, but are not limited to, the compounds disclosed in U.S. Patent Publication Nos. US2006/0264489, US2007/0066630, 2007/0173495 and US2008/0019978, each of which is incorporated herein by reference.

ACAT inhibitors useful in the present methods for treating or preventing a Condition include, but are not limited to, avasimibe, HL-004, lecimibide and CL-277082 (*N*-(2,4-difluorophenyl)-*N*-[[4-(2,2-dimethylpropyl)phenyl]-methyl]-N-heptylurea). See P. Chang et al., "Current, New and Future Treatments in Dyslipidaemia and Atherosclerosis", Drugs 2000 Jul;60(1); 55-93, which is incorporated by reference herein.

CETP inhibitors useful in the present methods for treating or preventing a Condition include, but are not limited to, those disclosed in International Publication No. WO 00/38721 and U.S. Patent No. 6,147,090, which are incorporated herein by reference.

LDL-receptor activators useful in the present methods for treating or preventing a Condition include, but are not limited to, HOE-402, an imidazolidinyl-pyrimidine derivative that directly stimulates LDL receptor activity. See M. Huettinger et al., "Hypolipidemic activity of HOE-402 is Mediated by Stimulation of the LDL Receptor Pathway", Arterioscler.Thromb. 1993; 13:1005-12.

Natural water-soluble fibers useful in the present methods for treating or preventing a Condition include, but are not limited to, psyllium, guar, oat and pectin.

Fatty acid esters of plant stanols useful in the present methods for treating or preventing a Condition include, but are not limited to, the sitostanol ester used in BENECOL® margarine.

Non-limiting examples of antidiabetic agents useful in the present methods for treating a Condition include insulin sensitizers, β-glucosidase inhibitors, DPP-IV inhibitors, insulin secretagogues, hepatic glucose output lowering compounds, antihypertensive agents, sodium glucose uptake transporter 2 (SGLT-2) inhibitors, insulin and insulin-containing compositions, and anti-obesity agents as set forth above.

In one embodiment, the antidiabetic agent is an insulin secretagogue. In one embodiment, the insulin secretagogue is a sulfonylurea.

Non-limiting examples of sulfonylureas useful in the present methods include glipizide, tolbutamide, glyburide, glimepiride, chlorpropamide, acetohexamide, gliamilide, gliclazide, gliquidone, glibenclamide and tolazamide.

In another embodiment, the insulin secretagogue is a meglitinide.

Non-limiting examples of meglitinides useful in the present methods for treating a Condition include repaglinide, mitiglinide, and nateglinide.

In still another embodiment, the insulin secretagogue is GLP-1 or a GLP-1 mimetic.

Non-limiting examples of GLP-1 mimetics useful in the present methods include Byetta-Exanatide, Liraglutinide, CJC-1131 (ConjuChem), Exanatide-LAR (Amylin), BIM-51077 (Ipsen/LaRoche), ZP-10 (Zealand Pharmaceuticals), and compounds disclosed in International Publication No. WO 00/07617.

Other non-limiting examples of insulin secretagogues useful in the present methods include exendin, GIP and secretin.

In another embodiment, the antidiabetic agent is an insulin sensitizer.

Non-limiting examples of insulin sensitizers useful in the present methods include PPAR activators or agonists, such as troglitazone, rosiglitazone, pioglitazone and englitazone; biguanidines such as metformin and phenformin; PTP-1B inhibitors; and glucokinase activators.

In another embodiment, the antidiabetic agent is a β-Glucosidase inhibitor.

Non-limiting examples of β-Glucosidase inhibitors useful the present methods include miglitol, acarbose, and voglibose.

In another embodiment, the antidiabetic agent is an hepatic glucose output lowering agent.

Non-limiting examples of hepatic glucose output lowering agents useful in the present methods include Glucophage and Glucophage XR.

In yet another embodiment, the antidiabetic agent is insulin, including all formulations of insulin, such as long acting and short acting forms of insulin.

Non-limiting examples of orally administrable insulin and insulin containing compositions include AL-401 from AutoImmune, and the compositions disclosed in U.S.

Patent Nos. 4,579,730; 4,849,405; 4,963,526; 5,642,868; 5,763,396; 5,824,638; 5,843,866; 6,153,632; 6,191,105; and International Publication No. WO 85/05029, each of which is incorporated herein by reference.

In another embodiment, the antidiabetic agent is a DPP-IV inhibitor.

Non-limiting examples of DPP-IV inhibitors useful in the present methods include sitagliptin, saxagliptin (Januvia™, Merck), denagliptin, vildagliptin (Galvus™, Novartis), alogliptin, alogliptin benzoate, ABT-279 and ABT-341 (Abbott), ALS-2-0426 (Alantos), ARI-2243 (Arisaph), BI-A and BI-B (Boehringer Ingelheim), SYR-322 (Takeda), MP-513 (Mitsubishi), DP-893 (Pfizer), RO-0730699 (Roche) or a combination of sitagliptin/metformin HCl (Janumet™, Merck).

In a further embodiment, the antidiabetic agent is a SGLT-2 inhibitor.

Non-limiting examples of SGLT-2 inhibitors useful in the present methods include dapagliflozin, sergliflozin, AVE2268 (Sanofi-Aventis) and T-1095 (Tanabe Seiyaku).

Non-limiting examples of antihypertensive agents useful in the present methods for treating a Condition include β-blockers and calcium channel blockers (e.g., diltiazem, verapamil, nifedipine, amlopidine, and mybefradil), ACE inhibitors (e.g., captopril, lisinopril, enalapril, spirapril, ceranopril, zefenopril, fosinopril, cilazopril, and quinapril), AT-1 receptor antagonists (e.g., losartan, irbesartan, and valsartan), renin inhibitors and endothelin receptor antagonists (e.g., sitaxsentan).

In one embodiment, the antidiabetic agent is an agent that slows or blocks the breakdown of starches and certain sugars.

Non-limiting examples of antidiabetic agents that slow or block the breakdown of starches and certain sugars and are suitable for use in the compositions and methods of the present invention include alpha-glucosidase inhibitors and certain peptides for increasing insulin production. Alpha-glucosidase inhibitors help the body to lower blood sugar by delaying the digestion of ingested carbohydrates, thereby resulting in a smaller rise in blood glucose concentration following meals. Non-limiting examples of suitable alpha-glucosidase inhibitors include acarbose, miglitol, camiglibose; certain polyamines as disclosed in WO 01/47528 (incorporated herein by reference) and voglibose. Non-limiting examples of suitable peptides for increasing insulin production including amlintide (CAS Reg. No. 122384-88-7 from Amylin; pramlintide, exendin, and certain compounds having Glucagon-like peptide-1 (GLP-1) agonistic activity as disclosed in International Publication No. WO 00/07617.

Other specific additional therapeutic agents useful in the present methods for treating or preventing a Condition include, but are not limited to, rimonabant, 2-methyl-6-(phenylethynyl)-pyridine, 3[(2-methyl-1,4-thiazol-4-yl)ethynyl]pyridine, Melanotan-II, dexfenfluramine, fluoxetine, paroxetine, fenfluramine, fluvoxamine, sertaline, imipramine, desipramine, talsupram, nomifensine, leptin, nalmefene, 3-methoxynaltrexone, naloxone, nalterxone, butabindide, axokine, sibutramine, topiramate, phytopharm compound 57, Cerulenin, theophylline, pentoxifylline, zaprinast, sildenafil, amrinone, milrinone, cilostamide, rolipram, cilomilast, phytanic acid, 4-[(E)-2-(5,6,7,8-tetramethyl-2-naphthalenyl)-1-propenyl]benzoic acid, retinoic acid, oleoyl-estrone, orlistat, lipstatin, tetrahydrolipstatin, teasaponin and diethylumbelliferyl phosphate.

In one embodiment, the present combination therapies for treating or preventing diabetes comprise administering a Bicyclic Heterocycle Derivative, an antidiabetic agent and/or an antiobesity agent.

In another embodiment, the present combination therapies for treating or preventing diabetes comprise administering a Bicyclic Heterocycle Derivative and an antidiabetic agent.

In another embodiment, the present combination therapies for treating or preventing diabetes comprise administering a Bicyclic Heterocycle Derivative and an anti-obesity agent.

In one embodiment, the present combination therapies for treating or preventing obesity comprise administering a Bicyclic Heterocycle Derivative, an antidiabetic agent and/or an antiobesity agent.

In another embodiment, the present combination therapies for treating or preventing obesity comprise administering a Bicyclic Heterocycle Derivative and an antidiabetic agent.

In another embodiment, the present combination therapies for treating or preventing obesity comprise administering a Bicyclic Heterocycle Derivative and an anti-obesity agent.

In one embodiment, the present combination therapies for treating or preventing metabolic syndrome comprise administering a Bicyclic Heterocycle Derivative and one or more additional therapeutic agents selected from: anti-obesity agents, antidiabetic agents, any agent useful for treating metabolic syndrome, any agent useful for treating a cardiovascular disease, cholesterol biosynthesis inhibitors, sterol absorption inhibitors, bile acid sequestrants, probucol derivatives, IBAT inhibitors, nicotinic acid receptor (NAR) agonists, ACAT inhibitors, cholesteryl ester transfer proten (CETP) inhibitors, low-denisity lipoprotein (LDL) activators, fish oil, water-soluble fibers, plant sterols, plant stanols and fatty acid esters of plant stanols.

In one embodiment, the additional therapeutic agent is a cholesterol biosynthesis inhibitor. In another embodiment, the cholesterol biosynthesis inhibitor is a squalene synthetase inhibitor. In another embodiment, the cholesterol biosynthesis inhibitor is a squalene epoxidase inhibitor. In still another embodiment, the cholesterol biosynthesis inhibitor is an HMG-CoA reductase inhibitor. In another embodiment, the HMG-CoA reductase inhibitor is a statin. In yet another embodiment, the statin is lovastatin, pravastatin, simvastatin or atorvastatin.

In one embodiment, the additional therapeutic agent is a cholesterol absorption inhibitor. In another embodiment, the cholesterol absorption inhibitor is ezetimibe.

In one embodiment, the additional therapeutic agent comprises a cholesterol absorption inhibitor and a cholesterol biosynthesis inhibitor. In another embodiment, the additional therapeutic agent comprises a cholesterol absorption inhibitor and a statin. In another embodiment, the additional therapeutic agent comprises ezetimibe and a statin. In another embodiment, the additional therapeutic agent comprises ezetimibe and simvastatin.

In one embodiment, the present combination therapies for treating or preventing metabolic syndrome comprise administering a Bicyclic Heterocycle Derivative, an antidiabetic agent and/or an antiobesity agent.

In another embodiment, the present combination therapies for treating or preventing metabolic syndrome comprise administering a Bicyclic Heterocycle Derivative and an antidiabetic agent.

In another embodiment, the present combination therapies for treating or preventing metabolic syndrome comprise administering a Bicyclic Heterocycle Derivative and an anti-obesity agent.

In one embodiment, the present combination therapies for treating or preventing a cardiovascular disease comprise administering one or more Bicyclic Heterocycle Derivatives, and an additional agent useful for treating or preventing a cardiovascular disease.

When administering a combination therapy to a patient in need of such administration, the therapeutic agents in the combination, or a pharmaceutical composition or compositions comprising the therapeutic agents, may be administered in any order such as, for example, sequentially, concurrently, together, simultaneously and the like. The amounts of the various actives in such combination therapy may be different amounts (different dosage amounts) or same amounts (same dosage amounts).

In one embodiment, the one or more Bicyclic Heterocycle Derivatives are administered during a time when the additional therapeutic agent(s) exert their prophylactic or therapeutic effect, or *vice versa.*

In another embodiment, the one or more Bicyclic Heterocycle Derivatives and the additional therapeutic agent(s) are administered in doses commonly employed when such agents are used as monotherapy for treating a Condition.

In another embodiment, the one or more Bicyclic Heterocycle Derivatives and the additional therapeutic agent(s) are administered in doses lower than the doses commonly employed when such agents are used as monotherapy for treating a Condition.

In still another embodiment, the one or more Bicyclic Heterocycle Derivatives and the additional therapeutic agent(s) act synergistically and are administered in doses lower than the doses commonly employed when such agents are used as monotherapy for treating a Condition.

In one embodiment, the one or more Bicyclic Heterocycle Derivatives and the additional therapeutic agent(s) are present in the same composition. In one embodiment, this composition is suitable for oral administration. In another embodiment, this composition is suitable for intravenous administration.

The one or more Bicyclic Heterocycle Derivatives and the additional therapeutic agent(s) can act additively or synergistically. A synergistic combination may allow the use of lower dosages of one or more agents and/or less frequent administration of one or more agents of a combination therapy. A lower dosage or less frequent administration of one or more agents may reduce any toxicity associated with the therapy without reducing the efficacy of the therapy.

In one embodiment, the administration of one or more Bicyclic Heterocycle Derivatives and the additional therapeutic agent(s) may inhibit the resistance of a Condition to these agents.

In one embodiment, when the patient is treated for diabetes or a diabetic complication, the additional therapeutic agent is an antidiabetic agent which is not a Bicyclic Heterocycle Derivative. In another embodiment, the additional therapeutic agent is an agent useful for reducing any potential side effect of a Bicyclic Heterocycle Derivative. Such potential side effects include, but are not limited to, nausea, vomiting, headache, fever, lethargy, muscle aches, diarrhea, general pain, and pain at an injection site.

In one embodiment, the additional therapeutic agent is used at its known therapeutically effective dose. In another embodiment, the additional therapeutic agent is used at its normally prescribed dosage. In another embodiment, the additional therapeutic agent is used at less than its normally prescribed dosage or its known therapeutically effective dose.

The doses and dosage regimen of the other agents used in the combination therapies of the present invention for the treatment or prevention of a Condition can be determined by the attending clinician, taking into consideration the the approved doses and dosage regimen in the package insert; the age, sex and general health of the patient; and the type and severity of the viral infection or related disease or disorder. When administered in combination, the Bicyclic Heterocycle Derivative(s) and the other agent(s) for treating diseases or conditions listed above can be administered simultaneously or sequentially. This particularly useful when the components of the combination are given on different dosing schedules, e.g., one component is administered once daily and another every six hours, or when the preferred pharmaceutical compositions are different, e.g. one is a tablet and one is a capsule. A kit comprising the separate dosage forms is therefore advantageous.

Generally, a total daily dosage of the one or more Bicyclic Heterocycle Derivatives and the additional therapeutic agent(s)can when administered as combination therapy, range from about 0.1 to about 2000 mg per day, although variations will necessarily occur depending on the target of the therapy, the patient and the route of administration. In one embodiment, the dosage is from about 0.2 to about 100 mg/day, administered in a single dose or in 2-4 divided doses. In another embodiment, the dosage is from about 1 to about 500 mg/day, administered in a single dose or in 2-4 divided doses. In another embodiment, the dosage is from about 1 to about 200 mg/day, administered in a single dose or in 2-4 divided doses. In still another embodiment, the dosage is from about 1 to about 100 mg/day, administered in a single dose or in 2-4 divided doses. In yet another embodiment, the dosage is from about 1 to about 50 mg/day, administered in a single dose or in 2-4 divided doses. In a further embodiment, the dosage is from about 1 to about 20 mg/day, administered in a single dose or in 2-4 divided doses.

### Compositions and Administration

In one embodiment, the invention provides compositions comprising an effective amount of one or more Bicyclic Heterocycle Derivatives or a pharmaceutically acceptable salt, solvate, ester, prodrug or stereoisomer thereof, and a pharmaceutically acceptable carrier.

For preparing compositions comprising one or more Bicyclic Heterocycle Derivatives, inert, pharmaceutically acceptable carriers can be either solid or liquid. Solid form preparations include powders, tablets, dispersible granules, capsules, cachets and suppositories. The powders and tablets may be comprised of from about 5 to about 95 percent active ingredient. Suitable solid carriers are known in the art, *e.g.* magnesium carbonate, magnesium stearate, talc, sugar or lactose. Tablets, powders, cachets and capsules can be used as solid dosage forms suitable for oral administration. Examples of pharmaceutically acceptable carriers and methods of manufacture for various compositions may be found in A. Gennaro (ed.), Remington's Pharmaceutical Sciences, 18th Edition, (1990), Mack Publishing Co., Easton, PA.

Liquid form preparations include solutions, suspensions and emulsions. As an example may be mentioned water or water-propylene glycol solutions for parenteral injection or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier, such as an inert compressed gas, *e.g.* nitrogen.

Also included are solid form preparations which are intended to be converted, shortly before use, to liquid form preparations for either oral or parenteral administration. Such liquid forms include solutions, suspensions and emulsions.

The compounds of the invention may also be deliverable transdermally. The transdermal compositions can take the form of creams, lotions, aerosols and/or emulsions and can be included in a transdermal patch of the matrix or reservoir type as are conventional in the art for this purpose.

In one embodiment, a Bicyclic Heterocycle Derivative is administered orally.

In one embodiment, the pharmaceutical preparation is in a unit dosage form. In such form, the preparation is subdivided into suitably sized unit doses containing appropriate quantities of the active component, e.g., an effective amount to achieve the desired purpose.

The quantity of active compound in a unit dose of preparation is from about 0.1 to about 2000 mg. Variations will necessarily occur depending on the target of the therapy, the patient and the route of administration. In one embodiment, the unit dose dosage is from about 0.2 to about 1000 mg. In another embodiment, the unit dose dosage is from about 1 to about 500 mg. In another embodiment, the unit dose dosage is from about 1 to about 100 mg/day. In still another embodiment, the unit dose dosage is from about 1 to about 50 mg. In yet another embodiment, the unit dose dosage is from about 1 to about 10 mg.

The actual dosage employed may be varied depending upon the requirements of the patient and the severity of the condition being treated. Determination of the proper dosage regimen for a particular situation is within the skill of the art. For convenience, the total daily dosage may be divided and administered in portions during the day as required.

The amount and frequency of administration of the compounds of the invention and/or the pharmaceutically acceptable salts thereof will be regulated according to the judgment of the attending clinician considering such factors as age, the condition and size of the patient, as well as severity of the symptoms being treated. A typical recommended daily dosage regimen for oral administration can range from about 1 mg/day to about 1000 mg/day, 1 mg/day to about 500 mg/day, 1 mg/day to about 300 mg/day, 1 mg/day to about 75 mg/day, 1 mg/day to about 50 mg/day, or 1 mg/day to about 20 mg/day, in one dose or in two to four divided doses.

When the invention comprises a combination of one or more Bicyclic Heterocycle Derivatives and an additional therapeutic agent, the two active components may be co-administered simultaneously or sequentially, or a single composition comprising one or more Bicyclic Heterocycle Derivatives and the additional therapeutic agent(s) in a pharmaceutically acceptable carrier can be administered. The components of the combination can be administered individually or together in any conventional dosage form such as capsule, tablet, powder, cachet, suspension, solution, suppository, nasal spray, etc. The dosage of the additional therapeutic agent can be determined from published material, and may range from about 1 to about 1000 mg per dose. In one embodiment, when used in combination, the dosage levels of the individual components are lower than the recommended individual dosages because of an advantageous effect of the combination.

In one embodiment, the components of a combination therapy regimen are to be administered simultaneously, they can be administered in a single composition with a pharmaceutically acceptable carrier.

In another embodiment, when the components of a combination therapy regimen are to be administered separately or sequentially, they can be administered in separate compositions, each containing a pharmaceutically acceptable carrier.

### Kits

In one aspect, the present invention provides a kit comprising an effective amount of one or more Bicyclic Heterocycle Derivatives, or a pharmaceutically acceptable salt, solvate, ester, prodrug or stereoisomer thereof, and a pharmaceutically acceptable carrier.

In another aspect the present invention provides a kit comprising an amount of one or more Bicyclic Heterocycle Derivatives, or a pharmaceutically acceptable salt, solvate, ester, prodrug or stereoisomer thereof, and an amount of at least one additional therapeutic agent listed above, wherein the combined amounts are effective for treating or preventing a Condition in a patient.

When the components of a combination therapy regimen are to be administered in more than one composition, they can be provided in a kit comprising a single package containing one or more containers, wherein one container contains one or more Bicyclic Heterocycle Derivatives in a pharmaceutically acceptable carrier, and a second, separate container comprises an additional therapeutic agent in a pharmaceutically acceptable carrier, with the active components of each composition being present in amounts such that the combination is therapeutically effective.

## Claims

1. A compound having the formula: 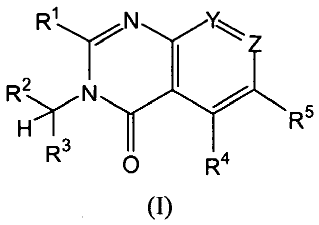 or a pharmaceutically acceptable salt or solvate thereof,
wherein
Y is -N- or -C(R⁷)-;
Z is -N- or -C(R⁶)-, such that at least one of Y and Z is other than -N-;
R¹ is -H, alkyl, -OH, -OR⁹, -SR⁹, or -N(R¹⁰)₂, wherein an alkyl group can be optionally substituted with one or more groups, which are each independently selected from halo, aryl, - OH, -O-haloalkyl, -O-alkyl, -CN, -N(R¹⁰)₂, -C(O)R⁹, -C(O)OR⁹, -C(O)N(R¹⁰)₂ and - NHC(O)R⁹;
R² and R³ are each independently selected from haloalkyl, -(alkylene)ₙ-aryl, -(alkylene)ₙ-heteroaryl, -(alkylene)ₙ-cycloalkyl and -alkylene-O-alkyl;
or R² and R³ and the carbon atom to which they are both attached, combine to form: 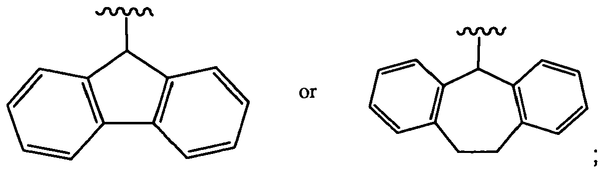
R⁴, R⁵, R⁶ and R⁷ are each independently selected from H, -(alkylene)ₙ-N(R⁸)₂, - S(O)ₚR¹³, -OR¹², -C(O)OR¹¹, -C(O)R¹¹, alkyl, halo, haloalkyl, -CN, cycloalkyl, heteroaryl, heterocycloalkyl, -C(O)N(R⁹)₂, -C(=NOH)-NH₂ and -(alkylene)ₙ-aryl, wherein any aryl, heteroaryl or heterocycloalkyl group can be optionally substituted with one or more groups, which are each independently selected from halo, alkyl, aryl, hydroxyalkyl, -O-alkyl, haloalkyl, -C(O)O-alkyl, -C(O)-alkyl, -NHC(O)O-alkyl, -C(O)NH-alkyl, -CN, -NO₂, -S(O)₂-alkyl and -S-alkyl, such that R⁴, R⁵, R⁶ and R⁷ are not each H, and wherein a heterocycloalkyl or heteroaryl group can be optionally fused to a benzene ring;
each occurrence of R⁸ is independently H, alkyl, -alkylene-C(O)OR¹¹, -(alkylene)ₙ-aryl, -(alkylene)ₙ-cycloalkyl, -(alkylene)ₙ-heterocycloalkyl, -(alkylene)ₙ-heteroaryl, -alkylene-O-alkyl, cyanoalkyl, alkenyl, alkynyl, haloalkyl or haloalkenyl, wherein an aryl, cycloalkyl, heterocycloalkyl or heteroaryl group can be optionally substituted with one or more groups, which are each independently selected from halo, alkyl, hydroxyalkyl, -OR¹⁰, haloalkyl, -CN, - NO₂, -O-haloalkyl, -S-alkyl, -S-haloalkyl, -alkylene-O-alkyl, -CN, -N(R¹²)₂, -C(O)R¹², - C(O)OR¹², -C(O)N(R¹²)₂, -NHC(O)R¹², -NHS(O)_{q}R¹⁴, -S(O)ₚR¹⁴ and -S(O)_{q}N(R¹²)₂;
each occurrence of R⁹ is alkyl, alkenyl, alkynyl, haloalkyl, -alkylene-O-aryl, -alkyleneS-aryl, -alkylene-N(R⁸)C(O)O-alkyl, -(alkylene)ₙ-aryl, -(alkylene)ₙ-cycloalkyl, -(alkylene)ₙ-cycloalkenyl, -(alkylene)ₙ-heterocycloalkyl or -(alkylene)ₙ-heteroaryl, wherein an aryl, cycloalkyl, cycloalkenyl, heterocycloalkyl or heteroaryl group can be optionally substituted with one or more groups, which are each independently selected from halo, alkyl, hydroxyalkyl, -OR¹⁰, haloalkyl, -CN, -NO₂, -O-haloalkyl, -S-haloalkyl, -alkylene-O-alkyl, - CN, -N(R¹²)₂, -C(O)R¹², -C(O)OR¹², -C(O)N(R¹²)₂, -NHC(O)R¹², -NHS(O)_{q}R¹⁴, -S(O)ₚR¹⁴ and -S(O)_{q}N(R¹²)₂;
each occurrence of R¹⁰ is independently H, alkyl, -(alkylene)ₙ-aryl, -(alkylene)ₙ-cycloalkyl, -(alkylene)ₙ-cycloalkenyl, -(alkylene)ₙ-heterocycloalkyl or -(alkylene)ₙ-heteroaryl, wherein any of the above groups, excluding H, can be optionally substituted with one or more groups, which are each independently selected from alkyl, haloalkyl, hydroxyalkyl, halo, -OH, -O-haloalkyl, -O-alkyl, -O-aryl, -alkylene-O-alkyl, -CN, -N(R¹²)₂, -C(O)H, -C(O)R¹², - C(O)OR¹², -C(O)N(R¹²)₂, -NHC(O)R¹², -NHS(O)_{q}R¹⁴, -S(O)ₚR¹⁴ and -S(O)_{q}N(R¹²)₂;
each occurrence of R¹¹ is independently H, alkyl, aryl, heterocycloalkyl, heteroaryl or cycloalkyl, wherein any of the above groups can be optionally substituted with one or more groups, which are each independently selected from alkyl, haloalkyl, hydroxyalkyl, halo, -OH, -O-haloalkyl, -O-alkyl, -O-aryl, -alkylene-O-alkyl, -CN, -N(R¹²)₂, -C(O)H, -C(O)R¹², - C(O)OR¹², -C(O)N(R¹²)₂, -NHC(O)R¹², -NHS(O)_{q}R¹⁴, -S(O)ₚR¹⁴ and -S(O)_{q}N(R¹²)₂;
each occurrence of R¹² is independently H, alkyl, -(alkylene)ₙ-aryl, heterocycloalkyl, heteroaryl or cycloalkyl;
each occurrence of R¹³ is independently alkyl, aryl, heterocycloalkyl, heteroaryl or cycloalkyl, wherein any of the above groups can be optionally substituted with one or more groups, which are each independently selected from alkyl, haloalkyl, hydroxyalkyl, halo, -OH, -O-haloalkyl, -O-alkyl, -O-aryl, -alkylene-O-alkyl, -CN, -N(R¹²)₂, -C(O)H, -C(O)R¹², - C(O)OR¹², -C(O)N(R¹²)₂, -NHC(O)R¹², -NHS(O)_{q}R¹⁴, -S(O)ₚR¹⁴ and -S(O)_{q}N(R¹²)₂;
each occurrence of R¹⁴ is independently alkyl, -(alkylene)ₙ-aryl, heterocycloalkyl, heteroaryl or cycloalkyl;
each occurrence of n is independently 0 or 1;
each occurrence of p is independently 0, 1 or 2; and
each occurrence of q is independently 1 or 2;
with the exception of 6-amino-3-(4,4'-dimethoxybenzhydryl)-2-propylquinazolin-4(3H)-one and 3-(4,4'-dimethoxybenzhydryl)-6-hydroxy-7-methoxyquinazolin-4(3H)-one.

2. The compound of claim 1, wherein Y is -C(R⁷)- and Z is -C(R⁶)-.

3. The compound of claim 1, wherein Y is -N- and Z is -C(R⁶)-.

4. The compound of claim 1, wherein R¹ is alkyl, -OH or -NH₂.

5. The compound of claim 4, wherein R¹ is methyl.

6. The compound of claim 1, wherein the group -CH(R²)(R³) is:

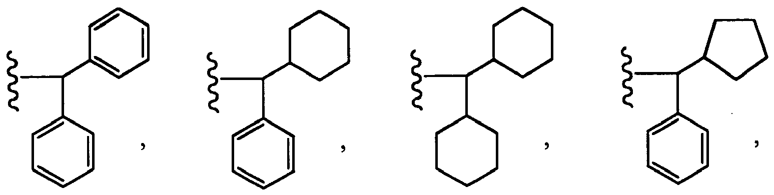

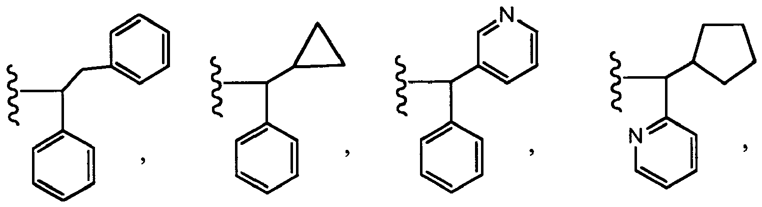

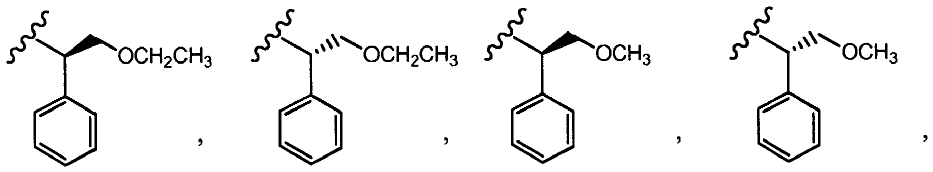

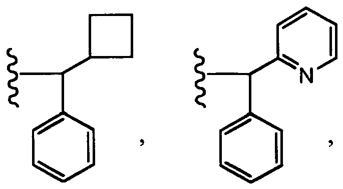

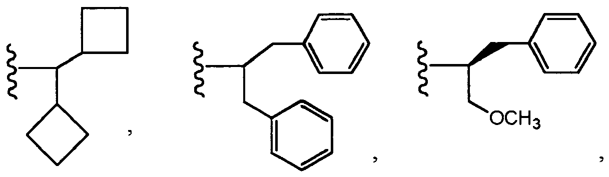

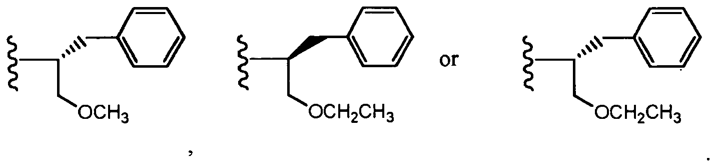

7. The compound of claim 1 or claim 5, wherein at least one of R² and R³ is phenyl.

8. The compound of claim 1, wherein R⁴ is H.

9. The compound of claim 1, wherein R⁵ is -N(R⁸)₂.

10. The compound of claim 9, wherein R⁵ is -NHC(O)OR¹¹, -NH-phenyl, or - N(alkyl)(phenyl), wherein the phenyl moiety of an -NH-phenyl or -N(alkyl)(phenyl) group can be unsubstituted or substituted as set forth in claim 1.

11. The compound of claim 4, wherein R¹ is methyl and R² and R³ are each independenlty selected from phenyl, benzyl, pyridyl, -cycloalkyl and -CH₂-O-alkyl.

12. The compound of claim 2, wherein R⁷ is H.

13. The compound of claim 2, wherein R⁶ is -N(R⁸)₂.

14. The compound of claim 1 having the formula: 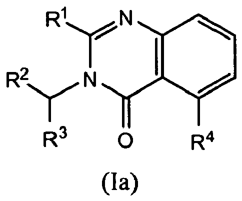 or 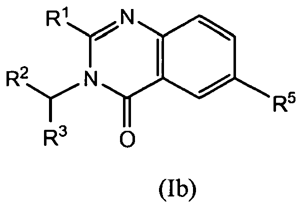 or 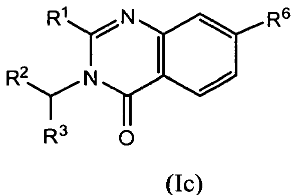 or a pharmaceutically acceptable salt or solvate thereof.

15. A compound of claim 1 having the structure 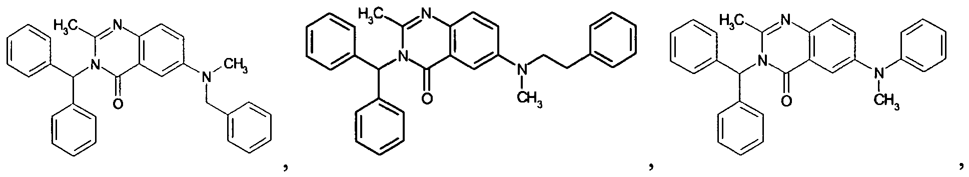 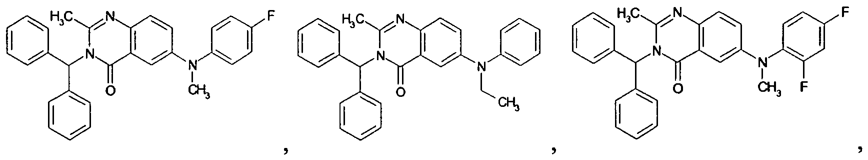 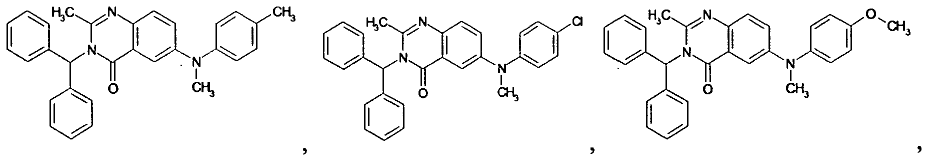 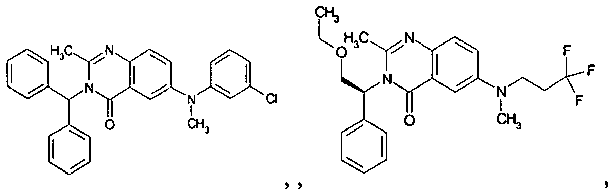 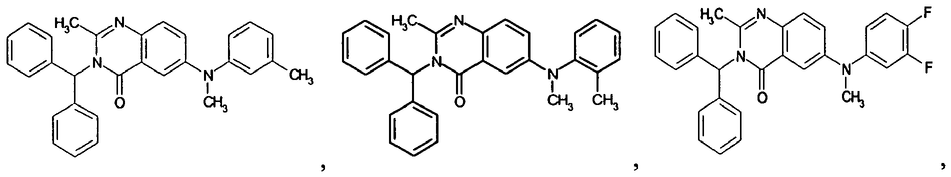 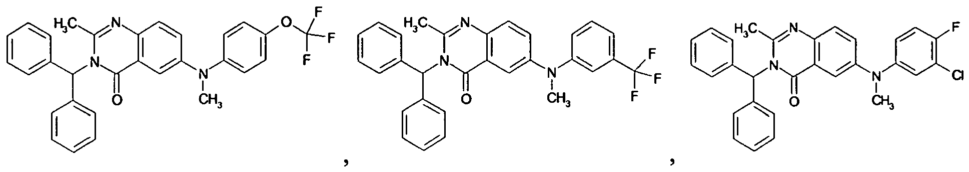 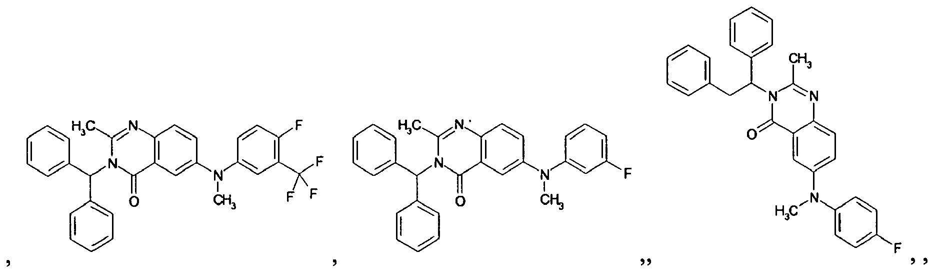 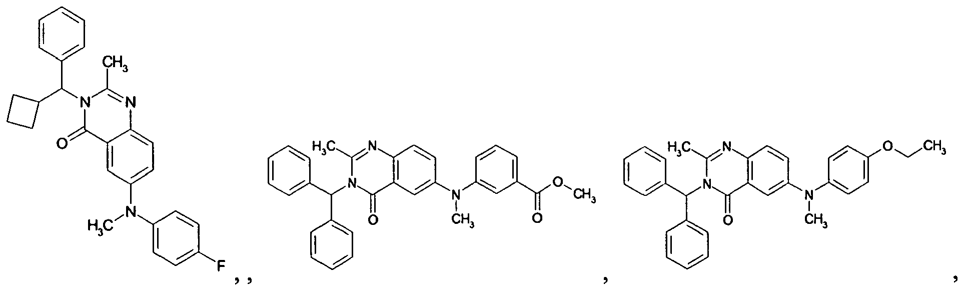 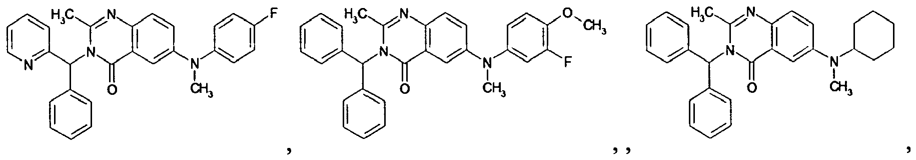 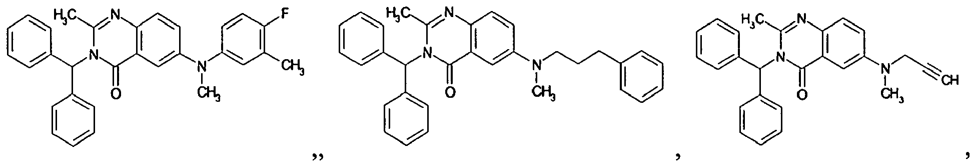 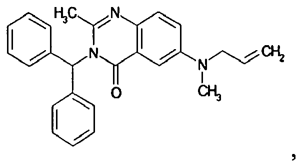 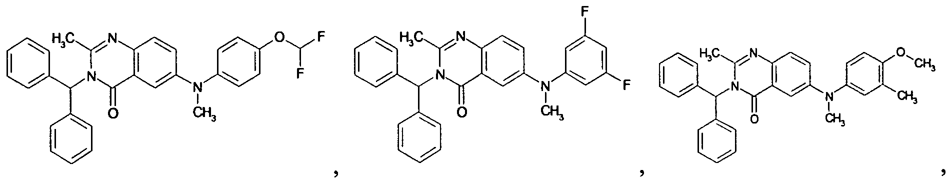 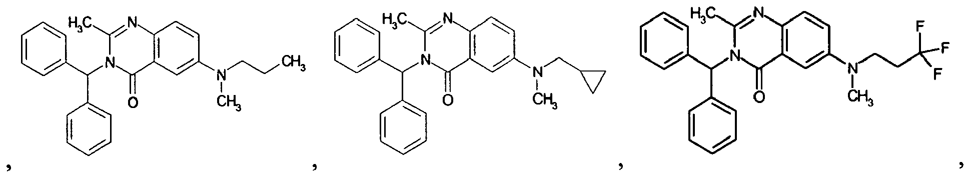 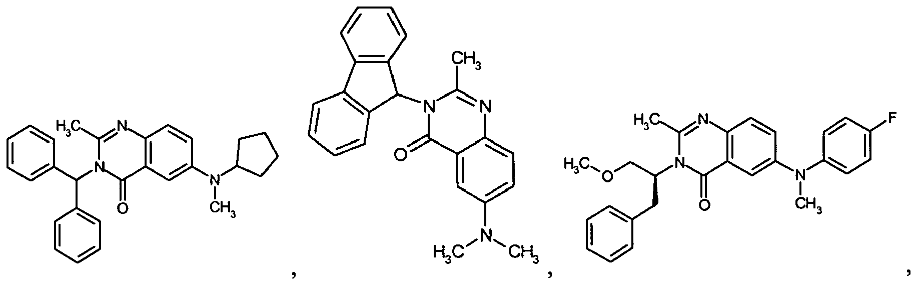 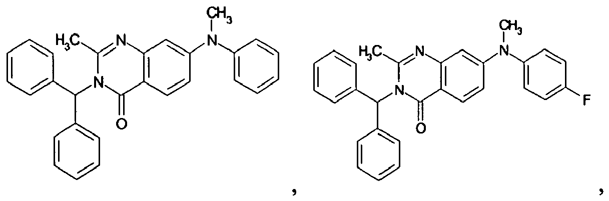 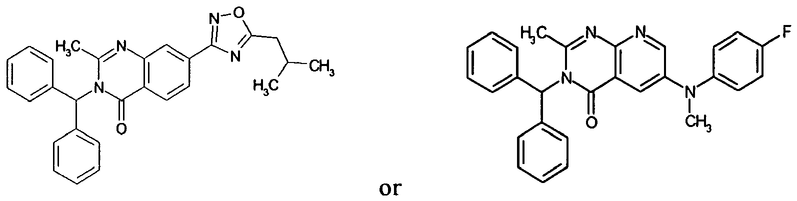 or a pharmaceutically acceptable salt or solvate thereof.

16. A composition comprising one or more compounds of any of claims 1-15 or a pharmaceutically acceptable salt or solvate thereof, and at least one pharmaceutically acceptable carrier.

17. A compound according to any of claims 1-15 for use in treating diabetes, a diabetic complication, obesity, metabolic syndrome or a cardiovascular disease in a patient, optionally in combination with at least one additional therapeutic agent that is not a compound of claim 1, wherein the additional agent is selected from an antidiabetic agent and an antiobesity agent.

## Patentansprüche

1. Eine Verbindung der Formel: 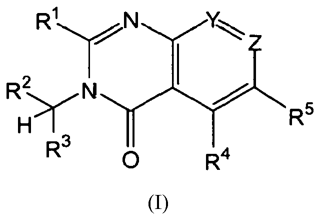 oder ein pharmazeutisch annehmbares Salz oder Solvat davon,
wobei
Y -N- oder -C(R⁷)- ist,
Z -N- oder -C(R⁶)- ist, so dass wenigstens eines von Y und Z anders als -N- ist,
**R¹** -H, Alkyl, -OH, -OR⁹, -SR⁹ oder -N(R¹⁰)₂ ist, wobei eine Alkylgruppe gegebenenfalls substituiert sein kann mit einer oder mehreren Gruppen, die jeweils unabhängig ausgewählt sind aus Halogen, Aryl, -OH, -O-Halogenalkyl, -O-Alkyl, -CN, -N(R¹⁰)₂, -C(O)R⁹, -C(O)OR⁹, -C(O)N(R¹⁰)₂ und -NHC(O)R⁹,
R² und R³ jeweils unabhängig ausgewählt sind aus Halogenalkyl, -(Alkylen)ₙ-aryl, -(Alkylen)ₙ-heteroaryl, -(Alkylen)ₙ-cycloalkyl und -Alkylen-O-alkyl,
oder R² und R³ und das Kohlenstoffatom, an das sie beide gebunden sind, kombiniert sind unter Bildung von: 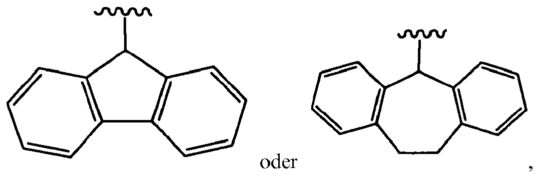
R⁴, R⁵, R⁶ und R⁷ jeweils unabhängig ausgewählt sind aus H, -(Alkylen)ₙ-N(R⁸)₂, -S(O)ₚR¹³, -OR¹², -C(O)OR¹¹, -C(O)R¹¹, Alkyl, Halogen, Halogenalkyl, -CN, Cycloalkyl, Heteroaryl, Heterocycloalkyl, -C(O)N(R⁸)₂, -C(=NOH)-NH₂ und -(Alkylen)ₙ-aryl, wobei jede Aryl-, Heteroaryl- oder Heterocycloalkylgruppe gegebenenfalls substituiert sein kann mit einer oder mehreren Gruppen, die jeweils unabhängig ausgewählt sind aus Halogen, Alkyl, Aryl, Hydroxyalkyl, -O-Alkyl, Halogenalkyl, -C(O)O-Alkyl, -C(O)-Alkyl, -NHC(O)O-Alkyl, -C(O)NH-Alkyl, -CN, -NO₂, -S(O)₂-Alkyl und -S-Alkyl, so dass R⁴, R⁵, R⁶ und R⁷ nicht jeweils H sind, und wobei eine Heterocycloalkyl- oder Heteroarylgruppe gegebenenfalls an einen Benzolring kondensiert sein kann,
jedes Auftreten von R⁸ unabhängig H, Alkyl, -Alkylen-C(O)OR¹¹, -(Alkylen)ₙ-aryl, -(Alkylen)ₙ-cycloalkyl, -(Alkylen)ₙ-heterocycloalkyl, -(Alkylen)ₙ-heteroaryl, -Alkylen-O-alkyl, Cyanoalkyl, Alkenyl, Alkinyl, Halogenalkyl oder Halogenalkenyl ist, wobei eine Aryl-, Cycloalkyl-, Heterocycloalkyl- oder Heteroarylgruppe gegebenenfalls substituiert sein kann mit einer oder mehreren Gruppen, die jeweils unabhängig ausgewählt sind aus Halogen, Alkyl, Hydroxyalkyl, -OR¹⁰, Halogenalkyl, -CN, -NO₂, -O-Halogenalkyl, -S-Alkyl, -S-Halogenalkyl, -Alkylen-O-alkyl, -CN, -N(R¹²)₂, -C(O)R¹², -C(O)OR¹², -C(O)N(R¹²)₂, -NHC(O)R¹², -NHS(O)_{q}R¹⁴, -S(O)ₚR¹⁴ und -S(O)_{q}N(R¹²)₂,
jedes Auftreten von R⁹ Alkyl, Alkenyl, Alkinyl, Halogenalkyl, -Alkylen-O-aryl, -Alkylen-S-aryl, -Alkylen-N(R⁸)C(O)O-alkyl, -(Alkylen)ₙ-aryl, -(Alkylen)ₙ-cycloalkyl, -(Alkylen)ₙ-cycloalkenyl, -(Alkylen)ₙ-heterocycloalkyl oder (Alkylen)ₙ-heteroaryl ist, wobei eine Aryl-, Cycloalkyl-, Cycloalkenyl-, Heterocycloalkyl- oder Heteroarylgruppe gegebenenfalls substituiert sein kann mit einer oder mehreren Gruppen, die jeweils unabhängig ausgewählt sind aus Halogen, Alkyl, Hydroxyalkyl, -OR¹⁰, Halogenalkyl, -CN, -NO₂, -O-Halogenalkyl, -S-Halogenalkyl, -Alkylen-O-alkyl, -CN, -N(R¹²)₂, -C(O)R¹², -C(O)OR¹², -C(O)N(R¹²)₂, -NHC(O)R¹², -NHS(O)_{q}R¹⁴, -S(O)ₚR¹⁴ und -S(O)_{q}N(R¹²)₂,
jedes Auftreten von R¹⁰ unabhängig H, Alkyl, -(Alkylen)ₙ-aryl, -(Alkylen)ₙ-cycloalkyl, -(Alkylen)ₙ-cycloalkenyl, -(Alkylen)ₙ-heterocycloalkyl oder -(Alkylen)ₙ-heteroaryl ist, wobei jede der obigen Gruppen mit Ausnahme von H gegebenenfalls substituiert sein kann mit einer oder mehreren Gruppen, die jeweils unabhängig ausgewählt sind aus Alkyl, Halogenalkyl, Hydroxyalkyl, Halogen, -OH, -O-Halogenalkyl, -O-Alkyl, -O-Aryl, -Alkylen-O-alkyl, -CN, -N(R¹²)₂, -C(O)H, -C(O)R¹², -C(O)OR¹², -C(O)N(R¹²)₂, -NHC(O)R¹², -NHS(O)_{q}R¹⁴, -S(O)ₚR¹⁴ und -S(O)qN(R¹²)₂,
jedes Auftreten von R¹¹ unabhängig H, Alkyl, Aryl, Heterocycloalkyl, Heteroaryl oder Cycloalkyl ist, wobei jede der obigen Gruppen gegebenenfalls substituiert sein kann mit einer oder mehreren Gruppen, die jeweils unabhängig ausgewählt sind aus Alkyl, Halogenalkyl, Hydroxyalkyl, Halogen, -OH, -O-Halogenalkyl, -O-Alkyl, -O-Aryl, -Alkylen-O-alkyl, -CN, -N(R¹²)₂, -C(O)H, -C(O)R¹², -C(O)OR¹², -C(O)N(R¹²)₂, -NHC(O)R¹², -NHS(O)_{q}R¹⁴, -S(O)ₚR¹⁴ und -S(O)_{q}N(R¹²),
jedes Auftreten von R¹² unabhängig H, Alkyl, -(Alkylen)ₙ-aryl, Heterocycloalkyl, Heteroaryl oder Cycloalkyl ist,
jedes Auftreten von R¹³ unabhängig Alkyl, Aryl, Heterocycloalkyl, Heteroaryl oder Cycloalkyl ist, wobei jede der obigen Gruppen gegebenenfalls substituiert sein kann mit einer oder mehreren Gruppen, die jeweils unabhängig ausgewählt sind aus Alkyl, Halogenalkyl, Hydroxyalkyl, Halogen, -OH, -O-Halogenalkyl, -O-Alkyl, -O-Aryl, -Alkylen-O-alkyl, -CN, -N(R¹²)₂, -C(O)H, -C(O)R¹², -C(O)OR¹², -C(O)N(R¹²)₂, -NHC(O)R¹², -NHS(O)_{q}R¹⁴, -S(O)ₚR¹⁴ und -S(O)_{q}N(R¹²)₂,
jedes Auftreten von R¹⁴ unabhängig Alkyl, -(Alkylen)ₙ-aryl, Heterocycloalkyl, Heteroaryl oder Cycloalkyl ist,
jedes Auftreten von n unabhängig 0 oder 1 ist,
jedes Auftreten von p unabhängig 0, 1 oder 2 ist und
jedes Auftreten von q unabhängig 1 oder 2 ist,
mit Ausnahme von 6-Amino-3-(4,4'-dimethoxybenzhydryl)-2-propylchinazolin-4(3H)-on und 3-(4,4'-Dimethoxybenzhydryl)-6-hydroxy-7-methoxychinazolin-4(3H)-on.

2. Die Verbindung nach Anspruch 1, wobei Y -C(R⁷)- ist und Z -C(R⁶)- ist.

3. Die Verbindung nach Anspruch 1, wobei Y -N- ist und Z -C(R⁶)- ist.

4. Die Verbindung nach Anspruch 1, wobei R¹ Alkyl, -OH oder -NH₂ ist.

5. Die Verbindung nach Anspruch 4, wobei R¹ Methyl ist.

6. Die Verbindung nach Anspruch 1, wobei die Gruppe -CH(R²)(R³) ist:

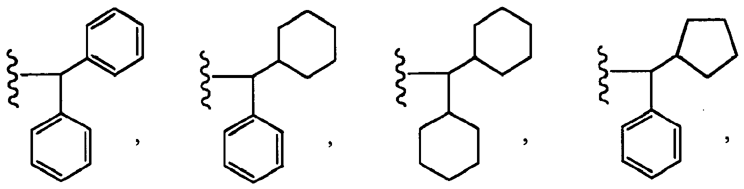

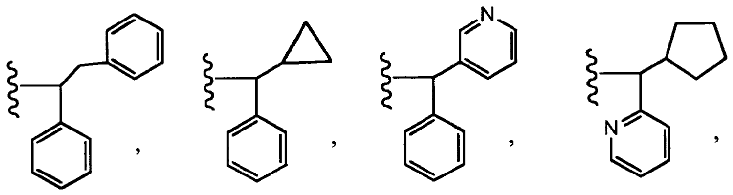

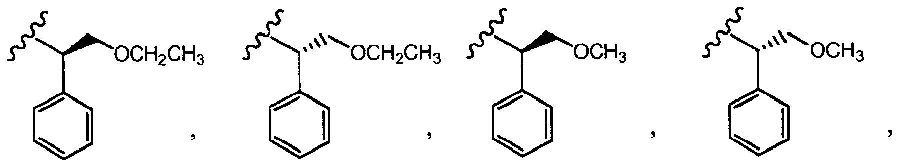

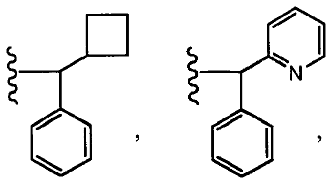

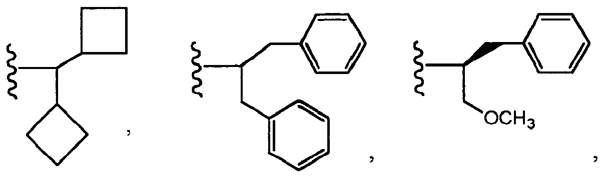

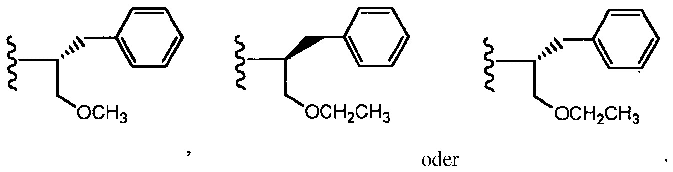

7. Die Verbindung nach Anspruch 1 oder Anspruch 5, wobei wenigstens eines von R² und R³ Phenyl ist.

8. Die Verbindung nach Anspruch 1, wobei R⁴ H ist.

9. Die Verbindung nach Anspruch 1, wobei R⁵ -N(R⁸)₂ ist.

10. Die Verbindung nach Anspruch 9, wobei R⁵ -NHC(O)OR¹¹, -NH-Phenyl oder -N(Alkyl)(phenyl) ist, wobei der Phenylrest einer -NH-Phenyl- oder -N(Alkyl)(phenyl)-Gruppe unsubstituiert oder wie in Anspruch 1 beschrieben substituiert sein kann.

11. Die Verbindung nach Anspruch 4, wobei R¹ Methyl ist und R² und R³ jeweils unabhängig ausgewählt ist aus Phenyl, Benzyl, Pyridyl, -Cycloalkyl und -CH₂-O-Alkyl.

12. Die Verbindung nach Anspruch 2, wobei R⁷ H ist.

13. Die Verbindung nach Anspruch 2, wobei R⁶ -N(R⁸)₂ ist.

14. Die Verbindung nach Anspruch 1 der Formel: 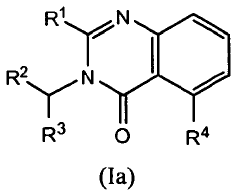 oder 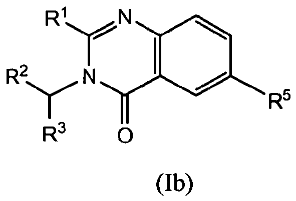 oder 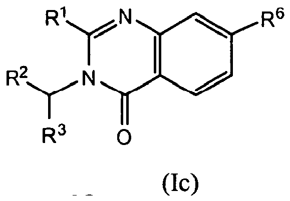 oder ein pharmazeutisch annehmbares Salz oder Solvat davon.

15. Eine Verbindung nach Anspruch 1 der Struktur 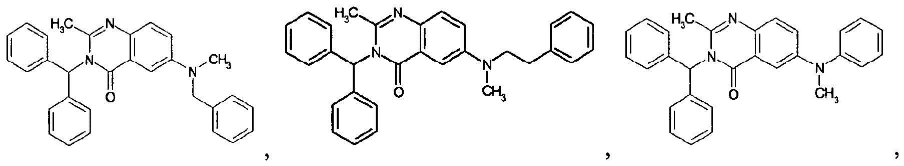 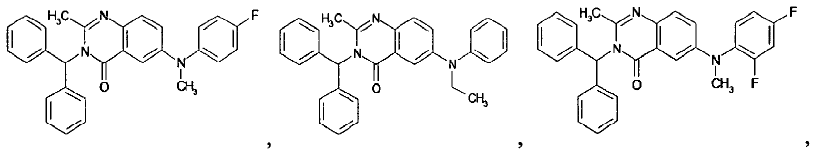 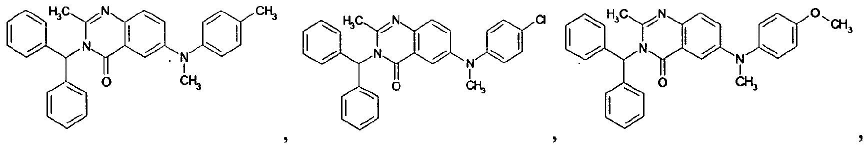 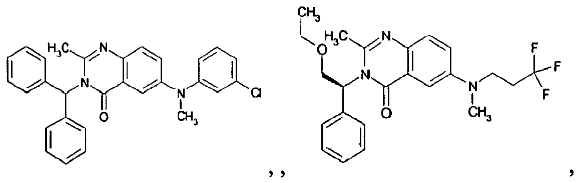 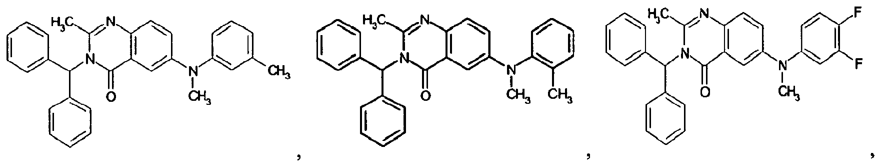 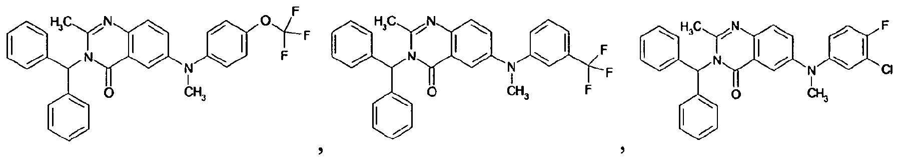 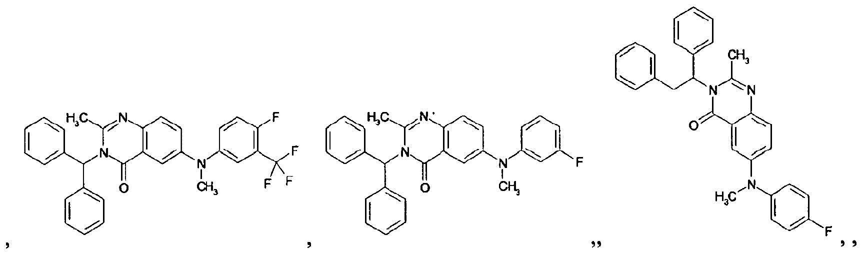 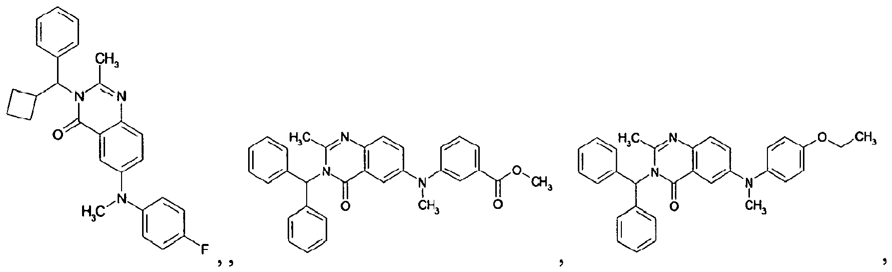 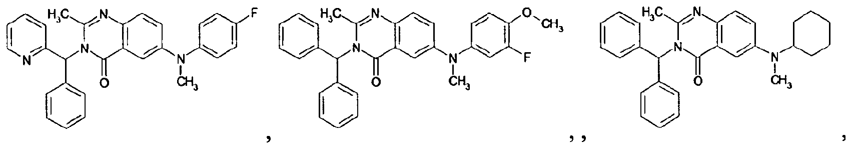 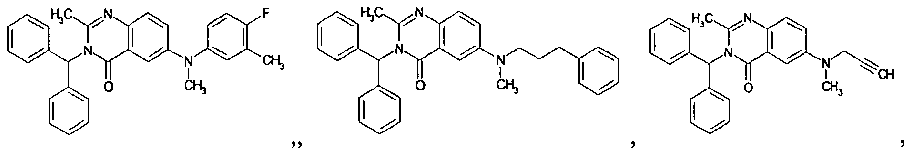 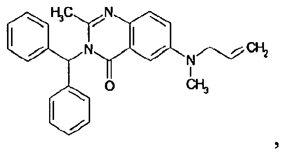 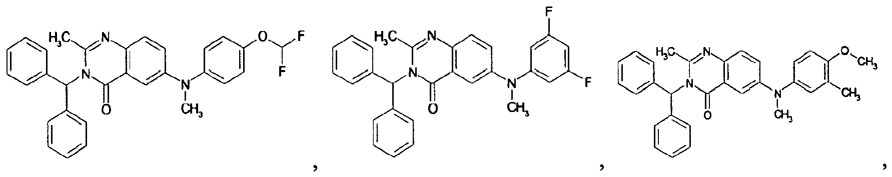 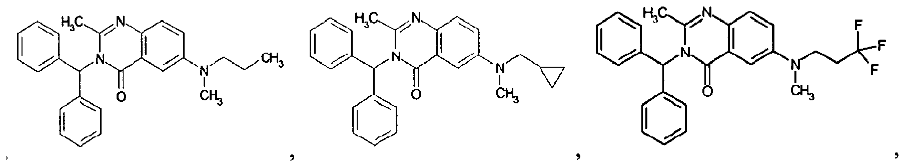 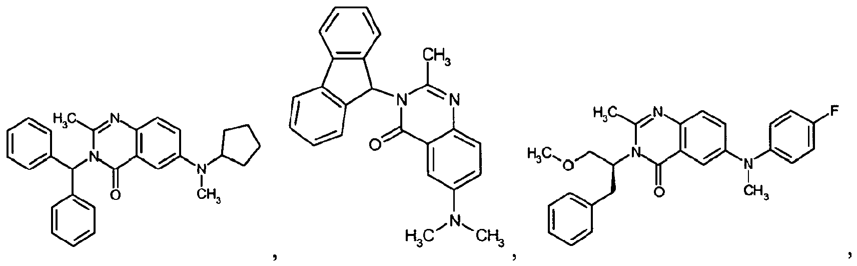 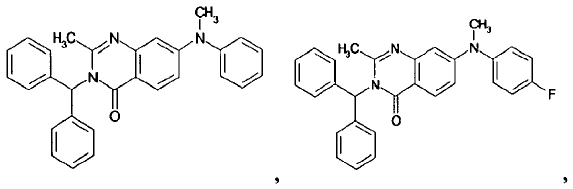 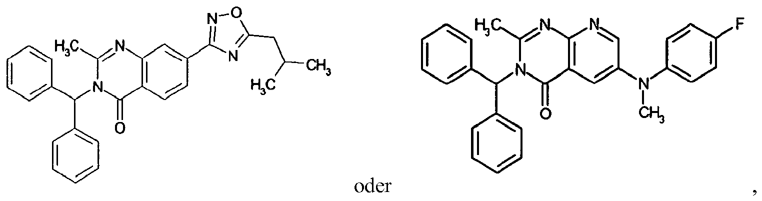 oder ein pharmazeutisch annehmbares Salz oder Solvat davon.

16. Eine Zusammensetzung, die eine oder mehrere Verbindung nach einem der Ansprüche 1-15 oder ein pharmazeutisch annehmbares Salz oder Solvat davon und wenigstens einen pharmazeutisch annehmbaren Träger umfasst.

17. Eine Verbindung gemäß einem der Ansprüche 1-15 zur Verwendung bei der Behandlung von Diabetes, einer diabetischen Komplikation, Adipositas, metabolischem Syndrom oder einer kardiovaskulären Erkrankung bei einem Patienten, gegebenenfalls in Kombination mit wenigstens einem weiteren therapeutischen Mittel, das keine Verbindung nach Anspruch 1 ist, wobei das weitere Mittel ausgewählt ist aus einem Antidiabetikum und einem Mittel gegen Adipositas.

## Revendications

1. Composé ayant la formule: 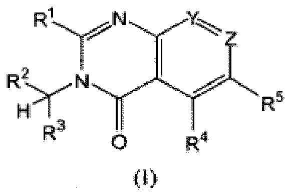 ou un sel ou solvate pharmaceutiquement acceptable de celui-ci,
dans lequel:
Y est -N- ou -C(R⁷)-,
Z est -N- ou -C(R⁶)-, de telle sorte qu'au moins l'un de Y et de Z est autre que - N-;
R¹ est -H, alkyle, -OH, -OR⁹, -SR⁹ ou -N(R¹⁰)₂, où un groupe alkyle peut être optionnellement substitué par un ou plusieurs groupes qui sont sélectionnés indépendamment parmi halo, aryle, -OH, -O-haloalkyle, -O-alkyle, -CN, -N(R¹⁰)₂, - C(O)R⁹, -C(O)OR⁹, -C(O)N(R¹⁰)₂ et -NHC(O)R⁹;
R² et R³ sont sélectionnés chacun indépendamment parmi haloalkyle, -(alkylène)ₙ-aryle, -(alkylène)ₙ-hétéroaryle, -(alkylène)ₙ-cycloalkyle et -alkylène-O-alkyle;
ou bien R² et R³ et l'atome de carbone auquel ils sont attachés tous les deux, se combinent pour former: 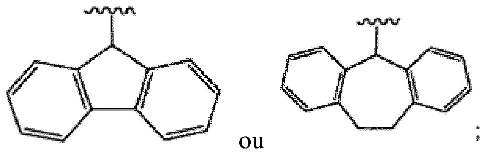
R⁴, R⁵, R⁶ et R⁷ sont sélectionnés chacun indépendamment parmi H, -(alkylène)n-N(R⁸)₂, -S(O)ₚR¹³, -OR¹², -C(O)OR¹¹, -C(O)R¹¹, alkyle, halo, haloalkyle, -CN, cycloalkyle, hétéroaryle, hétérocycloalkyle, -C(O)N(R⁸)₂, -C(=NOH)-NH₂ et - (alkylène)ₙ-aryle, où un groupe aryle, hétéroaryle ou hétérocycloalkyle quelconque peut être optionnellement substitué par un ou plusieurs groupes qui sont sélectionnés chacun indépendamment parmi halo, alkyle, aryle, hydroxyalkyle, -O-alkyle, haloalkyle, -C(O)O-alkyle, -C(O)-alkyle, -NHC(O)O-alkyle, -C(O)NH-alkyle, -CN, -NO₂, -S(O)₂-alkyle et - S-alkyle, de telle sorte que R⁴, R⁵, R⁶ et R⁷ ne sont pas chacun H et où un groupe hétérocycloalkyle ou hétéroaryle peut être optionnellement condensé avec un cycle benzène;
chaque occurrence de R⁸ est indépendamment H, alkyle, -alkylène-C(O)OR", - (alkylène)ₙ-aryle, -(alkylène)ₙ-cycloalkyle, -(alkylène)ₙ-hétérocycloalkyle, -(alkylène)ₙ-hétéroaryle, -alkylène-O-alkyle, cyanoalkyle, alcényle, alcynyle, haloalkyle ou haloalcényle, où un groupe aryle, cycloalkyle, hétérocycloalkyle ou hétéroaryle peut être optionnellement substitué par un ou plusieurs groupes qui sont sélectionnés chacun indépendamment parmi halo, alkyle, hydroxyalkyle, -OR¹⁰, haloalkyle, -CN, -NO₂, -O-haloalkyle, -S-alkyle, -S-haloalkyle, -alkylène-O-alkyle, -CN, -N(R¹²)₂, -C(O)R¹², - C(O)OR¹², -C(O)N(R¹²)₂, -NHC(O)R¹², -NHS(O)_{q}R¹⁴, -S(O)ₚR¹⁴ et -S(O)_{q}N(R¹²)₂,
chaque occurrence de R⁹ est alkyle, alcényle, alcynyle, haloalkyle, -alkylène-O-aryle, -alkylène-S-aryle, -alkylène-N(R⁸)C(O)O-alkyle, -(alkylène)ₙ-aryle, -(alkylène)ₙ-cycloalkyle, -(alkylène)ₙ-cycloalcényle, -(alkylène)ₙ-hétérocycloalkyle ou -(alkylène)ₙ-hétéroaryle, où un groupe aryle, cycloalkyle, cycloalcényle, hétérocycloalkyle ou hétéroaryle peut être optionnellement substitué par un ou plusieurs groupes qui sont sélectionnés chacun indépendamment parmi halo, alkyle, hydroxyalkyle, -OR¹⁰, haloalkyle, -CN, -NO₂, -O-haloalkyle, -S-haloalkyle, alkylène-O-alkyle, -CN, -N(R¹²)₂, - C(O)R¹², -C(O)OR¹², -C(O)N(R¹²)₂, -NHC(O)R¹², -NHS(O)_{q}R¹⁴, -S(O)ₚR¹⁴ et - S(O)_{q}N(R¹²)₂;
chaque occurrence de R¹⁰ est indépendamment H, alkyle, -(alkylène)ₙ-aryle, - (alkylène)ₙ-cycloalkyle, -(alkylène)ₙ-cycloalcényle, -(alkylène)ₙ-hétérocycloalkyle ou - (alkylène)ₙ-hétéroaryle, où l'un quelconque des groupes ci-dessus, à l'exception de H, peut être optionnellement substitué par un ou plusieurs groupes qui sont sélectionnés chacun indépendamment parmi alkyle, haloalkyle, hydroxyalkyle, halo, -OH, -O-haloalkyle, -O-alkyle, -O-aryle, -alkylène-O-alkyle, -CN, -N(R¹²)₂, -C(O)H, -C(O)R¹², -C(O)OR¹², - C(O)N(R¹²)₂, -NHC(O)R¹², -NHS(O)_{q}R¹⁴, -S(O)ₚR¹⁴ et -S(O)_{q}N(R¹²)₂;
chaque occurrence de R¹¹ est indépendamment H, alkyle, aryle, hétérocycloalkyle, hétéroaryle ou cycloalkyle, où l'un quelconque des groupes ci-dessus peut être optionnellement substitué par un ou plusieurs groupes qui sont sélectionnés chacun indépendamment parmi alkyle, haloalkyle, hydroxyalkyle, halo, -OH, -O-haloalkyle, -O-alkyle, -O-aryle, -alkylène-O-alkyle, -CN, -N(R¹²)₂, -C(O)H, -C(O)R¹², -C(O)OR¹², - C(O)N(R¹²)₂, -NHC(O)R¹², -NHS(O)_{q}R¹⁴, -S(O)ₚR¹⁴ et -S(O)_{q}N(R¹²)₂;
chaque occurrence de R¹² est indépendamment H, alkyle, -(alkylène)ₙ-aryle, hétérocycloalkyle, hétéroaryle ou cycloalkyle;
chaque occurrence de R¹³ est indépendamment alkyle, aryle, hétérocycloalkyle, hétéroaryle ou cycloalkyle, où l'un quelconque des groupes ci-dessus peut être optionnellement substitué par un ou plusieurs groupes qui sont sélectionnés chacun indépendamment parmi alkyle, haloalkyle, hydroxyalkyle, halo, -OH, -O-haloalkyle, -O-alkyle, -O-aryle, -alkylène-O-alkyle, -CN, -N(R¹²)₂, -C(O)H, -C(O)R¹², -C(O)OR¹², - C(O)N(R¹²)₂, -NHC(O)R¹², -NHS(O)_{q}R¹⁴, -S(O)ₚR¹⁴ et -S(O)_{q}N(R¹²)₂;
chaque occurrence de R¹⁴ est indépendamment alkyle, -(alkylène)ₙ-aryle, hétérocycloalkyle, hétéroaryle ou cycloalkyle;
chaque occurrence de n est indépendamment 0 ou 1 ;
chaque occurrence de p est indépendamment 0,1 ou 2; et
chaque occurrence de q est indépendamment 1 ou 2;
à l'exception de 6-amino-3-(4,4'-diméthoxybenzhydryl)-2-propylquinazolin-4(3H)-one et de 3-(4,4'-diméthoxybenzhydryl)-6-hydroxy-7-méthoxyquinazolin-4(3H)-one.

2. Composé selon la revendication 1, dans lequel Y est -C(R⁷)- et Z est -C(R⁶)-.

3. Composé selon la revendication 1, dans lequel Y est -N- et Z est -C(R⁶)-.

4. Composé selon la revendication 1, dans lequel R¹ est alkyle, -OH ou -NH₂.

5. Composé selon la revendication 4, dans lequel R¹ est méthyle.

6. Composé selon la revendication 1, dans lequel le groupe -CH(R²)(R³) est:

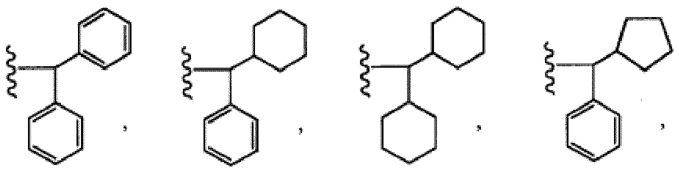

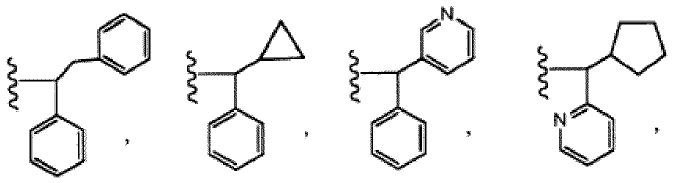

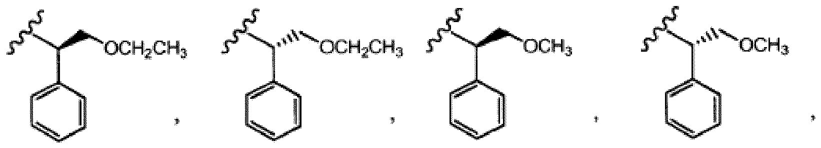

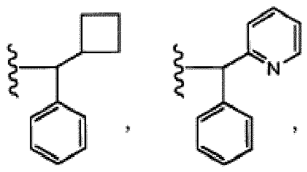

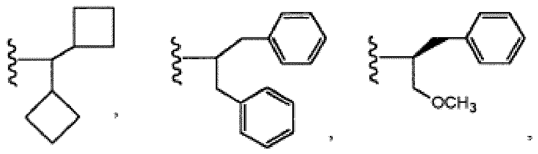

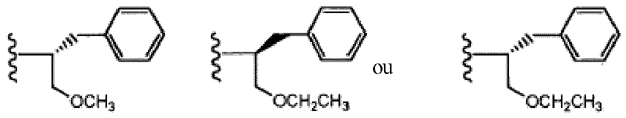

7. Composé selon la revendication 1 ou la revendication 5, dans lequel au moins l'un de R² et R³ est phényle.

8. Composé selon la revendication 1, dans lequel R⁴ est H.

9. Composé selon la revendication 1, dans lequel R⁵ est -N(R⁸)₂.

10. Composé selon la revendication 9, dans lequel R⁵ est -NHC(O)OR¹¹, -NH-phényle ou -N(alkyle)(phényle), où la fraction phényle d'un groupe -NH-phényle ou - N(alkyle)(phényle) peut être non substituée ou substituée comme stipulé dans la revendication 1.

11. Composé selon la revendication 4, dans lequel R¹ est méthyle et R² et R³ sont sélectionnés chacun indépendamment parmi phényle, benzyle, pyridyle, -cycloalkyle et - CH₂-O-alkyle.

12. Composé selon la revendication 2, dans lequel R⁷ est H.

13. Composé selon la revendication 2, dans lequel R⁶ est -N(R⁸)₂.

14. Composé selon la revendication 1 ayant la formule: 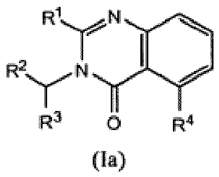 ou 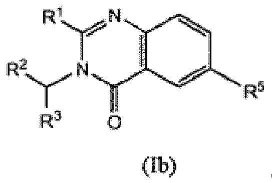 ou 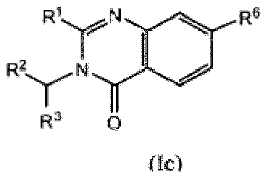 ou un sel ou solvate pharmaceutiquement acceptable de celui-ci.

15. Composé selon la revendication 1 ayant la structure: 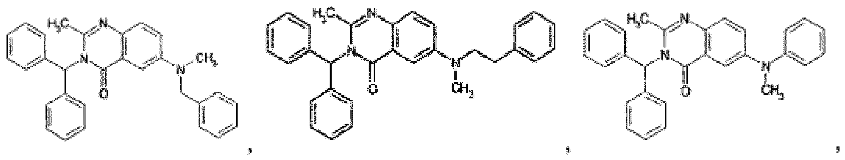 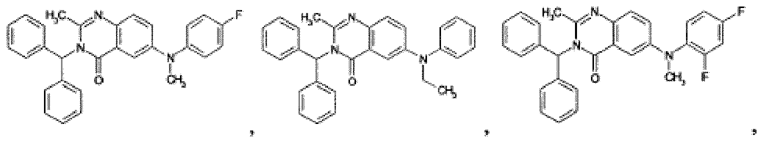 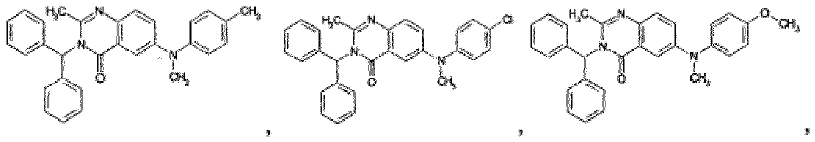 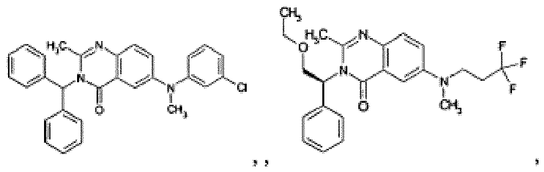 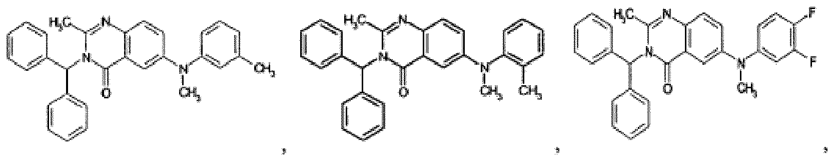 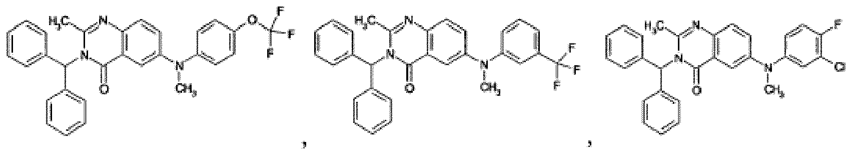 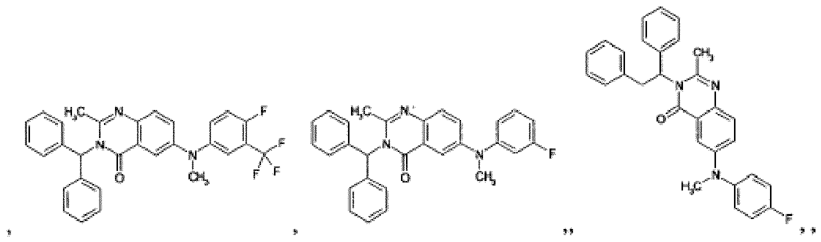 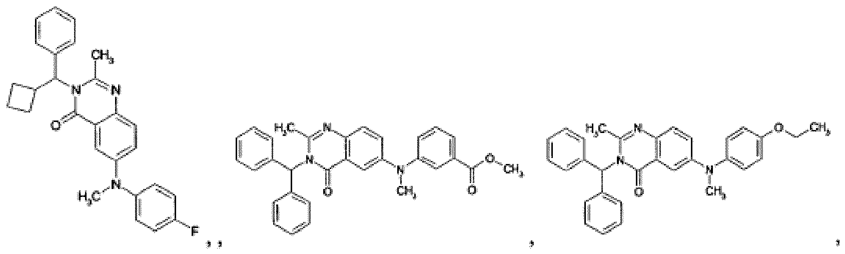 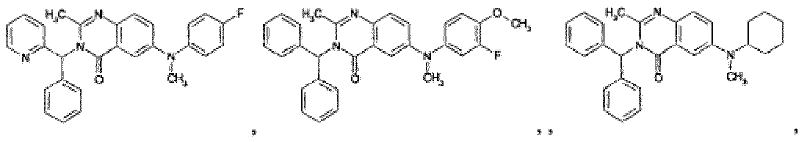 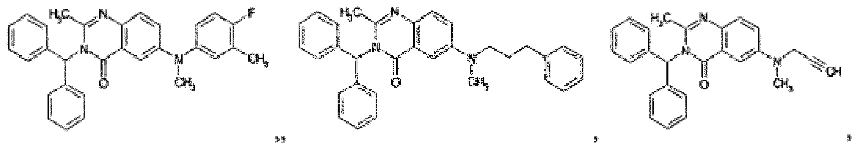 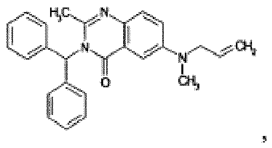 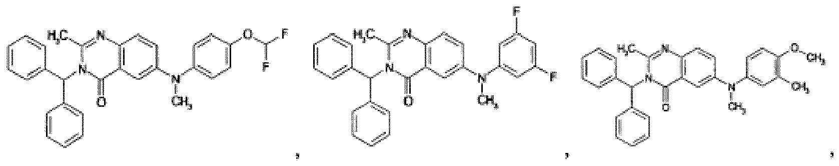 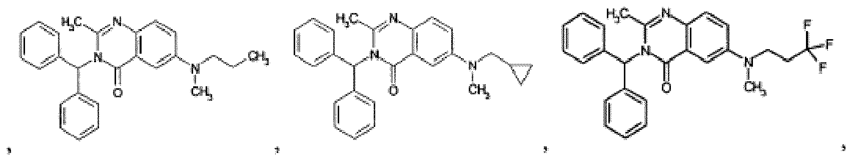 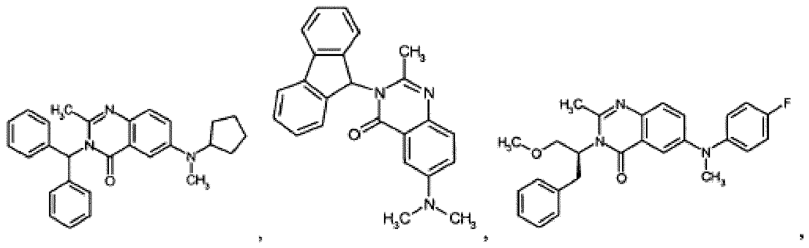 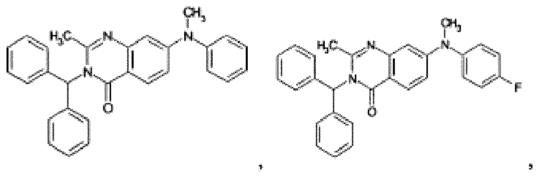 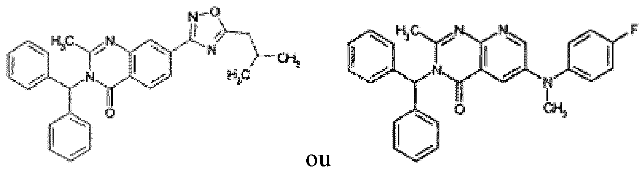 ou un sel ou solvate pharmaceutiquement acceptable de celui-ci.

16. Composition comprenant un ou plusieurs composés selon l'une quelconque des revendications 1-15 ou un sel ou solvate pharmaceutiquement acceptable de ceux-ci et au moins un véhicule pharmaceutiquement acceptable.

17. Composé selon l'une quelconque des revendications 1-15 à utiliser dans le traitement du diabète, d'une complication diabétique, de l'obésité, du syndrome métabolique ou d'une maladie cardiovasculaire chez un patient, optionnellement en combinaison avec au moins un agent thérapeutique supplémentaire qui n'est pas un composé selon la revendication 1, où l'agent supplémentaire est sélectionné parmi un agent antidiabétique et un agent anti-obésité.
